# EUROPEAN PATENT SPECIFICATION

(11) **EP 4 139 295 B1**
(45) Date of publication and mention of the grant of the patent: **09.04.2025**
(21) Application number: 21724005.0
(22) Date of filing: 22.04.2021
(51) Int. Cl.: C07D 401/14, C07D 413/14, C07D 417/14, C07D 471/04, A61K 31/4725, A61P 1/16, A61P 3/10, A61P 9/00, A61P 11/00, A61P 11/06, A61P 13/12, A61P 17/00, A61P 25/00, A61P 35/00

(54) **ISOQUINILINE NRF2 AGONISTS**
ISOCHINOLIN NRF2 AGONISTEN
ISOQUINOLINES COMME AGONISTES DU NRF2

(30) Priority: 22.04.2020 GB 202005852
(43) Date of publication of application: 01.03.2023
(73) Proprietor: C4x Discovery Limited, Manchester M1 3LD (GB)
(72) Inventor: LUCAS, Cathy Louise, Manchester M1 3LD (GB); BLANEY, Emma Louise, Manchester M1 3LD (GB); MARTIN, Barrie Phillip, Manchester M1 3LD (GB); NOWAK, Thorsten, Manchester M1 3LD (GB); RAY, Nicholas Charles, London NW1 1AD (GB); CRUMPLER, Simon Ross, Harlow Essex CM19 5TR (GB); SEWARD, Eileen Mary, Harlow Essex CM19 5TR (GB); HYND, George, Harlow Essex CM19 5TR (GB)
(74) Representative: HGF
(86) International application number: PCT/GB2021/050975
(87) International publication number: WO 2021/214472

(56) References cited:
- WO-A1-2013/067036
- WO-A1-2016/203400
- WO-A1-2018/181345
- WO-A1-2020/084300
- HONGBIN DAI ET AL: "Development of Novel Nrf2/ARE Inducers Bearing Pyrazino[2,1-a]isoquinolin Scaffold with Potent In Vitro Efficacy and Enhanced Physicochemical Properties", MOLECULES, vol. 22, no. 9, 1 September 2017 (2017-09-01), DE, pages 1541, XP055649795, ISSN: 1433-1373, DOI: 10.3390/molecules22091541
- BENJAMIN G. RICHARDSON ET AL: "Replacement of a Naphthalene Scaffold in Kelch-like ECH-Associated Protein 1 (KEAP1)/Nuclear Factor (Erythroid-derived 2)-like 2 (NRF2) Inhibitors", JOURNAL OF MEDICINAL CHEMISTRY, vol. 61, no. 17, 20 August 2018 (2018-08-20), US, pages 8029 - 8047, XP055642376, ISSN: 0022-2623, DOI: 10.1021/acs.jmedchem.8b01133
- DATABASE WPI Week 201871, Derwent World Patents Index; AN 2018-776846

## Description

### INTRODUCTION

The present invention relates to pharmaceutical compositions comprising tetrahydroisoquinoline compounds. More specifically, the present invention relates to pharmaceutical compositions comprising tetrahydroisoquinoline compounds that are Nrf2 activators. The present invention also relates to their use in the treatment of diseases or disorders associated with Nrf2 activation and/or inhibition of Keap1-Nrf2 protein-protein interactions.

### BACKGROUND OF THE INVENTION

Nuclear factor erythroid 2-related factor 2 (Nrf2) is a basic leucine zipper (bZIP) transcription factor and a member of the Cap 'n' Collar (CNC) family of transcription factors. It is a key master of the inducible cell defence system, mediating the expression of more than 100 oxidative stress-related genes that include phase I and II detoxification enzymes and antioxidant proteins. These genes all contain the antioxidant response element (ARE) in their promoter regulatory regions, which is the binding target of Nrf2. Under basal conditions the levels of Nrf2 are tightly regulated by the adaptor protein Keap1, a cytosolic actin-bound repressor protein, which binds to Nrf2 and leads to proteasomal degradation *via* the Cul3-based E3 ubiquitin ligase complex. Under conditions of oxidative stress, Keap1 is inactivated leading to an increase in the level of *de novo* synthesised Nrf2 which translocates to the nucleus, binds to AREs with a resulting up-regulation in cytoprotective gene expression.

It has been shown that Nrf2 mRNA expression in COPD subjects was significantly lower than that in control subjects and Nrf2 mRNA were negatively correlated with pack year. Nrf2 protein in COPD subjects was significantly lower than that in control subjects. CSE-induced A549 cell apoptosis was increased in a time-dependent and concentration-dependent manner, and was significantly increased by Nrf2 knockdown (Yamada, BMC Pulmonary Medicine, doi:10.1186/s12890-016-0189-1). Therefore, elevation of Nrf2 levels in the lungs of COPD patients should lead to a reduction in the inflammatory processes that lead to deleterious structural modifications of the lung and slow disease progression. Nrf2 may also be expected to show positive benefits in other respiratory diseases that exhibit oxidative stress components (Cho, Toxicol Appl Pharmacol, doi: 10.1016/j.taap.2009.07.024) such as acute, chronic and severe asthma (Sussan, Am J Physiol Lung Cell Mol Physiol, doi:10.1152/ajplung.00398.2014), acute lung injury/acute respiratory distress syndrome, with or without accompanying multi organ dysfunction syndrome (Yan, Free Radical Biol Med, doi:10.1016/j.freeradbiomed.2018.04.557; de la Vega Curr Pharmacol Rep, doi:10.1007/s40495-016-0053-2), pulmonary fibrosis, including idiopathic pulmonary fibrosis (Kikuchi, Respir Res, doi:10.1186/1465-9921-11-31) and cystic fibrosis (Chen, PLoS One, doi:10.1371/journal.pone.0003367). Indeed, the combination of cytoprotection by restoring redox and protein homeostasis, promoting resolution of inflammation, and facilitating repair in the lungs by Nrf2 activators has highlighted their potential for the treatment of COVID-19 (Cuadrado et al, Trends Pharmacol Sci, doi:10.1016/j.tips.2020.07.003).

The cardiac protective nature of Nrf2 in models of atherosclerosis, ischaemia, reperfusion, cardiac hypertrophy and heart failure has been demonstrated (Chen, Physiol Genomics, doi:10.1152/physiolgenomics.00041.2017). The Nrf2 activator Bardoxolone methyl has recently completed a Phase II study in patients with pulmonary arterial hypertension (PAH), with a Phase III study underway based on a significant improvement in 6 minute walking distance. Bardoxolone reacts covalently with Keap1 but compounds activating Nrf2 *via* alternative mechanisms of Keap1 binding should also be expected to be therapeutically useful in PAH, particularly in patients that also have an underlying connective tissue disorder (CTD), such as scleroderma or lupus erythematosus. Oxidative stress is elevated in the diseased myocardium, leading to raised levels of reactive oxygen species which impact negatively on cardiac function (Bolli, Circ, doi: 10.1161/circ.76.2.3111744). Nrf2 activation has been shown to suppress myocardial oxidative stress, cardiac apoptosis, hypertrophy, fibrosis and dysfunction in mouse models of pressure overload (Wang, J Card Failure, doi:10.1016/j.cardfail.2012.06.003) and to protect against cardiac ischemic/reperfusion injury in rodent models (Zhang, J Mol Cell Cardiol, doi:10.1016/j.yjmcc.2010.05.01). Furthermore, excessive production of oxidizing agents in detriment of antioxidant defences in the cardiovascular system has also been described in metabolic diseases such as obesity, metabolic syndrome and diabetes mellitus where activation of Nrf2 has also been suggested as a promising therapeutic strategy (da Costa, Front Pharmacol., doi:10.3389/fphar.2019.00382). In addition, the Nrf2 activator sulforaphane reduces hepatic glucose production and improves glucose control in patients with type 2 diabetes (Axelsson, Sci Transl Med., doi: 10.1126/scitranslmed.aah4477) and bardoxolone methyl has been shown to induce weight loss in generally obese patients in proportion to baseline BMI alongside improving glycaemic control (Chertow et al, J Diabetes Complications, doi: 10.1016/j.jdiacomp.2018.09.005). Age-associated mitochondrial dysfunction and oxidative damage are primary causes for multiple health problems including sarcopenia and cardiovascular disease and treatment of aging mice with the Nrf2 activator Sulforaphane restored Nrf2 activity, mitochondrial function, cardiac function, exercise capacity, glucose tolerance, and activation/differentiation of skeletal muscle satellite cells (Bose et al, Aging cell, doi:10.1111/acel.13261). Thus, it is expected that drugs leading to activation of Nrf2 should be useful in a number of cardiovascular and metabolic diseases including, but not limited to, atherosclerosis, hypertension, heart failure, myocardial infarction and repair, cardiac remodelling, cardiac arrhythmias, heart failure with reduced ejection fraction, diabetic cardiomyopathy, diabetic nephropathy, metabolic syndrome, obesity, diabetes mellitus (type 1 or type 2) and insulin resistance.

Subarachnoid haemorrhage (SAH) is a devastating condition with high morbidity and mortality rates due to the lack of effective therapy. Early brain injury (EBI) and cerebral vasospasm (CVS) are the two most important pathophysiological mechanisms for brain injury and poor outcomes for patients with SAH (clinicaltrials.gov/ct2/show/NCT0261474, *SFX01 After Subarachnoid Haemorrhage (SAS)).* Evidence from experimental SAH research indicates a protective role of the Nrf2/ARE pathway in EBI and CVS after SAH. Administration of sulforaphane (SFN) to rats following SAH enhances the activity of the Nrf2-ARE pathway, attenuates vasospasm in basilar arteries and suppresses the release of proinflammatory cytokines (Zhao, Brain Res., doi: 10.1016/j.brainres.2016.09.035). Intracerebral haemorrhage (ICH) is the primary event in 10-15% of the 15 million strokes occurring annually worldwide. *In vitro* studies demonstrated that Nrf2 activators rapidly increased HO-1 expression in astrocytes and reduced their vulnerability to haemoglobin or hemin. Systemic treatment with small molecule Nrf2 activators increased HO-1 expression in perivascular cells, particularly astrocytes. When tested in mouse or rat ICH models, Nrf2 activators were consistently protective, improving barrier function and attenuating edema, inflammation, neuronal loss and neurological deficits (Chen-Roetling, Curr Pharm Des, doi:10.2174/1381612822666161027150616). Ischemic stroke induces reactive oxygen species, causing oxidative and inflammatory responses in ischemic brain. To date, recombinant tissue plasminogen activator is the only available therapy for the treatment of ischemic stroke. However, the treatment does not prevent oxidative stress and inflammation in the ischemic brain. D3T, a sulfur-containing dithiolethione compound, is found in cruciferous vegetables and has been reported to induce anti-oxidant genes through activation of Nrf2. D3T has been shown (Yen, J Immunol 2017, 198 (1 supplement) 206.20) to attenuate brain infarct and ameliorate neurological deficits in stroke animals. In addition, D3T reduced CNS infiltrating inflammatory immune cells including neutrophils and monocytes in the ischemic brain. Moreover, D3T-induced suppression of inflammatory cytokine production was observed in wild-type but not in Nrf2-deficient microglia. Furthermore, the protective effect of D3T on the attenuation of ischemic brain infarct was abolished in Nrf2-deficient stroke animals and in the stroke animals administered with HO-1 inhibitor. These results suggest that D3T-mediated suppression of inflammation in the ischemic brain is mediated through Nrf2/HO-1 pathway, and thus that targeting the Nrf2/HO-1 pathway may be a promising therapeutic strategy for the amelioration of neuroinflammation in ischemic stroke.

Nrf2 is believed to play a key role in some hemoglobinopathies, such as beta-thalassemia and sickle cell disease (SCD). SCD is a recessive inherited disorder caused by a single missense mutation which leads to the mutated beta-globin protein haemoglobin S (HbS). At low oxygen concentrations HbS polymerises, leading to misshapen red blood cells which are prone to rupture, releasing free heme into plasma. The resulting oxidative stress and inflammation leads to damage in multiple organs of the body. Ablation of Keap1 and the resulting constitutive activation of Nrf2 has been shown to lead to improved outcomes in SCD model mice (Zhu, Blood, doi:10.1182/blood-2017-10-810531; Keleku-Lukwete PNAS, doi:10.1073/pnas.1509158112). Nrf2 activation has been shown to slow down the progression of haemolytic anemia and organ disfunction (Ghosh, JCI Insight, doi: 10.1172/jci.insight.81090) and loss of Nrf2 function worsens the pathophysiology of SCD in transgenic SCD mice (Zhu, Blood, doi.org/10.1182/blood-2017-10-810531). Global activation of Nrf2 with the known compound D3T reduces lethality in a haem-induced acute chest syndrome model in transgenic SCD mice (Ghosh, Brit. J. Haematology doi: 10.1111/bjh.15401). In addition, Nrf2 activators have also been shown to modulate foetal haemoglobin (HbF) expression through direct binding in the gamma-globin promoter and modification of chromatin structure in the beta-globin locus. In sickle erythroid cells, Nrf2 provides unique benefits through HbF induction to inhibit haemoglobin S polymerization and protection against oxidative stress due to chronic haemolysis (Zhu, Exp Biol Med doi: 10.1177/1535370219825859). Thus, the development of small molecule activators of Nrf2 has the potential to ameliorate the clinical severity of sickle cell disease and other diseases where increasing HbF is beneficial such as beta-thalassemia.

The function of Nrf2 is altered in many neurodegenerative disorders, such as Huntington's disease, Parkinson's Disease, Alzheimer's disease, amyotrophic lateral sclerosis, frontotemporal dementia, multiple sclerosis and Friedreich's ataxia (Dinkova-Kostova, FEBS, doi:10.1111/febs.14379). Nrf2 activation mitigates multiple pathogenic processes involved in these neurodegenerative disorders through upregulation of antioxidant defences, inhibition of inflammation, improvement of mitochondrial function, and maintenance of protein homeostasis. Small molecule pharmacological activators of Nrf2 have shown protective effects in numerous animal models of neurodegenerative diseases (Joshi, Neurobiol Aging, doi:10.1016/j.neurobiolaging.2014.09.004; Alarcon-Aguilar, Neurobiol Aging, doi:10.1016/j.neurobiolaging.2014.01.143 and in cultures of human cells expressing mutant proteins. Tecfidera (dimethyl fumarate) activates Nrf2 (in addition to other mechanisms) and is approved in the US to treat relapsing-remitting multiple sclerosis. The Nrf2 activator Omaveloxolone (RTA-408) currently in Phase II trials for the treatment of the inherited neurodegenerative disorder Friedrich's ataxia, has met its primary endpoint of change in the modified Friedrich's Ataxia Rating Scale (mFARS) relative to placebo after 48 weeks of treatment. Targeting Nrf2 signalling may therefore provide a therapeutic option to delay onset, slow progression, and ameliorate symptoms of neurodegenerative disorders. Due to the role of oxidative stress and mitochondrial dysfunction in disorders of the CNS, treatment of chronic pain and schizophrenia with Nrf2 activators has also been suggested.

Rheumatoid arthritis (RA) is an autoimmune disease that causes chronic inflammation of the joints and is characterized by periods of disease flares and remissions. Multiple joints can be affected sometimes resulting in permanent joint destruction and deformity. Nrf2 has been found to be activated in the joints of arthritic mice and of RA patients. Nrf2-knockout mice have more severe cartilage injuries and more oxidative damage, with the expression of Nrf2 target genes being enhanced in Nrf2-wild-type but not in knockout mice during antibody-induced arthritis (Wruck, BMJ Annals of Rheumatic Diseases, doi:10.1136/ard.2010.132720). Additionally, in an animal model of rheumatoid arthritis, using the transfer of serum from K/BxN transgenic mice to Nrf2(-/-) mice, Nrf2 deficiency accelerated the incidence of arthritis, and animals showed a widespread disease affecting both front and hind paws (Maicas, Antioxidants & Redox Signaling, doi:10.1089/ars.2010.3835).

Ulcerative colitis (UC) and Crohn's disease (CD) are chronic relapsing-remitting forms of inflammatory bowel disease (IBD) that are caused by dysfunction of the intestinal epithelium. Damage to the intestinal epithelial cells can disrupt the barrier function of the intestinal epithelium, facilitating an aberrant immune response and inflammatory conditions. Thus, the intact intestinal epithelium is critical for the healthy gut, and cytoprotective agents that could target the intestinal epithelial cells would be beneficial for the treatment of UC and CD. Antioxidant levels and oxidative stress biomarkers are typically correlated with disease severity in IBD and a number of genome-wide association studies have linked IBD-associated single nucleotide polymorphismsm (SNPs) to multiple genes involved in the response to oxidative stress, with many regulated by Nrf2 (Khor et al, Nature, doi:10.1038/nature10209). CPUY192018, a small-molecule inhibitor of the Keap1-Nrf2 protein-protein interaction (and hence Nrf2 activator) has demonstrated a cytoprotective effect in an experimental model of UC induced by dextran sodium sulphate in both NCM460 cells and mouse colon (Lu, Scientific Reports, doi:10.1038/srep26585). It has also been shown that Nrf2 knockout mice show an increased susceptibility to colitis-associated colorectal cancer (Khor, Cancer Prev Res (Phila), doi:10.1158/1940-6207).

Fumaderm, a mixture of dimethyl fumarate (DMF) and three salts of monoethyl fumarate, was licensed in Germany in 1994 for the treatment of psoriasis. The likely bioactive form of DMF, monomethyl fumarate (MMF) has been shown to increase total and nuclear Nrf2 levels in primary mouse keratinocytes and lead to enhanced mRNA expression of several Nrf2-downstream effectors such as heme oxygenase-1 and peroxiredoxin-6. (Helwa, J Pharmacol. Exp. Ther., doi:10.1124/jpet.116.239715). Other skin disorders may benefit from treatment with Nrf2 activators such as radiation-induced dermatitis/skin damage, atopic dermatitis and wound healing (Wu et al, Mol Med Rep. 2019 Aug;20(2):1761-1771).

Activation of Nrf2 has been shown to have beneficial effects in diseases of both the liver and kidney. NAFLD (Non-alcoholic fatty liver disease) is recognized as the leading cause of chronic liver disease worldwide. NAFLD represents a spectrum of diseases, some of which can progress to cirrhosis and hepatocellular carcinoma (HCC). Although all subtypes of NAFLD increase the risk for cardiovascular events and mortality, NASH (non-alcoholic steatohepatitis) is the main diagnostic subtype of NAFLD which predisposes patients to cirrhosis and liver-related complications. There are currently approved drug treatments for NAFLD and NASH. However, knockout of Nrf2 in mice profoundly predisposes to NASH stimulated by either a methionine- and choline-deficient (MCD) diet (Chowdry, Free Radic Biol Med, doi:10.1016/j.freeradbiomed.2009.11.007) or a high fat (HF) diet (Okada, J Gastroenterol, doi:10.1007/s00535-012-0659-z), and pharmacologic activation of Nrf2 has been shown to reverse NASH in mouse models (Sharma, Cell Mol Gastroenterol Hepatol, doi:10.1016/j.jcmgh.2017.11.016). Other liver diseases may benefit from treatment with Nrf2 activators such as toxin-induced liver disease, viral hepatitis and cirrhosis. Oxidative-stress molecules, such as reactive oxygen species, accumulate in the kidneys of animal models for acute kidney injury (AKI), in which Nrf2 is transiently and slightly activated. Genetic or pharmacological enhancement of Nrf2 activity in the renal tubules significantly ameliorates damage related to AKI and prevents AKI progression to chronic kidney disease (CKD) by reducing oxidative stress. However, a Phase III clinical trial of a KEAP1 inhibitor, CDDO-Me or bardoxolone-methyl, for patients with stage 4 CKD and type-2 diabetes mellitus (T2DM) was terminated due to the occurrence of cardiovascular events. Because recent basic studies have accumulated positive effects of KEAP1 inhibitors in moderate stages of CKD, Phase II trials have been restarted. The data from the ongoing projects demonstrate that a Nrf2 activator/KEAP1 inhibitor improves the glomerular filtration rate in patients with stage 3 CKD and T2DM without safety concerns (Nezu, Am J Nephrol, doi:10.1159/000475890). Inflammatory reactions and oxidative stress are implicated in the pathogenesis of focal segmental glomerulosclerosis (FSGS), a common chronic kidney disease with relatively poor prognosis and unsatisfactory treatment regimens. CXA-10 which upregulates Nrf2 pathways is currently in clinical trials for Focal Segmental Glomerulosclerosis (FIRSTx - A Study of Oral CXA-10 in Primary Focal Segmental Glomerulosclerosis (FSGS); clinicaltrials.gov/ct2/show/NCT03422510). Bardoxolone methyl has been shown in a Phil trial to lead to significant improvement in kidney function in patients with either autosomal dominant polycystic kidney disease (ADPKD), CKD associated with type 1 diabetes (T1D), IgA nephropathy (lgAN) or FSGS after 12 weeks of treatment (https://www.reatapharma.com/press-releases/reata-announces-positive-phase-2-data-for-bardoxolone-methyl-in-patients-with-focal-segmental-glomerulosclerosis-and-in-patients-from-all-four-cohorts-of-phoenix/). Alport Syndrome is the second most common inherited cause of kidney failure caused by a genetic defect in type IV collagen, a component in building the glomerular basement membrane. As bardoxolone methyl is thought to affect the underlying pathologic processes associated with mitochondrial dysfunction, inflammation and oxidative stress, it is currently being studied in these patients suggesting Nrf2 activators will be potentially useful in this disease.

Oxidative stress plays a critical role in the initiation and progression of cancer (Gorrini, Nat Rev Drug Discov., doi:10.1038/nrd4002). Due to its importance in the maintenance of redox cellular homeostasis, Nrf2 is considered a cytoprotective transcription factor and tumour suppressor. At lower homeostatic levels Nrf2 is able to eliminate ROS, carcinogens and other DNA-damaging agents, leading to the inhibition of tumour initiation and metastasis (Milkovic et al. Redox Biol. doi:10.1016/j.redox.2017.04.013). Evgen is currently evaluating SFX-01 (sulforaphane-cyclodextrin complex) in the Treatment and Evaluation of Metastatic Breast Cancer (STEM) (clinicaltrials.gov/ct2/show/NCT02970682) which includes ER+/HER- metastatic breast cancer. Bardoxolone derivatives have been shown to prevent lung cancer induced by vinyl carbamate in A/J mice (Liby, Cancer Res. doi:10.1158/0008-5472). Thus, activators of Nrf2 may have a role in the prevention of cancer.

Age related macular degeneration (AMD) is the principal cause of blindness in western countries and oxidative stress plays a major role in AMD pathogenesis and progression. It has been shown that Nrf2 activators are able to protect cells cultured to mimic the external layer of the retina from oxidative stress suggesting the potential for vision preservation in early AMD patients (Bellezza, Front Pharmacol. 2018; 9: 1280). In addition, Nrf2 activators may also be useful in other eye conditions such as Fuchs Endothelial Corneal Dystrophy and uveitis.

Recently, Nrf-2 activators have also been suggested to have benefit for the treatment of preeclampsia via suppression of oxidative stress and endothelial cell apoptosis (Jiang et al, Oxid Med Cell Longev doi:10.1155/2021/8839394).

Therefore, there is an ongoing need for agents capable of Nrf2 activation, given the role of Nrf2 in multiple indications.

WO 2013/067036 discloses bicyclic small molecule inhibitors of Keap1-Nrf2 interaction. WO 2018/181345 describes benzotriazole-linked bicyclic compounds having Nrf2-activating activity. WO 2020/084300 discloses tetrahydroisoquinoline compounds that are Nrf2 activators. Dai et al, Molecules (2017), 22, 1541 describe the development of Nrf2 activators featuring a pyrido[1,2-a]pyrazine scaffold. Richardson et al, J. Med. Chem. (2018), 61, 8029-8047 prepared compounds based on various heterocyclic scaffolds, in particular a 1,4-disubstituted isoquinoline scaffold, as inhibitors of Keap1-Nrf2 interaction.

### SUMMARY OF THE INVENTION

In one aspect, the present invention provides a pharmaceutical composition comprising a compound of the invention as defined in the appended claims, or a pharmaceutically acceptable salt thereof, and one or more pharmaceutically acceptable excipients.

In another aspect, the present invention relates to a pharmaceutical composition as defined in the appended claims, for use in therapy.

In another aspect, the present invention relates to a pharmaceutical composition as defined in the appended claims, for use in the treatment of diseases or disorders mediated by Nrf2 activation.

Examples of diseases or disorders mediated by Nrf2 activation include chronic obstructive pulmonary disease, asthma, pulmonary arterial hypertension, diabetes mellitus, chronic kidney disease, ulcerative colitis, Crohn's disease, inflammatory bowel disease, Friedreich's ataxia, sickle cell disease and non-alcoholic steatohepatitis.

In another aspect, the present invention provides a pharmaceutical composition as defined in the appended claims, for use in the treatment of chronic obstructive pulmonary disease, asthma, pulmonary arterial hypertension, diabetes mellitus, chronic kidney disease, ulcerative colitis, Crohn's disease, inflammatory bowel disease, Friedreich's ataxia, sickle cell disease or non-alcoholic steatohepatitis.

The present disclosure (not part of the invention) further provides a method of synthesising a compound, or a pharmaceutically acceptable salt thereof, as defined herein.

In another aspect, the present disclosure (not part of the invention) provides a compound, or a pharmaceutically acceptable salt thereof, obtainable by, or obtained by, or directly obtained by a method of synthesis as defined herein.

In another aspect, the present disclosure (not part of the invention) provides novel intermediates as defined herein which are suitable for use in any one of the synthetic methods set out herein.

Preferred, suitable, and optional features of any one particular aspect of the present invention are also preferred, suitable, and optional features of any other aspect.

### DETAILED DESCRIPTION OF THE INVENTION

### Definitions

Unless otherwise stated, the following terms used in the specification and claims have the following meanings set out below.

It is to be appreciated that references to "treating" or "treatment" include prophylaxis as well as the alleviation of established symptoms of a condition. "Treating" or "treatment" of a state, disorder or condition therefore includes: (1) preventing or delaying the appearance of clinical symptoms of the state, disorder or condition developing in a human that may be afflicted with or predisposed to the state, disorder or condition but does not yet experience or display clinical or subclinical symptoms of the state, disorder or condition, (2) inhibiting the state, disorder or condition, *i.e.,* arresting, reducing or delaying the development of the disease or a relapse thereof (in case of maintenance treatment) or at least one clinical or subclinical symptom thereof, or (3) relieving or attenuating the disease, *i.e.,* causing regression of the state, disorder or condition or at least one of its clinical or subclinical symptoms.

A "therapeutically effective amount" means the amount of a compound that, when administered to a mammal for treating a disease, is sufficient to effect such treatment for the disease. The "therapeutically effective amount" will vary depending on the compound, the disease and its severity and the age, weight, etc., of the mammal to be treated.

In this specification the term "alkyl" includes both straight and branched chain alkyl groups. References to individual alkyl groups such as "propyl" are specific for the straight chain version only and references to individual branched chain alkyl groups such as "isopropyl" are specific for the branched chain version only. For example, "(1-6C)alkyl" includes (1-4C)alkyl, (1-3C)alkyl, propyl, isopropyl and t-butyl. A similar convention applies to other radicals, for example "phenyl(1-6C)alkyl" includes phenyl(1-4C)alkyl, benzyl, 1-phenylethyl and 2-phenylethyl.

In this specification the term "alkylene" includes both straight and branched chain divalent alkyl groups. For example, "C₁₋₄alkylene" includes methylene (-CH₂-), ethylene (-CH₂CH₂-), propylene and butylene.

In this specification the term "alkoxy" includes both straight and branched chain alkyl groups singularly bonded to oxygen. For example, "C₁₋₄alkoxy" includes methoxy, ethoxy, iso-propoxy and t-butoxy.

The term "(m-nC)" or "(m-nC) group" used alone or as a prefix, refers to any group having m to n carbon atoms.

"Cycloalkyl" means a hydrocarbon monocyclic or bicyclic ring containing carbon atoms. Examples of monocyclic cycloalkyl groups include cyclopropyl, cyclobutyl, cyclopentyl, cyclohexyl, cycloheptyl. Bicyclic rings may be fused or spiro attached; examples of bicyclic cycloalkyl groups include bicyclo[2.2.2]octane, bicyclo[2.1.1]hexane, bicyclo[1.1.1]pentane, spiro[2.4]heptane, bicyclo[4.1.0]heptane and bicyclo[2.2.1]heptane.

The term "halo" refers to fluoro, chloro, bromo and iodo.

The term "haloalkyl" is used herein to refer to an alkyl group respectively in which one or more hydrogen atoms have been replaced by halogen (e.g. fluorine) atoms. Examples of haloalkyl groups include fluoroalkyl groups such as -CHF₂, -CH₂CF₃, or perfluoroalkyl/alkoxy groups such as -CF₃, or -CF₂CF₃.

The term "heterocyclyl", "heterocyclic" or "heterocycle" means a non-aromatic saturated or partially saturated monocyclic, fused, bridged, or spiro bicyclic heterocyclic ring system(s). Monocyclic heterocyclic rings contain from about 3 to 12 (suitably from 3 to 7) ring atoms, with from 1 to 5 (suitably 1, 2 or 3) heteroatoms selected from nitrogen, oxygen or sulfur in the ring. Bicyclic heterocycles contain from 7 to 17 member atoms, suitably 7 to 12 member atoms, in the ring. Bicyclic heterocyclic(s) rings may be fused, spiro, or bridged ring systems. Examples of heterocyclic groups include cyclic ethers such as oxiranyl, oxetanyl, tetrahydrofuranyl, dioxanyl, and substituted cyclic ethers. Heterocycles containing nitrogen include, for example, azetidinyl, pyrrolidinyl, piperidinyl, piperazinyl, tetrahydrotriazinyl, tetrahydropyrazolyl, and the like. Typical sulfur containing heterocycles include tetrahydrothienyl, dihydro-1,3-dithiol, tetrahydro-2H-thiopyran, and hexahydrothiepine. Other heterocycles include dihydro-oxathiolyl, dihydroisoxazolyl (such as 4,5-dihydroisoxazolyl), dihydropyridinyl (such as 1,2-dihydropyridinyl or 1,6-dihydropyridinyl), tetrahydro-oxazolyl, tetrahydro-oxadiazolyl, tetrahydro-dioxazolyl, tetrahydro-oxathiazolyl, hexahydrotriazinyl, tetrahydro-oxazinyl, morpholinyl, thiomorpholinyl, tetrahydropyrimidinyl, dioxolinyl, octahydrobenzofuranyl, octahydrobenzimidazolyl, and octahydrobenzothiazolyl. For heterocycles containing sulfur, the oxidized sulfur heterocycles containing SO or SO₂ groups are also included. Examples include the sulfoxide and sulfone forms of tetrahydrothienyl and thiomorpholinyl such as tetrahydrothiene 1,1-dioxide and thiomorpholinyl 1,1-dioxide. A suitable value for a heterocyclyl group which bears 1 or 2 oxo (=O) or thioxo (=S) substituents is, for example, 2-oxopyrrolidinyl, 2-thioxopyrrolidinyl, 2-oxoimidazolidinyl, 2-thioxoimidazolidinyl, 2-oxopiperidinyl, 2,5-dioxopyrrolidinyl, 2,5-dioxoimidazolidinyl or 2,6-dioxopiperidinyl. Particular heterocyclyl groups are saturated monocyclic 3 to 7 membered heterocyclyls containing 1, 2 or 3 heteroatoms selected from nitrogen, oxygen or sulfur, for example azetidinyl, tetrahydrofuranyl, tetrahydropyranyl, pyrrolidinyl, morpholinyl, tetrahydrothienyl, tetrahydrothienyl 1,1-dioxide, thiomorpholinyl, thiomorpholinyl 1,1-dioxide, piperidinyl, homopiperidinyl, piperazinyl or homopiperazinyl. As the skilled person would appreciate, any heterocycle may be linked to another group *via* any suitable atom, such as *via* a carbon or nitrogen atom. Suitably, the term "heterocyclyl", "heterocyclic" or "heterocycle" will refer to 4, 5, 6 or 7 membered monocyclic rings as defined above.

The term "heteroaryl" or "heteroaromatic" means an aromatic mono-, bi-, or polycyclic ring incorporating one or more (for example 1-4, particularly 1, 2 or 3) heteroatoms selected from nitrogen, oxygen or sulfur. Examples of heteroaryl groups are monocyclic and bicyclic groups containing from five to twelve ring members, and more usually from five to ten ring members. The heteroaryl group can be, for example, a 5- or 6-membered monocyclic ring or a 9- or 10-membered bicyclic ring, for example a bicyclic structure formed from fused five and six membered rings or two fused six membered rings. Each ring may contain up to about four heteroatoms typically selected from nitrogen, sulfur and oxygen. Typically, the heteroaryl ring will contain up to 3 heteroatoms, more usually up to 2, for example a single heteroatom. In one embodiment, the heteroaryl ring contains at least one ring nitrogen atom. The nitrogen atoms in the heteroaryl rings can be basic, as in the case of an imidazole or pyridine, or essentially non-basic as in the case of an indole or pyrrole nitrogen. In general, the number of basic nitrogen atoms present in the heteroaryl group, including any amino group substituents of the ring, will be less than five. Suitably, the term "heteroaryl" or "heteroaromatic" will refer to 5 or 6 membered monocyclic heteroaryl rings as defined above.

Non-limiting examples of heteroaryl include furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl, 1,3,5-triazenyl, benzofuranyl, indolyl, isoindolyl, benzothienyl, benzoxazolyl, benzimidazolyl, benzothiazolyl, indazolyl, purinyl, benzofurazanyl, quinolyl, isoquinolyl, quinazolinyl, quinoxalinyl, cinnolinyl, pteridinyl, naphthyridinyl, carbazolyl, phenazinyl, benzisoquinolinyl, pyridopyrazinyl, thieno[2,3-*b*]furanyl, 2*H*-furo[3,2-*b*]-pyranyl, 5*H*-pyrido[2,3-*d*]-o-oxazinyl, 1*H*-pyrazolo[4,3-*d*]-oxazolyl, 4*H*-imidazo[4,5-*d*]thiazolyl, pyrazino[2,3-*d*]pyridazinyl, imidazo[2,1-*b*]thiazolyl, imidazo[1,2-*b*][1,2,4]triazinyl. "Heteroaryl" also covers partially aromatic bi- or polycyclic ring systems wherein at least one ring is an aromatic ring and one or more of the other ring(s) is a non-aromatic, saturated or partially saturated ring, provided at least one ring contains one or more heteroatoms selected from nitrogen, oxygen or sulfur. Examples of partially aromatic heteroaryl groups include for example, tetrahydroisoquinolinyl, tetrahydroquinolinyl, 2-oxo-1,2,3,4-tetrahydroquinolinyl, dihydrobenzthienyl, dihydrobenzfuranyl, 2,3-dihydro-benzo[1,4]dioxinyl, benzo[1,3]dioxolyl, 2,2-dioxo-1,3-dihydro-2-benzothienyl, 4,5,6,7-tetrahydrobenzofuranyl, indolinyl, 1,2,3,4-tetrahydro-1,8-naphthyridinyl, 1,2,3,4-tetrahydropyrido[2,3-*b*]pyrazinyl, 3,4-dihydro-2*H*-pyrido[3,2-*b*][1,4]oxazinyl, 4,5,6,7-tetrahydrobenzo[*d*]isoxazolyl, 4,5,6,7-tetrahydro-[1,2,3]triazolo[1,5-*a*]pyridinyl, 5,6-dihydro-8*H*-[1,2,4]triazolo[3,4-*c*][1,4]oxazinyl, 5,6-dihydro-4*H*-pyrrolo[1,2-*c*][1,2,3]triazolyl, 6,7-dihydro-5*H*-pyrrolo[2,1-*c*][1,2,4]triazolyl, 5,6,7,8-tetrahydro-[1,2,4]triazolo[4,3-*a*]pyridinyl, 6,7-dihydro-4*H*-[1,2,3]triazolo[5,1-*c*][1,4]oxazinyl and 1,4,5,6-tetrahydrocyclopenta[*d*][1,2,3]triazol-5-yl.

Non-limiting examples of five membered heteroaryl groups include but are not limited to pyrrolyl, furanyl, thienyl, imidazolyl, furazanyl, oxazolyl, oxadiazolyl, oxatriazolyl, isoxazolyl, thiazolyl, isothiazolyl, pyrazolyl, triazolyl and tetrazolyl groups.

Non-limiting examples of six membered heteroaryl groups include but are not limited to pyridyl, pyrazinyl, pyridazinyl, pyrimidinyl and triazinyl.

Particular non-limiting examples of bicyclic heteroaryl groups containing a six membered ring fused to a five membered ring include but are not limited to benzofuranyl, benzothiophenyl, benzimidazolyl, benzoxazolyl, benzisoxazolyl, benzothiazolyl, benzisothiazolyl, isobenzofuranyl, indolyl, isoindolyl, indolizinyl, indolinyl, isoindolinyl, purinyl (e.g., adeninyl, guaninyl), indazolyl, benzodioxolyl, pyrrolopyridine, and pyrazolopyridinyl groups.

Particular non-limiting examples of bicyclic heteroaryl groups containing two fused six membered rings include but are not limited to quinolinyl, isoquinolinyl, chromanyl, thiochromanyl, chromenyl, isochromenyl, chromanyl, isochromanyl, benzodioxanyl, quinolizinyl, benzoxazinyl, benzodiazinyl, pyridopyridinyl, quinoxalinyl, quinazolinyl, cinnolinyl, phthalazinyl, naphthyridinyl and pteridinyl groups.

Particular non-limiting examples of bicyclic heteroaryl groups containing a five membered ring fused to a five membered ring include but are not limited to 6,7-dihydro-5*H*-pyrrolo[2,1-*c*][1,2,4]triazolyl, 5,6-dihydro-4*H*-pyrrolo[1,2-*c*][1,2,3]triazolyl and 1,4,5,6-tetrahydrocyclopenta[*d*][1,2,3]triazol-5-yl.

The term "aryl" means a cyclic or polycyclic aromatic ring having from 5 to 12 carbon atoms. The term aryl includes both monovalent species and divalent species. Examples of aryl groups include, but are not limited to, phenyl, biphenyl, naphthyl and the like. In this particular embodiment, an aryl is phenyl or naphthyl, especially phenyl.

The term "optionally substituted" refers to either groups, structures, or molecules that are substituted and those that are not substituted.

Where optional substituents are chosen from "one or more" groups it is to be understood that this definition includes all substituents being chosen from one of the specified groups or the substituents being chosen from two or more of the specified groups.

The phrase "compound of the invention" means those compounds which are disclosed herein according to Formula IC or Formula IF, both generically and specifically.

### Compounds of the Invention

In a first aspect, the present invention provides a pharmaceutical composition comprising a compound according to Formula IC or Formula IF, or a pharmaceutically acceptable salt thereof, and one or more pharmaceutically acceptable excipients: 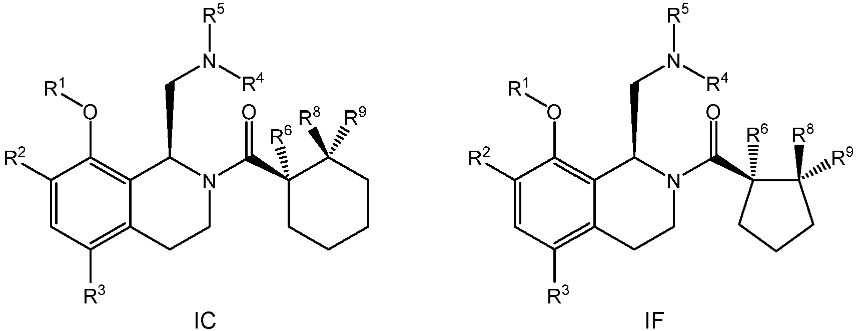 wherein:
R¹ is selected from C₁₋₄alkylene-R¹¹, heterocyclyl and 8-10 membered bicyclic heteroaryl, wherein said heterocyclyl is optionally substituted with one or more substituents independently selected from C₁₋₄alkyl, -C(O)-R¹², SO₂-R¹³, C₁₋₃alkylene-OR¹⁴ and heteroaryl which is optionally substituted with one or more substituents independently selected from C₁₋₄alkyl, C₃₋₇cycloalkyl, halo, OH, C₁₋₃alkoxy and cyano; and wherein said 8-10 membered bicyclic heteroaryl is optionally substituted with one or more substituents independently selected from C₁₋₄alkyl, C₃₋₇cycloalkyl, halo, OH and C₁₋₃alkoxy;
R² is selected from hydrogen, fluoro, chloro and C₁₋₃alkyl;
R³ is selected from hydrogen, fluoro, chloro, bromo, C₁₋₃alkoxy, C₁₋₃alkyl, C₁₋₃haloalkyl and cyano;
R⁴ is hydrogen or C₁₋₄alkyl;
R⁵ is -C(O)-C₁₋₄alkyl, -C(O)-C₃₋₇cycloalkyl, -C(O)-heteroaryl or -C(O)-aryl, wherein said heteroaryl and aryl are optionally substituted with one or more substituents selected from C₁₋₄alkyl, halo, hydroxy, C₁₋₃alkoxy, CO₂R¹⁵ and cyano; or
R⁴ and R⁵, taken together with the nitrogen atom to which they are attached, form a 4-, 5-, or 6-membered heterocyclyl ring, wherein said heterocyclyl ring:
   comprises one or more -C(O)- moieties attached to the nitrogen atom;
   optionally contains one or more additional heteroatoms selected from oxygen, nitrogen and sulfur;
   optionally is fused to an aryl or heteroaryl ring;
   optionally is spiro-attached to a C₃₋₇cycloalkyl group or a 3- to 6-membered heterocyclyl ring; and
   optionally is substituted with one or more substituents independently selected from C₁₋₄alkyl, halo, OH, C₁₋₃alkoxy, C₁₋₃haloalkyl, cyano, NR¹⁶R¹⁷, C(O)R¹⁸, S(O)R¹⁹ and SO₂R²⁰;
R⁶ is selected from hydrogen, C₁₋₄alkyl, and C₃₋₇cycloalkyl;
R⁸ is C(=O)NR^{8a}R^{8b} or -C(=O)R^{8c};
R^{8a} is hydrogen or C₁₋₆alkyl;
R^{8b} is hydrogen, C₁₋₄alkyl or C₃₋₅cycloalkyl, wherein the C₁₋₄alkyl group is optionally substituted with one or more substituents independently selected from halo, OH, C₁₋₃alkoxy, C₃₋₇cycloalkyl and cyano; or
R^{8a} and R^{8b}, taken together with the nitrogen atom to which they are attached, form a 3-, 4-, 5-, 6-, or 7-membered heterocyclyl ring, wherein the heterocyclyl ring:
   optionally contains one or more additional heteroatoms selected from oxygen, nitrogen and sulfur; and
   is optionally substituted with one or more substituents independently selected from C₁₋₄alkyl, C₃₋₇cycloalkyl, halo, OH, C₁₋₃alkoxy, C₁₋₃haloalkyl, cyano, NR¹⁶R¹⁷, C(O)R¹⁸, S(O)R¹⁹ and SO₂R²⁰;
R^{8c} is C₁₋₃alkyl or C₁₋₃haloalkyl;
R⁹ is methyl;
R¹¹ is selected from -C(O)-R²⁴, -SO₂-R²⁵, -NR²⁶C(O)-R²⁷, -NR²⁸SO₂-R²⁹, heterocyclyl, aryl and heteroaryl, wherein said aryl and heteroaryl groups are optionally substituted with one or more substituents independently selected from C₁₋₄alkyl, C₁₋₃haloalkyl, C₃₋₇cycloalkyl, C₁₋₄alkylene-R³⁰, halo, OH, C₁₋₃alkoxy, heterocyclyl and cyano; and said heterocyclyl group is optionally substituted with one or more substituents independently selected from C₁₋₄alkyl, C₁₋₃haloalkyl, C₃₋₇cycloalkyl, C₁₋₄alkylene-R³⁰, halo, OH, C₁₋₃alkoxy, oxo and cyano;
R¹² is selected from C₁₋₄alkyl, C₃₋₇cycloalkyl, OR³¹, NR³²R³³, aryl and heteroaryl, wherein said aryl and heteroaryl are optionally substituted with one or more substituents independently selected from C₁₋₄alkyl, halo, OH, C₁₋₃alkoxy and cyano;
R¹³ is selected from C₁₋₄alkyl, C₃₋₇cycloalkyl, heteroaryl, heterocyclyl and NR³⁴R³⁵, wherein said heteroaryl and heterocyclyl are optionally substituted with one or more substituents independently selected from C₁₋₄alkyl, halo, OH, C₁₋₃alkoxy and cyano;
R¹⁷ is selected from hydrogen, C₁₋₄alkyl, C(O)C₁₋₃alkyl and C(O)NR³⁶R³⁷,
R¹⁸, R¹⁹ and R²⁰ are independently selected from C₁₋₄alkyl, OH, C₁₋₃alkoxy and NR³⁸R³⁹;
R²⁴ is selected from C₁₋₄alkyl, NR⁴⁰R⁴¹ and OR⁴²;
R²⁵ is selected from C₁₋₄alkyl and NR⁴³R⁴⁴;
R²⁷ is selected from C₁₋₄alkyl, C₃₋₇cycloalkyl, C₁₋₃haloalkyl, heterocyclyl, aryl and heteroaryl, wherein said aryl and heteroaryl are optionally substituted with one or more substituents independently selected from C₁₋₄alkyl, C₁₋₃haloalkyl, C₃₋₇cycloalkyl, C₁₋₄alkylene-R⁴⁵, halo, OH, C₁₋₃alkoxy and cyano;
R²⁹ is selected from C₁₋₄alkyl, C₃₋₇cycloalkyl, C₁₋₃haloalkyl, aryl and heteroaryl, wherein said aryl and heteroaryl are optionally substituted with one or more substituents independently selected from C₁₋₄alkyl, C₁₋₃haloalkyl, C₃₋₇cycloalkyl, C₁₋₄alkylene-R⁴⁶ halo, OH, C₁₋₃alkoxy and cyano;
R³⁰ is selected from hydroxy, C₁₋₃alkoxy, C₃₋₇cycloalkyl, cyano and NR⁴⁷R⁴⁸;
R⁴⁰ is selected from hydrogen and C₁₋₄alkyl;
R⁴¹ is selected from hydrogen, C₁₋₄alkyl, C₃₋₇cycloalkyl, C₁₋₃alkoxy, aryl and heteroaryl; or
R⁴⁰ and R⁴¹, taken together with the nitrogen atom to which they are attached, form a 4-, 5-, or 6-membered heteroaryl or heterocyclyl ring, wherein said heteroaryl and heterocyclyl rings are optionally substituted with one or more substituents independently selected from C₁₋₄alkyl, halo, OH, C₁₋₃alkoxy, C₃₋₇cycloalkyl and cyano;
R⁴⁵ and R⁴⁶ are independently selected from hydroxy, C₁₋₃alkoxy and C₃₋₇cycloalkyl; and
R¹⁴, R¹⁵, R¹⁶, R²⁶, R²⁸, R³¹, R³², R³³, R³⁴, R³⁵, R³⁶, R³⁷, R³⁸, R³⁹, R⁴², R⁴³, R⁴⁴, R⁴⁷ and R⁴⁸ are independently selected from hydrogen, C₁₋₄alkyl and C₃₋₇cycloalkyl.

Particular pharmaceutical compositions of the invention include, for example, compounds of the formula IC or IF, or pharmaceutically acceptable salts thereof, wherein, unless otherwise stated, each of R¹, R², R³, R⁴, R⁵, L¹, L², X¹, X², R⁶, R⁷, R⁸, R^{8a}, R^{8b}, R^{8c}, R⁹, R¹⁰, R¹¹, R¹², R¹³, R¹⁷, R¹⁸, R¹⁹, R²⁰, R²⁴, R²⁵, R²⁷, R²⁹, R³⁰, R⁴⁰ and R⁴¹ has any of the meanings defined hereinbefore or in any of paragraphs (1) to (111) hereinafter unless contradicted by the appended claims. For the avoidance of doubt, the scope of the present invention encompasses pharmaceutical compositions comprising a compound of formula IC or IF, or pharmaceutically acceptable salts thereof, wherein any of the substituent definitions defined herein may be combined with any of the other substituent definitions also defined herein unless contradicted by the appended claims:-
(1) R¹ is C₁₋₄alkylene-R¹¹;
(2) R¹ is CH₂-R¹¹;
(3) R¹ is CH₂CH₂-R¹¹;
(4) R¹ is CH₂CH₂CH₂-R¹¹;
(5) R¹ is CH(Me)-R¹¹;
(6) R¹ is heterocyclyl, optionally substituted with one or more substituents independently selected from C₁₋₄alkyl, -C(O)-R¹², SO₂-R¹³, heteroaryl and C₁₋₃alkylene-OR¹⁴, wherein said heteroaryl is optionally substituted with one or more substituents independently selected from C₁₋₄alkyl, C₃₋₇cycloalkyl, halo, OH, C₁₋₃alkoxy and cyano;
(7) R¹ is heterocyclyl, optionally substituted with one or more substituents independently selected from C₁₋₄alkyl, -C(O)-R¹², SO₂-R¹³, heteroaryl and C₁₋₃alkylene-OR¹⁴, wherein said heteroaryl is optionally substituted with one or more substituents independently selected from C₁₋₄alkyl and C₃₋₇cycloalkyl;
(8) R¹ is piperidinyl or pyrrolidinyl, each optionally substituted with one or more substituents independently selected from -C(O)-R¹², SO₂-R¹³, heteroaryl and C₁₋₃alkylene-OR¹⁴, wherein said heteroaryl is optionally substituted with one or more substituents independently selected from C₁₋₄alkyl, C₃₋₇cycloalkyl, halo, OH, C₁₋₃alkoxy and cyano;
(9) R¹ is pyrrolidinyl, optionally substituted with one or more substituents independently selected from -C(O)-R¹², SO₂-R¹³, heteroaryl and C₁₋₃alkylene-OR¹⁴, wherein said heteroaryl is optionally substituted with C₁₋₄alkyl or C₃₋₇cycloalkyl;
(10) R¹ is selected from one of the following groups: 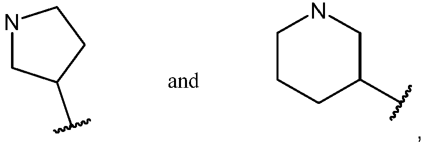 wherein 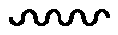 represents the point of attachment of the group to the oxygen atom of the rest of the compound and wherein each group is optionally substituted with one or more substituents independently selected from -C(O)-R¹², SO₂-R¹³, heteroaryl and C₁₋₃alkylene-OR¹⁴, wherein said heteroaryl is optionally substituted with C₁₋₄alkyl or C₃₋₇cycloalkyl;
(11) R¹ is an 8-10 membered bicyclic heteroaryl optionally substituted with one or more substituents independently selected from C₁₋₄alkyl, C₃₋₇cycloalkyl, halo, OH and C₁₋₃alkoxy;
(12) R¹ is an 8-membered bicyclic heteroaryl optionally substituted with one or more substituents independently selected from C₁₋₄alkyl, OH and C₁₋₃alkoxy;
(13) R¹ is selected from one of the following groups: 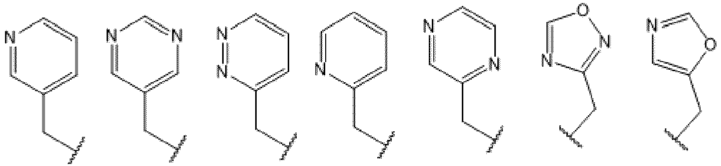 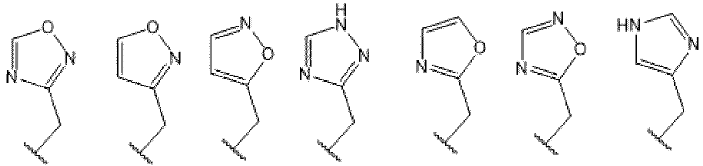 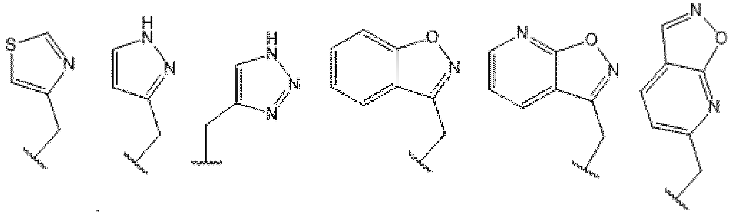 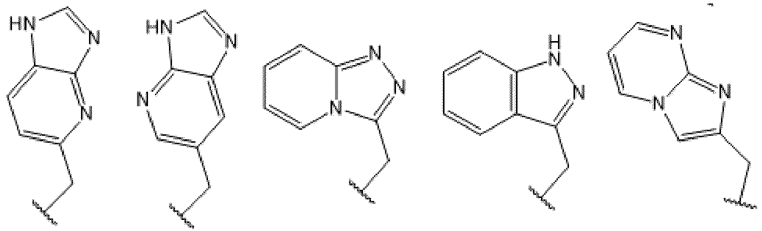 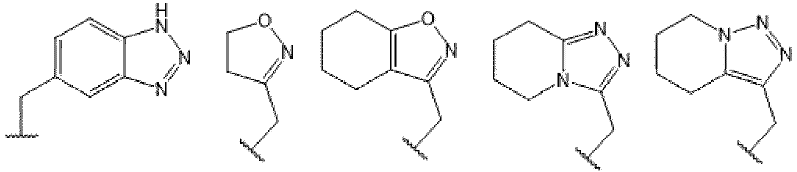 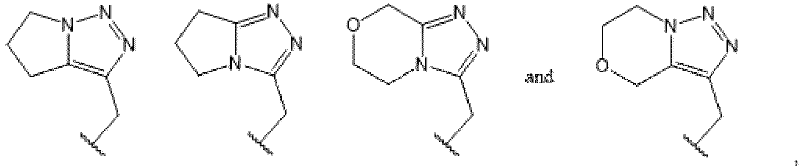 wherein 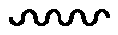 represents the point of attachment of the group to the oxygen atom of the rest of the compound and wherein each group is optionally substituted with one or more substituents independently selected from C₁₋₄alkyl, C₁₋₃haloalkyl, C₃₋₇cycloalkyl, C₁₋₄alkylene-R³⁰, halo, OH, C₁₋₃alkoxy, heterocycloalkyl and cyano;
(14) R¹ is selected from one of the following groups: 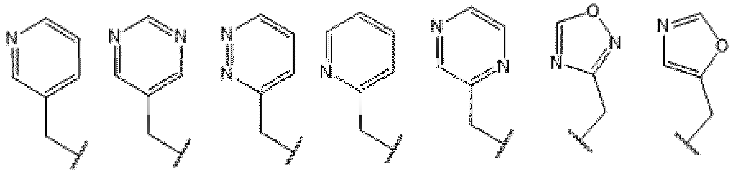 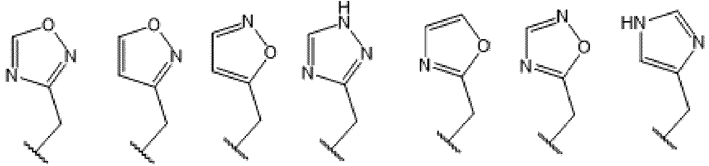 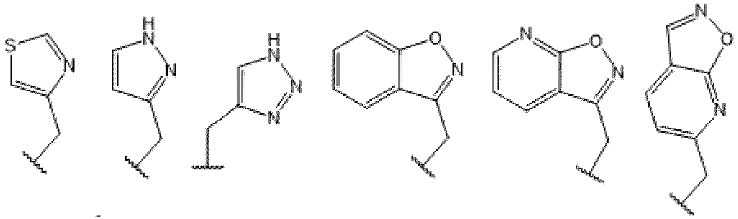 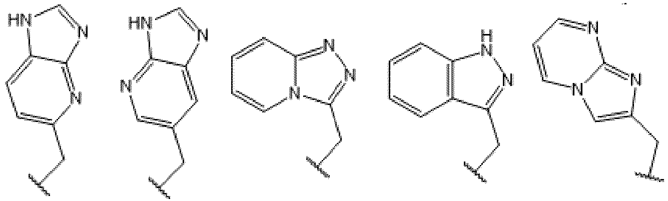 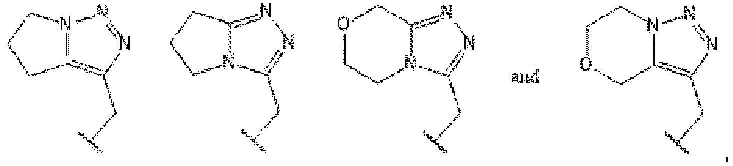 wherein 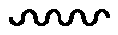 represents the point of attachment of the group to the oxygen atom of the rest of the compound and wherein each group is optionally substituted with one or more substituents independently selected from C₁₋₄alkyl, C₁₋₃haloalkyl, C₃₋₇cycloalkyl, C₁₋₄alkylene-R³⁰, C₁₋₃alkoxy and cyano;
(15) R¹ is selected from one of the following groups: 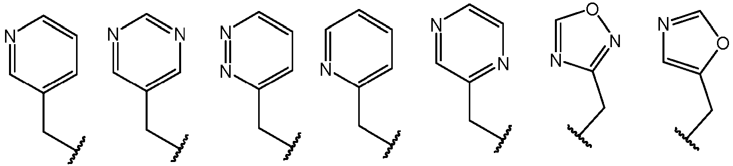 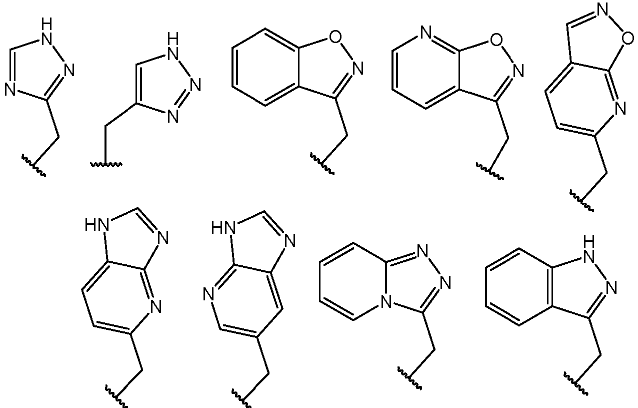 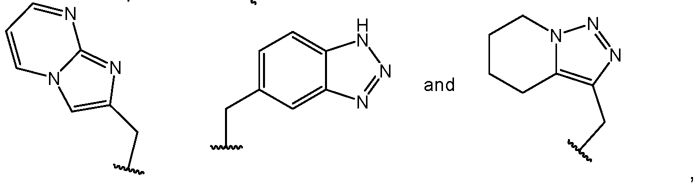 wherein 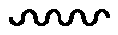 represents the point of attachment of the group to the oxygen atom of the rest of the compound and wherein each group is optionally substituted with one or more substituents independently selected from C₁₋₄alkyl, C₁₋₃haloalkyl, C₃₋₇cycloalkyl, C₁₋₄alkylene-R³⁰, halo, OH and C₁₋₃alkoxy;
(16) R¹ is: 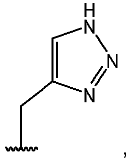 wherein 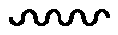 represents the point of attachment of the group to the oxygen atom of the rest of the compound and wherein the group is optionally substituted with one or more substituents independently selected from C₁₋₄alkyl, C₁₋₃haloalkyl, C₃₋₇cycloalkyl and C₁₋₄alkylene-R³⁰;
(17) R¹ is: 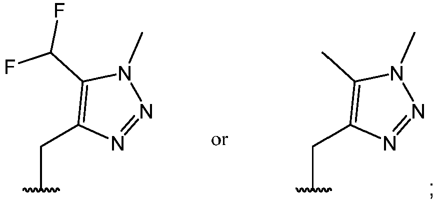
(18) R² is selected from hydrogen, fluoro and chloro;
(19) R² is hydrogen or fluoro;
(20) R³ is selected from hydrogen, chloro, bromo, C₁₋₃alkoxy, C₁₋₃alkyl, C₁₋₃haloalkyl and cyano;
(21) R³ is selected from hydrogen, chloro, bromo, methoxy, methyl, trifluoromethyl and cyano;
(22) R³ is selected from hydrogen and chloro;
(23) R³ is chloro;
(24) R⁴ is hydrogen;
(25) R⁵ is -C(O)-C₁₋₄alkyl, -C(O)-C₃₋₇cycloalkyl, or -C(O)-aryl, wherein said aryl group is optionally substituted with one or more substituents selected from C₁₋₄alkyl, halo, hydroxy, C₁₋₃alkoxy, CO₂R¹⁵ and cyano;
(26) R⁴ is hydrogen and R⁵ is -C(O)-C₁₋₄alkyl or -C(O)-aryl, wherein said aryl group is optionally substituted with one or more substituents selected from C₁₋₄alkyl, halo, hydroxy, C₁₋₃alkoxy, CO₂R¹⁵ and cyano;
(27) R⁴ is hydrogen and R⁵ is -C(O)-C₁₋₄alkyl;
(28) R⁴ and R⁵, taken together with the nitrogen atom to which they are attached, form a 5-, or 6-membered heterocyclyl ring, wherein said heterocyclyl ring:
   comprises one or more -C(O)- moieties attached to the nitrogen atom;
   optionally contains one or more additional heteroatoms selected from oxygen, nitrogen and sulfur;
   optionally is fused to an aryl ring;
   optionally is spiro-attached to a C₃₋₇cycloalkyl group or a 3- to 5-membered heterocyclyl ring; and
   optionally is substituted with one or more substituents independently selected from C₁₋₄alkyl, halo, OH, C₁₋₃alkoxy, C₁₋₃haloalkyl, cyano, NR¹⁶R¹⁷, C(O)R¹⁸, S(O)R¹⁹ and SO₂R²⁰;
(29) R⁴ and R⁵, taken together with the nitrogen atom to which they are attached, form a 5-, or 6-membered heterocyclyl ring, wherein said heterocyclyl ring:
   optionally contains one or more additional heteroatoms selected from oxygen, and nitrogen;
   optionally is fused to an aryl ring;
   optionally is spiro-attached to a C₃₋₇cycloalkyl group; and
   optionally is substituted with one or more substituents independently selected from C₁₋₄alkyl, halo, OH, C₁₋₃alkoxy, C₁₋₃haloalkyl, cyano, NR¹⁶R¹⁷, C(O)R¹⁸, S(O)R¹⁹ and SO₂R²⁰;
(30) R⁴ and R⁵, taken together with the nitrogen atom to which they are attached, form a 5-membered heterocyclyl ring, wherein said heterocyclyl ring:
   comprises one or more -C(O)- moieties attached to the nitrogen atom;
   optionally contains one or more additional heteroatoms selected from oxygen, and nitrogen;
   optionally is fused to an aryl ring;
   optionally is spiro-attached to a C₃₋₇cycloalkyl group; and
   optionally is substituted with one or more substituents independently selected from C₁₋₄alkyl, halo and OH;
(31) R⁴ and R⁵, taken together with the nitrogen atom to which they are attached, form a 5-membered heterocyclyl ring, wherein said heterocyclyl ring comprises a -C(O)-moiety attached to the nitrogen atom and is optionally fused to a phenyl ring, or optionally spiro-attached to a cyclopropyl group; and said heterocyclyl ring is optionally substituted with one or more substituents independently selected from methyl, fluoro and OH;
(32) R⁴ and R⁵, taken together with the nitrogen atom to which they are attached, form a heterocyclic moiety selected from one of the following: 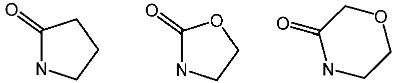 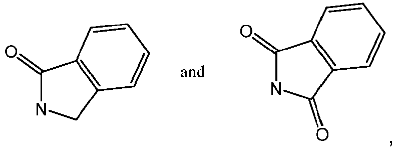 wherein the saturated ring of the heterocyclic moiety is optionally spiro-attached to a C₃₋₇cycloalkyl group, and wherein said heterocyclic moiety is optionally substituted with one or more substituents independently selected from C₁₋₄alkyl, halo and OH;
(33) R⁴ and R⁵, taken together with the nitrogen atom to which they are attached, form a heterocyclic moiety selected from one of the following: 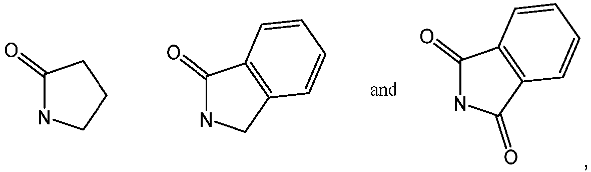 wherein the saturated ring of the heterocyclic moiety is optionally spiro-attached to a cyclopropyl group, and wherein said heterocyclic moiety is optionally substituted with one or more substituents independently selected from methyl, fluoro and OH;
(34) R⁴ and R⁵, taken together with the nitrogen atom to which they are attached, form the following heterocyclic moiety: 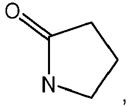 wherein the heterocyclic moiety is optionally spiro-attached to a cyclopropyl group and is optionally substituted with one or more substituents independently selected from methyl and fluoro;
(35) R⁴ and R⁵, taken together with the nitrogen atom to which they are attached, form the following heterocyclic moiety: 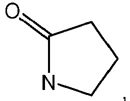 wherein the heterocyclic moiety is optionally spiro-attached to a cyclopropyl group, and is optionally substituted with C₁₋₃alkyl, wherein said cyclopropyl and/or C₁₋₃alkyl group is attached to the heterocyclic ring at a position either alpha or beta to the carbonyl group;
(36) X¹ is a group -CR⁶ and X² is a group -C*R⁹;
(37) X¹ is a group -CR⁶ and X² is nitrogen;
(38) X¹ is nitrogen and X² is a group -C*R⁹;
(39) L¹ and L² are independently selected from a bond and -CH₂-;
(40) L¹ is a bond and L² is -CH₂-;
(41) L¹ is CH₂- and L² is a bond;
(42) L¹ and L² are both bonds;
(43) R⁶ and R⁷ are independently selected from hydrogen and C₁₋₄alkyl;
(44) R⁶ and R⁷ are independently selected from hydrogen and methyl;
(45) R⁶ is hydrogen;
(46) R⁷ is hydrogen;
(47) R⁶ and R⁷ are both hydrogen;
(48) R⁶ and R⁷, taken together with the carbon atom to which they are attached, form a 3-, 4-, 5-, or 6-membered cycloalkyl ring;
(49) X¹ is a group -CR⁶, X² is nitrogen or a group -C*R⁹ and R³ is -C(=O)NR^{8a}R^{8b};
(50) X¹ is a group -CR⁶, X² is a group -C*R⁹ and R⁸ is -C(=O)NR^{8a}R^{8b};
(51) X¹ is nitrogen, X² is a group -C*R⁹ and R⁸ is -C(=O)NR^{8a}R^{8b};
(52) X¹ is a group -CR⁶, X² is nitrogen and R⁸ is -C(=O)NR^{8a}R^{8b};
(53) X¹ is a group -CR⁶, X² is nitrogen and R⁸ is -C(=O)R^{8c};
(54) R⁸ is C(=O)NR^{8a}R^{8b};
(55) R⁸ is -C(=O)R^{8c};
(56) R^{8a} is hydrogen or methyl;
(57) R^{8a} is hydrogen;
(58) R^{8b} is hydrogen, C₁₋₆alkyl or C₃₋₇cycloalkyl, wherein the C₁₋₆alkyl group is optionally substituted with one or more substituents independently selected from halo, OH, C₁₋₃alkoxy, C₃₋₇cycloalkyl, cyano, NR¹⁶R¹⁷, C(O)R¹⁸, S(O)R¹⁹ and SO₂R²⁰;
(59) R^{8b} is hydrogen, C₁₋₄alkyl or C₃₋₅cycloalkyl, wherein the C₁₋₄alkyl group is optionally substituted with one or more substituents independently selected from halo, OH, C₁₋₃alkoxy C₃₋₇cycloalkyl and cyano;
(60) R^{8b} is C₁₋₄alkyl or C₃₋₅cycloalkyl, wherein the C₁₋₄alkyl group is optionally substituted with one or more substituents independently selected from fluoro, OH, C₁₋₃alkoxy C₃₋₇cycloalkyl and cyano;
(61) R^{8b} is hydrogen, methyl, ethyl, n-propyl, cyclopropyl, cyclobutyl or cyclopropylmethyl wherein the methyl, ethyl or n-propyl groups are optionally substituted with one or more substituents independently selected from fluoro, OH, methoxy and cyano;
(62) R^{8b} is methyl, ethyl, n-propyl, cyclopropyl, cyclobutyl or cyclopropylmethyl;
(63) R^{8b} is methyl;
(64) R^{8a} is hydrogen and R^{8b} is hydrogen, C₁₋₆alkyl or C₃₋₇cycloalkyl, wherein the C₁₋₆alkyl group is optionally substituted with one or more substituents independently selected from halo, OH, C₁₋₃alkoxy, C₃₋₇cycloalkyl, cyano, NR¹⁶R¹⁷, C(O)R¹⁸, S(O)R¹⁹ and SO₂R²⁰;
(65) R^{8a} is hydrogen and R^{8b} is C₁₋₆alkyl or C₃₋₇cycloalkyl, wherein the C₁₋₆alkyl group is optionally substituted with one or more substituents independently selected from fluoro, OH, C₁₋₃alkoxy, C₃₋₇cycloalkyl, cyano, NR¹⁶R¹⁷, C(O)R¹⁸, S(O)R¹⁹ and SO₂R²⁰;
(66) R^{8a} is hydrogen and R^{8b} is methyl, ethyl, n-propyl, cyclopropyl, cyclobutyl or cyclopropylmethyl;
(67) R^{8a} is hydrogen and R^{8b} is methyl;
(68) R^{8a} and R^{8b}, taken together with the nitrogen atom to which they are attached, form a 3-, 4-, or 5-membered heterocyclyl ring, wherein the heterocyclyl ring optionally contains one or more additional heteroatoms selected from oxygen, nitrogen and sulfur, and is optionally substituted with one or more substituents independently selected from C₁₋₄alkyl, halo, OH, C₁₋₃alkoxy, C₁₋₃haloalkyl, C₃₋₇cycloalkyl, cyano, NR¹⁶R¹⁷, C(O)R¹⁸, S(O)R¹⁹ and SO₂R²⁰;
(69) R^{8a} and R^{8b}, taken together with the nitrogen atom to which they are attached, form a 4-, or 5-membered heterocyclyl ring, wherein the heterocyclyl ring is optionally substituted with one or more substituents independently selected from C₁₋₄alkyl, halo, OH, C₁₋₃alkoxy, C₁₋₃haloalkyl, C₃₋₇cycloalkyl, cyano, NR¹⁶R¹⁷, C(O)R¹⁸, S(O)R¹⁹ and SO₂R²⁰;
(70) R^{8a} and R^{8b}, taken together with the nitrogen atom to which they are attached, form an azetidinyl ring optionally substituted with one or more substituents independently selected from methyl, fluoro, OH, methoxy and C₁₋₃fluoroalkyl;
(71) R^{8c} is C₁₋₃alkyl or C₁₋₃fluoroalkyl;
(72) R^{8c} is C₁₋₃fluoroalkyl;
(73) R^{8c} is fluoromethyl, difluoromethyl or trifluoromethyl;
(74) R⁹ is selected from hydrogen, C₁₋₄alkyl, C₁₋₃alkoxy and halo;
(75) R⁹ is selected from hydrogen, methyl, methoxy and fluoro;
(76) R⁹ is selected from C₁₋₄alkyl, C₁₋₃alkoxy and halo;
(77) R⁹ is selected from methyl, methoxy and fluoro;
(78) R⁹ is hydrogen or C₁₋₄alkyl;
(79) R⁹ is hydrogen or methyl;
(80) R⁹ is methyl;
(81) R¹⁰ is selected from hydrogen and methyl;
(82) R⁹ and R¹⁰, taken together with the carbon atom to which they are attached, form a 3-, 4-, 5-, or 6-membered cycloalkyl ring;
(83) L² is a bond and R⁷ and R¹⁰, taken together with the atoms to which they are attached, form a 4-, 5- or 6-membered cycloalkyl or heterocyclyl ring, wherein:
   said heterocyclyl ring contains 1 or 2 heteroatoms independently selected from nitrogen, oxygen and sulfur;
   said cycloalkyl ring optionally comprises 1 or 2 carbon-carbon double bonds and is optionally bridged by a C₁₋₃alkylene group connecting two carbon atoms of the ring, or R⁹ is optionally a C₁₋₃alkylene group connecting C* to a carbon atom of the ring;
   said cycloalkyl and heterocyclyl rings are optionally substituted with one or more substituents independently selected from C₁₋₄alkyl, halo, OH, C₁₋₃alkoxy and deuterium; and
   said cycloalkyl and heterocyclyl rings are optionally spiro-attached to a C₃₋₇cycloalkyl group;
(84) L¹ and L² are both bonds and R⁷ and R¹⁰, taken together with the atoms to which they are attached, form a 5- or 6-membered cycloalkyl or heterocyclyl ring, wherein:
   said heterocyclyl ring contains 1 or 2 heteroatoms independently selected from nitrogen, oxygen and sulfur;
   said cycloalkyl ring optionally comprises a carbon-carbon double bond and is optionally bridged by a C₁₋₃alkylene group connecting two carbon atoms of the ring, or R⁹ is optionally a C₁₋₃alkylene group connecting C* to a carbon atom of the ring;
   said cycloalkyl and heterocyclyl rings are optionally substituted with one or more substituents independently selected from C₁₋₄alkyl, halo, OH, C₁₋₃alkoxy and deuterium; and
   said cycloalkyl and heterocyclyl rings are optionally spiro-attached to a C₃₋₇cycloalkyl group;
(85) L² is a bond, X¹ is a group -CR⁶, X² is a group -C*R⁹ and R⁷ and R¹⁰, taken together with the atoms to which they are attached, form a 5- or 6-membered cycloalkyl ring, wherein said cycloalkyl ring is optionally bridged by a C₁₋₃alkylene group connecting two carbon atoms of the ring, or R⁹ is optionally a C₁₋₃alkylene group connecting C* to a carbon atom of the ring, and said cycloalkyl ring is optionally substituted with one or more substituents independently selected from C₁₋₄alkyl, halo, OH, C₁₋₃alkoxy and deuterium;
(86) L² is a bond, X¹ is a group -CR⁶, X² is a group -CR⁹ and R⁷ and R¹⁰, taken together with the atoms to which they are attached, form a cyclohexyl ring, wherein said cyclohexyl ring optionally comprises a carbon-carbon double bond and is optionally bridged by a C₁₋₃alkylene group connecting two carbon atoms of the ring, and said cyclohexyl ring is optionally substituted with one or more substituents independently selected from C₁₋₄alkyl, halo, OH and deuterium;
(87) L¹ and L² are both bonds, X¹ is a group -CR⁶, X² is nitrogen and R⁷ and R¹⁰, taken together with the atoms to which they are attached, form a 5- or 6-membered heterocyclyl ring, wherein said heterocyclyl ring is optionally substituted with one or more substituents independently selected from C₁₋₄alkyl, halo, OH, C₁₋₃alkoxy and deuterium; and said heterocyclyl ring is optionally spiro-attached to a C₃₋₇cycloalkyl group;
(88) L¹ and L² are both bonds, X¹ is a group -CR⁶, X² is nitrogen and R⁷ and R¹⁰, taken together with the atoms to which they are attached, form a pyrrolidinyl or piperidinyl ring, wherein said ring is optionally substituted with one or more substituents independently selected from methyl, halo, OH, methoxy and deuterium; and said ring is optionally spiro-attached to a cyclopropyl group;
(89) L¹ and L² are both bonds, X¹ is nitrogen, X² is a group -CR⁹ and R⁷ and R¹⁰, taken together with the atoms to which they are attached, form a 5- or 6-membered heterocyclyl ring, wherein said heterocyclyl ring is optionally substituted with one or more substituents independently selected from C₁₋₄alkyl, halo, OH, C₁₋₃alkoxy and deuterium; and said heterocyclyl ring is optionally spiro-attached to a C₃₋₇cycloalkyl group;
(90) L¹ and L² are both bonds, X¹ is nitrogen, X² is a group -CR⁹ and R⁷ and R¹⁰, taken together with the atoms to which they are attached, form a pyrrolidinyl or piperidinyl ring, wherein said ring is optionally substituted with one or more substituents independently selected from methyl, halo, OH, methoxy and deuterium; and said ring is optionally spiro-attached to a cyclopropyl group;
(91) R¹¹ is selected from -C(O)-R²⁴, -SO₂-R²⁶, -NR²⁶C(O)-R²⁷, -NR²⁸SO₂-R²⁹, and heteroaryl wherein said heteroaryl is optionally substituted with one or more substituents independently selected from C₁₋₄alkyl, C₁₋₃haloalkyl, C₃₋₇cycloalkyl, C₁₋₄alkylene-R³⁰, halo, OH, C₁₋₃alkoxy, heterocyclyl and cyano;
(92) R¹¹ is -C(O)-R²⁴ and R²⁴ is selected from NR⁴⁰R⁴¹ and OR⁴²;
(93) R¹¹ is -NR²⁶C(O)-R²⁷ and R²⁷ is selected from C₁₋₄alkyl, C₃₋₇cycloalkyl, heterocyclyl, aryl and heteroaryl, wherein said aryl and heteroaryl are optionally substituted with one or more substituents independently selected from C₁₋₄alkyl and C₃₋₇cycloalkyl;
(94) R¹¹ is -NR²⁸SO₂-R²⁹ and R²⁹ is selected from C₁₋₄alkyl, C₃₋₇cycloalkyl and heteroaryl, wherein said heteroaryl is optionally substituted with one or more substituents independently selected from C₁₋₄alkyl and C₃₋₇cycloalkyl;
(95) R¹¹ is heteroaryl optionally substituted with one or more substituents independently selected from C₁₋₄alkyl, C₁₋₃haloalkyl, C₃₋₇cycloalkyl, C₁₋₄alkylene-R³⁰, halo, OH, C₁₋₃alkoxy, heterocyclyl and cyano;
(96) R¹¹ is heteroaryl optionally substituted with one or more substituents independently selected from C₁₋₄alkyl, C₁₋₃haloalkyl, C₃₋₇cycloalkyl, C₁₋₄alkylene-R³⁰, C₁₋₃alkoxy, and cyano;
(97) R¹¹ is heteroaryl optionally substituted with one or more substituents independently selected from methyl, ethyl, isopropyl, difluoromethyl, trifluoromethyl, chloro, fluoro, cyclopropyl, methoxy, CH₂-R³⁰ and CH₂CH₂-R³⁰;
(98) R¹¹ is pyridyl, pyrimidinyl, pyridazinyl, pyrazinyl, oxazolyl, isoxazolyl, oxadiazolyl, 1,2,3-triazolyl, 1,2,4-triazolyl, 1,2,4-oxadiazolyl, indazolyl, benzotriazolyl, benzisoxazolyl, isoxazolopyridinyl, imidazopyridinyl or triazolopyridinyl, each optionally substituted with one or more substituents independently selected from C₁₋₄alkyl, C₁₋₃haloalkyl, C₃₋₇cycloalkyl, C₁₋₄alkylene-R³⁰, halo, OH, C₁₋₃alkoxy, heterocyclyl and cyano;
(99) R¹¹ is heteroaryl selected from: 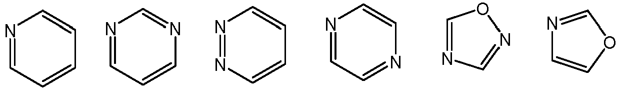 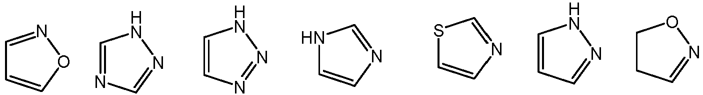 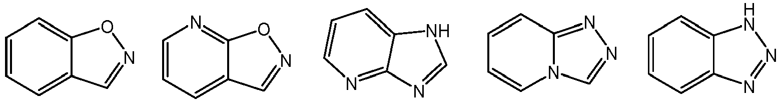 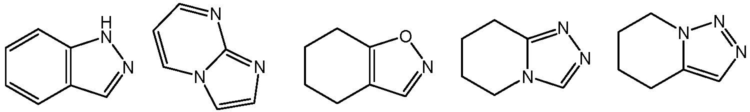 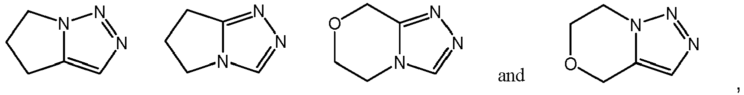 each heteroaryl being optionally substituted with one or more substituents independently selected from C₁₋₄alkyl, C₁₋₃haloalkyl, C₃₋₇cycloalkyl, C₁₋₄alkylene-R³⁰, halo, OH, C₁₋₃alkoxy, heterocycloalkyl and cyano;
(100) R¹¹ is oxazolyl, isoxazolyl or 1,2,3-triazolyl, each optionally substituted with one or more substituents independently selected from C₁₋₄alkyl, C₁₋₃haloalkyl, C₃₋₇cycloalkyl, C₁₋₄alkylene-R³⁰, halo, OH, C₁₋₃alkoxy, heterocycloalkyl and cyano;
(101) R¹¹ is 1,2,3-triazolyl optionally substituted with one or more substituents independently selected from C₁₋₄alkyl, C₁₋₃haloalkyl, C₃₋₇cycloalkyl, C₁₋₄alkylene-R³⁰, halo, OH, C₁₋₃alkoxy, heterocycloalkyl and cyano;
(102) R¹¹ is heterocyclyl optionally substituted with one or more substituents independently selected from C₁₋₄alkyl, C₁₋₃haloalkyl, C₃₋₇cycloalkyl, C₁₋₄alkylene-R³⁰, halo, OH, C₁₋₃alkoxy, oxo and cyano;
(103) R²⁹ is selected from C₁₋₄alkyl, C₃₋₇cycloalkyl, C₁₋₃haloalkyl and heteroaryl, wherein said heteroaryl is optionally substituted with one or more substituents independently selected from C₁₋₄alkyl and C₁₋₃haloalkyl;
(104) R²⁹ is methyl, ethyl or cyclopropyl;
(105) R³⁰ is selected from hydroxy, C₁₋₃alkoxy, C₃₋₇cycloalkyl and cyano;
(106) R³⁰ is selected from hydroxy, methoxy, cyclopropyl and cyano;
(107) R⁴⁰ is selected from hydrogen and methyl;
(108) R⁴¹ is selected from hydrogen, methyl, cyclopropyl, methoxy and phenyl;
(109) R⁴⁰ and R⁴¹, taken together with the nitrogen atom to which they are attached, form 5-membered heteroaryl or heterocyclyl ring, wherein said heteroaryl and heterocyclyl rings are optionally substituted with one or more substituents independently selected from C₁₋₄alkyl, halo, OH, C₁₋₃alkoxy, C₃₋₇cycloalkyl and cyano;
(110) R⁴⁰ and R⁴¹, taken together with the nitrogen atom to which they are attached, form 5-membered heterocyclyl ring, wherein said heterocyclyl ring is optionally substituted with one or more substituents independently selected from C₁₋₄alkyl, halo and OH;
(111) R⁴⁰ and R⁴¹, taken together with the nitrogen atom to which they are attached, form 5-membered heterocyclyl ring, wherein said heterocyclyl ring is optionally substituted with one or more substituents independently selected from methyl, fluoro and OH.

Suitably, R¹ is as defined in any one of paragraphs (1) to (17) above. In an embodiment, R¹ is as defined in paragraph (2) or (3) above. In another embodiment, R¹ is as defined in paragraph (10) above. In a further embodiment, R¹ is as defined in paragraphs (16) to (17) above.

Suitably, R² is as defined in any one of paragraphs (18) to (19) above. In an embodiment, R² is as defined in paragraph (19) above.

Suitably, R³ is as defined in any one of paragraphs (20) to (23) above. In another embodiment, R³ is as defined in paragraphs (22) to (23) above.

Suitably, R⁴ is as defined in paragraph (24) above.

Suitably, R⁵ is as defined in paragraph (25) above.

Suitably, R⁴ and R⁵ are as defined in any one of paragraphs (26) to (35) above. In another embodiment, R⁴ and R⁵ are as defined in paragraph (27) above. In an embodiment, R⁴ and R⁵ are as defined in paragraphs (32) to (35) above. In a further embodiment, R⁴ and R⁵ are as defined in paragraph (35) above.

Suitably, X¹ and X² are as defined in any one of paragraphs (36) to (38) above. Preferably, X¹ and X² are as defined in paragraph (36) above. In another embodiment, X¹ and X² are as defined in any one of paragraphs (49) to (53) above. More preferably, X¹ and X² are as defined in paragraph (50) above.

Suitably, L¹ and L² are as defined in any one of paragraphs (39) to (42) above. In another embodiment, L¹ and L² are as defined in paragraph (42) above.

Suitably, R⁶ and R⁷ are as defined in any one of paragraphs (43) to (48) above. In another embodiment, R⁶ and R⁷ are as defined in paragraph (47) above.

Suitably, R⁸ is as defined in any one of paragraphs (54) to (55) above. In an embodiment, R⁸ is as defined in paragraph (54) above.

Suitably, R^{8a} is as defined in any one of paragraphs (56) to (57) above. In an embodiment, R^{8a} is as defined in paragraph (57) above.

Suitably, R^{8b} is as defined in any one of paragraphs (59) to (63) above. In an embodiment, R^{8b} is as defined in paragraph (62) or (63) above.

Suitably, R^{8a} and R^{8b} are as defined in any one of paragraphs (66) to (70) above. In an embodiment, R^{8a} and R^{8b} are as defined in paragraph (67) or (70) above.

In a particular embodiment, in any of the above paragraphs, it is provided that R^{8a} and R^{8b} cannot both be equal to hydrogen.

Suitably, R^{8c} is as defined in any one of paragraphs (71) to (73) above. In an embodiment, R^{8c} is as defined in paragraph (73) above.

R⁹ is as defined in paragraph (80) above.

Suitably, R¹⁰ is as defined in paragraph (81) above.

Suitably, R⁹ and R¹⁰ are as defined in paragraph (82) above.

Suitably, L¹, L², R⁷ and R¹⁰ are as defined in any one of paragraphs (83) to (90) above. In another embodiment, L¹, L², R⁷ and R¹⁰ are as defined in paragraphs (84) to (86) above. In an embodiment, L², R⁷ and R¹⁰ are as defined in paragraph (86) above.

Suitably, R¹¹ is as defined in any one of paragraphs (91) to (102) above. In another embodiment, R¹¹ is as defined in paragraphs (92) to (93) above. In another embodiment, R¹¹ is as defined in paragraphs (99) to (101) above. In a further embodiment, R¹ is as defined in paragraph (2) above and R¹¹ is as defined in paragraphs (99) to (101) above.

Suitably, R²⁹ is as defined in paragraphs (103) to (104) above. In an embodiment, R²⁹ is as defined in paragraph (104) above.

Suitably, R³⁰ is as defined in paragraphs (105) to (106) above. In an embodiment, R³⁰ is as defined in paragraph (106) above.

Suitably, R⁴⁰ is as defined in paragraph (107) above.

Suitably, R⁴¹ is as defined in paragraph (108) above.

Suitably, R⁴⁰ and R⁴¹ are as defined in any one of paragraphs (109) to (111) above. In another embodiment, R⁴⁰ and R⁴¹ are as defined in paragraph (111) above.

In an embodiment the pharmaceutical composition comprises a compound having the structural formula IC shown below: 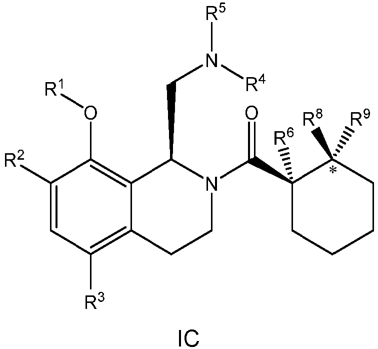 wherein R¹ to R⁶, R⁸ and R⁹ are as defined in the appended claims.

In an embodiment the compound has the structural formula IC shown above, wherein R¹ is as defined in any one of paragraphs (2) to (3), (10) or (16) to (17) above; R² is as defined in paragraph (19) above; R³ is as defined in any one of paragraphs (22) to (23) above; R⁴ and R⁶ are as defined in any one of paragraphs (27) and (32) to (35) above; R⁶ is as defined in paragraph (45) above; R⁸ is as defined in any one of paragraphs (54) to (55) above; and R⁹ is as defined in paragraph (80) above.

In an embodiment the pharmaceutical composition comprises a compound having the structural formula ID shown below: 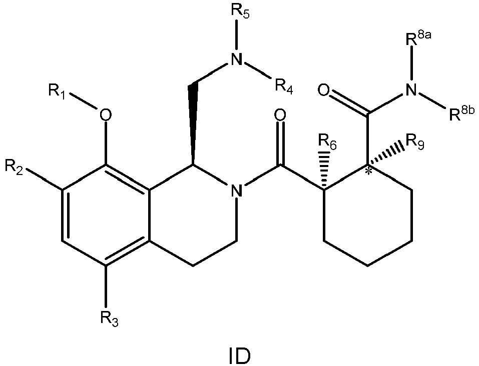 wherein R¹ to R⁶, R^{8a}, R^{8b} and R⁹ are as defined hereinbefore.

In an embodiment the compound has the structural formula ID shown above, wherein R¹ is as defined in any one of paragraphs (2) to (3), (10) or (16) to (17) above; R² is as defined in paragraph (18) above; R³ is as defined in any one of paragraphs (22) to (23) above; R⁴ and R⁵ are as defined in any one of paragraphs (27) and (32) to (35) above; R⁶ is as defined in paragraph (44) above; R^{8a} is as defined in any one of paragraphs (56) to (57) above; R^{8b} is as defined in any one of paragraphs (59) to (63) above; or R^{8a} and R^{8b} combined are as defined in any one of paragraphs (68) to (70) above; and R⁹ is as defined in paragraph (80) above.

In an embodiment the pharmaceutical composition comprises a compound having the structural formula IE shown below: 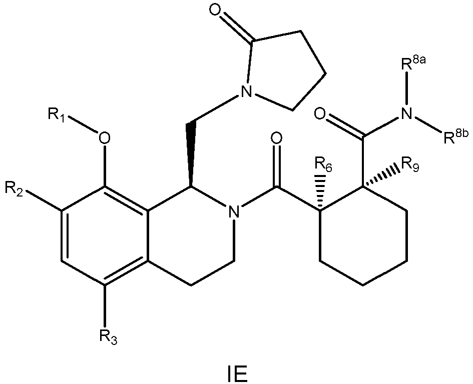 wherein R¹ to R³, R⁶, R^{8a}, R^{8b} and R⁹ are as defined hereinbefore.

In an embodiment the compound has the structural formula IE shown above, wherein R¹ is as defined in any one of paragraphs (2) to (3), (10) or (16) to (17) above; R² is as defined in paragraph (19) above; R³ is as defined in any one of paragraphs (22) to (23) above; R⁶ is as defined in paragraph (44) above; R^{8a} is as defined in any one of paragraphs (56) to (57) above; R^{8b} is as defined in any one of paragraphs (59) to (63) above; or R^{8a} and R^{8b} combined are as defined in any one of paragraphs (68) to (70) above; and R⁹ is as defined in paragraph (80) above.

In an embodiment the compound has the structural formula IE shown above, wherein R¹ is as defined in any one of paragraphs (2) to (3), (10) or (16) to (17) above; R² is as defined in paragraph (19) above; R³ is as defined in any one of paragraphs (22) to (23) above; R⁶ is as defined in paragraph (45) above; R^{8a} is as defined in paragraph (57) above; R^{8b} is as defined in paragraph (63) above; or R^{8a} and R^{8b} combined are as defined in paragraph (70) above; and R⁹ is as defined in paragraph (80) above.

In an embodiment the pharmaceutical composition comprises a compound have the structural formula IF shown below: 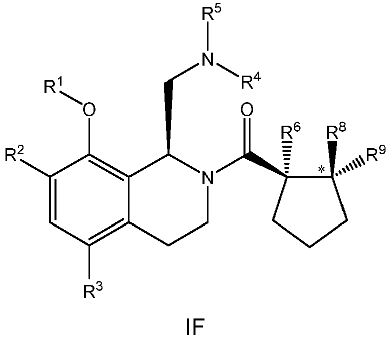 wherein R¹ to R⁶, R⁸ and R⁹ are as defined hereinbefore.

In an embodiment the compound has the structural formula IF shown above, wherein R¹ is as defined in any one of paragraphs (2) to (3), (10) or (16) to (17) above; R² is as defined in paragraph (19) above; R³ is as defined in any one of paragraphs (22) to (23) above; R⁴ and R⁵ are as defined in any one of paragraphs (27) and (32) to (35) above; R⁶ is as defined in paragraph (45) above; R⁸ is as defined in any one of paragraphs (54) to (55) above; and R⁹ is as defined in paragraph (80) above.

In an embodiment the compound has the structural formula IF shown above, wherein R¹ is as defined in any one of paragraphs (2) to (3), (10) or (16) to (17) above; R² is as defined in paragraph (19) above; R³ is as defined in any one of paragraphs (22) to (23) above; R⁴ and R⁵ are as defined in any one of paragraphs (27) and (32) to (35) above; R⁶ is as defined in paragraph (45) above; R⁸ is as defined in paragraph (54) above; and R⁹ is as defined in paragraph (80) above.

In an embodiment the pharmaceutical composition comprises a compound having the structural formula IG shown below: 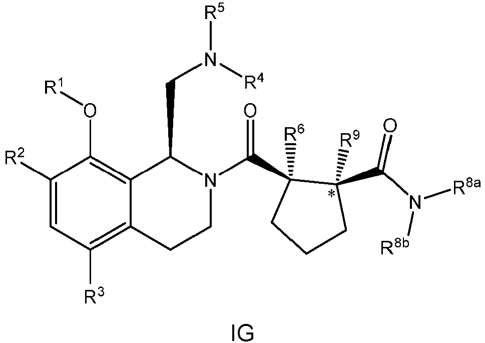 wherein R¹ to R⁶, R^{8a}, R^{8b} and R⁹ are as defined hereinbefore.

In an embodiment the compound has the structural formula IG shown above, wherein R¹ is as defined in any one of paragraphs (2) to (3), (10) or (16) to (17) above; R² is as defined in paragraph (19) above; R³ is as defined in any one of paragraphs (22) to (23) above; R⁴ and R⁵ are as defined in any one of paragraphs (27) and (32) to (35) above; R⁶ is as defined in paragraph (45) above; R^{8a} is as defined in any one of paragraphs (56) to (57) above; R^{8b} is as defined in any one of paragraphs (59) to (63) above; or R^{8a} and R^{8b} combined are as defined in any one of paragraphs (67) to (70) above; and R⁹ is as defined in paragraph (80) above.

In an embodiment the pharmaceutical composition comprises a compound, the compound having the structural formula IC or IF shown above, wherein R¹ is as defined in any one of paragraphs (2) to (3), (10) or (16) to (17) above; R² is as defined in paragraph (19) above; R³ is as defined in any one of paragraphs (22) to (23) above; R⁴ and R⁵ are as defined in any one of paragraphs (27) and (32) to (35) above; R⁶ is as defined in paragraph (45) above; R⁸ is as defined in any one of paragraphs (54) to (55) above; and R⁹ is methyl.

In an embodiment the pharmaceutical composition comprises a compound, the compound having the structural formula IC or IF shown above, wherein R¹ is as defined in any one of paragraphs (2) to (3), (10) or (16) to (17) above; R² is as defined in paragraph (19) above; R³ is as defined in any one of paragraphs (22) to (23) above; R⁴ and R⁵ are as defined in any one of paragraphs (27) and (32) to (35) above; R⁶ is as defined in paragraph (45) above; R⁸ is as defined in paragraph (54) above; and R⁹ is methyl.

In an embodiment the pharmaceutical composition comprises a compound, the compound having the structural formula IH shown below: 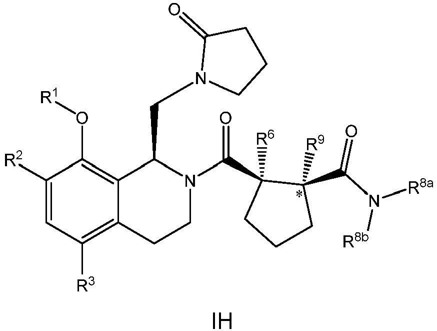 wherein R¹ to R³, R⁶, R^{8a}, R^{8b} and R⁹ are as defined hereinbefore.

In an embodiment the compound has the structural formula IH shown above, wherein R¹ is as defined in any one of paragraphs (2) to (3), (10) or (16) to (17) above; R² is as defined in paragraph (19) above; R³ is as defined in any one of paragraphs (22) to (23) above; R⁶ is as defined in paragraph (45) above; R^{8a} is as defined in any one of paragraphs (56) to (57) above; R^{8b} is as defined in any one of paragraphs (59) to (63) above; or R^{8a} and R^{8b} combined are as defined in any one of paragraphs (67) to (70) above; and R⁹ is as defined in paragraph (80) above.

In an embodiment the pharmaceutical composition comprises a compound, the compound having the Formula IP or IQ, or a pharmaceutically acceptable salt thereof: 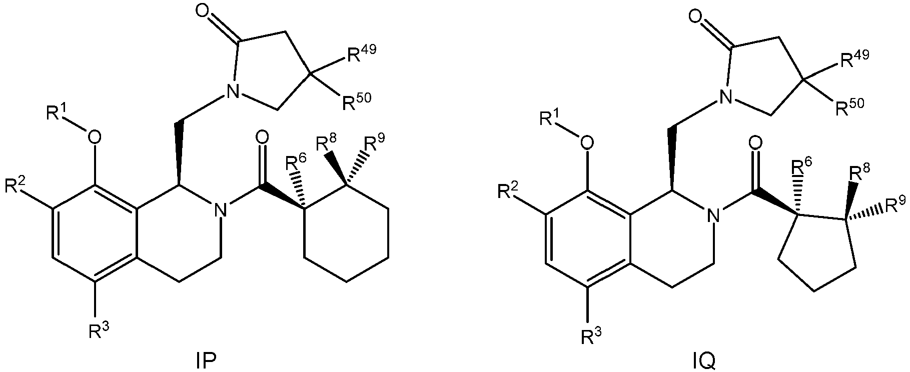 wherein R¹ to R³, R⁶and R⁸ are as defined hereinbefore, R⁹ is methyl and R⁴⁹ and R⁵⁰ are independently selected from hydrogen, C₁₋₄alkyl and halo; or R⁴⁹ and R⁵⁰, together with the carbon atom to which they are attached, form a cyclopropyl or cyclobutyl ring.

In an embodiment the compound has the structural formula IP or IQ shown above, wherein R¹ is as defined in any one of paragraphs (2) to (3), (10) or (16) to (17) above; R² is as defined in paragraph (19) above; R³ is as defined in any one of paragraphs (22) to (23) above; R⁶ is as defined in paragraph (45) above; R⁸ is as defined in any one of paragraphs (54) to (55) above; R⁹ is methyl; and R⁴⁹ and R⁵⁰ are independently selected from hydrogen and C₁₋₃alkyl; or R⁴⁹ and R⁵⁰, together with the carbon atom to which they are attached, form a cyclopropyl or cyclobutyl ring.

In an embodiment the compound has the structural formula IP or IQ shown above, wherein R¹ is as defined in any one of paragraphs (2) to (3), (10) or (16) to (17) above; R² is as defined in paragraph (19) above; R³ is chloro; R⁶ is as defined in paragraph (45) above; R³ is as defined in paragraph (54) above; R⁹ is methyl; and R⁴⁹ and R⁵⁰ are independently selected from hydrogen and methyl; or R⁴⁹ and R⁵⁰, together with the carbon atom to which they are attached, form a cyclopropyl ring.

In an embodiment, the pharmaceutical composition comprises a compound selected from one of the following:
(1S,2R)-2-((S)-5-chloro-8-((5-(difluoromethyl)-1-methyl-1H-1,2,3-triazol-4-yl)methoxy)-1-((2-oxopyrrolidin-1-yl)methyl)-1,2,3,4-tetrahydroisoquinoline-2-carbonyl)-N,1-dimethylcyclohexane-1-carboxamide;
(1S,2R)-2-((S)-8-(benzo[d]isoxazol-3-ylmethoxy)-5-chloro-1-((2-oxopyrrolidin-1-yl)methyl)-1,2,3,4-tetrahydroisoquinoline-2-carbonyl)-N,1-dimethylcyclohexane-1-carboxamide;
(1S,2R)-2-((S)-5-chloro-8-((5-(difluoromethyl)-1-methyl-1H-1,2,3-triazol-4-yl)methoxy)-1-((2-oxopyrrolidin-1-yl)methyl)-1,2,3,4-tetrahydroisoquinoline-2-carbonyl)-N,1-dimethylcyclopentane-1-carboxamide;
(1S,2R)-2-((S)-5-chloro-8-((1,5-dimethyl-1H-1,2,3-triazol-4-yl)methoxy)-1-((2-oxopyrrolidin-1-yl)methyl)-1,2,3,4-tetrahydroisoquinoline-2-carbonyl)-N,1-dimethylcyclohexane-1-carboxamide;
(1S,2R)-2-((S)-5-chloro-8-((1-methyl-5-(trifluoromethyl)-1H-1,2,3-triazol-4-yl)methoxy)-1-((2-oxopyrrolidin-1-yl)methyl)-1,2,3,4-tetrahydroisoquinoline-2-carbonyl)-N,1-dimethylcyclohexane-1-carboxamide;
(1S,2R)-2-((S)-5-chloro-8-((1-methyl-1H-1,2,3-triazol-4-yl)methoxy)-1-((2-oxopyrrolidin-1-yl)methyl)-1,2,3,4-tetrahydroisoquinoline-2-carbonyl)-N,1-dimethylcyclohexane-1-carboxamide;
(1S,2R)-2-((S)-5-chloro-8-((5-(difluoromethyl)-1-methyl-1H-1,2,3-triazol-4-yl)methoxy)-1-((6-oxo-5-azaspiro[2.4]heptan-5-yl)methyl)-1,2,3,4-tetrahydroisoquinoline-2-carbonyl)-N,1-dimethylcyclopentane-1-carboxamide;
(1S,2R)-2-((1S)-5-chloro-8-((5-(difluoromethyl)-1-methyl-1H-1,2,3-triazol-4-yl)methoxy)-1-((3-methyl-2-oxopyrrolidin-1-yl)methyl)-1,2,3,4-tetrahydroisoquinoline-2-carbonyl)-N,1-dimethylcyclopentane-1-carboxamide;
(1S,2R)-2-((1S)-5-chloro-8-((5-(difluoromethyl)-1-methyl-1H-1,2,3-triazol-4-yl)methoxy)-1-((3-methyl-2-oxopyrrolidin-1-yl)methyl)-1,2,3,4-tetrahydroisoquinoline-2-carbonyl)-N,1-dimethylcyclohexane-1-carboxamide;
(1S,2R)-2-((S)-5-chloro-8-((1-methyl-5-(trifluoromethyl)-1H-1,2,3-triazol-4-yl)methoxy)-1-((6-oxo-5-azaspiro[2.4]heptan-5-yl)methyl)-1,2,3,4-tetrahydroisoquinoline-2-carbonyl)-N,1-dimethylcyclohexane-1-carboxamide;
(1S,2R)-2-((S)-5-chloro-8-((5-cyclopropyl-1-methyl-1H-1,2,3-triazol-4-yl)methoxy)-1-((2-oxopyrrolidin-1-yl)methyl)-1,2,3,4-tetrahydroisoquinoline-2-carbonyl)-N,1-dimethylcyclohexane-1-carboxamide;
(1S,2R)-2-((S)-5-chloro-8-((5-(difluoromethyl)-1-methyl-1H-1,2,3-triazol-4-yl)methoxy)-1-((6-oxo-5-azaspiro[2.4]heptan-5-yl)methyl)-1,2,3,4-tetrahydroisoquinoline-2-carbonyl)-1-methylcyclohexane-1-carboxamide;
(1S,2R)-2-((S)-5-chloro-8-((1,5-dimethyl-1H-1,2,3-triazol-4-yl)methoxy)-1-((6-oxo-5-azaspiro[2.4]heptan-5-yl)methyl)-1,2,3,4-tetrahydroisoquinoline-2-carbonyl)-N,1-dimethylcyclohexane-1-carboxamide;
(1S,2R)-2-((S)-5-chloro-8-((1-methyl-1H-1 ,2,3-triazol-4-yl)methoxy)-1-((6-oxo-5-azaspiro[2.4]heptan-5-yl)methyl)-1,2,3,4-tetrahydroisoquinoline-2-carbonyl)-N, 1-dimethylcyclohexane-1-carboxamide;
(1S,2R)-2-((S)-5-chloro-8-((5-cyclopropyl-1-methyl-1H-1,2,3-triazol-4-yl)methoxy)-1-((6-oxo-5-azaspiro[2.4]heptan-5-yl)methyl)-1,2,3,4-tetrahydroisoquinoline-2-carbonyl)-N,1-dimethylcyclohexane-1-carboxamide;
(1S,2R)-2-((S)-5-chloro-8-((1-methyl-1H-1 ,2,3-triazol-4-yl)methoxy)-1-(((R)-4-methyl-2-oxopyrrolidin-1-yl)methyl)-1,2,3,4-tetrahydroisoquinoline-2-carbonyl)-N,1-dimethylcyclohexane-1-carboxamide;
(1S,2R)-2-((S)-5-chloro-8-((5-(methoxymethyl)-1-methyl-1H-1,2,3-triazol-4-yl)methoxy)-1-(((R)-4-methyl-2-oxopyrrolidin-1-yl)methyl)-1,2,3,4-tetrahydroisoquinoline-2-carbonyl)-N,1-dimethylcyclohexane-1-carboxamide;
(1S,2R)-2-((S)-5-chloro-8-((5-(difluoromethyl)-1-methyl-1H-1,2,3-triazol-4-yl)methoxy)-7-fluoro-1-((6-oxo-5-azaspiro[2.4]heptan-5-yl)methyl)-1,2,3,4-tetrahydroisoquinoline-2-carbonyl)-N,1-dimethylcyclohexane-1-carboxamide;
(1S,2R)-2-((S)-5-chloro-8-((5-(difluoromethyl)-1-methyl-1H-1,2,3-triazol-4-yl)methoxy)-7-fluoro-1-((6-oxo-5-azaspiro[2.4]heptan-5-yl)methyl)-1,2,3,4-tetrahydroisoquinoline-2-carbonyl)-1-methylcyclohexane-1-carboxamide;
(1S,2R)-2-((S)-5-chloro-8-((5-(difluoromethyl)-1-methyl-1H-1,2,3-triazol-4-yl)methoxy)-7-fluoro-1-((3-oxomorpholino)methyl)-1,2,3,4-tetrahydroisoquinoline-2-carbonyl)-N,1-dimethylcyclohexane-1-carboxamide;
(1S,2R)-2-((S)-5-chloro-8-((5-(difluoromethyl)-1-methyl-1H-1,2,3-triazol-4-yl)methoxy)-7-fluoro-1-((2-oxopyrrolidin-1-yl)methyl)-1,2,3,4-tetrahydroisoquinoline-2-carbonyl)-N,1-dimethylcyclohexane-1-carboxamide;
(1S,2R)-2-((S)-5-chloro-7-fluoro-8-((1-methyl-1H-1,2,3-triazol-4-yl)methoxy)-1-((6-oxo-5-azaspiro[2.4]heptan-5-yl)methyl)-1,2,3,4-tetrahydroisoquinoline-2-carbonyl)-N, 1-dimethylcyclohexane-1-carboxamide;
(1S,2R)-2-((S)-5-chloro-7-fluoro-1-((6-oxo-5-azaspiro[2.4]heptan-5-yl)methyl)-8-((4,5,6,7-tetrahydro-[1,2,3]triazolo[1,5-a]pyridin-3-yl)methoxy)-1,2,3,4-tetrahydroisoquinoline-2-carbonyl)-1-methylcyclohexane-1-carboxamide;
(1S,2R)-2-((S)-5-chloro-8-((1,5-dimethyl-1H-1,2,3-triazol-4-yl)methoxy)-7-fluoro-1-((6-oxo-5-azaspiro[2.4]heptan-5-yl)methyl)-1,2,3,4-tetrahydroisoquinoline-2-carbonyl)-1-methylcyclohexane-1-carboxamide; or
(1S,2R)-2-((S)-5-chloro-8-((5-cyclopropyl-1-methyl-1H-1,2,3-triazol-4-yl)methoxy)-1-(((R)-4-methyl-2-oxopyrrolidin-1-yl)methyl)-1,2,3,4-tetrahydroisoquinoline-2-carbonyl)-N,1-dimethylcyclohexane-1-carboxamide;
or a pharmaceutically acceptable salt thereof.

The various functional groups and substituents making up the compounds of Formula IC or Formula IF are typically chosen such that the molecular weight of the compound does not exceed 1000. More usually, the molecular weight of the compound will be less than 750, for example less than 700, or less than 650.

A suitable pharmaceutically acceptable salt of a compound of Formula IC or Formula IF is, for example, an acid-addition salt of a compound of the invention which is sufficiently basic, for example, an acid-addition salt with, for example, an inorganic or organic acid, for example hydrochloric, hydrobromic, sulfuric, phosphoric, trifluoroacetic, formic, citric or maleic acid. In addition a suitable pharmaceutically acceptable salt of a compound of Formula IC or Formula IF which is sufficiently acidic is an alkali metal salt, for example a sodium or potassium salt, an alkaline earth metal salt, for example a calcium or magnesium salt, an ammonium salt or a salt with an organic base which affords a physiologically-acceptable cation, for example a salt with methylamine, dimethylamine, trimethylamine, piperidine, morpholine or tris-(2-hydroxyethyl)amine.

Compounds that have the same molecular formula but differ in the nature or sequence of bonding of their atoms or the arrangement of their atoms in space are termed "isomers". Isomers that differ in the arrangement of their atoms in space are termed "stereoisomers". Stereoisomers that are not mirror images of one another are termed "diastereomers" and those that are non-superimposable mirror images of each other are termed "enantiomers". When a compound has an asymmetric centre, for example, it is bonded to four different groups, a pair of enantiomers is possible. An enantiomer can be characterized by the absolute configuration of its asymmetric centre and is described by the R- and S-sequencing rules of Cahn and Prelog, or by the manner in which the molecule rotates the plane of polarized light and designated as dextrorotatory or levorotatory (i.e., as (+) or (-)-isomers respectively). A chiral compound can exist as either individual enantiomer or as a mixture thereof. A mixture containing equal proportions of the enantiomers is called a "racemic mixture".

The compounds of Formula IC or Formula IF typically possess one or more asymmetric centers; such compounds can therefore be produced as individual (R)- or (S)-stereoisomers or as mixtures thereof. Unless indicated otherwise, the description or naming of a particular compound in the specification and claims is intended to include both individual enantiomers, diastereoisomers and mixtures, racemic or otherwise, thereof. The methods for the determination of stereochemistry and the separation of stereoisomers are well-known in the art (see discussion in Chapter 4 of "Advanced Organic Chemistry", 4th edition J. March, John Wiley and Sons, New York, 2001), for example by synthesis from optically active starting materials or by resolution of a racemic form. Some of the compounds of the invention may have geometric isomeric centres (E- and Z- isomers). It is to be understood that the present invention encompasses all optical, diastereoisomers and geometric isomers and mixtures thereof that possess Nrf2 activation activity.

The present invention also encompasses compounds of Formula IC or Formula IF as defined herein which comprise one or more isotopic substitutions. For example, H may be in any isotopic form, including ¹H, ²H (D) and ³H (T); C may be in any isotopic form including ¹²C, ¹³C, and ¹⁴C; and O may be in any isotopic form, including ¹⁶O and ¹⁸O; and the like.

It is also to be understood that certain compounds of Formula IC or Formula IF may exist in solvated as well as unsolvated forms such as, for example, hydrated forms. It is to be understood that the invention encompasses all such solvated forms that possess Nrf2 activation activity.

It is also to be understood that certain compounds of Formula IC or Formula IF may exhibit polymorphism, and that the invention encompasses all such forms that possess Nrf2 activation activity.

Compounds of Formula IC or Formula IF may exist in a number of different tautomeric forms and references to compounds of the invention include all such forms. For the avoidance of doubt, where a compound can exist in one of several tautomeric forms, and only one is specifically described or shown, all others are nevertheless embraced by compounds of Formula IC or Formula IF. Examples of tautomeric forms include keto-, enol-, and enolate-forms, as in, for example, the following tautomeric pairs: keto/enol (illustrated below), imine/enamine, amide/imino alcohol, amidine/amidine, nitroso/oxime, thioketone/enethiol, and nitro/aci-nitro.

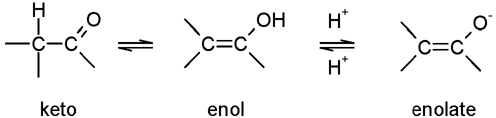

Compounds of Formula IC or Formula IF containing an amine function may also form N-oxides. A reference herein to a compound of Formula IC or Formula IF that contains an amine function also includes the N-oxide. Where a compound contains several amine functions, one or more than one nitrogen atom may be oxidised to form an N-oxide. Particular examples of N-oxides are the N-oxides of a tertiary amine or a nitrogen atom of a nitrogen-containing heterocycle. N-Oxides can be formed by treatment of the corresponding amine with an oxidizing agent such as hydrogen peroxide or a per-acid (e.g. a peroxycarboxylic acid), see for example Advanced Organic Chemistry, by Jerry March, 4th Edition, Wiley Interscience, pages. More particularly, N-oxides can be made by the procedure of L. W. Deady (Syn. Comm. 1977, 7, 509-514) in which the amine compound is reacted with m-chloroperoxybenzoic acid (MCPBA), for example, in an inert solvent such as dichloromethane.

The compounds of Formula IC or Formula IF may be administered in the form of a pro-drug which is broken down in the human or animal body to release a compound of Formula IC or Formula IF n. A pro-drug may be used to alter the physical properties and/or the pharmacokinetic properties of a compound of Formula IC or Formula IF. A pro-drug can be formed when the compound of Formula IC or Formula IF contains a suitable group or substituent to which a property-modifying group can be attached.

Accordingly, the present invention includes those compounds of Formula IC or Formula IF as defined hereinbefore when made available by organic synthesis and when made available within the human or animal body by way of cleavage of a pro-drug thereof. Accordingly, the present invention includes those compounds of Formula IC or Formula IF that are produced by organic synthetic means and also such compounds that are produced in the human or animal body by way of metabolism of a precursor compound, that is a compound of Formula IC or Formula IF may be a synthetically-produced compound or a metabolically-produced compound.

A suitable pharmaceutically acceptable pro-drug of a compound of Formula IC or Formula IF is one that is based on reasonable medical judgement as being suitable for administration to the human or animal body without undesirable pharmacological activities and without undue toxicity.

Various forms of pro-drug have been described, for example in the following documents:
a) Methods in Enzymology, Vol. 42, p. 309-396, edited by K. Widder, et al. (Academic Press, 1985);
b) Design of Pro-drugs, edited by H. Bundgaard, (Elsevier, 1985);
c) A Textbook of Drug Design and Development, edited by Krogsgaard-Larsen and H. Bundgaard, Chapter 5 "Design and Application of Pro-drugs", by H. Bundgaard p. 113-191 (1991);
d) H. Bundgaard, Advanced Drug Delivery Reviews, 8, 1-38 (1992);
e) H. Bundgaard, et al., Journal of Pharmaceutical Sciences, 77, 285 (1988);
f) N. Kakeya, et al., Chem. Pharm. Bull., 32, 692 (1984);
g) T. Higuchi and V. Stella, "Pro-Drugs as Novel Delivery Systems", A.C.S. Symposium Series, Volume 14; and
h) E. Roche (editor), "Bioreversible Carriers in Drug Design", Pergamon Press, 1987.

The *in vivo* effects of a compound of Formula IC or Formula IF may be exerted in part by one or more metabolites that are formed within the human or animal body after administration of a compound of Formula IC or Formula IF. As stated hereinbefore, the *in vivo* effects of a compound of Formula IC or Formula IF may also be exerted by way of metabolism of a precursor compound (a pro-drug).

It shall also be appreciated that compounds of Formula IC or Formula IF may also be covalently linked (at any suitable position) to other groups such as, for example, solubilising moieties (for example, PEG polymers), moieties that enable them to be bound to a solid support (such as, for example, biotin-containing moieties), and targeting ligands (such as antibodies or antibody fragments).

### Synthesis

In the description of the synthetic methods described below and in the referenced synthetic methods that are used to prepare the starting materials, it is to be understood that all proposed reaction conditions, including choice of solvent, reaction atmosphere, reaction temperature, duration of the experiment and workup procedures, can be selected by a person skilled in the art.

It is understood by one skilled in the art of organic synthesis that the functionality present on various portions of the molecule must be compatible with the reagents and reaction conditions utilised.

Necessary starting materials may be obtained by standard procedures of organic chemistry. The preparation of such starting materials is described in conjunction with the following representative process variants and within the accompanying Examples. Alternatively, necessary starting materials are obtainable by analogous procedures to those illustrated which are within the ordinary skill of an organic chemist.

It will be appreciated that during the synthesis of the compounds of the invention in the processes defined below, or during the synthesis of certain starting materials, it may be desirable to protect certain substituent groups to prevent their undesired reaction. The skilled chemist will appreciate when such protection is required, and how such protecting groups may be put in place, and later removed.

For examples of protecting groups see one of the many general texts on the subject, for example, "Protecting groups in Organic Synthesis (3rd Ed), John Wiley & Sons, NY (1999)", T. Greene & P. Wuts. Protecting groups may be removed by any convenient method described in the literature or known to the skilled chemist as appropriate for the removal of the protecting group in question, such methods being chosen so as to effect removal of the protecting group with the minimum disturbance of groups elsewhere in the molecule.

Thus, if reactants include, for example, groups such as amino, carboxy or hydroxy it may be desirable to protect the group in some of the reactions mentioned herein.

By way of example, a suitable protecting group for an amino or alkylamino group is, for example, an acyl group, for example an alkanoyl group such as acetyl, an alkoxycarbonyl group, for example a methoxycarbonyl, ethoxycarbonyl or tert-butoxycarbonyl group, an arylmethoxycarbonyl group, for example benzyloxycarbonyl, or an aroyl group, for example benzoyl. The deprotection conditions for the above protecting groups necessarily vary with the choice of protecting group. Thus, for example, an acyl group such as an alkanoyl or alkoxycarbonyl group or an aroyl group may be removed by, for example, hydrolysis with a suitable base such as an alkali metal hydroxide, for example lithium or sodium hydroxide. Alternatively, an acyl group such as a tert-butoxycarbonyl group may be removed, for example, by treatment with a suitable acid as hydrochloric, sulfuric or phosphoric acid or trifluoroacetic acid and an arylmethoxycarbonyl group such as a benzyloxycarbonyl group may be removed, for example, by hydrogenation over a catalyst such as palladium-on-carbon, or by treatment with a Lewis acid for example BF₃.OEt₂. A suitable alternative protecting group for a primary amino group is, for example, a phthaloyl group which may be removed by treatment with an alkylamine, for example dimethylaminopropylamine, or with hydrazine.

The person skilled in the art will recognise that the compounds of the invention may be prepared, in known manner, in a variety of ways. Compounds of Formula IC or Formula IF can be prepared by the methods given below, by the methods given in the experimental or by analogous methods. The routes described are merely illustrative of some of the methods that can be employed for the synthesis of compounds of formula I and the person skilled in the art will appreciate that the order of the reaction steps is not limited to those described. It will also be appreciated that the assignment of nucleophile and electrophile is not limited to that described herein and in some cases it may be appropriate for the assignment to be reversed. Different approaches to synthetic chemistry strategy are described in "Organic Synthesis: The Disconnection Approach", 2nd edition, S. Warren and P. Wyatt (2008).

The tetrahydroisoquinoline (THIQ) scaffold, wherein R³ is bromo, may be constructed according to the route outlined in Scheme 1.

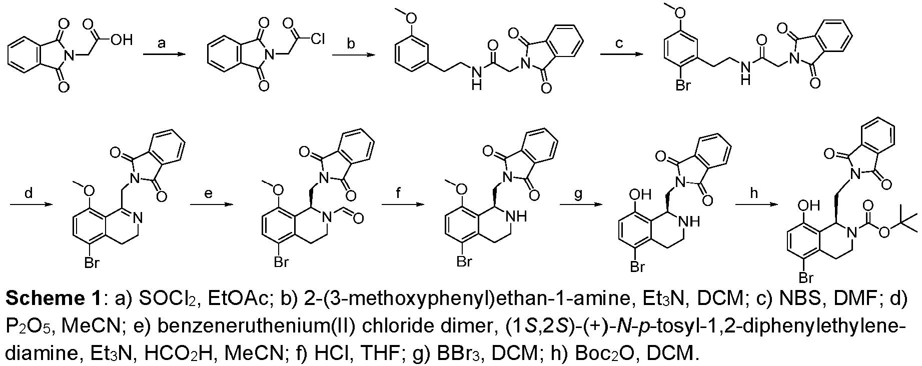

Variations at the R³ position can be conveniently prepared from compounds of the invention or intermediates thereof wherein R³ is bromo from chemistry well known in the art, as illustrated in Schemes 2 and 3. Compounds of the invention wherein R³ is H, or Cl may be prepared from intermediates where R³ is Br according to the method outlined in Scheme 2.

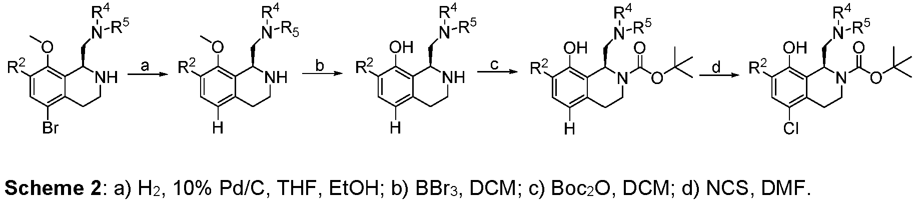

As shown in Scheme 3, the conversion of the bromo R³ substituent into a suitable boronate or boronic acid allows preparation of the fluoro and trifluoro derivatives. The bromo can be converted to an alkyl group through reaction with a suitable alkyl boronate with a suitable palladium catalyst; for example, where alkyl is methyl, a suitable boronate is 2,4,6-trimethyl-1,3,5,2,4,6-trioxatriborinane and a suitable palladium catalyst is Pd(dppf)Cl₂. Compounds wherein R³ is cyano can be prepared from bromo derivatives by treatment with a cyanating agent such as Zn(CN)₂ with a palladium catalyst such as Pd(PPh₃)₄.

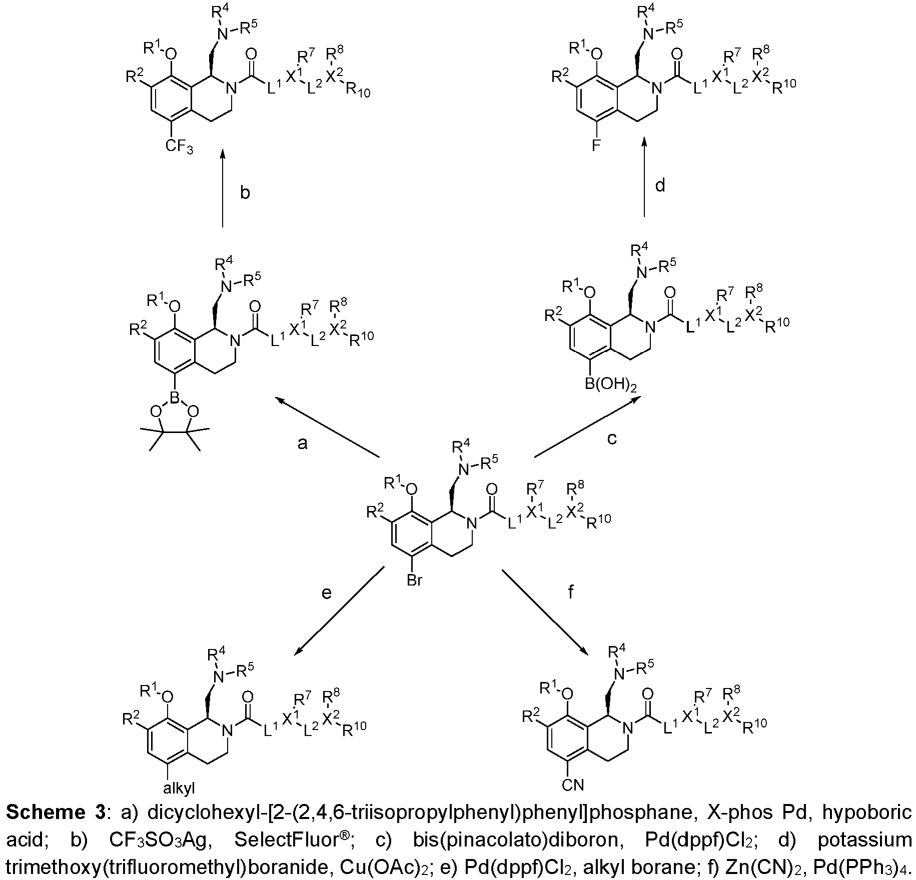

Compounds of the invention wherein -NR⁴R⁵ represents an indolinone may be prepared by reduction of the corresponding phthalimide sequentially with sodium borohydride and then triethylsilane, according to Scheme 4.

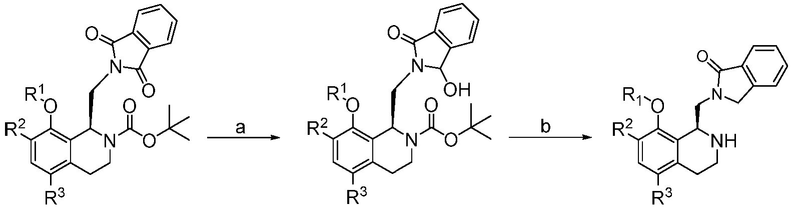

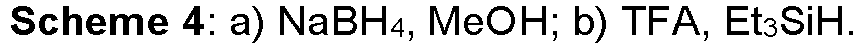

Compounds of the invention wherein the group -NR⁴R⁵ represents a pyrrolidinone may be prepared from intermediates wherein -NR⁴R⁵ represents a phthalimide group by removal of the phthalimide group with hydrazine, followed by conversion of the resulting primary amine to a pyrrolidinone by reaction with an appropriate lactone or ω-halo ester, or to an indolinone according to the routes outlined in Scheme 5.

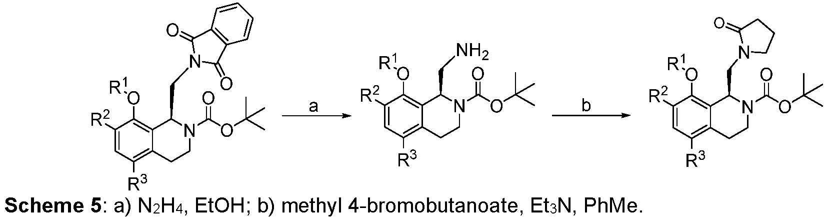

Compounds of the invention wherein R² is a halo substituent may be prepared from intermediates or Examples of the invention wherein R² is hydrogen by treatment with a suitable halogenating agent, such as N-chlorosuccinimide, as shown in Scheme 6.

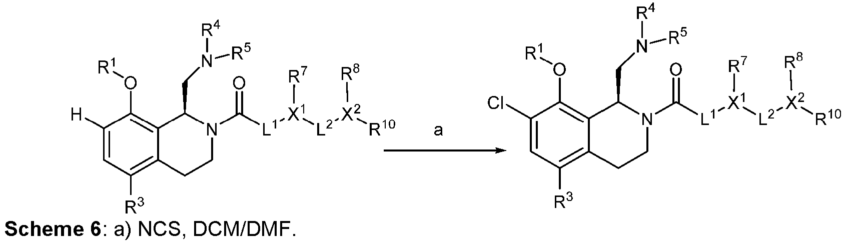

### General Method A

In a typical synthetic procedure the phthalimide, when used as a protecting group, is removed using typical reagents (e.g. hydrazine) and the amine is reacted with appropriate reagents to install the desired substitution NR⁴R⁵. A third step involves installation of the required ether through conventional methods such as alkylation with an alkyl halide or activated alcohol (e.g. mesylate, triflate) or Mitsunobu reaction using reagents such as DBAD or DEAD and an appropriate phosphine. The Boc protecting group is then removed typically by treatment with HCl. The L¹X¹(R⁷)L²X²(CO₂R)R¹⁰ group may be introduced from the appropriately substituted and protected bis-acid derivative; ideally where one of the acid groups is activated for reaction with the amine of the tetrahydroisoquinoline (THIQ) scaffold and the other acid group is suitably protected, for example as a benzyl or dimethoxybenzyl ester, or by ring opening of an appropriate cyclic anhydride, or by reaction with an acid chloride. Typical amide coupling reagents such as HATU are used to effect acid activation.

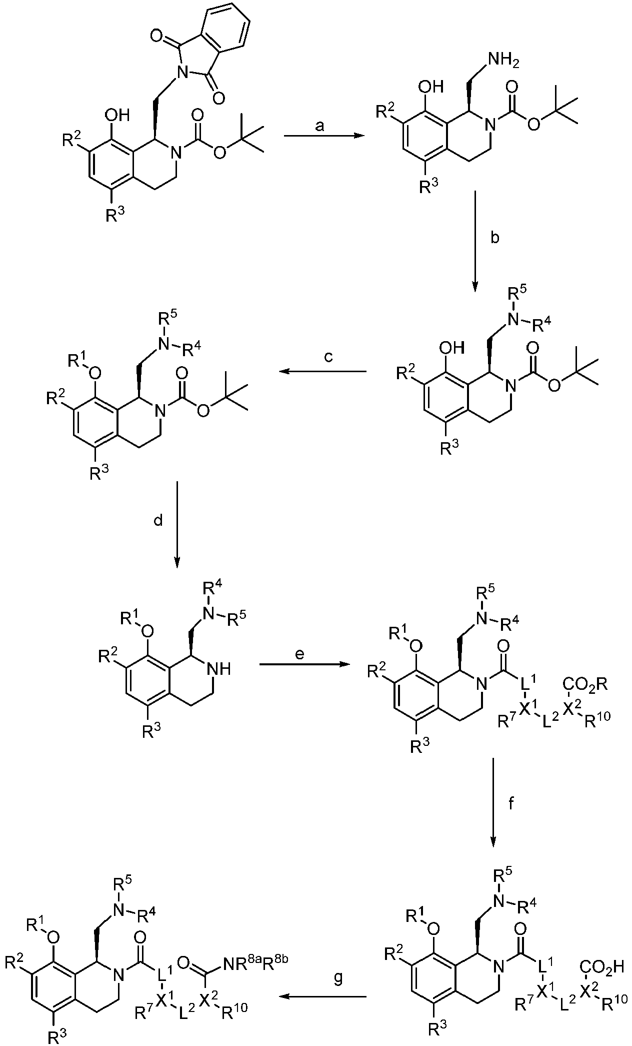

In a penultimate step, the protecting group is removed by the appropriate methodology such as hydrolysis, hydrogenolysis, strong acid such as HCl or TFA or lewis acid such as BBr₃ to give the carboxylic acid. In a final step the carboxylic acid is converted to a primary, secondary or tertiary amide using amide coupling reactions and agents well known in the art, such as HATU and 1,1'-carbonyldiimidazole (CDI).

### General Method B

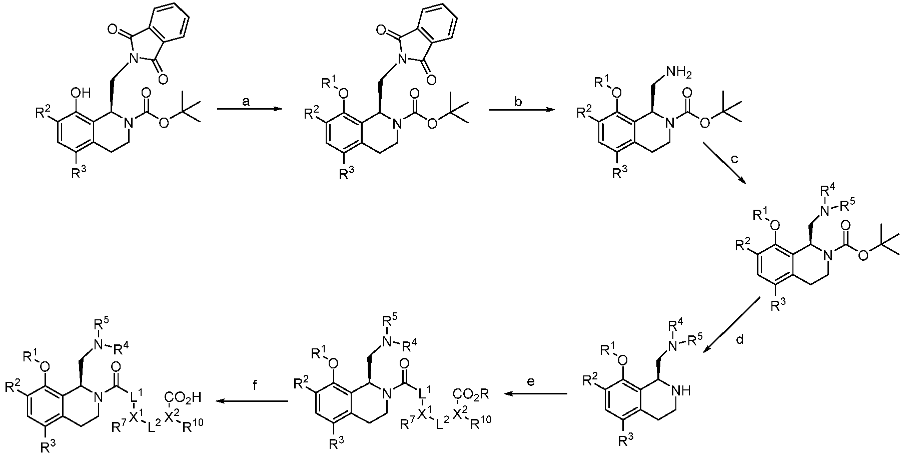

In a further typical procedure, the order of steps can be altered compared with general method A. The required ether is installed in a first step, again through conventional methods as described in the above General Method A. The phthalimide protecting group is removed in a second step and the amine is reacted with appropropriate reagents to install the desired substitution NR⁴R⁵. The Boc protecting group is then removed and the L¹X¹(R⁷)L²X²(CO₂R)R¹⁰ group may then be introduced in a fifth step. The final step comprises removal of the carboxylic acid protecting group as described in the above general methods.

Syntheses of example carboxylic acid compounds are described in the co-pending application PCT/GB2019/053012 (WO2020/084300).

### General Method C

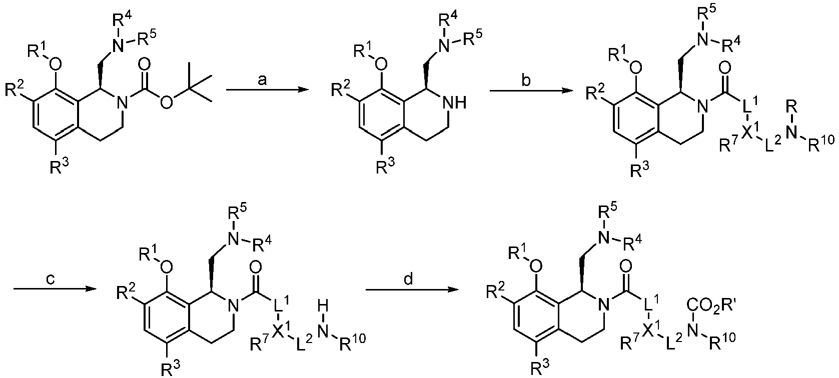

In a further typical procedure the L¹X¹(R⁷)L²N(R)R¹⁰ group may be introduced from the appropriately substituted and protected mono-acid derivative; ideally where the acid group is activated for reaction with the amine of the tetrahydroisoquinoline (THIQ) scaffold and the amine group is suitably protected, for example as a tert-butoxycarbonyl, or by reaction with an appropriate acid chloride.

### Pharmaceutical Compositions

There is provided a pharmaceutical composition which comprises a compound according to Formula IC or Formula IF, or a pharmaceutically acceptable salt thereof, and one or more pharmaceutically acceptable excipients, diluent or carrier.

The pharmaceutical compositions of the invention may be prepared and packaged in bulk form wherein a safe and effective amount of a compound of the invention can be extracted and then given to the patient such as with powders or syrups. Alternatively, the pharmaceutical compositions of the invention may be prepared and packaged in unit dosage form wherein each physically discrete unit contains a safe and effective amount of a compound of the invention. When prepared in unit dosage form, the pharmaceutical compositions of the invention typically contain from 1 mg to 1000 mg.

The compositions of the invention may be in a form suitable for oral use (for example as tablets, capsules, caplets, pills, troches, powders, syrups, elixirs, suspensions, solutions, emulsions, sachets, and cachets), for topical use (for example as creams, ointments, lotions, solutions, pastes, sprays, foams, and gels), for transdermal administration such as *via* transdermal patches, for administration by inhalation (for example as a dry powders, aerosols, suspensions, and solutions), for administration by insufflation (for example as a finely divided powder) or for parenteral administration (for example as a sterile aqueous or oily solution for intravenous, subcutaneous, intramuscular, intraperitoneal or intramuscular dosing or as a suppository for rectal dosing).

As used herein, "pharmaceutically-acceptable excipient" means a pharmaceutically acceptable material, composition or vehicle involved in giving form or consistency to the pharmaceutical composition. Each excipient must be compatible with the other ingredients of the pharmaceutical composition when commingled such that interactions which would substantially reduce the efficacy of the compound of the invention when administered to a patient and interactions which would result in pharmaceutical compositions that are not pharmaceutically acceptable are avoided. In addition, each excipient must be of sufficiently high purity to render it pharmaceutically-acceptable.

Suitable pharmaceutically-acceptable excipients will vary depending upon the particular dosage form chosen. In addition, suitable pharmaceutically-acceptable excipients may be chosen for a particular function that they may serve in the composition. For example, certain pharmaceutically-acceptable excipients may be chosen for their ability to facilitate the production of uniform dosage forms. Certain pharmaceutically-acceptable excipients may be chosen for their ability to facilitate the production of stable dosage forms. Certain pharmaceutically-acceptable excipients may be chosen for their ability to facilitate the carrying or transporting of the compound or compounds of the invention once administered to the patient from one organ, or portion of the body, to another organ, or portion of the body. Certain pharmaceutically-acceptable excipients may be chosen for their ability to enhance patient compliance.

Suitable pharmaceutically-acceptable excipients include the following types of excipients: diluents, fillers, binders, disintegrants, lubricants, glidants, granulating agents, coating agents, wetting agents, solvents, co-solvents, suspending agents, emulsifiers, sweeteners, flavoring agents, flavor masking agents, coloring agents, anticaking agents, humectants, chelating agents, plasticizers, viscosity increasing agents, antioxidants, preservatives, stabilizers, surfactants, and buffering agents. The person skilled in the art will appreciate that certain pharmaceutically-acceptable excipients may serve more than one function and may serve alternative functions depending on how much of the excipient is present in the formulation and what other ingredients are present in the formulation.

Persons skilled in the art possess the knowledge and skill to enable them to select suitable pharmaceutically-acceptable excipients in appropriate amounts for use in the invention. In addition, there are a number of resources that are available to the skilled artisan which describe pharmaceutically-acceptable excipients and may be useful in selecting suitable pharmaceutically-acceptable excipients. Examples include Remington's Pharmaceutical Sciences (Mack Publishing Company), The Handbook of Pharmaceutical Additives (Gower Publishing Limited), and The Handbook of Pharmaceutical Excipients (the American Pharmaceutical Association and the Pharmaceutical Press).

The pharmaceutical compositions of the invention are prepared using techniques and methods known to those skilled in the art. Some of the methods commonly used in the art are described in Remington's Pharmaceutical Sciences (Mack Publishing Company).

The amount of active ingredient that is combined with one or more excipients to produce a single dosage form will necessarily vary depending upon the host treated and the particular route of administration. For example, a formulation intended for oral administration to humans will generally contain, for example, from 0.5 mg to 0.5 g of active agent (more suitably from 0.5 to 100 mg, for example from 1 to 30 mg) compounded with an appropriate and convenient amount of excipients which may vary from about 5 to about 98 percent by weight of the total composition.

The size of the dose for therapeutic or prophylactic purposes of a compound of Formula IC or Formula IF will naturally vary according to the nature and severity of the conditions, the age and sex of the animal or patient and the route of administration, according to well-known principles of medicine.

In using a compound of the invention for therapeutic or prophylactic purposes it will generally be administered so that a daily dose in the range, for example, 0.1 mg/kg to 75 mg/kg body weight is received, given if required in divided doses. In general, lower doses will be administered when a parenteral route is employed. Thus, for example, for intravenous or intraperitoneal administration, a dose in the range, for example, 0.1 mg/kg to 30 mg/kg body weight will generally be used. Similarly, for administration by inhalation, a dose in the range, for example, 0.05 mg/kg to 25 mg/kg body weight will be used. Oral administration may also be suitable, particularly in tablet form. Typically, unit dosage forms will contain about 0.5 mg to 0.5 g of a compound of this invention.

### Routes of Administration

The pharmaceutical composition comprising the active compound may be administered to a subject by any convenient route of administration, whether systemically/peripherally or topically (i.e. at the site of desired action).

Routes of administration include, but are not limited to, oral (e.g., by ingestion); buccal; sublingual; transdermal (including, e.g., by a patch, plaster, etc.); transmucosal (including, e.g., by a patch, plaster, etc.); intranasal (e.g., by nasal spray); ocular (e.g., by eyedrops); pulmonary (e.g., by inhalation or insufflation therapy using, e.g., *via* an aerosol, e.g., through the mouth or nose); rectal (e.g., by suppository or enema); vaginal (e.g., by pessary); parenteral, for example, by injection, including subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, and intrasternal; by implant of a depot or reservoir, for example, subcutaneously or intramuscularly.

In a preferred embodiment, a pharmaceutical composition as defined herein is administered orally or *via* inhalation.

### Therapeutic Uses and Applications

The compounds of Formula IC or Formula IF are activators of Nrf2. As a consequence, they are potentially useful therapeutic agents for the treatment of diseases or conditions mediated by Nrf2 activation.

Thus, in one aspect, the present invention relates to a pharmaceutical composition as defined herein, for use in therapy.

In another aspect, the present invention relates to a pharmaceutical composition as defined herein, for use in the treatment of diseases or disorders mediated by Nrf2 activation.

Examples of particular diseases or conditions that the compounds of formula (I) and their pharmaceutically acceptable salts may be used to treat include, but are not limited to, any one of the following: chronic obstructive pulmonary disease, acute, chronic and severe asthma, acute lung injury/acute respiratory distress syndrome with or without accompanying multi organ dysfunction syndrome, pulmonary fibrosis including idiopathic pulmonary fibrosis, cystic fibrosis, COVID-19, diabetes, atherosclerosis, hypertension, heart failure, myocardial infarction and repair, cardiac remodelling, cardiac arrhythmias, cardiac hypertrophy, heart failure with preserved ejection fraction, diabetic cardiomyopathy, sarcopenia, obesity, metabolic syndrome, diabetes mellitus, insulin resistance, pulmonary arterial hypertension, subarachnoid haemorrhage, intracerebral haemorrhage, ischemic stroke, beta-thalassemia, sickle cell disease, rheumatoid arthritis, irritable bowel disorder, ulcerative colitis, Crohn's disease, psoriasis, radiation-induced dermatitis, atopic dermatitis, non-alcoholic fatty liver disease, non-alcoholic steatohepatitis, toxin-induced liver disease, viral hepatitis and cirrhosis, chronic kidney disease, diabetic nephropathy, autosomal dominant polycystic kidney disease, CKD associated with type 1 diabetes (T1D), IgA nephropathy (IgAN), Alport Syndrome, focal segmental glomerulosclerosis, Huntington's disease, Parkinson's disease, Alzheimer's disease, amyotrophic lateral sclerosis, frontotemporal dementia, multiple sclerosis, Friedreich's ataxia, chronic pain, schizophrenia, lung cancer, breast cancer, colon cancer, age related macular degeneration (AMD), Fuchs Endothelial Corneal Dystrophy, uveitis or preeclampsia.

In particular, the pharmaceutical compositions of the invention may be used in the treatment of chronic obstructive pulmonary disease, asthma, pulmonary arterial hypertension, diabetes mellitus, chronic kidney disease, ulcerative colitis, Crohn's disease, inflammatory bowel disease, Friedreich's ataxia, sickle cell disease or non-alcoholic steatohepatitis.

In another aspect, the present invention provides a pharmaceutical composition as defined herein, for use in the treatment of chronic obstructive pulmonary disease, acute, chronic and severe asthma, acute lung injury/acute respiratory distress syndrome with or without accompanying multi organ dysfunction syndrome, pulmonary fibrosis including idiopathic pulmonary fibrosis, cystic fibrosis, COVID-19, diabetes, atherosclerosis, hypertension, heart failure, myocardial infarction and repair, cardiac remodelling, cardiac arrhythmias, cardiac hypertrophy, heart failure with preserved ejection fraction, diabetic cardiomyopathy, sarcopenia, obesity, metabolic syndrome, diabetes mellitus, insulin resistance, pulmonary arterial hypertension, subarachnoid haemorrhage, intracerebral haemorrhage, ischemic stroke, beta-thalassemia, sickle cell disease, rheumatoid arthritis, irritable bowel disorder, ulcerative colitis, Crohn's disease, psoriasis, radiation-induced dermatitis, atopic dermatitis, non-alcoholic fatty liver disease, non-alcoholic steatohepatitis, toxin-induced liver disease, viral hepatitis and cirrhosis, chronic kidney disease, diabetic nephropathy, autosomal dominant polycystic kidney disease, CKD associated with type 1 diabetes (T1D), IgA nephropathy (IgAN), Alport Syndrome, focal segmental glomerulosclerosis, Huntington's disease, Parkinson's disease, Alzheimer's disease, amyotrophic lateral sclerosis, frontotemporal dementia, multiple sclerosis, Friedreich's ataxia, chronic pain, schizophrenia, lung cancer, breast cancer, colon cancer, age related macular degeneration (AMD), Fuchs Endothelial Corneal Dystrophy, uveitis or preeclampsia.

In another aspect, the present invention provides a pharmaceutical composition as defined herein, for use in the treatment of chronic obstructive pulmonary disease, asthma, pulmonary arterial hypertension, diabetes mellitus, chronic kidney disease, ulcerative colitis, Crohn's disease, inflammatory bowel disease, Friedreich's ataxia, sickle cell disease or non-alcoholic steatohepatitis.

In another aspect, the present disclosure (not part of the invention) provides a method of activating Nrf2 *in vitro,* said method comprising administering an effective amount of a compound, or a pharmaceutically acceptable salt thereof.

### Combination Therapies

The compounds of Formula IC or Formula IF may be administered alone as a monotherapy or may administered in combination with one or more additional therapeutic agents. The selection of the one or more additional therapeutic agents will of course vary depending on the disease or condition to be treated and its severity.

It is commonplace to use combination therapies to treat certain medical conditions.

According to a particular aspect of the disclosure (not part of the invention) there is provided a combination suitable for use in the treatment of a disease or condition in which Nrf2 activation is implicated, comprising a compound of the invention as defined hereinbefore, or a pharmaceutically acceptable salt thereof, and another therapeutic agent.

According to this aspect of the disclosure (not part of the invention) there is provided a combination suitable for use in the prevention or treatment of chronic obstructive pulmonary disease, acute, chronic and severe asthma, acute lung injury/acute respiratory distress syndrome with or without accompanying multi organ dysfunction syndrome, pulmonary fibrosis including idiopathic pulmonary fibrosis, cystic fibrosis, COVID-19, diabetes, atherosclerosis, hypertension, heart failure, myocardial infarction and repair, cardiac remodelling, cardiac arrhythmias, cardiac hypertrophy, heart failure with preserved ejection fraction, diabetic cardiomyopathy, sarcopenia, obesity, metabolic syndrome, diabetes mellitus, insulin resistance, pulmonary arterial hypertension, subarachnoid haemorrhage, intracerebral haemorrhage, ischemic stroke, beta-thalassemia, sickle cell disease, rheumatoid arthritis, irritable bowel disorder, ulcerative colitis, Crohn's disease, psoriasis, radiation-induced dermatitis, atopic dermatitis, non-alcoholic fatty liver disease, non-alcoholic steatohepatitis, toxin-induced liver disease, viral hepatitis and cirrhosis, chronic kidney disease, diabetic nephropathy, autosomal dominant polycystic kidney disease, CKD associated with type 1 diabetes (T1D), IgA nephropathy (IgAN), Alport Syndrome, focal segmental glomerulosclerosis, Huntington's disease, Parkinson's disease, Alzheimer's disease, amyotrophic lateral sclerosis, frontotemporal dementia, multiple sclerosis, Friedreich's ataxia, chronic pain, schizophrenia, lung cancer, breast cancer, colon cancer, age related macular degeneration (AMD), Fuchs Endothelial Corneal Dystrophy, uveitis or preeclampsia, the combination comprising a compound of the invention as defined hereinbefore, or a pharmaceutically acceptable salt thereof, and one or more additional therapeutic agents.

In a further aspect of the disclosure (not part of the invention) there is provided a compound of the invention or a pharmaceutically acceptable salt thereof, in combination with one or more additional therapeutic agents.

Herein, where the term "combination" is used it is to be understood that this refers to simultaneous, separate or sequential administration. In one aspect of the disclosure (not part of the invention) "combination" refers to simultaneous administration. In another aspect of the disclosure (not part of the invention) "combination" refers to separate administration. In a further aspect of the disclosure (not part of the invention) "combination" refers to sequential administration. Where the administration is sequential or separate, the delay in administering the second component should not be such as to lose the beneficial effect of the combination.

According to a further aspect of the disclosure there is provided a pharmaceutical composition which comprises a compound of Formula IC or Formula IF, or a pharmaceutically acceptable salt thereof in combination with one or more additional therapeutic agents in association with a pharmaceutically acceptable diluent or carrier.

The one or more additional therapeutic agents may comprise a further compound of the present invention. Therefore, in an embodiment, there is provided a pharmaceutical composition which comprises two compounds of the invention, or pharmaceutically acceptable salts thereof, in association with a pharmaceutically acceptable diluent or carrier.

According to a particular aspect of the disclosure there is provided a combination suitable for use in the prevention or treatment of allergic disease, inflammatory disease or autoimmune disease (e.g. asthma or COPD); a cardiovascular or metabolic disease (e.g. diabetes); a neurodegenerative disease; a chronic kidney or liver disease; sickle cell disease; pulmonary arterial hypertension; cancer; or for aiding transplantation.

According to a particular aspect of the disclosure there is provided a combination suitable for use in the prevention or treatment of chronic obstructive pulmonary disease, asthma, pulmonary arterial hypertension, diabetes mellitus, chronic kidney disease, ulcerative colitis, Crohn's disease, inflammatory bowel disease, Friedreich's ataxia, sickle cell disease or non-alcoholic steatohepatitis.

Examples of other therapeutic agents that may be used as part of a combination therapy with a compound of the present disclosure (e.g. as one of two or more active agents as part of double or triple combinations) include, but are not limited to, the following:
(i) beta2-adrenoreceptor agonists (which may be a racemate or a single enantiomer) including salmeterol, salbutamol, formoterol, salmefamol, fenoterol, carmoterol, etanterol, naminterol, clenbuterol, pirbuterol, flerbuterol, reproterol, bambuterol, indacaterol, terbutaline, vilanterol, olodaterol and salts thereof;
(ii) anticholinergic agents that act as antagonists at the muscarinic receptors that include ipratropium (for example, as the bromide, CAS 22254-24-6, sold under the name Atrovent), oxitropium and tiotropium (for example, as the bromide, CAS 136310-93-5, sold under the name Spiriva), revatropate, LAS- 34273, Aclidinium, Glycopyrronium, Umeclidinium and salts thereof;
(iii) Corticosteroid anti-inflammatory agents. Examples include methyl prednisolone, prednisolone, dexamethasone, fluticasone propionate, fluticasone furoate, beclomethasone esters (for example the 17-propionate ester or the 17,21 -dipropionate ester), budesonide, flunisolide, mometasone esters (for example mometasone furoate), triamcinolone acetonide, rofleponide, ciclesonide, butixocort propionate, RPR-106541, and ST-126;
(iv) Anti-inflammatory agents including non-steroidal anti-inflammatory drugs (NSAIDs). Examples of NSAID's include sodium cromoglycate, nedocromil sodium, phosphodiesterase (PDE) inhibitors (for example, theophylline, PDE4 inhibitors or mixed PDE3/PDE4 inhibitors), leukotriene antagonists, JAK inhibitors, Pi3K inhibitors, inhibitors of leukotriene synthesis (for example montelukast), iNOS inhibitors, tryptase and elastase inhibitors, beta-2 integrin antagonists and adenosine receptor agonists or antagonists (e.g. adenosine 2a agonists), cytokine antagonists (for example chemokine antagonists, such as a CCR3 antagonist) or inhibitors of cytokine synthesis, or 5 -lipoxygenase inhibitors;
(v) Vasodilator and anti-proliferative agents (e.g. prostanoids and PDE5 inhibitors) including Epoprostenol (Flolan), Treprostinil (Remodulin), Iloprost (Ventavis), Treprostinil (Tyvaso), Bosentan (Tracleer), Ambrisentan (Letairis), Sildenafil (Revatio), Tadalafil (Adcirca);
(vi) Anti-diabetic medications including insulins, biguanides (e.g. metformin), sulfonylureas (e.g. glimepiride), meglitinides (e.g. repaglinide), thiazolidinediones (e.g., pioglitazone), dipeptidyl peptidase IV inhibitors (e.g. sitagliptin), incretin mimetics/GLP-1 analogues (e.g. liraglutide, exenatide, dulaglutide), sodium glucose co-transporter-2 (SGLT2) inhibitors (e.g. canagliflozin, dapagliflozin and empagliflozin) and α-glucosidase inhibitors (e.g. acarbose);
(vii) Hydroxyurea and other agents used to treat sickle cell disease such as L-glutamine, NCX1443, GBT440 (voxelotor), pan-Selectin antagonists (GMI-1070, rivipansel), humanized anti-P-Selectin antibody (SelG1, crinalizumab), P-selectin aptamers, sevuparin, Regadenoson, Ticagrelor, N-Acetyl-Cysteine (NAC), phosphodiesterase 9 inhibitors (e.g. PF-04447943, IMR-687, BAY 73-6691, BAY 41-2271); and
(viii) ASK1 inhibitors such as selonsertib, FXR agonists such as obeticholic acid, GS-9674, Px-102, ACC inhibitors such as GS-0976 and PPARα/δ agonists such as Elafibranor.

The combinations referred to above may conveniently be presented for use in the form of a pharmaceutical formulation and thus pharmaceutical formulations comprising a combination as defined above together with a pharmaceutically acceptable diluent or carrier represent a further aspect of the disclosure.

Such conjoint/combination treatment may be achieved by way of the simultaneous, sequential or separate dosing of the individual components of the treatment. In one embodiment, the individual compounds will be administered simultaneously in a combined pharmaceutical formulation.

Such combination therapies employ the compounds of this disclosure within the dosage range described herein and the other pharmaceutically active agent within approved dosage ranges and/or the dosage such as described in the relevant publication reference.

### EXAMPLES

### General Procedures:

Methods for preparing the compounds of this disclosure are illustrated in the following Examples. Starting materials are made according to procedures known in the art, or as illustrated herein, or are available commercially. Commercial reagents were used without further purification. Where no reaction temperature is included, the reaction was performed at ambient temperature which is typically 18-27°C.

Where compounds described in the disclosure are characterized by ¹H NMR spectroscopy, spectra were recorded on 500 MHz Bruker, 400 MHz Bruker, 250 MHz Bruker, 300 MHz JEOL or 400 MHz JEOL instruments. Where no temperature is included, the spectra were recorded at ambient temperature. Chemical shift values are expressed in parts per million (ppm). Where NMR spectra are complex due to the presence of interconverting isomers, approximate partial integrations of signals are reported, or characterisation for the major isomer only is reported. The following abbreviations are used for the multiplicity of the NMR signals: s=singlet, b=broad, t=triplet, q=quartet, m=multiplet, d=doublet.

### Analytical LCMS

Where compounds described in the disclosure are characterized by LCMS data, retention time and molecular weight are determined using the methods listed in the table below. In cases where compounds of the invention appear as slowly interconverting stereoisomers, multiple retention times are reported.

| **Method** | **Instrument** | **Column** | | **Eluents** | **Gradient** |
|---|---|---|---|---|---|
| **1** | Waters Acquity UPLC (diode array 210-350 nm) and SQD mass detector | XBridge BEH C18 (2.5 µm, 2.1 x 50 mm) | 40°C | A: 10 mM ammonium bicarbonate pH 10 | 2 - 98% B from 0.0 to 4.0 min |
| | | | | B: MeCN | |
| **2** | Waters Alliance 2695 HPLC (diode array 215-350 nm) and ZQ Mass Spectrometer | XBridge C18 (2.5 µm, 3.0 x 150 mm) | 40°C | A: 0.05% formic acid in water | 0 - 95% B from 0.0 to 6.0 min, 95% B to 11.1 min |
| | | | | B: MeCN | |
| **3** | Acquity UPLC (binary pump with PDA detector) and ZQ Mass Spectrometer | Acquity UPLC BEH C18 (1.7 µm, 2.1 x 100 mm) | 40°C | A: 0.1% formic acid in water | 5 - 95% B from 0.4 to 6.0 min |
| | | | | B: MeCN (containing 0.1% formic acid) | |
| **4** | Acquity UPLC + Waters DAD + Waters SQD2, single quadrapole UPLC-MS | Acquity UPLC HSS C₁₈ (1.8 µm, 2.1 x 100 mm) | 40°C | A: 0.1% formic acid in water | 5 - 95% B from 0.4 to 6.0 min |
| | | | | B: MeCN (containing 0.1% formic acid) | |
| **5** | Acquity UPLC + Waters DAD + Waters SQD2, single quadrapole UPLC-MS | Acquity UPLC BEH Shield RP18 (1.7 µm 2.1 x 100 mm) | 40 °C | A: 10 mM ammonium bicarbonate pH 10 | 5 - 95% B from 0.4 to 6.0 min |
| | | | | B: MeCN | |
| **6** | Waters Acquity UPLC (diode array 210-350 nm) and QDa mass detector | XBridge C18 (2.5 µm, 2.1 x 50 mm) | 40°C | A: 10 mM ammonium bicarbonate pH 10 | 2 - 98% B from 0.0 to 4.6 min |
| | | | | B: MeCN | |
| **7** | Waters Acquity UPLC (diode array 210-350 nm) and SQD mass detector | XBridge C18 (2.5 µm, 2.1 x 50 mm) | 40°C | A: water | 2 - 95% B in A (5% C throughout) from 0.0 to 5.0 min |
| | | | | B: MeCN | |
| | | | | C: 2% ammonia in water | |
| **8** | Waters Acquity UPLC (diode array 210-350 nm) and SQD mass detector | XBridge C18 (2.5 µm, 2.1 x 50 mm) | 40°C | A: 10 mM ammonium bicarbonate pH 10 | 2 - 95% B in A from 0.0 to 4.0 min, 95% B to 4.7 min |
| | | | | B: MeCN | |
| 9 | Waters Acquity Sample manager (Quaternary pump with PDA 210 - 350 nm and ELS and SQD mass detector) | BEH C18 (1.7 µm, 2.1 x 50 mm) | 40°C | A: water | 2% B with 5% C and 93% A to 95% B and 5% C from 0.0 to 4.50 min, 95% B 5% C to 5.00 min |
| | | | | B: MeCN | |
| | | | | C: 2% ammonia in water | |
| **10** | Waters Acquity UPLC H-Class system (Quaternary pump with PDA 210 - 350 nm) and QDa mass detector | CSH C18 (1.7 µm, 2.1 x 50 mm) | 40°C | A: water | 2 - 95% B in A (5% C throughout) from 0.0 to 4.0 min, 95% B 5% C to 4.60 min |
| | | | | B: MeCN | |
| | | | | C: 2% formic acid in water | |
| **11** | Waters Acquity UPLC H-Class system (Quaternary pump with PDA 210 - 350 nm) and QDa mass detector | BEH C18 (1.7 µm, 2.1 x 50 mm) | 40°C | A: water | 2 - 95% B in A (5% C throughout) from 0.0 to 4.0 min, 95% B 5% C to 4.60 min |
| | | | | B: MeCN | |
| | | | | C: 2% ammonia in water | |
| **12** | Waters Acquity UPLC H-Class system (Quaternary pump with PDA 210 - 350 nm) and QDa mass detector | CSH C18 (1.7 µm, 2.1 x 50 mm) | 40°C | A: 0.1% formic acid in water | 3 - 99% B in A from 0.0 to 1.5 min, 95% B 5% A to 1.90 min |
| | | | | B: MeCN containing (0.1% formic acid in water) | |
| **13** | Waters Acquity Sample manager (Quaternary pump with PDA 210 - 350 nm and ELS and SQD mass detector) | CSH C18 (1.7 µm, 2.1 x 50 mm) | 40°C | A: 0.1% formic acid in water | 2 - 95% B in A (5% C throughout) from 0.0 to 4.5 min, 95% B 5% C to 5.00 min |
| | | | | B: MeCN | |
| | | | | C: 0.1% formic acid in water | |
| **14** | Acquity UPLC (binary pump with PDA detector) and ZQ Mass Spectrometer | Acquity UPLC BEH C18 (1.7 µm, 2.1 x 100 mm) | 40°C | A: 0.1% ammonia in water | 5 - 95% B from 0.4 to 6.0 min |
| | | | | B: MeCN (containing 0.1% ammonia) | |
| **15** | Waters Acquity UPLC H-Class system (Quaternary pump with PDA 210 - 350 nm) and QDa mass detector | BEH C18 (1.7 µm, 2.1 x 50 mm) | 40°C | A: 0.1% ammonia in water | 3 - 95% B from 0.2 to 2.2 min |
| | | | | B: MeCN (containing 0.1% ammonia) | |
| **16** | Waters Acquity UPLC H-Class system (Quaternary pump with PDA 210 - 350 nm) and QDa mass detector | BEH C18 (1.7 µm, 2.1 x 50 mm) | 40°C | A: water | 2 - 95% B in A (5% C throughout) from 0.0 to 1.2 min, 95% B 5% C to 1.40 min |
| | | | | B: MeCN | |
| | | | | C: 2% ammonia in water | |

### Preparative HPLC

Preparative HPLC was performed using a variety of preparative systems with variable wavelength UV detection or Mass Directed AutoPrep (MDAP) systems as listed in the table below. Collection was triggered by UV, MS or a combination of the two. The UV detection was at a selected wavelength generally 210 nm, 230 nm or 280 nm. Mass spectra were recorded on a mass spectrometer using an alternate-scan positive and negative electrospray ionization.

| **Method** | **Instrument** | **Column** | | **Eluents** | **Gradient** |
|---|---|---|---|---|---|
| **1** | Agilent 1260 infinity purifications system. Agilent 6100 series single Quadrupole LC/MS | XSELECT CSH Prep C18 (5 µm OBD, 21 x 250 mm) | rt | A: 0.1% formic acid in water | 10% to 95% B over 22 min, centered around a specific focused gradient |
| | | | | B: MeCN (containing 0.1% formic acid) | |
| **2** | Agilent 1260 infinity purifications system. Agilent 6100 series single Quadrupole LC/MS | XBridge Prep C18 (5 µm OBD, 21 x 250 mm) | rt | A: 0.1% ammonia in water | 10% to 95% B over 22 min, centered around a specific focused gradient |
| | | | | B: MeCN (containing 0.1% ammonia) | |
| **3** | Waters Fractionlynx preparative HPLC system (2525 pump, 2996/2998 UV/VIS detector, 2767 liquid handler) with Waters Acquity systems with Waters SQD LCMS detection | Waters Sunfire OBD Phenomenex Luna Phenyl Hexyl (10 µm, 19 x 150 mm) | rt | A: MeOH | Gradient as specified |
| | | | | B: MeCN (containing 10mM ammonium bicarbonate) | |

### Abbreviations:

| | |
|---|---|
| CDI | 1,1'-Carbonyldiimidazole |
| DBAD | Di-*tert*-butyl azodicarboxylate |
| DCM | Dichloromethane |
| DIAD | Diisopropyl azodicarboxylate |
| DIPEA | *N,N*-Diisopropylethylamine |
| DMF | *N,N*-Dimethylformamide |
| DMSO | Dimethylsulfoxide |
| EDTA | Ethylenediaminetetraacetic acid |
| Et₂O | Diethyl ether |
| EtOAc | Ethyl acetate |
| EtOH | Ethanol |
| h | Hour(s) |
| HATU | *N*-[(Dimethylamino)-1*H*-1,2,3-triazolo-[4,5-*b*]pyridin-1-ylmethylene]-*N-*methylmethanaminium hexafluorophosphate *N*-oxide |
| HPLC | High Performance Liquid Chromatography |
| IMS | Industrial methylated spirits |
| LCMS | Liquid Chromatography Mass Spectrometry |
| MDAP | Mass Directed Auto Purification |
| MeCN | Acetonitrile |
| MeOH | Methanol |
| min | Minute(s) |
| NMR | Nuclear Magnetic Resonance |
| Pd/C | Palladium on carbon |
| rt | Room Temperature |
| TBME | *tert*-Butyl methyl ether |
| TFA | Trifluoroacetic acid |
| THF | Tetrahydrofuran |

### Intermediates

The following intermediates and their synthetic routes are described in co-pending application PCT/GB2019/053012:

| | | |
|---|---|---|
| **Intermediate 1** | 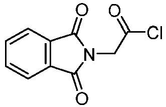 | 2-(1,3-dioxoisoindolin-2-yl)acetyl chloride |
| **Intermediate 2** | 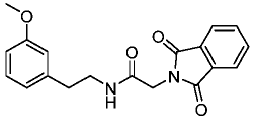 | 2-(1,3-dioxoisoindolin-2-yl)-*N*-(3-methoxyphenethyl)acetamide |
| **Intermediate 3** | 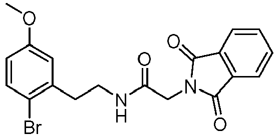 | *N*-(2-bromo-5-methoxyphenethyl)-2-(1,3-dioxoisoindolin-2-yl)acetamide |
| **Intermediate 4** | 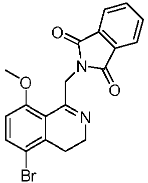 | 2-((5-bromo-8-methoxy-3,4-dihydroisoquinolin-1-yl)methyl)isoindoline-1,3-dione |
| **Intermediate 5** | 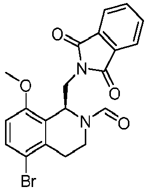 | (*S*)-5-bromo-1-((1,3-dioxoisoindolin-2-yl)methyl)-8-methoxy-3,4-dihydroisoquinoline-2(1*H*)-carbaldehyde |
| **Intermediate 6** | 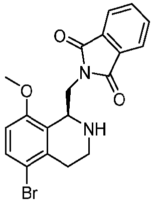 | (*S*)-2-((5-bromo-8-methoxy-1,2,3,4-tetrahydroisoquinolin-1-yl)methyl)isoindoline-1,3-dione |
| **Intermediate 7** | 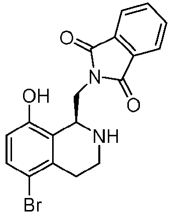 | (*S*)-2-((5-bromo-8-hydroxy-1,2,3,4-tetrahydroisoquinolin-1-yl)methyl)isoindoline-1,3-dione |
| **Intermediate 8** | 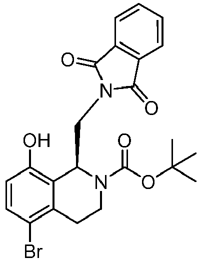 | *tert-*butyl (*S*)-5-bromo-1-((1,3-dioxoisoindolin-2-yl)methyl)-8-hydroxy-3,4-dihydroisoquinoline-2(1*H*)-carboxylate |
| **Intermediate 9** | 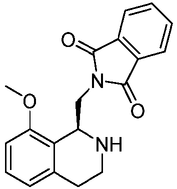 | (*S*)-2-((8-methoxy-1,2,3,4-tetrahydroisoquinolin-1-yl)methyl)isoindoline-1,3-dione |
| **Intermediate 10** | 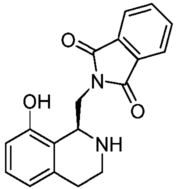 | (*S*)-2-((8-hydroxy-1,2,3,4-tetrahydroisoquinolin-1-yl)methyl)isoindoline-1,3-dione |
| **Intermediate 11** | 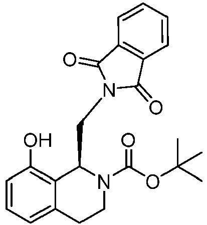 | *tert*-butyl (*S*)-1-((1,3-dioxoisoindolin-2-yl)methyl)-8-hydroxy-3,4-dihydroisoquinoline-2(1*H*)-carboxylate |
| **Intermediate 12** | 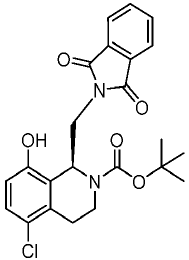 | *tert*-butyl (*S*)-5-chloro-1-((1,3-dioxoisoindolin-2-yl)methyl)-8-hydroxy-3,4-dihydroisoquinoline-2(1*H*)-carboxylate |
| **Intermediate 13** | 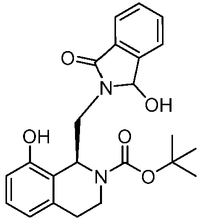 | *tert*-butyl (1*S*)-8-hydroxy-1-((1-hydroxy-3-oxoisoindolin-2-yl)methyl)-3,4-dihydroisoquinoline-2(1*H*)-carboxylate |
| **Intermediate 14** | 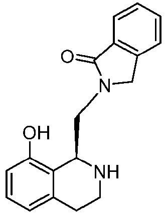 | (*S*)-2-((8-hydroxy-1,2,3,4-tetrahydroisoquinolin-1-yl)methyl)isoindolin-1-one |
| **Intermediate 15** | 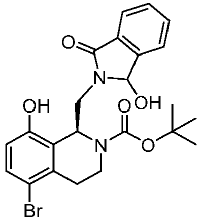 | *tert*-butyl (1*S*)-5-bromo-8-hydroxy-1-((1-hydroxy-3-oxoisoindolin-2-yl)methyl)-3,4-dihydroisoquinoline-2(1*H*)-carboxylate |
| **Intermediate 16** | 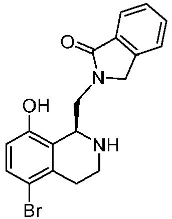 | (*S*)-2-((5-bromo-8-hydroxy-1,2,3,4-tetrahydroisoquinolin-1-yl)methyl)isoindolin-1-one |
| **Intermediate 17** | 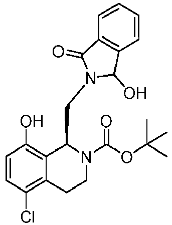 | *tert*-butyl (1*S*)-5-chloro-8-hydroxy-1-((1-hydroxy-3-oxoisoindolin-2-yl)methyl)-3,4-dihydroisoquinoline-2(1*H*)-carboxylate |
| **Intermediate 18** | 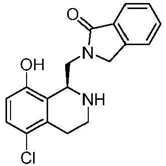 | (*S*)-2-((5-chloro-8-hydroxy-1,2,3,4-tetrahydroisoquinolin-1-yl)methyl)isoindolin-1-one |
| **Intermediate 19** | 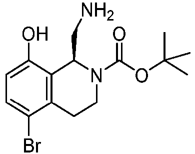 | *tert*-butyl (*S*)-1-(aminomethyl)-5-bromo-8-hydroxy-3,4-dihydroisoquinoline-2(1*H*)-carboxylate |
| **Intermediate 20** | 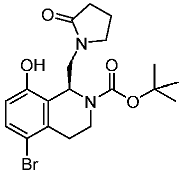 | *tert*-butyl (*S*)-5-bromo-8-hydroxy-1-((2-oxopyrrolidin-1-yl)methyl)-3,4-dihydroisoquinoline-2(1*H*)-carboxylate |
| **Intermediate 21** | 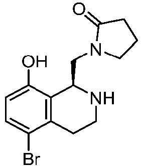 | (*S*)-1-((5-bromo-8-hydroxy-1,2,3,4-tetrahydroisoquinolin-1-yl)methyl)pyrrolidin-2-one |
| **Intermediate 22** | 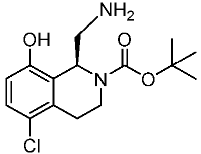 | *tert*-butyl (*S*)-1-(aminomethyl)-5-chloro-8-hydroxy-3,4-dihydroisoquinoline-2(1*H*)-carboxylate |
| **Intermediate 23** | 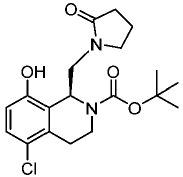 | *tert*-butyl (*S*)-5-chloro-8-hydroxy-1-((2-oxopyrrolidin-1-yl)methyl)-3,4-dihydroisoquinoline-2(1*H*)-carboxylate |
| **Intermediate 24** | 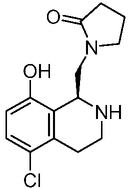 | (*S*)-1-((5-chloro-8-hydroxy-1,2,3,4-tetrahydroisoquinolin-1-yl)methyl)pyrrolidin-2-one |
| **Intermediate 25** | 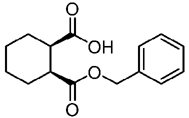 | (1*R*,2*S*)-2-((benzyloxy)carbonyl)cyclohexane-1-carboxylic acid |
| **Intermediate 26** | 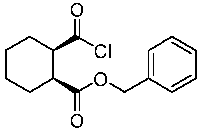 | benzyl (1*S*,2*R*)-2-(chlorocarbonyl)cyclohexane-1-carboxylate |
| **Intermediate 27** | 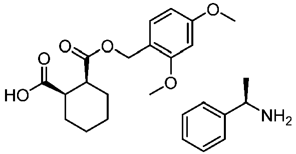 | (1*R*,2*S*)-2-[(2,4-dimethoxyphenyl) methoxycarbonyl] cyclohexane-carboxylic acid; (1*R*)-1-phenylethanamine |
| **Intermediate 28** | 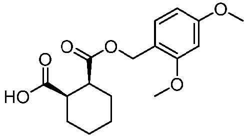 | (1*R*,2*S*)-2-(((2,4-dimethoxybenzyl)oxy) carbonyl)cyclohexane-1-carboxylic acid |
| **Intermediate 29** | 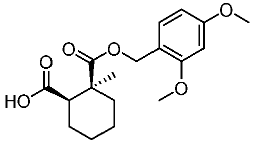 | (1*R*,2*S*)-2-(((2,4-dimethoxybenzyl)oxy)carbonyl)-2-methylcyclohexane-1-carboxylic acid |
| **Intermediate 30** | 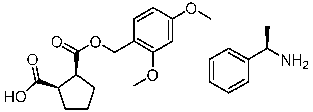 | (1*R*,2*S*)-2-[(2,4-dimethoxyphenyl) methoxycarbonyl]cyclopentanecarboxylic acid; (1*R*)-1-phenylethanamine |
| **Intermediate 31** | 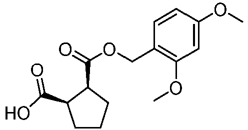 | (1*R*,2*S*)-2-(((2,4-dimethoxybenzyl)oxy)carbonyl) cyclopentane-1-carboxylic acid |
| **Intermediate 32** | 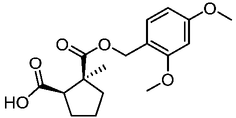 | (1*R*,2*S*)-2-(((2,4-dimethoxybenzyl)oxy)carbonyl)-2-methylcyclopentane-1-carboxylic acid |
| **Intermediate 33** | 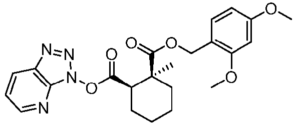 | 2-(3*H*-[1,2,3]triazolo[4,5-*b*]pyridin-3-yl) 1-(2,4-dimethoxybenzyl) (1*S*,2*R*)-1-methylcyclohexane-1,2-dicarboxylate |
| **Intermediate 34** | 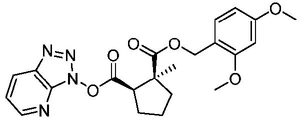 | 2-(3*H*-[1,2,3]triazolo[4,5-*b*]pyridin-3-yl) 1-(2,4-dimethoxybenzyl) (1*S*,2*R*)-1-methylcyclopentane-1,2-dicarboxylate |
| **Intermediate 35** | 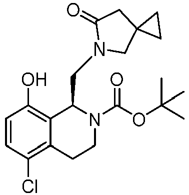 | *tert*-butyl (*S*)-5-chloro-8-hydroxy-1-((6-oxo-5-azaspiro[2.4]heptan-5-yl)methyl)-3,4-dihydroisoquinoline-2(1*H*)-carboxylate |
| **Intermediate 36** | 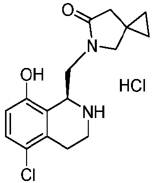 | (*S*)-5-((5-chloro-8-hydroxy-1,2,3,4-tetrahydroisoquinolin-1-yl)methyl)-5-azaspiro[2.4]heptan-6-one hydrochloride |
| **Intermediate 37** | 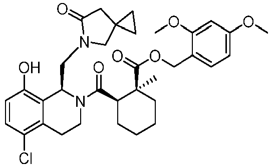 | 2,4-dimethoxybenzyl (1*S*,2*R*)-2-((*S*)-5-chloro-8-hydroxy-1-((6-oxo-5-azaspiro[2.4]heptan-5-yl)methyl)-1,2,3,4-tetrahydroisoquinoline-2-carbonyl)-1-methylcyclohexane-1-carboxylate |
| **Intermediate 38** | 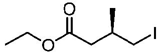 | ethyl (*R*)-4-iodo-3-methylbutanoate |
| **Intermediate 39** | 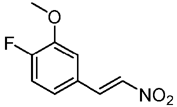 | (*E*)-1-fluoro-2-methoxy-4-(2-nitrovinyl)benzene |
| **Intermediate 40** | 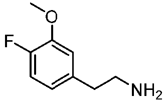 | 2-(4-fluoro-3-methoxyphenyl)ethan-1-amine |
| **Intermediate 41** | 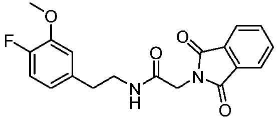 | 2-(1,3-dioxoisoindolin-2-yl)-*N*-(4-fluoro-3-methoxyphenethyl)acetamide |
| **Intermediate 42** | 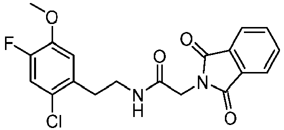 | *N*-(2-chloro-4-fluoro-5-methoxyphenethyl)-2-(1,3-dioxoisoindolin-2-yl)acetamide |
| **Intermediate 43** | 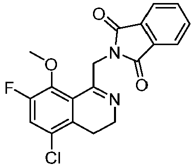 | 2-((5-chloro-7-fluoro-8-methoxy-3,4-dihydroisoquinolin-1-yl)methyl)isoindoline-1,3-dione |
| **Intermediate 44** | 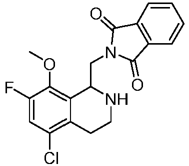 | 2-((5-chloro-7-fluoro-8-methoxy-1,2,3,4-tetrahydroisoquinolin-1-yl)methyl)isoindoline-1,3-dione |
| **Intermediate 45** | 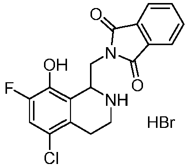 | 2-((5-chloro-7-fluoro-8-hydroxy-1,2,3,4-tetrahydroisoquinolin-1-yl)methyl)isoindoline-1,3-dione hydrobromide |
| **Intermediate 46** | 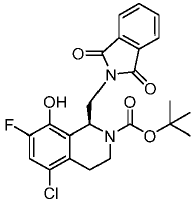 | *tert*-butyl (*S*)-5-chloro-1-((1,3-dioxoisoindolin-2-yl)methyl)-7-fluoro-8-hydroxy-3,4-dihydroisoquinoline-2(1*H*)-carboxylate |
| **Intermediate 47** | 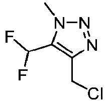 | 4-(chloromethyl)-5-(difluoromethyl)-1-methyl-1*H-*1,2,3-triazole |

### Synthesis of Example Compounds

For methodology to synthesise the required carboxylic acids, see experimental methods in PCT/GB2019/053012 (WO 2020/084300), and the representative synthesis below:

### (1S,2R)-2-((S)-1-((1,3-dioxoisoindolin-2-yl)methyl)-8-(4-(methylamino)-4-oxobutoxy)-1,2,3,4-tetrahydroisoquinoline-2-carbonyl)cyclohexane-1-carboxylic acid

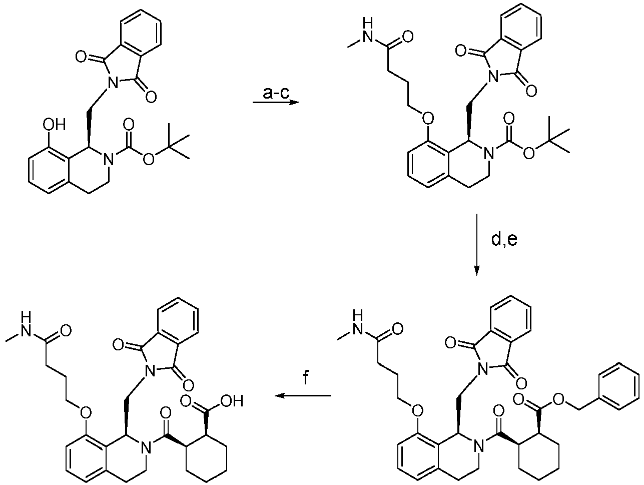

**Step a.** To a stirred solution of Intermediate 11 (1.0 g, 2.45 mmol) in anhydrous THF (15 mL) was added benzyl 4-hydroxybutanoate (570 mg, 2.94 mmol; CAS: 91970-62-6) and triphenylphosphine (963 mg, 3.67 mmol; CAS: 603-35-0) and to this was added a solution of DBAD (845 mg, 3.67 mmol) in anhydrous THF (5 mL) dropwise over 15 min. The reaction mixture was stirred at rt for 18 h and concentrated *in vacuo.* Flash column chromatography (silica, 20% EtOAc in Heptanes) gave *tert*-butyl (*S*)-8-(4-(benzyloxy)-4-oxobutoxy)-1-((1,3-dioxoisoindolin-2-yl)methyl)-3,4-dihydroisoquinoline-2(1*H*)-carboxylate (1.47 g, assumed quantitative). LCMS (Method 1 with 2 - 98% B in 0.80 min, 98% B to 1.30 min): 1.05 min, 585.5 [M+H]⁺.

**Step b.** A suspension of the above intermediate (1.47 g, 2.51 mmol) and 10% Pd/C (535 mg, 0.251 mmol) in anhydrous THF (20 mL) was stirred under an atmosphere of hydrogen (atmospheric pressure) at rt for 18 h. The reaction mixture was filtered through Celite^{®}, washed with methanol, and the filtrate concentrated *in vacuo.* Flash column chromatography (silica, 30 - 50% EtOAc in Heptanes) gave (*S*)-4-((2-(*tert-*butoxycarbonyl)-1-((1,3-dioxoisoindolin-2-yl)methyl)-1,2,3,4-tetrahydroisoquinolin-8-yl)oxy)butanoic acid (1.00 g, 80%). LCMS (Method 1): 1.62 min, 495.2 [M+H]⁺.

**Step c.** To a stirred solution of the above intermediate (200 mg, 0.40 mmol) in anhydrous THF (2 mL) at rt was added CDI (262 mg, 1.62 mmol; CAS: 530-62-1) in one portion. After 1 h, the reaction mixture was cooled to 0 °C and methylamine (2M in THF; 0.40 mL, 1.62 mmol; CAS: 74-89-5) was added. The reaction mixture was stirred for 18 h at rt, diluted with water, extracted with EtOAc and the combined organics washed with brine, dried over MgSO₄, filtered and concentrated *in vacuo.* Flash column chromatography (silica, EtOAc) gave *tert-*butyl (*S*)-1-((1,3-dioxoisoindolin-2-yl)methyl)-8-(4-(methylamino)-4-oxobutoxy)-3,4-dihydroisoquinoline-2(1*H*)-carboxylate (120 mg, 58%). LCMS (Method 2 with XBridge IS C18 2.5 µm 2.1 x 20 mm column, 0 - 95% B 0.18 to 2.00 min, 95% B to 2.60 min): 1.99 min, 508.2 [M+H]⁺.

**Step d.** To a stirred solution of the above intermediate (120 mg, 0.24 mmol) in 1,4-dioxane (1 mL) was added hydrogen chloride (3 M in dioxane, 2 mL) and the reaction mixture stirred at rt for 3 h. The suspension was concentrated *in vacuo* and triturated from TBME to give (*S*)-4-((1-((1,3-dioxoisoindolin-2-yl)methyl)-1,2,3,4-tetrahydroisoquinolin-8-yl)oxy)-*N*-methylbutanamide hydrochloride (105 mg, assumed quantitative), used without further purification. LCMS (Method 2 with XBridge IS C18 2.5 µm 2.1 x 20 mm column, 0 - 95% B 0.18 to 2.00 min, 95% B to 2.60 min): 1.68 min, 408.2 [M+H]⁺.

**Step e.** To a stirred solution of the above intermediate (120 mg, 0.27 mmol) and triethylamine (0.11 mL, 0.81 mmol) in anhydrous DCM (1.5 mL) at 0 °C was added a solution of Intermediate 26 (83 mg, 0.297 mmol) in DCM (0.5 mL) dropwise. The reaction mixture was stirred for 0.5 h at 0 °C then at rt 1 h. To this was added water, the mixture was extracted with EtOAc and the combined organics were washed with brine, dried over MgSO₄, filtered and concentrated *in vacuo.* Purification by flash column chromatography (silica, 20% EtOAc in heptanes, followed by 100% EtOAc) gave benzyl (1*S*,2*R*)-2-((*S*)-1-((1,3-dioxoisoindolin-2-yl)methyl)-8-(4-(methylamino)-4-oxobutoxy)-1,2,3,4-tetrahydroisoquinoline-2-carbonyl)cyclohexane-1-carboxylate (80 mg, 45%). LCMS (Method 1 with 2 - 98% B in 0.80 min, 98% B to 1.30 min): 0.93 min, 652.6 [M+H]⁺.

**Step f.** A suspension of the above intermediate (80 mg, 0.12 mmol) and 10% Pd/C (26 mg, 0.12 mmol) in anhydrous THF (2 mL) was stirred under an atmosphere of hydrogen (atmospheric pressure) at rt for 24 h. The reaction mixture was filtered through Celite^{®}, washed with methanol, and the filtrate concentrated *in vacuo.* Purification by flash column chromatography (0 - 10% MeOH in DCM) gave the title compound (6.1 mg, 9%). LCMS (Method 1): 1.56 min, 562.4 [M+H]⁺.

### Amide Coupling Methods

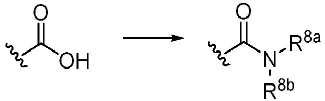

### Amide Coupling Method A: Reference Example 2: (1S,2R)-2-((S)-1-((1,3-dioxoisoindolin-2-yl)methyl)-8-(4-(methylamino)-4-oxobutoxy)-1,2,3,4-tetrahydroisoquinoline-2-carbonyl)-N-methylcyclohexane-1-carboxamide

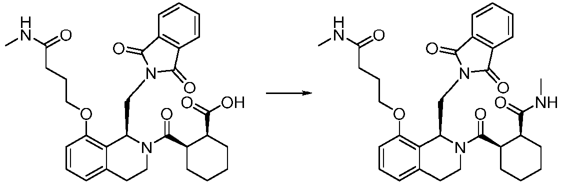

To a stirred solution of (1*S*,2*R*)-2-((*S*)-1-((1,3-dioxoisoindolin-2-yl)methyl)-8-(4-(methylamino)-4-oxobutoxy)-1,2,3,4-tetrahydroisoquinoline-2-carbonyl)cyclohexane-1-carboxylic acid (50 mg, 0.09 mmol; synthesis above) in THF (5 mL) was added 1,1'-carbonyldiimidazole (58 mg, 0.36 mmol; CAS: 530-62-1) and the reaction mixture was heated to 50 °C for 2 h. The reaction mixture was cooled to 0 °C and to this was added methylamine (2M in THF; 0.9 mL, 1.8 mmol; CAS: 74-89-5). After 45 min, the the mixture was diluted with 0.5 M aqueous HCl (8 mL), the mixture extracted with EtOAc (2 x 10 mL) and the combined organics dried (MgSO₄), filtered and concentrated *in vacuo.* The residue was purified by reverse phase column chromatography (15 - 80 % MeCN in pH10 ammonium hydrogen carbonate aqueous buffer solution, 12 g C18 cartridge) to give the title compound (16 mg, 31%). LCMS (Method 1): 2.01 min, 575.4 [M+H]⁺. ¹H NMR (400 MHz, DMSO-d₆) δ 7.89-7.77 (m, 4H), 7.71 (m, 1H), 7.23-7.13 (m, 2H), 6.86 (d, 1H), 6.78 (d, 1H), 6.01 (dd, 1H), 4.18-4.03 (m, 2H), 3.98 (m, 1H), 3.85 (m, 1H), 3.76 (dd, 1H), 3.63 (m, 1H), 3.26 (m, 1H), 2.96 (m, 1H), 2.76 (m, 1H), 2.59 (d, 3H), 2.55-2.37 (m, 2H), 2.35 (d, 3H), 2.15 (m, 2H), 2.04 (m, 1H), 1.92 (m, 1H), 1.53 (m, 1H), 1.41 (m, 1H), 1.35-1.19 (m, 2H), 0.93 (m, 1H), 0.66 (m, 1H), 0.17 (m, 1H).

### Amide Coupling Method B: Reference Example 6: N-(2-(((S)-1-((1,3-dioxoisoindolin-2-yl)-methyl)-2-((1R,2S)-2-(ethylcarbamoyl)cyclohexane-1-carbonyl)-1,2,3,4-tetrahydroisoquinolin-8-yl)oxy)ethyl)-5-methylisoxazole-3-carboxamide

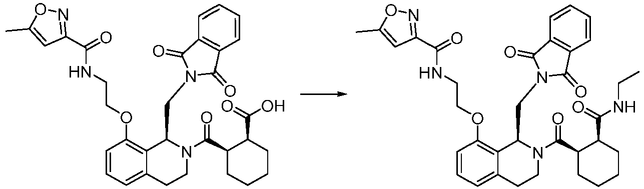

To a stirred solution of (1*S*,2*R*)-2-((*S*)-1-((1,3-dioxoisoindolin-2-yl)methyl)-8-(2-(5-methylisoxazole-3-carboxamido)ethoxy)-1,2,3,4-tetrahydroisoquinoline-2-carbonyl)cyclohexane-1-carboxylic acid (50 mg, 0.08 mmol) in THF (1 mL) was added ethylamine solution (2M in THF; 0.08 mL, 0.16 mmol), triethylamine (0.01 mL, 0.72 mmol) and HATU (37 mg, 0.096 mmol) and the reaction mixture stirred at rt for 18 h. To this was added 10% aqueous citric acid (1 mL), the mixture was extracted with EtOAc, tthe combined organics were dried (MgSO₄) and concentrated in vacuo. Purification by reverse phase column chromatography (30 - 80% MeCN in pH10 ammonium hydrogen carbonate aqueous buffer solution, 12 g spherical bead C18 cartridge) to give the title compound (23 mg, 44%). LCMS (Method 1): 2.36 min, 642.5 [M+H]⁺. ¹H NMR (300 MHz, DMSO) δ 8.76 (t, 1H), 7.91-7.76 (m, 4H), 7.24-7.12 (m, 2H), 6.89 (d, 1H), 6.80 (d, 1H), 6.54 (m, 1H), 6.03 (dd, 1H), 4.25-4.04 (m, 3H), 4.02-3.70 (m, 4H), 3.62 (m, 1H), 3.25 (m, 1H), 3.03-2.67 (m, 4H), 2.35 (s, 3H), 2.10-1.82 (m, 2H), 1.58-1.38 (m, 2H), 1.37-1.15 (m, 2H), 0.94 (m, 1H), 0.82-0.63 (m, 4H), 0.23 (m, 1H).

### Reference Example 38: (1S,2R)-2-((S)-1-(cyclopropanecarboxamidomethyl)-8-(((S)-1-(thiazole-5-carbonyl)pyrrolidin-3-yl)oxy)-1,2,3,4-tetrahydroisoquinoline-2-carbonyl)-N-methylcyclohexane-1-carboxamide

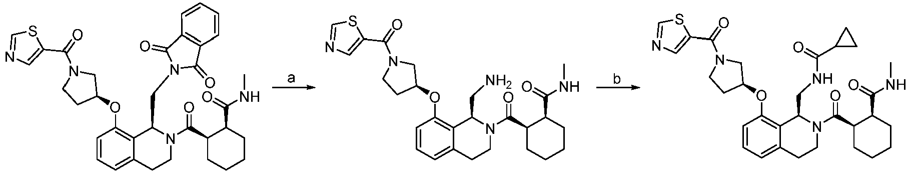

**Step a.** To a stirred solution of Example 17 (58 mg, 0.088 mmol) in IMS (1 mL) under argon was added hydrazine hydrate (6 uL, 0.18 mmol; CAS: 10217-52-4) and the reaction mixture was heated at 80 °C for 2h. The cooled reaction mixture was diluted with water, extracted with EtOAc, the combined organics concentrated *in vacuo* to give (1*S*,2*R*)-2-((*S*)-1-(aminomethyl)-8-(((*S*)-1-(thiazole-5-carbonyl)pyrrolidin-3-yl)oxy)-1,2,3,4-tetrahydroisoquinoline-2-carbonyl)-*N*-methylcyclohexane-1-carboxamide (46 mg, assumed quantitative). The crude product was used without further purification.

Step b. To a stirred solution of the above intermediate (46 mg, 0.088 mmol) and triethylamine (25 uL, 0.18 mmol) in DCM (1 mL) under argon at 0 °C was added a solution of cyclopropanecarbonyl chloride (9 uL, 0.098 mmol; CAS: 4023-34-1) in DCM (0.5 mL) dropwise. The reaction mixture was allowed to warm to rt and stirred at rt for 18 h. The mixture was diluted with water, extracted with DCM, the combined organics passed through a phase separator and concentrated *in vacuo.* Purification by MDAP (Method 1) provided the title compound (6 mg, 11%). ¹H NMR (400 MHz, DMSO-d₆; rotamers observed, all reported) δ 9.30-9.14 (m, 1H), 8.59-8.03 (m, 2H), 7.48 (m, 0.4H), 7.29-7.11 (m, 1.6H), 7.10-6.85 (m, 1H), 6.84-6.71 (m, 1H), 5.80-5.65 (m, 0.6H), 5.39-5.21 (m, 0.5H), 5.20-5.08 (m, 0.5H), 5.06-4.93 (m, 0.4H), 4.54-2.97 (m, 9H), 2.97-2.04 (m, 9H), 2.00-0.77 (m, 8H), 0.76-0.47 (m, 4H).

### Reference Example 39: (1S,2R)-2-((S)-8-((1H-pyrazol-5-yl)methoxy)-1-((1,3-dioxoisoindolin-2-yl)methyl)-1,2,3,4-tetrahydroisoquinoline-2-carbonyl)-N-methylcyclohexane-1-carboxamide

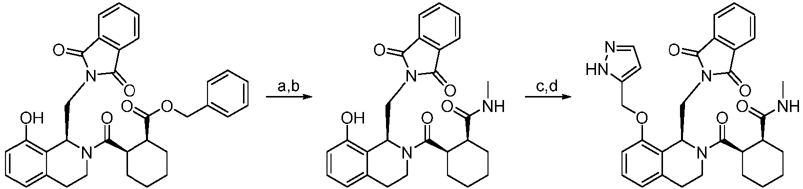

**Step a.** To a solution of benzyl (1*S*,2*R*)-2-((*S*)-1-((1,3-dioxoisoindolin-2-yl)methyl)-8-hydroxy-1,2,3,4-tetrahydroisoquinoline-2-carbonyl)cyclohexane-1-carboxylate (527 mg, 0.95 mmol; PCT/GB2019/053012 Example 2, step a) in THF (15 mL) was added Pd/C (10%; 200 mg). The mixture was stirred at rt under an atmosphere of hydrogen for 18 h at atmospheric pressure. The mixture was then filtered through a pad of Celite^{®} and the filtrate was concentrated *in vacuo* to give (1*S*,2*R*)-2-((*S*)-1-((1,3-dioxoisoindolin-2-yl)methyl)-8-hydroxy-1,2,3,4-tetrahydroisoquinoline-2-carbonyl)cyclohexane-1-carboxylic acid (392 mg, 89%).

**Step b.** (1*S*,2*R*)-2-((*S*)-1-((1,3-dioxoisoindolin-2-yl)methyl)-8-hydroxy-1,2,3,4-tetrahydroisoquinoline-2-carbonyl)-*N*-methylcyclohexane-1-carboxamide (541 mg, assumed quantitative) was prepared from the above intermediate (392 mg, 0.85 mmol) using a procedure similar to that described for Example 92, step g. LCMS (Method 12): 1.27 min, 476.2 [M+H]⁺.

**Step c.** *tert*-Butyl 5-((((*S*)-1-((1,3-dioxoisoindolin-2-yl)methyl)-2-((1*R*,2*S*)-2-(methylcarbamoyl)cyclohexane-1-carbonyl)-1,2,3,4-tetrahydroisoquinolin-8-yl)oxy)methyl)-1*H*-pyrazole-1-carboxylate (110 mg, 54%) was prepared from the above intermediate (156 mg, 0.32 mmol) and *tert*-butyl 5-(bromomethyl)-1*H*-pyrazole-1-carboxylate (107 mg, 0.41 mmol; CAS: 186551-69-9) using a procedure similar to that described for Example 67 step a. LCMS (Method 12): 1.61 min, 678.3 [M+H]⁺.

**Step d.** To a stirred solution of the above intermediate (110 mg, 0.17 mmol) in DCM (5 mL) was added TFA (1 mL) and the reaction mixture was stirred at rt for 1 h. The mixture was concentrated in vacuo and azeotroped with toluene. Purification by MDAP (Method 1) provided the title compound (39 mg, 41%). LCMS (Method 3): 4.00 min, 556.2 [M+H]⁺. ¹H NMR (400 MHz, DMSO-d₆) δ 12.78 (bs, 1H), 7.89-7.66 (m, 5H), 7.19 (t, 1H), 7.14 (m, 1H), 7.05 (d, 1H), 6.80 (d, 1H), 6.65 (bm, 1H), 6.05 (dd, 1H), 5.25 (d, 1H), 5.19 (d, 1H), 4.17 (dd, 1H), 3.81 (m, 1H), 3.73 (dd, 1H), 3.63 (m, 1H), 3.24 (m, 1H), 2.97 (m, 1H), 2.78 (m, 1H), 2.35 (d, 3H), 2.03 (m, 1H), 1.93 (m, 1H), 1.52 (m, 1H), 1.42-1.18 (m, 3H), 0.92 (m, 1H), 0.61 (m, 1H), 0.18 (m, 1H).

### Reference Example 44: (1S,2R)-2-((S)-1-(acetamidomethyl)-8-(((S)-1-(thiazole-5-carbonyl)pyrrolidin-3-yl)oxy)-1,2,3,4-tetrahydroisoquinoline-2-carbonyl)-N-methylcyclohexane-1-carboxamide

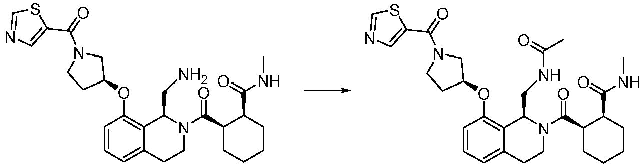

To a stirred solution of (1*S*,2*R*)-2-((*S*)-1-(aminomethyl)-8-(((*S*)-1-(thiazole-5-carbonyl)pyrrolidin-3-yl)oxy)-1,2,3,4-tetrahydroisoquinoline-2-carbonyl)-*N-*methylcyclohexane-1-carboxamide (50 mg, 0.095 mmol; Example 38 step a) and triethylamine (15 uL, 0.10 mmol) in DCM (1 mL) at 0 °C under argon was added acetyl chloride (8 uL, 0.10 mmol) and the reaction mixture allowed to warm to rt and stirred for 3 h. To this was added water, the mixture was extracted with DCM and the combined organics concentrated *in vacuo.* Purification by MDAP (Method 2) gave the title compound (12 mg, 22%. LCMS (Method 3): 3.05 min, 590.2 [M+Na]⁺. ¹H NMR (400 MHz, DMSO-d₆; rotamers observed, all reported) δ 9.26 (s, 0.3H), 9.25 (s, 0.3H), 9.23 (s, 0.2H), 9.18 (s, 0.2H), 8.45-8.37 (m, 0.8H), 8.27 (s, 0.2H), 8.05-7.97 (m, 0.5H), 7.95 (m, 0.3H), 7.87 (m, 0.2H), 7.41 (m, 0.5H), 7.27-7.10 (m, 1.5H), 7.00-6.84 (m, 1H), 6.81-6.69 (m, 1H), 5.77-5.61 (m, 0.5H), 5.30 (m, 0.2H), 5.25 (m, 0.3H), 5.19-5.09 (m, 0.5H), 5.08-4.94 (m, 0.5H), 4.43-2.96 (m, 9H), 2.95-1.96 (m, 9H), 1.95-0.73 (m, 10H).

### Reference Example 45: (1S,2R)-N-methyl-2-((S)-1-((2-oxooxazolidin-3-yl)methyl)-8-(((S)-1-(thiazole-5-carbonyl)pyrrolidin-3-yl)oxy)-1,2,3,4-tetrahydroisoquinoline-2-carbonyl)cyclohexane-1-carboxamide

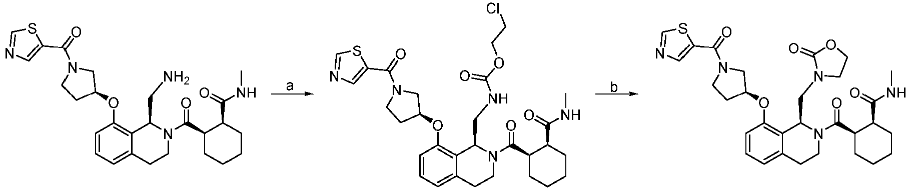

**Step a.** 2-Chloroethyl (((*S*)-2-((1*R*,2*S*)-2-(methylcarbamoyl)cyclohexane-1-carbonyl)-8-(((*S*)-1-(thiazole-5-carbonyl)pyrrolidin-3-yl)oxy)-1,2,3,4-tetrahydroisoquinolin-1-yl)methyl)carbamate (68 mg, assumed quantitative) was prepared from (1*S*,2*R*)-2-((*S*)-1-(aminomethyl)-8-(((*S*)-1-(thiazole-5-carbonyl)pyrrolidin-3-yl)oxy)-1,2,3,4-tetrahydroisoquinoline-2-carbonyl)-*N*-methylcyclohexane-1-carboxamide (50 mg, 0.095 mmol; Example 38 step a) and 2-chloroethyl chloroformate (11 uL, 0.10 mmol; CAS: 627-11-2) using a procedure similar to that described for Example 44.

**Step b.** To a mixture of the above intermediate (68 mg, 0.1 mmol) and sodium hydride (60% dispersion in mineral oil; 5 mg, 0.1 mmol) under argon at 0 °C was added anhydrous DMF (1 mL) and the reaction mixture allowed to warm to rt and stirred for 48 h. To the mixture was added saturated ammonium chloride solution, the mixture was extracted with DCM and the combined organics passed through a phase separator and concentrated *in vacuo.* Purification by MDAP (Method 2) gave the title compound (14 mg, 24%). LCMS (Method 3): 3.15 min, 618.2 [M+Na]⁺. ¹H NMR (400 MHz, DMSO-d₆; rotamers observed, both reported) δ 9.26 (s, 0.5H), 9.24 (s, 0.5H), 8.47 (s, 0.5H), 8.40 (s, 0.5H), 7.31-7.14 (m, 2H), 6.99-6.88 (m, 1H), 6.84-6.72 (m, 1H), 5.88-5.73 (m, 1H), 5.35-5.02 (m, 1H), 4.46-3.40 (m, 10H), 3.39-3.24 (m, 2H), 3.15 (m, 1H), 2.92 (m, 1H), 2.73 (m, 1H), 2.59-2.08 (m, 7H), 1.85-1.59 (m, 3H), 1.55-1.06 (m, 4H).

### Reference Example 46: 4-(((S)-1-((1,3-dioxoisoindolin-2-yl)methyl)-2-((1R,2S)-2-(methylcarbamoyl)cyclohexane-1-carbonyl)-1,2,3,4-tetrahydroisoquinolin-8-yl)oxy)butanoic acid

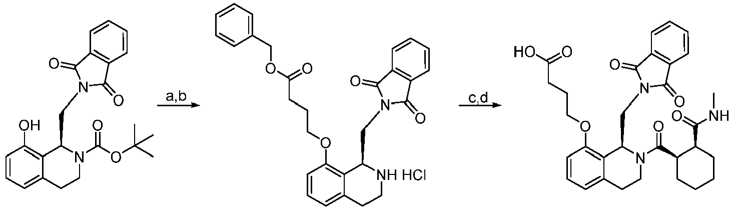

**Step a.** To a stirred solution of triphenyl phosphine (6.09 g, 23.2 mmol) in THF (50 mL) at 0 °C was added DIAD (4.5 mL, 23.2 mmol) dropwise and the reaction mixture stirred for 0.5 h. To this was added a solution of benzyl 4-hydroxybutanoate (1.35 g, 6.96 mmol; CAS: 91970-62-6) in THF (10 mL) and stirred at 0 °C for 30 min, followed by addition of Intermediate 11 (1.9 g, 4.64 mmol) in THF (20 mL). The reaction mixture was allowed to warm to rt and stirred for 16 h. The reaction mixture was diluted with water, extracted with EtOAc and the combined organics dried over Na₂SO₄, filtered and concentrated *in vacuo.* Purification by flash column chromatography (silica gel, 0 - 50% EtOAc in n-hexane) gave *tert*-butyl (*S*)-8-(4-(benzyloxy)-4-oxobutoxy)-1-((1,3-dioxoisoindolin-2-yl)methyl)-3,4-dihydroisoquinoline-2(1*H*)-carboxylate (520 mg, 23%).

**Step b.** Benzyl (*S*)-4-((1-((1,3-dioxoisoindolin-2-yl)methyl)-1,2,3,4-tetrahydro-isoquinolin-8-yl)oxy)butanoate hydrochloride (1.07 g, assumed quantitative) was prepared from the above intermediate (1.14 g, 1.95 mmol) using a procedure similar to that described for Example 67, step b. LCMS (Method 12): 1.11 min, 485 [M+H]⁺.

**Step c.** To a stirred solution of (1*R*,2*S*)-2-(methylcarbamoyl)-cyclohexanecarboxylic acid (43 mg, 0.23 mmol; CAS: 1821740-00-4) in DMF (0.5 mL) at rt under argon was added triethylamine (80 uL, 0.58 mmol) and HATU (88 mg, 0.23 mmol) and the reaction mixture stirred for 10 mins. To this was added a solution of the above intermediate (104 mg, 0.19 mmol) in DMF (1 mL) dropwise and the reaction mixture stirred for 23 h. The mixture was diluted with saturated sodium hydrogen carbonate solution, extracted with EtOAc and the combined organics washed with saturated ammonium chloride, dried over Na₂SO₄ and concentrated *in vacuo.* Purification by flash column chromatography (12g Puriflash silica cartridge, 0 - 5% MeOH in DCM) to provide benzyl 4-(((*S*)-1-((1,3-dioxoisoindolin-2-yl)methyl)-2-((1*R*,2*S*)-2-(methylcarbamoyl)cyclohexane-1-carbonyl)-1,2,3,4-tetrahydroisoquinolin-8-yl)oxy)butanoate (120 mg, 80%). LCMS (Method 12): 1.63 min, 652 [M+H]⁺.

**Step d.** A solution of the above intermediate (118 mg, 0.18 mmol) in MeOH (0.1 M; 1.8 mL) was passed through an H-Cube^{®} with a 10% Pd/C cartridge at 1 mL/min at 50 °C with hydrogen at 50 bar pressure. The solution was passed through a second time and then concentrated *in vacuo.* Purification by MDAP (Method 1) gave the title compound (23 mg, 23%). LCMS (Method 3): 4.05 min, 584.2 [M+H]⁺. ¹H NMR (400 MHz, DMSO-d₆) δ 12.11 (bs, 1H), 7.92-7.75 (m, 4H), 7.26-7.06 (m, 2H), 6.86 (d, 1H), 6.79 (d, 1H), 6.00 (dd, 1H), 4.19-4.06 (m, 2H), 4.01 (m, 1H), 3.85 (m, 1H), 3.76 (m, 1H), 3.63 (m, 1H), 3.26 (m, 1H), 2.97 (m, 1H), 2.82-2.46 (m, 3H), 2.36 (d, 3H), 2.23-2.10 (m, 2H), 2.04 (m, 1H), 1.93 (m, 1H), 1.53 (m, 1H), 1.41 (m, 1H), 1.35-1.19 (m, 2H), 0.93 (m, 1H), 0.66 (m, 1H), 0.17 (m, 1H).

### Reference Example 48: (R)-1-((S)-1-((1,3-dioxoisoindolin-2-yl)methyl)-8-(((S)-1-(thiazole-5-carbonyl)pyrrolidin-3-yl)oxy)-1,2,3,4-tetrahydroisoquinoline-2-carbonyl)-N-methylpiperidine-2-carboxamide

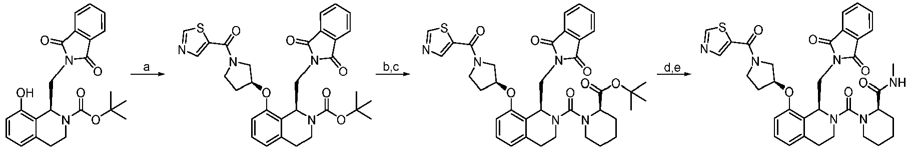

**Step a.** To a stirred solution of Intermediate 11 (5.0 g, 12.2 mmol) and (R)-(3-hydroxypyrrolidin-1-yl)(thiazol-5-yl)methanone (3.16 g, 15.9 mmol, CAS: 1690066-04-6) in anhydrous THF (100 mL) under argon was added tributylphosphine (3.98 mL, 15.9 mmol) followed by a solution of DBAD (3.66 g, 15.9 mmol; CAS: 870-50-8) in THF (50 mL) over 10 mins. After 2 h of stirring additional protions of tributylphosphine (3.98 mL, 15.9 mmol) and DBAD (3.66 g, 15.9 mmol) in THF (50 mL) were added and the reaction mixture stirred at rt for 18 h. Additional (*R*)-(3-hydroxypyrrolidin-1-yl)(thiazol-5-yl)methanone (3.16 g, 15.9 mmol, CAS: 1690066-04-6), tributylphosphine (3.98 mL, 15.9 mmol) and DBAD (3.66 g, 15.9 mmol) in THF (50 mL) were added sequentially and the reaction mixture stirred for 3 h. To the reaction mixture was added water and the mixture was concentrated *in vacuo.* Purification by flash column chromatography on the Interchim 4125 (330 g silica column Puriflash HP, 0 - 10% MeOH in EtOAc) followed by flash column chromatography on the Interchim 4100 (300 g silica Biotage SNAP, 0 - 10% MeOH in EtOAc) gave *tert*-butyl (*S*)-1-((1,3-dioxoisoindolin-2-yl)methyl)-8-(((*S*)-1-(thiazole-5-carbonyl)pyrrolidin-3-yl)oxy)-3,4-dihydroisoquinoline-2(1*H*)-carboxylate (6.48 g, 90%). LCMS (Method 3): 1.47 min, 611 [M+Na]⁺.

**Step b.** 2-(((*S*)-8-(((*S*)-1-(thiazole-5-carbonyl)pyrrolidin-3-yl)oxy)-1,2,3,4-tetrahydroisoquinolin-1-yl)methyl)isoindoline-1,3-dione hydrochloride (498 mg, assumed quantitative) was prepared from the above intermediate (600 mg, 1.02 mmol) using a procedure similar to that described for Example 67, step b.

**Step c.** To a stirred solution of the above intermediate (150 mg, 0.29 mmol) in DCM (1.5 mL) under argon was added triethylamine (120 uL, 0.86 mmol) and a solution of triphosgene (34 mg, 0.11 mmol) in DCM (1 mL). The reaction mixture was stirred at rt for 20 min, and to this was added a solution of *tert-*butyl (*R*)-piperidine-2-carboxylate hydrochloride (66mg, 0.30 mmol; CAS: 140646-13-5) in DCM (1.5 mL) and triethylamine (200 uL, 1.43 mmol). The reaction mixture was stirred at rt for 2 h, then heated to 40 °C for 5 days. The mixture was diluted with saturated sodium hydrogen carbonate, extracted with DCM and the combined organics washed with brine, dried over Na2SO4 and concentrated *in vacuo.* Purification by flash column chromatography (12g Puriflash silica cartridge, 0 - 10% MeOH in DCM) gave *tert*-Butyl (*R*)-1-((*S*)-1-((1,3-dioxoisoindolin-2-yl)methyl)-8-(((*S*)-1-(thiazole-5-carbonyl)pyrrolidin-3-yl)oxy)-1,2,3,4-tetrahydroisoquinoline-2-carbonyl)piperidine-2-carboxylate (128 mg, 64%). LCMS (Method 12): 1.61 min, 700 [M+H]⁺.

**Step d.** To a stirred solution of the above intermediate (127 mg, 0.18 mmol) in DCM (3 mL) was added TFA (1.5 mL) and the reaction mixture stirred at rt for 2 h 40 mins. The mixture was concentrated in vacuo and azeotroped with toluene to give (*R*)-1-((*S*)-1-((1,3-dioxoisoindolin-2-yl)methyl)-8-(((*S*)-1-(thiazole-5-carbonyl)pyrrolidin-3-yl)oxy)-1,2,3,4-tetrahydroisoquinoline-2-carbonyl)piperidine-2-carboxylic acid (117 mg, assumed quantitative), used without further purification.

**Step e.** The title compound (50 mg, 42%) was prepared from the above intermediate (117 mg, 0.18 mmol) using a procedure similar to that described for Example 92 step g. LCMS (Method 3): 3.92 min, 657.3 [M+H]⁺. ¹H NMR (400 MHz, DMSO-d₆; rotamers observed, both reported) δ 9.24 (s, 1H), 8.42 (s, 0.5H), 8.39 (s, 0.5H), 7.90-7.78 (m, 4H), 7.41 (m, 1H), 7.29-7.16 (m, 1H), 6.97 (d, 0.5H), 6.93 (d, 0.5H), 6.81 (d, 0.5H), 6.79 (d, 0.5H), 5.31-5.17 (m, 2H), 4.37-4.23 (m, 1H), 4.10 (m, 0.5H), 4.02-3.72 (m, 4.5H), 3.65-3.47 (m, 2H), 3.45-3.23 (m, 1H), 2.98 (m, 1H), 2.92-2.63 (m, 3H), 2.58-2.22 (m, 5H), 1.79-1.62 (m, 1H), 1.41-0.79 (m, 5H).

### Reference Example 67: (S)-2-((S)-5-chloro-8-((5-(difluoromethyl)-1-methyl-1H-1,2,3-triazol-4-yl)methoxy)-1-((2-oxopyrrolidin-1-yl)methyl)-1,2,3,4-tetrahydroisoquinoline-2-carbonyl)-N-methylpyrrolidine-1-carboxamide

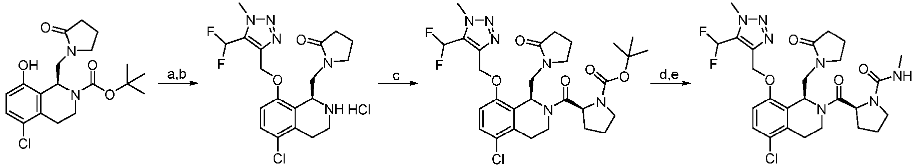

Step a. To a stirred solution of Intermediate 23 (2.6 g, 6.83 mmol) in DMF (59 mL) was added 4-(chloromethyl)-5-(difluoromethyl)-1-methyl-triazole (1.29 g, 7.09 mmol, CAS: 2138555-23-2) and caesium carbonate (8.01 g, 24.6 mmol) and the reaction mixture stirred under argon at rt for 19 h. The reaction was filtered and the solid washed with EtOAc (20 ml) and the filtrate concentrated *in vacuo.* The residue was partitioned between EtOAc and water, the organics washed with brine, dried (MgSO₄) and concentrated *in vacuo.* The crude product was purified by flash column chromatography on the Teledyne ISCO CombiFlash^{®} Rf+ (80 g silica column Puriflash HC, 0 - 10% MeOH in DCM) to provide *tert-*butyl (*S*)-5-chloro-8-((5-(difluoromethyl)-1-methyl-1*H*-1,2,3-triazol-4-yl)methoxy)-1-((2-oxopyrrolidin-1-yl)methyl)-3,4-dihydroisoquinoline-2(1*H*)-carboxylate (0.63 g). The caesium carbonate residue was partitioned between DCM/H₂O, and the aqueous extracted with DCM, the combined organics dried over Mg₂SO₄, filtered and concentrated *in vacuo* to give another crop of the product (2.35 g). The batches were combined to give *tert-*butyl (*S*)-5-chloro-8-((5-(difluoromethyl)-1-methyl-1*H*-1,2,3-triazol-4-yl)methoxy)-1-((2-oxopyrrolidin-1-yl)methyl)-3,4-dihydroiso-quinoline-2(1H)-carboxylate (2.98 g, 83%). LCMS (Method 12): 1.42 min, 548.3 [M+Na]⁺.

**Step b.** To the above intermediate (1.11 g, 2.11 mmol) was added hydrochloric acid (4 M dioxane; 10.55 mL, 42.2 mmol) and the reaction mixture was stirred at rt for 20 min. MeOH was added to the reaction mixture and the mixture concentrated *in vacuo* to give (*S*)-1-((5-chloro-8-((5-(difluoromethyl)-1-methyl-1*H*-1,2,3-triazol-4-yl)methoxy)-1,2,3,4-tetrahydroisoquinolin-1-yl)methyl)pyrrolidin-2-one (0.99 g, assumed quantitative), used without further purification. LCMS (Method 12): 0.77 min, 426.0 [M+H]⁺.

**Step c.** To a stirred solution of *N*-(i-butoxycarbonyl)-L-proline (78 mg, 0.36 mmol; CAS: 15761-39-4) in anhydrous DMF (1 mL) under argon was added DIPEA (0.17 mL, 0.98 mmol) and HATU (137 mg, 0.36 mmol; CAS: 148893-10-1). The reaction mixture was stirred at rt for 10 min, then to this was added the above intermediate (151 mg, 0.33 mmol) in anhydrous DMF (2 mL) dropwise. The reaction mixture was stirred at rt for 21 h, diluted with saturated aqueous NaHCO₃ solution, extracted with EtOAc and the combined organics were washed with brine (30 mL), dried over MgSO₄, filtered, and concentrated *in vacuo.* The crude product was purified by flash column chromatography on the Teledyne ISCO CombiFlash^{®} Rf200 (12 g silica column Puriflash HC, 0 - 10% MeOH in DCM) to provide *tert-*butyl (*S*)-2-((*S*)-5-chloro-8-((5-(difluoromethyl)-1-methyl-1*H*-1,2,3-triazol-4-yl)methoxy)-1-((2-oxopyrrolidin-1-yl)methyl)-1,2,3,4-tetrahydroisoquinoline-2-carbonyl)-pyrrolidine-1-carboxylate (159 mg, 78%). LCMS (Method 12): 1.30 min, 623.3 [M+H]⁺.

**Step d.** To a stirred solution of the above intermediate (158 mg, 0.25 mmol) in anhydrous 1,4-dioxane (4 mL) was added hydrogen chloride (4 M in dioxane; 4.0 mL, 16 mmol). The reaction mixture was stirred at rt for 2.5 h then concentrated *in vacuo* to provide 1-(((*S*)-2-(*L*-prolyl)-5-chloro-8-((5-(difluoromethyl)-1-methyl-1*H*-1,2,3-triazol-4-yl)methoxy)-1,2,3,4-tetrahydroisoquinolin-1-yl)methyl)pyrrolidin-2-one hydrochloride (154 mg, assumed quantitative). LCMS (Method 12): 0.84 min, 523.3 [M+H]⁺.
Step e. To a stirred mixture of the above intermediate (152 mg, 0.27 mmol) in anhydrous DCM (5 mL) was added *N*-methyl-1-imidazolecarboxamide (46 mg, 0.37 mmol; CAS: 72002-25-6), then triethylamine (0.1 mL, 0.73 mmol) and the resulting solution was stirred at rt under argon for 17 h. Additional N-methyl-1-imidazolecarboxamide (15 mg, 0.12 mmol) was added and the solution was stirred for a further 7 h. The solution was diluted with saturated NaHCO₃ solution, extracted with DCM and the combined organic extracts washed with brine, dried (MgSO₄), filtered, and concentrated *in vacuo.* The residue was purified by MDAP (Method 3) to provide the title compound (115 mg, 73%). LCMS (Method 5): 3.03 min, 580.4 [M+H]⁺. ¹H NMR (400 MHz, DMSO-d₆) δ 7.58 (t, 1H), 7.41 (d, 1H), 7.18 (d, 1H), 5.99 (m, 1H), 5.67 (dd, 1H), 5.35-5.25 (m, 2H), 4.70 (m, 1H), 4.17 (s, 3H), 4.02 (dd, 1H), 3.87 (dd, 1H), 3.57 (m, 1H), 3.40 (m, 1H), 3.36-3.27 (m, 1H), 3.20 (m, 1H), 2.94 (dd, 1H), 2.87-2.69 (m, 3H), 2.46 (d, 3H), 2.17 (m, 1H), 2.05-1.60 (m, 7H).

### Reference Example 84: 1-(((S)-5-chloro-2-((S)-1-(2,2-difluoroacetyl)piperidine-2-carbonyl)-8-((5-(difluoromethyl)-1-methyl-1H-1,2,3-triazol-4-yl)methoxy)-1,2,3,4-tetrahydroisoquinolin-1-yl)methyl)pyrrolidin-2-one

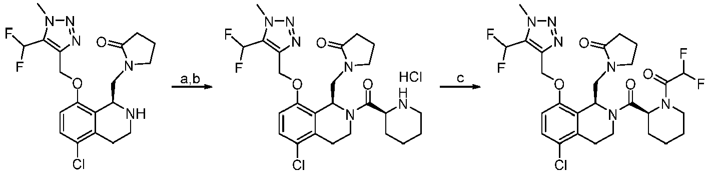

**Steps a & b.** These steps were carried out with (*S*)-(-)-1-(*tert*-butoxycarbonyl)-2-piperidinecarboxylic acid (162 mg, 0.70 mmol; CAS: 26250-84-0) with methods analogous to Example 67 steps c and d.

**Step c.** To a stirred solution of 1-(((*S*)-5-chloro-8-((5-(difluoromethyl)-1-methyl-1*H*-1,2,3-triazol-4-yl)methoxy)-2-((*S*)-piperidine-2-carbonyl)-1,2,3,4-tetrahydroisoquinolin-1-yl)methyl)pyrrolidin-2-one (126 mg, 0.22 mmol) in DCM (1.5 mL) under nitrogen was added *N*-(3-dimethylaminopropyl)-*N*'-ethylcarbodiimide hydrochloride (0.07 mL, 0.26 mmol; CAS: 25952-53-8), 1-hydroxybenzotriazole hydrate (40 mg, 0.26 mmol; CAS: 123333-53-9) and DIPEA (0.15 mL, 0.88 mmol). The reaction mixture was stirred at rt for 10 min and to this was added 2,2-difluoroacetic acid (0.02 mL, 0.26 mmol; CAS: 381-73-7). The reaction mixture was stirred at rt for 3 days, diluted with DCM and directly purified by chromatography on silica (Puriflash 12g, 0 - 50% EtOAc in isohexane) followed by chromatography on silica (Puriflash 12g, 0 - 10% MeOH in DCM) and MDAP (Method 3) to give the title compound (7 mg, 5%). LCMS (Method 3) 4.24 min, 615.2 [M+H]⁺. ¹H NMR (400 MHz, DMSO-d₆) δ 7.57 (t, 1H), 7.40 (d, 1H), 7.17 (d, 1H), 6.72 (t, 1H), 5.71 (m, 1H), 5.33-5.25 (m, 2H), 5.22 (m, 1H), 4.17 (s, 3H), 4.00 (m, 1H), 3.87 (dd, 1H), 3.75 (m, 1H), 3.70-3.20 (m, 2H), 2.99 (m, 1H), 2.85 (m, 2H), 2.72 (m, 1H), 2.19 (m, 1H), 2.02 (m, 1H), 1.93-1.53 (m, 7H), 1.50-1.13 (m, 2H).

### Reference Example 88: (S)-6-((S)-5-chloro-8-((5-(difluoromethyl)-1-methyl-1H-1,2,3-triazol-4-yl)methoxy)-1-((2-oxopyrrolidin-1-yl)methyl)-1,2,3,4-tetrahydroisoquinoline-2-carbonyl)-N-methyl-5-azaspiro[2.4]heptane-5-carboxamide

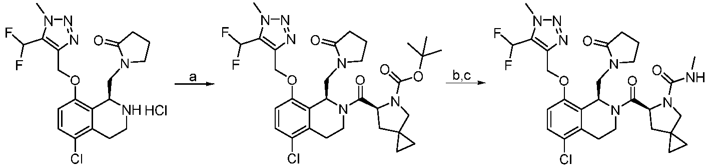

**Steps a, b.** These steps were carried out with (*S*)-5-(*tert*-butoxycarbonyl)-5-azaspiro[2.4]heptane-6-carboxylic acid (86 mg, 0.36 mmol; CAS: 1129634-44-1) with methods analogous to Example 67 steps c and d.

**Step c.** To a solution of 1-(((*S*)-5-chloro-8-((5-(difluoromethyl)-1-methyl-1*H*-1,2,3-triazol-4-yl)methoxy)-2-((*S*)-5-azaspiro[2.4]heptane-6-carbonyl)-1,2,3,4-tetrahydro-isoquinolin-1-yl)methyl)pyrrolidin-2-onehydrochloride (75 mg, 0.12 mmol) in DCM (1.9 mL) cooled to 2 °C was added triethylamine (0.04 mL, 0.26 mmol) followed by *N-*methylcarbamoyl chloride (13.14 mg, 0.140 mmol; CAS: 6452-47-7). The reaction mixture was warmed to ambient temperature and stirred for 1 h and the mixture was combined with another batch (0.031 mmol). To the combined mixture was added water, the mixture extracted with DCM and the combined organics concentrated *in vacuo.* The crude product was purified by automated reverse phase column chromatography on the Biotage Isolera One^{™} (200-400 nm diode array detector, 12 g C18 column, 5 - 40% MeCN in pH10 0.1 M NH₄HCO₃ buffer solution) to provide the title compound (50 mg, 54%). LCMS (Method 6): 1.63 min, 606.3 [M+H]⁺. ¹H NMR (400 MHz, DMSO-d₆) δ 7.58 (t, 1H), 7.41 (d, 1H), 7.18 (d, 1H), 5.95 (m, 1H), 5.68 (m, 1H), 5.38-5.21 (m, 2H), 4.79 (dd, 1H), 4.17 (s, 3H), 3.98 (m, 1H), 3.86 (dd, 1H), 3.53 (m, 1H), 3.45-3.21 (m, 2H), 3.18 (d, 1H), 2.94 (dd, 1H), 2.86-2.72 (m, 3H), 2.45 (d, 3H), 2.22-2.09 (m, 2H), 1.98 (m, 1H), 1.89-1.66 (m, 2H), 1.52 (dd, 1H), 0.60-0.34 (m, 4H).

### Example 98: (1R,2S)-2-((R)-5-chloro-8-((5-(difluoromethyl)-1-methyl-1H-1,2,3-triazol-4-yl)methoxy)-1-((6-oxo-5-azaspiro[2.4]heptan-5-yl)methyl)-1,2,3,4-tetrahydroisoquinoline-2-carbonyl)-N,1-dimethylcyclohexane-1-carboxamide

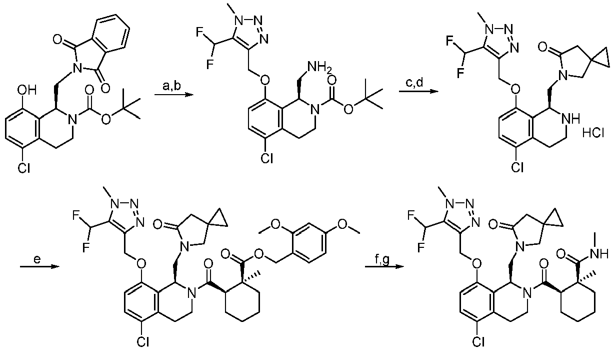

**Step a.** To a stirred solution of Intermediate 12 (16.5 g, 37.3 mmol) and cesium carbonate (43.7g, 134 mmol) in DMF (300 mL) was added 4-(chloromethyl)-5-(difluoromethyl)-1-methyl-triazole hydrochloride (8.12 g, 37.3 mmol) and the reaction mixture was stirred at rt for 18 h. The reaction mixture was diluted with water and the precipitate isolated. The solid was azeotroped with MeOH, dissolved in Et₂O and concentrated *in vacuo* to give *tert*-butyl (*S*)-5-chloro-8-((5-(difluoromethyl)-1-methyl-1*H-*1,2,3-triazol-4-yl)methoxy)-1-((1,3-dioxoisoindolin-2-yl)methyl)-3,4-dihydroisoquinoline-2(1*H*)-carboxylate (18.5 g, 84%) LCMS (Method 16): 1.13 min, 488.2 [M-CO₂*^{t}*Bu+H]⁺.

**Step b.** To a solution of the above intermediate (18.5 g, 31.4 mmol) in EtOH (569 mL) was added hydrazine hydrate (7.65 mL, 157 mmol). The reaction mixture was heated at 65 °C for 18 h then cooled to rt. The suspension was filtered and the solid rinsed with EtOH. The combined filtrates were concentrated *in vacuo.* The residue was washed with diethyl ether and filtered. The filtrate was concentrated *in vacuo,* azeotroped with IMS and the precipitate dissolved in DCM. Precipitate was removed by filtration and the combined organics dried over MgSO₄, filtered and concentrated *in vacuo* to give *tert*-butyl (*S*)-1-(aminomethyl)-5-chloro-8-((5-(difluoromethyl)-1-methyl-1*H*-1,2,3-triazol-4-yl)methoxy)-3,4-dihydroisoquinoline-2(1*H*)-carboxylate (13.5 g, 94%). LCMS (Method 16): 0.90 min, 458.2 [M+H]⁺.

**Step c.** A stirred mixture of the above intermediate (11.0 g, 22.0 mmol), triethylamine (4.60 mL, 33.0 mmol) and methyl 2-(1-(bromomethyl)-cyclopropyl)acetate (5.92 g, 28.6 mmol, CAS: 855473-50-6) in MeCN (210 mL) was heated at reflux for 72 h. The mixture was concentrated *in vacuo.* Purification by flash column chromatography (silica, 0 - 2% MeOH in DCM) gave *tert-*butyl (*S*)-5-chloro-8-((5-(difluoromethyl)-1-methyl-1*H*-1,2,3-triazol-4-yl)methoxy)-1-((6-oxo-5-azaspiro[2.4]-heptan-5-yl)methyl)-3,4-dihydroisoquinoline-2(1*H*)-carboxylate (11.5 g, 95%). LCMS (Method 16): 1.02 min, 452.2 [M-CO₂*^{t}*Bu+H]⁺.

**Step d.** (*S*)-5-((5-Chloro-8-((5-(difluoromethyl)-1-methyl-1*H*-1,2,3-triazol-4-yl)methoxy)-1,2,3,4-tetrahydroisoquinolin-1-yl)methyl)-5-azaspiro[2.4]heptan-6-one hydrochloride (4.08 g, assumed quantitative) was prepared from the above intermediate (4.54 g, 8.22 mmol) using a procedure similar to that described for Example 67, step b and was used without further purification. LCMS (Method 12): 0.99 min, 452.2 [M+H]⁺.

**Step e.** A mixture of the above intermediate (12.4 g, 25.4 mmol), Intermediate 33 (17.3 g, 38.1 mmol) and DIPEA (8.85 mL, 50.8 mmol) in DMF (30 mL) was stirred under nitrogen at rt for 6 days. The mixture was diluted with water (120 mL) and extracted with EtOAc. The combined organics were dried (MgSO₄), filtered and concentrated *in vacuo.* The crude product was purified by flash column chromatography on the Teledyne ISCO CombiFlash^{®} Rf+ (330 g silica column Puriflash HC, 0 - 5% MeOH in DCM) followed by another purification on the Teledyne ISCO CombiFlash^{®} Rf+ (330 g silica column Puriflash HC, 0 - 5% MeOH in DCM) to give 2,4-dimethoxybenzyl (1*S*,2*R*)-2-((*S*)-5-chloro-8-((5-(difluoromethyl)-1-methyl-1*H*-1,2,3-triazol-4-yl)methoxy)-1-((6-oxo-5-azaspiro[2.4]heptan-5-yl)methyl)-1,2,3,4-tetrahydroisoquinoline-2-carbonyl)-1-methylcyclohexane-1-carboxylate (14.7 g, 75%). LCMS (Method 3): 1.71 min, 770.5 [M+H]⁺.

**Step f.** A solution of the above intermediate (550 mg, 0.71 mmol) in hydrogen chloride in dioxane (4 M; 17.8 mL, 71.4 mmol) was stirred at rt for 10 mins and concentrated *in vacuo.* The residue was triturtated with methanol, then purified by reverse phase flash column chromatography on the Biotage Isolera (Biotage C18 SNAP 60 g, 20 - 60% MeCN in water, 0.1 % formic acid throughout) to provide (1*S*,2*R*)-2-((*S*)-5-chloro-8-((5-(difluoromethyl)-1-methyl-1*H*-1,2,3-triazol-4-yl)methoxy)-1-((6-oxo-5-azaspiro[2.4]-heptan-5-yl)methyl)-1,2,3,4-tetrahydroisoquinoline-2-carbonyl)-1-methylcyclohexane-1-carboxylic acid (250 mg, 56%). LCMS (Method 10): 2.52 min, 620.3 [M+H]⁺. ¹H NMR (300 MHz, DMSO-d₆) δ 11.84 (bs, 1H), 7.57 (t, 1H), 7.38 (d, 1H), 7.14 (d, 1H), 5.77 (m, 1H), 5.28 (s, 2H), 4.16 (s, 3H), 4.04-3.86 (m, 2H), 3.59 (m, 1H), 3.38-3.24 (m, 1H), 3.06-2.90 (m, 2H), 2.84 (m, 1H), 2.77-2.65 (m, 1H), 2.61 (d, 1H), 2.37-2.15 (m, 2H), 2.10 (d, 1H), 1.75-1.16 (m, 7H), 1.10 (s, 3H), 0.67-0.32 (m, 4H).

**Step f Method 2.** To a stirred solution of the above intermediate (14.7 g, 19.1 mmol) in DCM (92 mL) was added triethylsilane (3.05 mL, 19.1 mmol) followed by dropwise addition of TFA (1.76 mL, 22.9 mmol). The resulting solution was stirred for 1.5 h. The precipitate was removed by filtration, and the filtrate was concentrated *in vacuo* to ~5mL. The residue was purified by flash column chromatography on the Teledyne ISCO CombiFlash^{®} Rf⁺ (220 g silica column Puriflash HC, 0 - 3% MeOH in DCM). The product was then dissolved in 10mL of warm EtOH and left to cool to rt and the resulting precipitate was collected by filtration and the precipitate was washed with cold EtOH. The material was then slurried in MeCN (30 mL) and collected by filtration to provide (1*S*,2*R*)-2-((*S*)-5-chloro-8-((5-(difluoromethyl)-1-methyl-1*H*-1,2,3-triazol-4-yl)methoxy)-1-((6-oxo-5-azaspiro[2.4]heptan-5-yl)methyl)-1,2,3,4-tetrahydroisoquinoline-2-carbonyl)-1-methylcyclohexane-1-carboxylic acid (9.45 g, 78%). LCMS (Method 3): 4.57 min, 620.4 [M+H]⁺.

**Step g.** To a stirred solution of the above intermediate (100 mg, 0.160 mmol) in DMF (0.5 mL) was added HATU (92 mg, 0.240 mmol; CAS: 148893-10-1), methylamine (2 M in THF; 0.81 mL, 1.61 mmol; CAS: 74-89-5) and DIPEA (0.17 mL, 0.97 mmol) and the reaction mixture was stirred at rt for 18 h. The reaction mixture was diluted with water, extracted with EtOAc and the combined organics washed with 10 % citric acid, brine, dried over MgSO₄ and concentrated *in vacuo.* Purification using the Isolera (Biotage C18 SNAP 30 g, 5 - 60 % MeCN in water, 0.1 % formic acid throughout) gave the title compound (18 mg, 17%). LCMS (Method 9): 2.33 min, 633.5 [M+H]⁺. ¹H NMR (400 MHz, CDCl₃) δ 7.80 (bm, 1H), 7.24 (d, 1H), 6.93 (t, 1H), 6.89 (d, 1H), 5.97 (m, 1H), 5.32-5.18 (m, 2H), 4.17 (s, 3H), 4.07 (dd, 1H), 3.96 (m, 1H), 3.85 (m, 1H), 3.52 (d, 1H), 3.07 (m, 1H), 3.02-2.89 (m, 3H), 2.83 (m, 1H), 2.71 (d, 3H), 2.55 (m, 1H), 2.37 (d, 1H), 2.20 (d, 1H), 1.82-1.31 (m, 6H), 1.22-1.07 (m, 4H), 0.69-0.47 (m, 4H).

Compounds in Table 1 were synthesised by methods analogous to the above Examples.

**Table 1**

| **Ex.** | **Structure** | **Name** | **Analogous Example** | **¹H NMR δ ppm** | **LCMS** |
|---|---|---|---|---|---|
| **Ref. Ex. 1** | 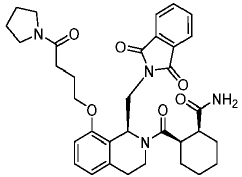 | (1*S*,2*R*)-2-((*S*)*-*1-((1,3-dioxoisoindolin-2-yl)methyl)-8-(4-oxo-4-(pyrrolidin-1-yl)butoxy)-1,2,3,4-tetrahydroisoquinoline-2-carbonyl)cyclohexane-1-carboxamide | 2 | ¹H NMR (400 MHz, DMSO-d₆) δ 7.87-7.76 (m, 4H), 7.18 (m, 1H), 6.86 (m, 1H), 6.80-6.72 (m, 2H), 6.51 (bs, 1H), 6.02 (m, 1H), 4.20-4.06 (m, 2H), 4.00 (m, 1H), 3.89-3.71 (m, 2H), 3.64 (m, 1H), 3.52-3.20 (m, 4H), 2.99 (m, 1H), 2.75 (m, 1H), 2.65 (m, 2H), 2.14 (m, 2H), 2.05-1.88 (m, 2H), 1.87-1.69 (m, 4H), 1.49 (m, 1H), 1.44-1.08 (m, 4H), 0.91 (m, 1H), 0.63 (m, 1H), 0.15 (m, 1H) | Method 1: 1.90 min, 601.1 [M+H]⁺ |
| **Ref. Ex. 3** | 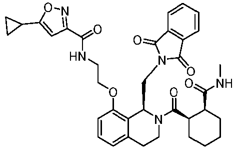 | 5-cyclopropyl-*N*-(2-(((*S*)-1-((1,3-dioxoisoindolin-2-yl)methyl)-2-((1*R*,2*S*)-2-(methylcarbamoyl)cyclohe xane-1-carbonyl)-1,2,3,4-tetrahydroisoquinolin-8-yl)oxy)ethyl)isoxazole-3-carboxamide | 2 | ¹H NMR (300 MHz, DMSO-d₆) δ 8.71 (t, 1H), 7.90-7.77 (m, 4H), 7.25-7.11 (m, 2H), 6.89 (d, 1H), 6.80 (d, 1H), 6.48 (s, 1H), 6.02 (m, 1H), 4.24-4.05 (m, 3H), 4.00-3.73 (m, 4H), 3.60 (m, 1H), 3.39-3.22 (m, 1H), 2.96 (m, 1H), 2.75 (m, 1H), 2.35 (d, 3H), 2.15-1.83 (m, 2H), 1.76 (m, 1H), 1.60-1.39 (m, 2H), 1.37-0.66 (m, 8H), 0.22 (m, 1H). | Method 1: 2.42 min, 654.5 [M+H]⁺ |
| **Ref. Ex. 4** | 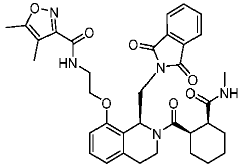 | *N*-(2-(((*S*)-1-((1,3-dioxoisoindolin-2-yl)methyl)-2-((1*R*,2*S*)-2-(methylcarbamoyl)cyclohe xane-1-carbonyl)-1,2,3,4-tetrahydroisoquinolin-8-yl)oxy)ethyl)-4,5-dimethylisoxazole-3-carboxamide | 2 | ¹H NMR (300 MHz, DMSO-d₆) δ 8.69 (t, 1H), 7.91-7.75 (m, 4H), 7.25-7.09 (m, 2H), 6.90 (d, 1H), 6.80 (d, 1H), 6.02 (dd, 1H), 4.22-4.06 (m, 3H), 4.04-3.68 (m, 4H), 3.61 (m, 1H), 3.26 (m, 1H), 2.96 (m, 1H), 2.75 (m, 1H), 2.35 (d, 3H), 2.25 (s, 3H), 2.13-1.79 (m, 5H), 1.59-1.39 (m, 2H), 1.37-0.62 (m, 4H), 0.21 (m, 1H). | Method 1: 2.44 min, 642.5 [M+H]⁺ |
| **Ref. Ex. 5** | 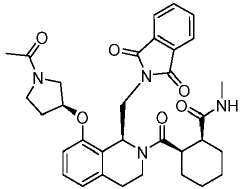 | (1*S*,2*R*)-2-((*S*)-8-(((*S*)-1-acetylpyrrolidin-3-yl)oxy)-1-((1,3-dioxoisoindolin-2-yl)methyl)-1,2,3,4-tetrahydroisoquinoline-2-carbonyl)-*N-*methylcyclohexane-1-carboxamide | 2 | ¹H NMR (400 MHz, DMSO-d₆; rotamers observed, both reported) δ 7.91-7.75 (m, 4H), 7.25-7.10 (m, 2H), 6.89 (m, 1H), 6.81 (m, 1H), 5.92 (m, 1H), 5.16 (m, 0.6H), 5.10 (m, 0.4H), 4.15-3.52 (m, 8H), 3.25 (m, 1H), 2.95 (m, 1H), 2.80-2.70 (m, 1H), 2.60-1.81 (m, 10H), 1.52 (m, 1H), 1.42 (m, 1H), 1.36-1.17 (m, 2H), 1.01-0.76 (m, 1H), 0.75-0.62 (m, 1H), 0.31-0.04 (m, 1H). | Method 1: 1.89 & 1.97 min, 587.5 [M+H]⁺ (rotamers) |
| **Ref. Ex. 7** | 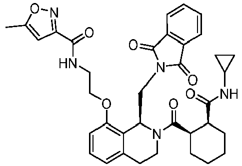 | *N*-(2-(((*S*)-2-((1*R,2S)-2-*(cyclopropylcarbamoyl)cy clohexane-1-carbonyl)-1-((1,3-dioxoisoindolin-2-yl)methyl)-1,2,3,4-tetrahydroisoquinolin-8-yl)oxy)ethyl)-5-methylisoxazole-3-carboxamide | 6 | ¹H NMR (300 MHz, DMSO-d₆) δ 8.77 (m, 1H), 7.91-7.75 (m, 4H), 7.26-7.11 (m, 2H), 6.90 (m, 1H), 6.80 (m, 1H), 6.54 (m, 1H), 6.03 (m, 1H), 4.25-4.05 (m, 3H), 4.03-3.72 (m, 4H), 3.62 (m, 1H), 3.23 (m, 1H), 2.96 (m, 1H), 2.75 (m, 1H), 2.42-2.25 (m, 4H), 2.10-1.79 (m, 2H), 1.53-1.37 (m, 2H), 1.36-1.16 (m, 2H), 0.92 (m, 1H), 0.74 (m, 1H), 0.44-0.02 (m, 5H). | Method 1: 2.37 min, 654.5 [M+H]⁺ |
| **Ref. Ex. 8** | 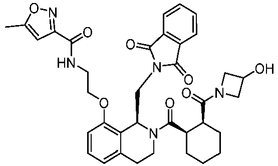 | *N*-(2-(((*S*)-1-((1,3-dioxoisoindolin-2-yl)methyl)-2-((1*R*,2*S*)-2-(3-hydroxyazetidine-1-carbonyl)cyclohexane-1-carbonyl)-1,2,3,4-tetrahydroisoquinolin-8-yl)oxy)ethyl)-5-methylisoxazole-3-carboxamide | 6 | ¹H NMR (300 MHz, DMSO-d₆) δ 8.78 (t, 1H), 7.98-7.77 (m, 4H), 7.20 (t, 1H), 6.90 (m, 1H), 6.80 (m, 1H), 6.55 (m, 1H), 6.07 (m, 1H), 5.60 (m, 1H), 4.37-3.48 (m, 12H), 3.08-2.66 (m, 3H), 2.44-2.22 (m, 4H), 1.95 (m, 1H), 1.69-0.90 (m, 5H), 0.73 (m, 1H), 0.51 (m, 1H). | Method 1: 2.12 min, 670.6 [M+H]⁺ |
| **Ref. Ex. 9** | 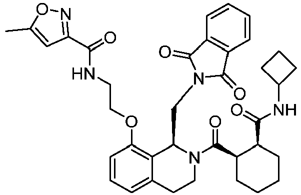 | *N*-(2-(((*S*)-2-((1*R,2S)-2-*(cyclobutylcarbamoyl)cycl ohexane-1-carbonyl)-1-((1,3-dioxoisoindolin-2-yl)methyl)-1,2,3,4-tetrahydroisoquinolin-8-yl)oxy)ethyl)-5-methylisoxazole-3-carboxamide | 6 | ¹H NMR (300 MHz, DMSO-d₆) δ 8.77 (t, 1H), 7.91-7.77 (m, 4H), 7.36 (d, 1H), 7.19 (t, 1H), 6.89 (m, 1H), 6.79 (m, 1H), 6.54 (m, 1H), 6.02 (m, 1H), 4.24-4.04 (m, 3H), 4.01-3.71 (m, 5H), 3.62 (m, 1H), 3.24 (m, 1H), 2.96 (m, 1H), 2.74 (m, 1H), 2.35 (s, 3H), 2.13-1.17 (m, 12H), 0.93 (m, 1H), 0.73 (m, 1H), 0.23 (m, 1H). | Method 1: 2.53 min, 668.6 [M+H]⁺ |
| **Ref. Ex. 10** | 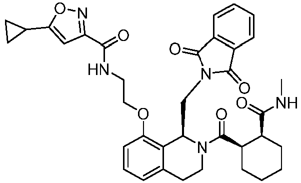 | 5-cyclopropyl-*N*-(2-(((*S*)-1-((1,3-dioxoisoindolin-2-yl)methyl)-2-((1*R*,2*S*)-2-(methylcarbamoyl)cyclohe xane-1-carbonyl)-1,2,3,4-tetrahydroisoquinolin-8-yl)oxy)ethyl)isoxazole-3-carboxamide | 6 | ¹H NMR (400 MHz, DMSO-d₆) δ 8.87 (t, 1H), 7.89-7.78 (m, 4H), 7.20 (t, 1H), 7.15 (m, 1H), 6.90 (d, 1H), 6.81 (d, 1H), 6.79 (s, 1H), 6.03 (dd, 1H), 4.23 (m, 1H), 4.17-4.08 (m, 2H), 3.94-3.75 (m, 4H), 3.62 (m, 1H), 3.26 (m, 1H), 2.97 (m, 1H), 2.76 (m, 1H), 2.35 (d, 3H), 2.05 (m, 1H), 1.99-1.87 (m, 2H), 1.52 (m, 1H), 1.44 (m, 1H), 1.36-1.21 (m, 2H), 1.02-0.86 (m, 3H), 0.79-0.65 (m, 3H), 0.24 (m, 1H). | Method 1: 2.29 min, 654.6 [M+H]⁺ |
| **Ref. Ex. 11** | 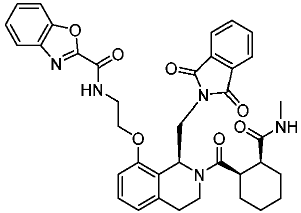 | *N*-(2-(((*S*)-1-((1,3-dioxoisoindolin-2-yl)methyl)-2-((1*R*,2*S*)-2-(methylcarbamoyl)cyclohe xane-1-carbonyl)-1,2,3,4-tetrahydroisoquinolin-8-yl)oxy)ethyl)benzo[*d*]oxaz ole-2-carboxamide | 6 | ¹H NMR (400 MHz, DMSO-d₆) δ 9.30 (t, 1H), 7.92-7.74 (m, 4H), 7.71 (m, 1H), 7.44 (m, 1H), 7.36-7.26 (m, 2H), 7.21 (t, 1H), 7.16 (m, 1H), 6.92 (d, 1H), 6.81 (d, 1H), 6.08 (dd, 1H), 4.28-4.21 (m, 2H), 4.17 (dd, 1H), 4.02 (m, 1H), 3.94-3.75 (m, 3H), 3.60 (m, 1H), 3.27 (m, 1H), 2.95 (m, 1H), 2.75 (m, 1H), 2.35 (d, 3H), 2.04 (m, 1H), 1.89 (m, 1H), 1.57-1.20 (m, 4H), 0.93 (m, 1H), 0.74 (m, 1H), 0.21 (m, 1H). | Method 1: 2.42 min, 664.6 [M+H]⁺ |
| **Ref. Ex. 12** | 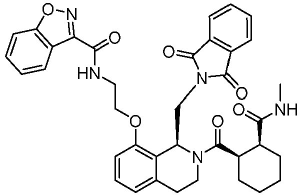 | *N*-(2-(((*S*)-1-((1,3-dioxoisoindolin-2-yl)methyl)-2-((1*R*,2*S*)-2-(methylcarbamoyl)cyclohe xane-1-carbonyl)-1,2,3,4-tetrahydroisoquinolin-8-yl)oxy)ethyl)benzo[*d*]isoxa zole-3-carboxamide | 6 | ¹H NMR (400 MHz, DMSO-d₆) δ 9.12 (t, 1H), 8.12 (m, 1H), 7.84-7.77 (m, 4H), 7.75 (m, 1H), 7.66 (m, 1H), 7.46 (m, 1H), 7.21 (t, 1H), 7.17 (m, 1H), 6.93 (d, 1H), 6.81 (d, 1H), 6.04 (dd, 1H), 4.32-4.19 (m, 2H), 4.15 (dd, 1H), 4.06 (m, 1H), 3.95-3.75 (m, 3H), 3.61 (m, 1H), 3.26 (m, 1H), 2.96 (m, 1H), 2.75 (m, 1H), 2.35 (d, 3H), 2.04 (m, 1H), 1.90 (m, 1H), 1.52 (m, 1H), 1.45 (m, 1H), 1.35-1.20 (m, 2H), 0.93 (m, 1H), 0.74 (m, 1H), 0.20 (m, 1H). | Method 1: 2.62 min, 664.6 [M+H]⁺ |
| **Ref. Ex. 13** | 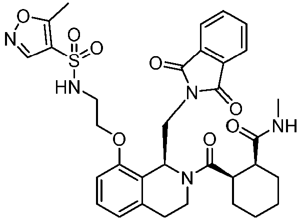 | (1*S*,2*R*)-2-((*S*)-1-((1,3-dioxoisoindolin-2-yl)methyl)-8-(2-((5-methylisoxazole)-4-sulfonamido)ethoxy)-1,2,3,4-tetrahydroisoquinoline-2-carbonyl)-*N*-methylcyclohexane-1-carboxamide | 6 | ¹H NMR (400 MHz, DMSO-d₆) δ 8.85 (bs, 1H), 7.89-7.76 (m, 4H), 7.19 (t, 1H), 7.15 (m, 1H), 6.83 (d, 1H), 6.78 (d, 1H), 6.13 (t, 1H), 5.96 (dd, 1H), 4.26-4.14 (m, 2H), 3.99 (m, 1H), 3.86 (m, 1H), 3.75 (dd, 1H), 3.62 (m, 1H), 3.45-3.21 (m, 3H), 2.94 (m, 1H), 2.75 (m, 1H), 2.34 (d, 3H), 2.07-1.86 (m, 2H), 1.81 (s, 3H), 1.55-1.39 (m, 2H), 1.35-1.21 (m, 2H), 0.93 (m, 1H), 0.73 (m, 1H), 0.23 (m, 1H). | Method 1: 1.63 min, 664.5 [M+H]⁺ |
| **Ref. Ex. 14** | 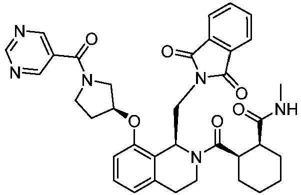 | (1*S*,2*R*)-2-((*S*)-1-((1,3-dioxoisoindolin-2-yl)methyl)-8-(((*S*)-1-(pyrimidine-5-carbonyl)pyrrolidin-3-yl)oxy)-1,2,3,4-tetrahydroisoquinoline-2-carbonyl)-*N-*methylcyclohexane-1-carboxamide | 6 | ¹H NMR (400 MHz, DMSO-d₆; rotamers observed, both reported) δ 9.32 (s, 0.6H), 9.27 (s, 0.4H), 9.09 (s, 1.2H), 9.04 (s, 0.8H), 7.89-7.75 (m, 4H), 7.24 (t, 0.4H), 7.19 (t, 0.6H), 7.14 (m, 0.6H), 7.08 (m, 0.4H), 6.99 (d, 0.4H), 6.88 (d, 0.6H), 6.84 (d, 0.4H), 6.79 (d, 0.6H), 6.01 (dd, 0.4H), 5.87 (dd, 0.6H), 5.25 (m, 0.4H), 5.13 (m, 0.6H), 4.22 (dd, 0.6H), 4.14-3.46 (m, 7.4H), 3.24 (m, 1H), 2.94 (m, 1H), 2.84-2.69 (m, 1H), 2.56-2.20 (m, 5H), 2.15-1.97 (m, 1H), 1.96-1.78 (m, 1H), 1.50 (m, 1H), 1.45-1.35 (m, 1H), 1.33-1.18 (m, 2H), 0.92 (m, 1H), 0.70 (m, 1H), 0.25 (m, 0.4H), 0.13 (m, 0.6H). | Method 1: 1.95 min, 651.6 [M+H]⁺ |
| **Ref. Ex. 15** | 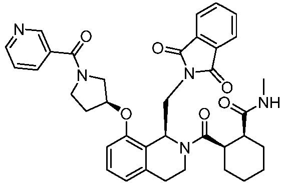 | (1*S*,*2*R)-2-((*S*)-1-((1,3-dioxoisoindolin-2-yl)methyl)-8-(((*S*)-1-nicotinoylpyrrolidin-3-yl)oxy)-1,2,3,4-tetrahydroisoquinoline-2-carbonyl)-*N-*methylcyclohexane-1-carboxamide | 6 | ¹H NMR (400 MHz, DMSO-d₆; rotamers observed, both reported) δ 8.82 (m, 0.5H), 8.76 (m, 0.5H), 8.67 (dd, 0.5H), 8.64 (dd, 0.5H), 8.06 (m, 0.5H), 8.00 (m, 0.5H), 7.88-7.76 (m, 4H), 7.52 (dd, 0.5H), 7.45 (dd, 0.5H), 7.27-7.09 (m, 2H), 6.97 (d, 0.5H), 6.86 (d, 0.5H), 6.83 (d, 0.5H), 6.79 (d, 0.5H), 6.01 (dd, 0.5H), 5.89 (dd, 0.5H), 5.23 (m, 0.5H), 5.12 (m, 0.5H), 4.14-3.53 (m, 8H), 3.26 (m, 1H), 2.95 (m, 1H), 2.84-2.69 (m, 1H), 2.59-2.21 (m, 5H), 2.18-1.77 (m, 2H), 1.51 (m, 1H), 1.43 (m, 1H), 1.35-1.17 (m, 2H), 1.01-0.78 (m, 1H), 0.71 (m, 1H), 0.19 (m, 1H). | Method 1: 2.00 min, 650.6 [M+H]⁺ |
| **Ref. Ex. 16** | 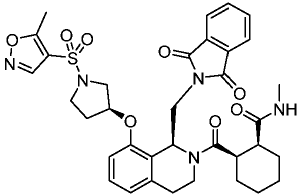 | (1*S*,2*R*)-2-((*S*)-1-((1,3-dioxoisoindolin-2-yl)methyl)-8-(((*S*)-1-((5-methylisoxazol-4-yl)sulfonyl)pyrrolidin-3-yl)oxy)-1,2,3,4-tetrahydroisoquinoline-2-carbonyl)-*N-*methylcyclohexane-1-carboxamide | 6 | ¹H NMR (400 MHz, DMSO-d₆) δ 7.90-7.75 (m, 5H), 7.24-7.13 (m, 2H), 6.86 (m, 1H), 6.78 (m, 1H), 5.93 (m, 1H), 4.98 (m, 1H), 4.11 (dd, 1H), 4.03 (m, 1H), 3.86 (m, 1H), 3.75 (m, 1H), 3.68-3.29 (m, 4H), 3.25 (m, 1H), 2.95 (m, 1H), 2.74 (m, 1H), 2.35 (d, 3H), 2.26-2.15 (m, 2H), 2.09-1.84 (m, 5H), 1.55-1.38 (m, 2H), 1.35-1.19 (m, 2H), 0.93 (m, 1H), 0.71 (m, 1H), 0.23 (m, 1H). | Method 1: 1.75 min, 690.6 [M+H]⁺ |
| **Ref. Ex. 17** | 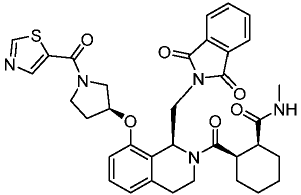 | (1*S,*2*R*)-2-((*S*)-1-((1,3-dioxoisoindolin-2-yl)methyl)-8-(((*S*)-1-(thiazole-5-carbonyl)pyrrolidin-3-yl)oxy)-1,2,3,4-tetrahydroisoquinoline-2-carbonyl)-*N-*methylcyclohexane-1-carboxamide | 6 | ¹H NMR (400 MHz, DMSO-d₆; rotamers observed, both reported) δ 9.26 (s, 0.6H), 9.22 (s, 0.4H), 8.44 (s, 0.6H), 8.39 (s, 0.4H), 7.86-7.74 (m, 4H), 7.27-7.17 (m, 1H), 7.16-7.05 (m, 1H), 6.99-6.90 (m, 1H), 6.85-6.77 (m, 1H), 5.97 (m, 0.4H), 5.91 (m, 0.6H), 5.21 (m, 1H), 4.33 (dd, 0.6H), 4.25-3.69 (m, 6.4H), 3.62 (m, 1H), 3.24 (m, 1H), 2.95 (m, 1H), 2.75 (m, 1H), 2.60-2.22 (m, 5H), 2.14-1.96 (m, 1H), 1.88 (m, 1H), 1.58-1.36 (m, 2H), 1.34-1.05 (m, 2H), 1.02-0.63 (m, 2H), 0.19 (m, 1H). | Method 1: 2.02 min, 656.4 [M+H]⁺ |
| **Ref. Ex. 18** | 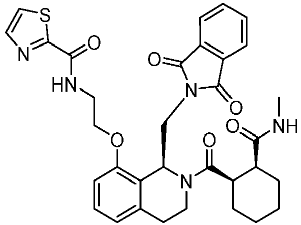 | *N*-(2-(((*S*)-1-((1,3-dioxoisoindolin-2-yl)methyl)-2-((1*R*,2*S*)-2-(methylcarbamoyl)cyclohe xane-1-carbonyl)-1,2,3,4-tetrahydroisoquinolin-8-yl)oxy)ethyl)thiazole-2-carboxamide | 6 | ¹H NMR (400 MHz, DMSO-d₆) δ 8.97 (t, 1H), 7.96 (d, 1H), 7.91-7.77 (m, 4H), 7.69 (d, 1H), 7.24-7.12 (m, 2H), 6.90 (d, 1H), 6.80 (d, 1H), 6.04 (dd, 1H), 4.22-4.07 (m, 3H), 4.00 (m, 1H), 3.94-3.74 (m, 3H), 3.60 (m, 1H), 3.27 (m, 1H), 2.95 (m, 1H), 2.74 (m, 1H), 2.35 (d, 3H), 2.04 (m, 1H), 1.90 (m, 1H), 1.53 (m, 1H), 1.46 (m, 1H), 1.36-1.19 (m, 2H), 0.95 (m, 1H), 0.76 (m, 1H), 0.22 (m, 1H). | Method 1: 2.25 min, 630.4 [M+H]⁺ |
| **Ref. Ex. 19** | 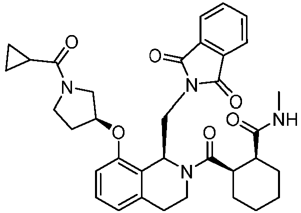 | (1*S*,2*R*)-2-((*S*)-8-(((*S*)-1-(cyclopropanecarbonyl)py rrolidin-3-yl)oxy)-1-((1,3-dioxoisoindolin-2-yl)methyl)-1,2,3,4-tetrahydroisoquinoline-2-carbonyl)-*N-*methylcyclohexane-1-carboxamide | 6 | ND | Method 1: 2.14 min, 613.5 [M+H]⁺ |
| **Ref. Ex. 20** | 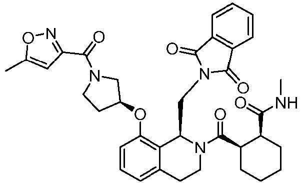 | (1*S*,2*R*)-2-((*S*)-1-((1,3-dioxoisoindolin-2-yl)methyl)-8-(((*S*)-1-(5-methylisoxazole-3-carbonyl)pyrrolidin-3-yl)oxy)-1,2,3,4-tetrahydroisoquinoline-2-carbonyl)-*N*-methylcyclohexane-1-carboxamide | 6 | ¹H NMR (400 MHz, DMSO-d₆; rotamers observed, both reported) δ 7.87-7.75 (m, 4H), 7.26-7.17 (m, 1H), 7.14 (m, 1H), 6.94 (m, 0.4H), 6.89 (m, 0.6H), 6.85-6.77 (m, 1H), 6.55 (m, 0.4H), 6.51 (m, 0.6H), 5.93 (m, 0.4H), 5.88 (m, 0.6H), 5.18 (m, 1H), 4.20-3.70 (m, 7H), 3.61 (m, 1H), 3.25 (m, 1H), 3.16 (m, 3H), 2.94 (m, 1H), 2.75 (m, 1H), 2.60-2.23 (m, 5H), 2.17-1.81 (m, 2H), 1.60-1.36 (m, 2H), 1.33-1.17 (m, 2H), 1.02-0.77 (m, 1H), 0.69 (m, 1H), 0.17 (m, 1H). | Method 1: 2.24 & 2.35 min, 654.6 [M+H]+ (rotamers) |
| **Ref. Ex. 21** | 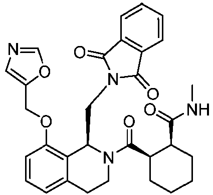 | (1*S*,2*R*)-2-((*S*)-1-((1,3-dioxoisoindolin-2-yl)methyl)-8-(oxazol-5-ylmethoxy)-1,2,3,4-tetrahydroisoquinoline-2-carbonyl)-*N-*methylcyclohexane-1-carboxamide | 6 | ¹H NMR (400 MHz, DMSO-d₆) δ 8.43 (s, 1H), 7.85-7.75 (m, 4H), 7.52 (s, 1H), 7.23 (t, 1H), 7.14 (m, 1H), 7.07 (d, 1H), 6.84 (d, 1H), 5.98 (dd, 1H), 5.33 (d, 1H), 5.25 (d, 1H), 4.08 (dd, 1H), 3.83 (m, 1H), 3.73 (dd, 1H), 3.62 (m, 1H), 3.24 (m, 1H), 2.97 (m, 1H), 2.77 (m, 1H), 2.35 (d, 3H), 2.03 (m, 1H), 1.91 (m, 1H), 1.52 (m, 1H), 1.42-1.19 (m, 3H), 0.92 (m, 1H), 0.63 (m, 1H), 0.16 (m, 1H). | Method 1: 2.07 min, 557.4 [M+H]⁺ |
| **Ref. Ex. 22** | 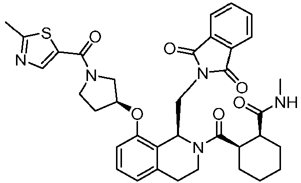 | (1*S*,2*R*)-2-((*S*)-1-((1,3-dioxoisoindolin-2-yl)methyl)-8-(((*S*)-1-(2-methylthiazole-5-carbonyl)pyrrolidin-3-yl)oxy)-1,2,3,4-tetrahydroisoquinoline-2-carbonyl)-*N-*methylcyclohexane-1-carboxamide | 6 | ¹H NMR (400 MHz, DMSO-d₆; rotamers observed, both reported) δ 8.14 (s, 0.6H), 8.12 (s, 0.4H), 7.90-7.74 (m, 4H), 7.26-7.16 (m, 1H), 7.11 (m, 1H), 6.99-6.87 (m, 1H), 6.86-6.77 (m, 1H), 5.99-5.86 (m, 1H), 5.27-5.09 (m, 1H), 4.29 (dd, 0.6H), 4.24-3.70 (m, 6.4H), 3.61 (m, 1H), 3.24 (m, 1H), 2.94 (m, 1H), 2.82-2.60 (m, 4H), 2.56-2.20 (m, 5H), 2.13-1.96 (m, 1H), 1.88 (m, 1H), 1.50 (m, 1H), 1.42 (m, 1H), 1.34-1.18 (m, 2H), 0.92 (m, 1H), 0.70 (m, 1H), 0.18 (m, 1H). | Method 1: 2.07 min, 670.4 [M+H]⁺ |
| **Ref. Ex. 23** | 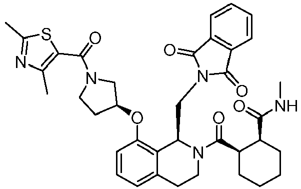 | (1*S*,2*R*)-2-((*S*)-8-(((*S*)-1-(2,4-dimethylthiazole-5-carbonyl)pyrrolidin-3-yl)oxy)-1-((1,3-dioxoisoindolin-2-yl)methyl)-1,2,3,4-tetrahydroisoquinoline-2-carbonyl)-*N-*methylcyclohexane-1-carboxamide | 6 | ¹H NMR (400 MHz, DMSO-d₆) δ 7.96-7.71 (m, 4H), 7.28-7.08 (m, 2H), 6.97-6.74 (m, 2H), 5.93 (m, 1H), 5.17 (m, 1H), 4.16-3.52 (m, 8H), 3.25 (m, 1H), 2.96 (m, 1H), 2.75 (m, 1H), 2.61 (m, 3H), 2.45-2.16 (m, 8H), 2.15-1.81 (m, 2H), 1.59-1.38 (m, 2H), 1.35-1.17 (m, 2H), 0.93 (m, 1H), 0.71 (m, 1H), 0.19 (m, 1H). | Method 1: 2.16 min, 684.2 [M+H]⁺ |
| **Ref. Ex. 24** | 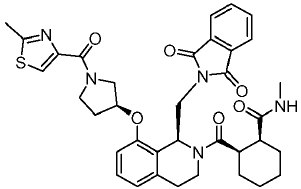 | (1*S*,2*R*)-2-((*S*)-1-((1,3-dioxoisoindolin-2-yl)methyl)-8-(((*S*)-1-(2-methylthiazole-4-carbonyl)pyrrolidin-3-yl)oxy)-1,2,3,4-tetrahydroisoquinoline-2-carbonyl)-*N-*methylcyclohexane-1-carboxamide | 6 | ¹H NMR (400 MHz, DMSO-d₆; rotamers observed, both reported) δ 8.07 (s, 0.4H), 8.04 (s, 0.6H), 7.97-7.74 (m, 4H), 7.27-7.17 (m, 1H), 7.14 (m, 1H), 6.93 (d, 0.4H), 6.89 (d, 0.6H), 6.82 (d, 0.4H), 6.79 (d, 0.6H), 5.94 (dd, 0.4H), 5.88 (dd, 0.6H), 5.19 (m, 0.6H), 5.14 (m, 0.4H), 4.32-3.69 (m, 7H), 3.61 (m, 1H), 3.24 (m, 1H), 3.02-2.86 (m, 1H), 2.83-2.58 (m, 4H), 2.55-2.09 (m, 5H), 2.02 (m, 1H), 1.88 (m, 1H), 1.60-1.47 (m, 1H), 1.46-1.36 (m, 1H), 1.33-1.18 (m, 2H), 0.91 (m, 1H), 0.69 (m, 1H), 0.16 (m, 1H). | Method 1: 2.20 min, 670.4 [M+H]⁺ |
| **Ref. Ex. 25** | 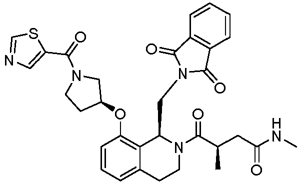 | (*R*)-4-((*S*)-1-((1,*3-*dioxoisoindolin-2-yl)methyl)-8-(((*S*)-1-(thiazole-5-carbonyl)pyrrolidin-3-yl)oxy)-3,4-dihydroisoquinolin-2(1*H*)-yl)-*N*,3-dimethyl-4-oxobutanamide | 6 | ¹H NMR (400 MHz, DMSO-d₆; rotamers observed, both reported) δ 9.28-9.18 (m, 1H), 8.42 (s, 0.5H), 8.38 (s, 0.5H), 7.87-7.72 (m, 4H), 7.51 (m, 1H), 7.28-7.17 (m, 1H), 7.00-6.88 (m, 1H), 6.86-6.76 (m, 1H), 5.93 (m, 0.5H), 5.88 (m, 0.5H), 5.22 (m, 1H), 4.35-3.74 (m, 7H), 3.65 (m, 1H), 3.10-2.75 (m, 3H), 2.55-2.22 (m, 5H), 2.14 (m, 1H), 1.83 (m, 1H), 0.56 (d, 3H). | Method 1: 1.78 min, 616.3 [M+H]⁺ |
| **Ref.** Ex. **26** | 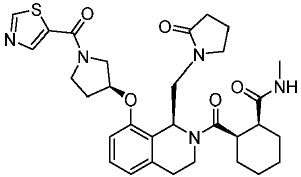 | (1*S*,2*R*)-*N*-methyl-2-((*S*)-1-((2-oxopyrrolidin-1-yl)methyl)-8-(((*S*)-1-(thiazole-5-carbonyl)pyrrolidin-3-yl)oxy)-1,2,3,4-tetrahydroisoquinoline-2-carbonyl)cyclohexane-1-carboxamide | 6 | ¹H NMR (400 MHz, DMSO-d₆; rotamers observed, both reported) δ 9.26 (s, 1H), 8.46 (s, 0.5H), 8.42 (s, 0.5H), 7.31-7.11 (m, 2H), 6.91 (d, 1H), 6.82-6.71 (m, 1H), 5.80 (m, 0.5H), 5.74 (m, 0.5H), 5.17 (m, 0.5H), 5.12 (m, 0.5H), 4.34-3.22 (m, 9H), 3.11-2.80 (m, 3H), 2.71 (m, 1H), 2.46-1.51 (m, 14H), 1.50-1.32 (m, 2H), 1.31-1.08 (m, 2H). | Method 1: 1.42 min, 594.3 [M+H]⁺ |
| **Ref. Ex. 27** | 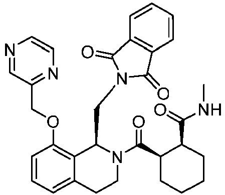 | (1*S*,2*R*)-2-((*S*)-1-((1,3-dioxoisoindolin-2-yl)methyl)-8-(pyrazin-2-ylmethoxy)-1,2,3,4-tetrahydroisoquinoline-2-carbonyl)-*N-*methylcyclohexane-1-carboxamide | 6 | ¹H NMR (400 MHz, DMSO-d₆) δ 9.27 (m, 1H), 8.71-8.67 (m, 2H), 7.86-7.78 (m, 4H), 7.25 (t, 1H), 7.15 (m, 1H), 7.08 (d, 1H), 6.87 (d, 1H), 6.14 (dd, 1H), 5.41 (d, 1H), 5.33 (d, 1H), 4.18 (dd, 1H), 3.90-3.75 (m, 2H), 3.67 (m, 1H), 3.40-3.20 (m, 1H), 3.00 (m, 1H), 2.82 (m, 1H), 2.36 (d, 3H), 2.05 (m, 1H), 1.93 (m, 1H), 1.53 (m, 1H), 1.44-1.15 (m, 3H), 0.92 (m, 1H), 0.60 (m, 1H), 0.17 (m, 1H). | Method 3: 4.16 min, 568.2 [M+H]⁺ |
| **Ref. Ex. 28** | 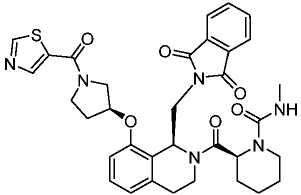 | (*S*)-2-((*S*)-1-((1,3-dioxoisoindolin-2-yl)methyl)-8-(((*S*)-1-(thiazole-5-carbonyl)pyrrolidin-3-yl)oxy)-1,2,3,4-tetrahydroisoquinoline-2-carbonyl)-*N-*methylpiperidine-1-carboxamide | 67 | ¹H NMR (400 MHz, DMSO-d₆; rotamers observed, both reported) δ 9.25 (s, 0.6H), 9.23 (s, 0.4H), 8.43 (s, 0.6H), 8.39 (s, 0.4H), 7.86-7.79 (m, 4H), 7.23 (m, 1H), 6.95 (m, 1H), 6.82 (m, 1H), 6.23 (m, 1H), 5.88 (m, 1H), 5.25-5.18 (m, 1H), 4.99-4.92 (m, 1H), 4.32 (dd, 0.6H), 4.25-3.92 (m, 3.4H), 3.92-3.77 (m, 4H), 3.65 (m, 1H), 3.42-3.23 (m, 1H), 3.04-2.91 (m, 2H), 2.79 (m, 1H), 2.47-2.24 (m, 4H), 1.47-1.37 (m, 2H), 1.24 (m, 1H), 1.07 (m, 1H), 0.91 (m, 1H), 0.31 (m, 1H). | Method 3: 3.91 min, 657.2 [M+H]⁺ |
| **Ref. Ex. 29** | 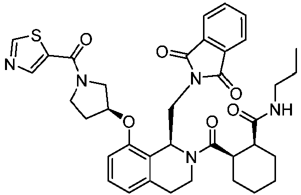 | (1*S*,2*R*)-2-((*S*)-1-((1,3-dioxoisoindolin-2-yl)methyl)-8-(((*S*)-1-(thiazole-5-carbonyl)pyrrolidin-3-yl)oxy)-1,2,3,4-tetrahydroisoquinoline-2-carbonyl)-*N-*propylcyclohexane-1-carboxamide | 6 | ¹H NMR (400 MHz, DMSO-d₆; rotamers observed, both reported) δ 9.25 (s, 0.6H), 9.23 (s, 0.4H), 8.41 (s, 0.6H), 8.39 (s, 0.4H), 7.86-7.75 (m, 4H), 7.21 (m, 1H), 7.03 (m, 1H), 6.93 (m, 1H), 6.80 (m, 1H), 5.97 (dd, 0.4H), 5.91 (dd, 0.6H), 5.26-5.15 (m, 1H), 4.31 (dd, 0.6H), 4.25-3.69 (m, 6.4H), 3.62 (m, 1H), 3.22 (m, 1H), 2.92 (m, 1H), 2.82-2.63 (m, 3H), 2.56-2.24 (m, 2H), 2.09-1.87 (m, 2H), 1.48-1.37 (m, 2H), 1.35-1.20 (m, 2H), 1.12-0.85 (m, 3H), 0.72 (m, 1H), 0.46 (t, 1.2H), 0.37 (t, 1.8H), 0.24 (m, 1H). | Method 3: 4.38 min, 684.2 [M+H]⁺ |
| **Ref. Ex. 30** | 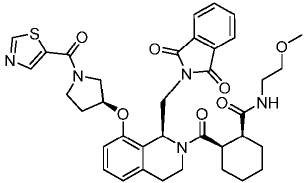 | (1*S*,2*R*)-2-((*S*)-1-((1,3-dioxoisoindolin-2-yl)methyl)-8-(((*S*)-1-(thiazole-5-carbonyl)pyrrolidin-3-yl)oxy)-1,2,3,4-tetrahydroisoquinoline-2-carbonyl)-*N*-(2-methoxyethyl)cyclohexan e-1-carboxamide | 6 | ¹H NMR (400 MHz, DMSO-d₆; rotamers observed, both reported) δ 9.25 (s, 0.6H), 9.23 (s, 0.4H), 8.42 (s, 0.6H), 8.39 (s, 0.4H), 7.84-7.75 (m, 4H), 7.22 (m, 1H), 7.13 (m, 1H), 6.93 (m, 1H), 6.81 (m, 1H), 5.98 (dd, 0.4H), 5.92 (dd, 0.6H), 5.25-5.16 (m, 1H), 4.32 (dd, 0.6H), 4.24-3.70 (m, 6.4H), 3.62 (m, 1H), 3.22 (m, 1H), 3.01-2.87 (m, 8H), 2.76 (m, 1H), 2.59-2.24 (m, 2H), 2.05 (m, 1H), 1.92 (m, 1H), 1.50-1.38 (m, 2H), 1.35-1.20 (m, 2H), 0.93 (m, 1H), 0.72 (m, 1H), 0.24 (m, 1H). | Method 3: 4.13 min, 700.2 [M+H]⁺ |
| **Ref. Ex. 31** | 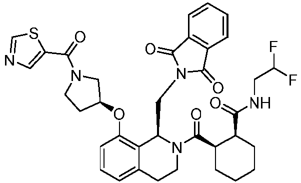 | (1*S,2R)-N-(2,2-*difluoroethyl)-2-((*S*)-1-((1,3-dioxoisoindolin-2-yl)methyl)-8-(((*S*)-1-(thiazole-5-carbonyl)pyrrolidin-3-yl)oxy)-1,2,3,4-tetrahydroisoquinoline-2-carbonyl)cyclohexane-1-carboxamide | 6 | ¹H NMR (400 MHz, DMSO-d₆; rotamers observed, both reported) δ 9.25 (s, 0.6H), 9.23 (s, 0.4H), 8.42 (s, 0.6H), 8.38 (s, 0.4H), 7.85-7.74 (m, 4H), 7.57 (m, 1H), 7.27-7.17 (m, 1H), 6.99-6.89 (m, 1H), 6.86-6.76 (m, 1H), 5.97 (m, 0.4H), 5.91 (m, 0.6H), 5.80-5.42 (m, 1H), 5.27-5.16 (m, 1H), 4.32 (dd, 0.6H), 4.27-3.71 (m, 6.4H), 3.63 (m, 1H), 3.45-2.84 (m, 4H), 2.76 (m, 1H), 2.58-2.24 (m, 2H), 2.19-2.06 (m, 1H), 1.92 (m, 1H), 1.63-1.39 (m, 2H), 1.38-1.20 (m, 2H), 1.04-0.87 (m, 1H), 0.75 (m, 1H), 0.26 (m, 1H). | Method 3: 4.42 min, 706.2 [M+H]⁺ |
| **Ref. Ex. 32** | 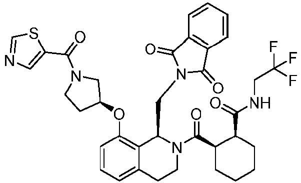 | (1*S*,2*R*)-2-((*S*)-1-((1,3-dioxoisoindolin-2-yl)methyl)-8-(((*S*)-1-(thiazole-5-carbonyl)pyrrolidin-3-yl)oxy)-1,2,3,4-tetrahydroisoquinoline-2-carbonyl)-*N*-(2,2,2-trifluoroethyl)cyclohexane-1-carboxamide | 6 | ¹H NMR (400 MHz, DMSO-d₆; rotamers observed, both reported) δ 9.25 (s, 0.6H), 9.23 (s, 0.4H), 8.42 (s, 0.6H), 8.38 (s, 0.4H), 7.89-7.72 (m, 5H), 7.28-7.16 (m, 1H), 6.99-6.89 (m, 1H), 6.86-6.75 (m, 1H), 5.97 (m, 0.4H), 5.91 (m, 0.6H), 5.21 (m, 1H), 4.38-3.43 (m, 10H), 3.21 (m, 1H), 2.89 (m, 1H), 2.76 (m, 1H), 2.55-2.22 (m, 2H), 2.16 (m, 1H), 1.94 (m, 1H), 1.62-1.41 (m, 2H), 1.37-1.21 (m, 2H), 0.97 (m, 1H), 0.76 (m, 1H), 0.25 (m, 1H). | Method 3: 4.60 min, 724.2 [M+H]⁺ |
| **Ref. Ex. 33** | 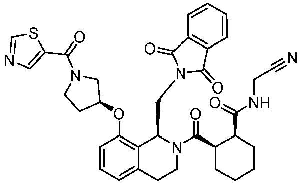 | (1*S*,2*R*)-*N*-(cyanomethyl)-2-((S)-1-((1,3-dioxoisoindolin-2-yl)methyl)-8-(((*S*)-1-(thiazole-5-carbonyl)pyrrolidin-3-yl)oxy)-1,2,3,4-tetrahydroisoquinoline-2-carbonyl)cyclohexane-1-carboxamide | 6 | ¹H NMR (400 MHz, DMSO-d₆; rotamers observed, both reported) δ 9.26 (s, 0.6H), 9.23 (s, 0.4H), 8.42 (s, 0.6H), 8.39 (s, 0.4H), 7.98-7.88 (m, 1H), 7.85-7.76 (m, 4H), 7.22 (m, 1H), 6.94 (m, 1H), 6.82 (m, 1H), 5.96 (m, 0.4H), 5.90 (m, 0.6H), 5.27-5.14 (m, 1H), 4.32 (dd, 0.6H), 4.22-3.56 (m, 9.4H), 3.22-3.20 (m, 1H), 2.94 (m, 1H), 2.77 (m, 1H), 2.57-2.24 (m, 2H), 2.15 (m, 1H), 1.89 (m, 1H), 1.60-1.42 (m, 2H), 1.36-1.22 (m, 2H), 0.95 (m, 1H), 0.74 (m, 1H), 0.17 (m, 1H). | Method 3: 4.23 min, 681.2 [M+H]⁺ |
| **Ref. Ex. 34** | 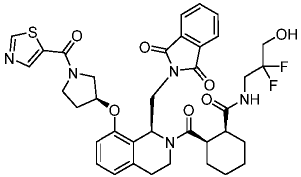 | (1*S*,2*R*)-*N*-(2,2-difluoro-3-hydroxypropyl)-2-((*S*)-1-((1,3-dioxoisoindolin-2-yl)methyl)-8-(((*S*)-1-(thiazole-5-carbonyl)pyrrolidin-3-yl)oxy)-1,2,3,4-tetrahydroisoquinoline-2-carbonyl)cyclohexane-1-carboxamide | 6 | ¹H NMR (400 MHz, DMSO-d₆; rotamers observed, both reported) δ 9.25 (s, 0.6H), 9.23 (s, 0.4H), 8.42 (s, 0.6H), 8.38 (s, 0.4H), 7.85-7.76 (m, 4H), 7.56 (t, 1H), 7.21 (m, 1H), 6.94 (m, 1H), 6.80 (m, 1H), 6.02-5.88 (m, 1H), 5.22 (m, 1H), 5.04 (m, 1H), 4.32 (dd, 0.6H), 4.23-3.71 (m, 6.4H), 3.71-3.55 (m, 1H), 3.42-3.18 (m, 5H), 2.89 (m, 1H), 2.76 (m, 1H), 2.57-2.25 (m, 2H), 2.16 (m, 1H), 1.93 (m, 1H), 1.58-1.41 (m, 2H), 1.36-1.22 (m, 2H), 0.97 (m, 1H), 0.76 (m, 1H), 0.29 (m, 1H). | Method 3: 4.27 min, 736.2 [M+H]⁺ |
| **Ref. Ex. 35** | 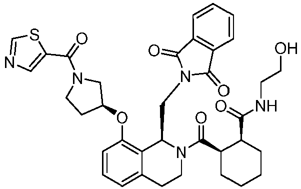 | (1*S*,2*R*)-2-((*S*)-1-((1,3-dioxoisoindolin-2-yl)methyl)-8-(((*S*)-1-(thiazole-5-carbonyl)pyrrolidin-3-yl)oxy)-1,2,3,4-tetrahydroisoquinoline-2-carbonyl)-N-(2-hydroxyethyl)cyclohexane -1-carboxamide | 6 | ¹H NMR (400 MHz, DMSO-d₆; rotamers observed, both reported) δ 9.25 (s, 0.6H), 9.22 (s, 0.4H), 8.44 (s, 0.6H), 8.39 (s, 0.4H), 7.84-7.75 (m, 4H), 7.22 (m, 1H), 7.08 (m, 1H), 6.94 (m, 1H), 6.81 (m, 1H), 5.98 (m, 0.4H), 5.92 (m, 0.6H), 5.21 (m, 1H), 4.39 (m, 1H), 4.33 (dd, 0.6H), 4.23-3.69 (m, 6.4H), 3.62 (m, 1H), 3.23 (m, 1H), 3.18-3.09 (m, 2H), 3.01-2.88 (m, 2H), 2.82 (m, 1H), 2.73 (m, 1H), 2.58-2.22 (m, 2H), 2.06 (m, 1H), 1.92 (m, 1H), 1.55-1.37 (m, 2H), 1.33-1.20 (m, 2H), 0.93 (m, 1H), 0.71 (m, 1H), 0.23 (m, 1H). | Method 3: 3.87 min, 686.2 [M+H]⁺ |
| **Ref. Ex. 36** | 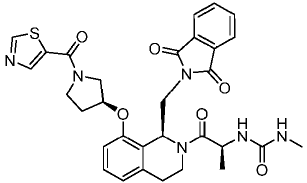 | 1-((*S*)-1-((*S*)-1-((1,3-dioxoisoindolin-2-yl)methyl)-8-(((*S*)-1-(thiazole-5-carbonyl)pyrrolidin-3-yl)oxy)-3,4-dihydroisoquinolin-2(1*H*)-yl)-1-oxopropan-2-yl)-3-methylurea | 67 | ¹H NMR (400 MHz, DMSO-d₆; rotamers observed, both reported) δ 9.26-9.22 (m, 1H), 8.42 (s, 0.5H), 8.38 (s, 0.5H), 7.88-7.75 (m, 4H), 7.30-7.17 (m, 1H), 6.99 (d, 0.5H), 6.95 (d, 0.5H), 6.88-6.77 (m, 1H), 6.00-5.82 (m, 2H), 5.62 (m, 1H), 5.25 (m, 1H), 4.46 (m, 1H), 4.35-4.22 (m, 1H), 4.21-3.56 (m, 7H), 2.96-2.75 (m, 2H), 2.57-2.24 (m, 5H), 0.77-0.68 (m, 3H). | Method 3: 3.52 min, 617.1 [M+H]⁺ |
| **Ref. Ex. 37** | 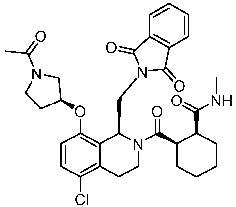 | (1*S,*2*R*)-2-((*S*)-8-(((*S*)-1-acetylpyrrolidin-3-yl)oxy)-5-chloro-1-((1,3-dioxoisoindolin-2-yl)methyl)-1,2,3,4-tetrahydroisoquinoline-2-carbonyl)-*N-*methylcyclohexane-1-carboxamide | 6 | ¹H NMR (400 MHz, DMSO-d₆; rotamers observed, both reported) δ 7.88-7.78 (m, 4H), 7.39 (d, 0.4H), 7.37 (d, 0.6H), 7.19 (m, 0.6H), 7.13 (m, 0.4H), 7.02-6.96 (m, 1H), 5.97-5.89 (m, 1H), 5.20-5.15 (m, 0.6H), 5.15-5.10 (m, 0.4H), 4.08 (m, 1H), 4.01-3.54 (m, 7H), 3.27 (m, 1H), 2.90-2.70 (m, 2H), 2.55-2.26 (m, 4.4H), 2.19 (m, 0.6H), 2.13-1.81 (m, 5H), 1.52 (m, 1H), 1.41 (m, 1H), 1.34-1.21 (m, 2H), 0.93 (m, 1H), 0.69 (m, 1H), 0.31-0.08 (m, 1H). | Method 3: 4.40 min, 621.3 [M+H]⁺ |
| **Ref. Ex. 40** | 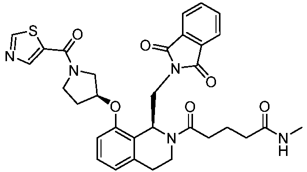 | 5-((*S*)-1-((1,3-dioxoisoindolin-2-yl)methyl)-8-(((*S*)-1-(thiazole-5-carbonyl)pyrrolidin-3-yl)oxy)-3,4-dihydroisoquinolin-2(1*H*)-yl)-N-methyl-5-oxopentanamide | 6 | ¹H NMR (400 MHz, DMSO-d₆; rotamers observed, both reported) δ 9.26 (s, 0.5H), 9.24 (s, 0.5H), 8.41 (s, 0.5H), 8.39 (s, 0.5H), 7.89-7.75 (m, 4H), 7.49 (m, 1H), 7.28-7.18 (m, 1H), 7.00-6.91 (m, 1H), 6.87-6.78 (m, 1H), 5.93 (m, 0.5H), 5.88 (m, 0.5H), 5.23 (m, 1H), 4.35-4.16 (m, 1H), 4.13-3.97 (m, 2H), 3.96-3.73 (m, 4H), 3.65 (m, 1H), 2.92-2.77 (m, 2H), 2.58-2.23 (m, 5H), 2.10 (t, 2H), 1.81 (t, 2H), 1.46-1.17 (m, 2H). | Method 3: 3.52 min, 616.2 [M+H]⁺ |
| **Ref. Ex. 41** | 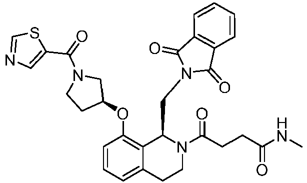 | 4-((S)-1-((1,3-dioxoisoindolin-2-yl)methyl)-8-(((*S*)-1-(thiazole-5-carbonyl)pyrrolidin-3-yl)oxy)-3,4-dihydroisoquinolin-2(1*H*)-yl)-*N*-methyl-4-oxobutanamide | 6 | ¹H NMR (400 MHz, DMSO-d₆; rotamers observed, both reported) δ 9.25 (s, 0.5H), 9.24 (s, 0.5H), 8.41 (s, 0.5H), 8.38 (s, 0.5H), 7.86-7.77 (m, 4H), 7.53 (m, 1H), 7.28-7.18 (m, 1H), 6.96 (d, 0.5H), 6.93 (d, 0.5H), 6.87-6.78 (m, 1H), 5.91 (dd, 0.5H), 5.85 (dd, 0.5H), 5.22 (m, 1H), 4.35-3.75 (m, 7H), 3.74-3.60 (m, 1H), 2.93-2.81 (m, 2H), 2.49-2.19 (m, 7H), 2.10-1.84 (m, 2H). | Method 3: 3.49 min, 602.2 [M+H]⁺ |
| **Ref. Ex. 42** | 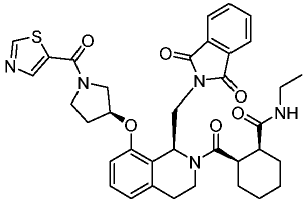 | (1*S*,2*R*)-2-((*S*)-1-((1,3-dioxoisoindolin-2-yl)methyl)-8-(((*S*)-1-(thiazole-5-carbonyl)pyrrolidin-3-yl)oxy)-1,2,3,4-tetrahydroisoquinoline-2-carbonyl)-*N-*ethylcyclohexane-1-carboxamide | 6 | ¹H NMR (400 MHz, DMSO-d₆; rotamers observed, both reported) δ 9.25 (s, 0.6H), 9.23 (s, 0.4H), 8.42 (s, 0.6H), 8.39 (s, 0.4H), 7.84-7.76 (m, 4H), 7.26-7.16 (m, 1H), 7.13-7.02 (m, 1H), 6.95 (d, 0.4H), 6.92 (d, 0.6H), 6.86-6.85 (m, 1H), 5.97 (dd, 0.4H), 5.91 (dd, 0.6H), 5.21 (m, 1H), 4.31 (dd, 0.6H), 4.24-3.69 (m, 6.4H), 3.62 (m, 1H), 3.25-3.19 (m, 1H), 2.94 (m, 1H), 2.85-2.69 (m, 3H), 2.57-2.23 (m, 2H), 2.01 (m, 1H), 1.91 (m, 1H), 1.50-1.38 (m, 2H), 1.34-1.28 (m, 2H), 0.92 (m, 1H), 0.76-0.60 (m, 4H), 0.24 (m, 1H). | Method 3: 4.23 min, 670.3 [M+H]⁺ |
| **Ref. Ex. 43** | 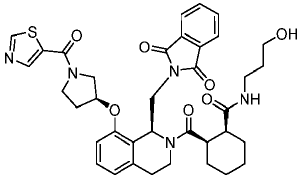 | (1*S*,2*R*)*-*2-((*S*)-1-((1,3-dioxoisoindolin-2-yl)methyl)-8-(((*S*)-1-(thiazole-5-carbonyl)pyrrolidin-3-yl)oxy)-1,2,3,4-tetrahydroisoquinoline-2-carbonyl)-*N*-(3-hydroxypropyl)cyclohexan e-1-carboxamide | 6 | ¹H NMR (400 MHz, DMSO-d₆; rotamers observed, both reported) δ 9.25 (s, 0.6H), 9.23 (s, 0.4H), 8.43 (s, 0.6H), 8.39 (s, 0.4H), 7.85-7.73 (m, 4H), 7.27-7.16 (m, 1H), 7.11 (m, 1H), 6.99-6.88 (m, 1H), 6.85-6.76 (m, 1H), 5.97 (m, 0.4H), 5.92 (m, 0.6H), 5.21 (m, 1H), 4.32 (m, 0.6H), 4.27-3.53 (m, 7.4H), 3.23 (m, 1H), 3.18-3.06 (m, 2H), 3.00-2.20 (m, 6H), 2.04 (m, 1H), 1.92 (m, 1H), 1.56-1.36 (m, 2H), 1.35-1.14 (m, 4H), 0.93 (m, 1H), 0.71 (m, 1H), 0.22 (m, 1H). | Method 3: 3.91 min, 700.3 [M+H]⁺ |
| **Ref. Ex. 47** | 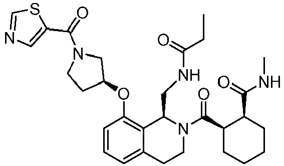 | (1S,2R)-N-methyl-2-((*S*)-1-(propionamidomethyl)-8-(((S)-1-(thiazole-5-carbonyl)pyrrolidin-3-yl)oxy)-1,2,3,4-tetrahydroisoquinoline-2-carbonyl)cyclohexane-1-carboxamide | 38 | ¹H NMR (400 MHz, DMSO-d₆; rotamers observed, all reported) δ 9.26 (s, 0.3H), 9.24 (s, 0.3H), 9.23 (s, 0.2H), 9.18 (s, 0.2H), 8.46-8.37 (m, 0.8H), 8.28 (s, 0.2H), 7.99-7.90 (m, 0.6H), 7.80 (m, 0.2H), 7.73 (m, 0.2H), 7.42 (m, 0.4H), 7.28-7.09 (m, 1.6H), 7.00-6.69 (m, 2H), 5.76-5.64 (m, 0.5H), 5.30 (m, 0.2H), 5.26 (m, 0.3H), 5.19-5.09 (m, 0.5H), 5.02 (m, 0.3H), 4.97 (m, 0.2H), 4.42-2.96 (m, 9H), 2.96-1.90 (m, 11H), 1.90-0.75 (m, 10H). | Method 3: 3.23 min, 582.3 [M+H]⁺ |
| **Ref. Ex. 49** | 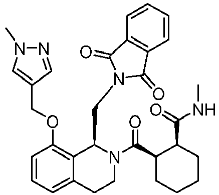 | (1*S*,2*R*)-2-((*S*)-1-((1,3-dioxoisoindolin-2-yl)methyl)-8-((1-methyl-1*H*-pyrazol-4-yl)methoxy)-1,2,3,4-tetrahydroisoquinoline-2-carbonyl)-*N-*methylcyclohexane-1-carboxamide | 46 | ¹H NMR (400 MHz, DMSO-d₆) δ 7.99 (m, 1H), 7.88-7.75 (m, 4H), 7.64 (m, 1H), 7.20 (t, 1H), 7.14 (m, 1H), 7.02 (d, 1H), 6.80 (d, 1H), 6.03 (dd, 1H), 5.14 (d, 1H), 5.06 (d, 1H), 4.12 (dd, 1H), 3.84 (m, 4H), 3.72 (dd, 1H), 3.63 (m, 1H), 3.24 (m, 1H), 2.96 (m, 1H), 2.78 (m, 1H), 2.35 (d, 3H), 2.03 (m, 1H), 1.93 (m, 1H), 1.53 (m, 1H), 1.44-1.15 (m, 3H), 0.92 (m, 1H), 0.61 (m, 1H), 0.18 (m, 1H). | Method 3: 4.23 min, 570.3 [M+H]⁺ |
| **Ref. Ex. 50** | 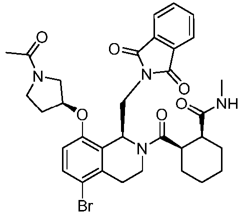 | (1*S*,2*R*)-2-((*S*)-8-(((*S*)-1-acetylpyrrolidin-3-yl)oxy)-5-bromo-1-((1,3-dioxoisoindolin-2-yl)methyl)-1,2,3,4-tetrahydroisoquinoline-2-carbonyl)-*N-*methylcyclohexane-1-carboxamide | 6 | ¹H NMR (400 MHz, DMSO-d₆; rotamers observed, both reported) δ 7.89-7.76 (m, 4H), 7.58-7.50 (m, 1H), 7.19 (m, 0.6H), 7.13 (m, 0.4H), 6.98-6.90 (m, 1H), 5.99-5.88 (m, 1H), 5.18 (m, 0.6H), 5.13 (m, 0.4H), 4.12-4.03 (m, 1H), 4.02-3.53 (m, 7H), 3.49-3.19 (m, 1H), 2.88-2.64 (m, 2H), 2.58-2.14 (m, 5H), 2.13-1.79 (m, 5H), 1.52 (m, 1H), 1.46-1.35 (m, 1H), 1.35-1.18 (m, 2H), 0.93 (m, 1H), 0.69 (m, 1H), 0.20 (m, 1H). | Method 3: 4.45 min, 665.3 [M+H]⁺ |
| **Ref. Ex. 51** | 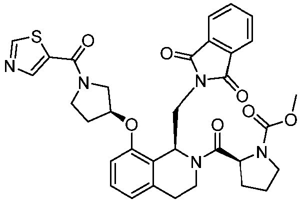 | methyl (*S*)-2-((*S*)-1-((1,3-dioxoisoindolin-2-yl)methyl)-8-(((S)-1-(thiazole-5-carbonyl)pyrrolidin-3-yl)oxy)-1,2,3,4-tetrahydroisoquinoline-2-carbonyl)pyrrolidine-1-carboxylate | 67 | ¹H NMR (400 MHz, CDCl₃; rotamers observed, all reported) δ 8.94-8.84 (m, 1H), 8.43 (s, 0.1H), 8.41 (s, 0.4H), 8.38 (s, 0.2H), 8.35 (s, 0.3H), 7.90-7.59 (m, 4H), 7.28-7.11 (m, 1H), 6.87-6.62 (m, 2H), 6.19-5.36 (m, 1H), 5.23-5.01 (m, 1H), 4.65-3.71 (m, 9H), 3.51 (s, 1.8H), 3.46-3.35 (m, 1.2H), 3.34-2.49 (m, 5H), 2.47-0.68 (m, 5H). | Method 3: 4.00 min, 644.4 [M+H]⁺ |
| **Ref. Ex. 52** | 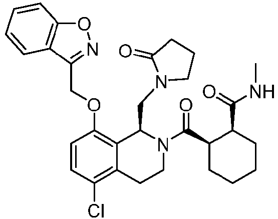 | (1S,2R)-2-((S)-8-(benzo[*d*]isoxazol-3-ylmethoxy)-5-chloro-1-((2-oxopyrrolidin-1-yl)methyl)-1,2,3,4-tetrahydroisoquinoline-2-carbonyl)-*N-*methylcyclohexane-1-carboxamide | 98 | ¹H NMR (400 MHz, DMSO-d₆) δ 8.05 (m, 1H), 7.83 (m, 1H), 7.72 (m, 1H), 7.47 (m, 1H), 7.43 (d, 1H), 7.26 (m, 1H), 7.20 (d, 1H), 5.85 (dd, 1H), 5.71 (d, 1H), 5.62 (d, 1H), 3.94 (dd, 1H), 3.86 (dd, 1H), 3.68-3.51 (m, 1H), 3.38-3.29 (m, 1H), 3.25 (m, 1H), 2.98 (dd, 1H), 2.89-2.70 (m, 2H), 2.59 (m, 1H), 2.42 (d, 3H), 2.24-2.04 (m, 3H), 1.95 (m, 1H), 1.77-1.59 (m, 5H), 1.51-1.31 (m, 2H), 1.29-1.07 (m, 2H). | Method 3: 4.33 min, 579.1 [M+H]⁺ |
| **Ref. Ex. 53** | 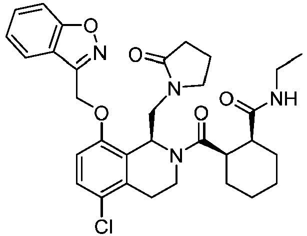 | (1*S*,2*R*)-2-((*S*)-8-(benzo[*d*]isoxazol-3-ylmethoxy)-5-chloro-1-((2-oxopyrrolidin-1-yl)methyl)-1,2,3,4-tetrahydroisoquinoline-2-carbonyl)-*N-*ethylcyclohexane-1-carboxamide | 98 | ¹H NMR (400 MHz, DMSO-d₆) δ 8.04 (m, 1H), 7.83 (m, 1H), 7.72 (m, 1H), 7.47 (m, 1H), 7.42 (d, 1H), 7.26 (t, 1H), 7.19 (d, 1H), 5.87 (dd, 1H), 5.71 (d, 1H), 5.62 (d, 1H), 3.98-3.80 (m, 2H), 3.60 (m, 1H), 3.36-3.22 (m, 2H), 3.02-2.72 (m, 5H), 2.60 (m, 1H), 2.24-2.04 (m, 3H), 1.96 (m, 1H), 1.75-1.60 (m, 5H), 1.51-1.33 (m, 2H), 1.32-1.10 (m, 2H), 0.79 (t, 3H). | Method 3: 4.56 min, 593.1 [M+H]⁺ |
| **54 (Ref. Cpd)** | 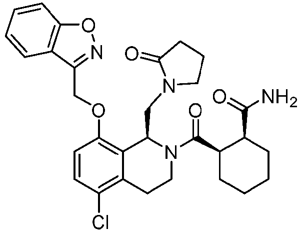 | (1*S*,2*R*)-2-((*S*)-8-(benzo[*d*]isoxazol-3-ylmethoxy)-5-chloro-1-((2-oxopyrrolidin-1-yl)methyl)-1,2,3,4-tetrahydroisoquinoline-2-carbonyl)cyclohexane-1-carboxamide | 98 | ¹H NMR (400 MHz, DMSO-d₆) δ 8.06 (m, 1H), 7.83 (m, 1H), 7.72 (m, 1H), 7.48 (m, 1H), 7.41 (d, 1H), 7.18 (d, 1H), 6.85 (bs, 1H), 6.55 (bs, 1H), 5.87 (dd, 1H), 5.70 (d, 1H), 5.61 (d, 1H), 3.94 (m, 1H), 3.86 (dd, 1H), 3.59 (m, 1H), 3.37 (m, 1H), 3.24 (m, 1H), 2.97 (dd, 1H), 2.90-2.75 (m, 2H), 2.58 (m, 1H), 2.27-2.04 (m, 3H), 1.94 (m, 1H), 1.75-1.59 (m, 5H), 1.52-1.33 (m, 2H), 1.28-1.11 (m, 2H). | Method 3: 4.18 min, 565.1 [M+H]⁺ |
| **Ref. Ex. 55** | 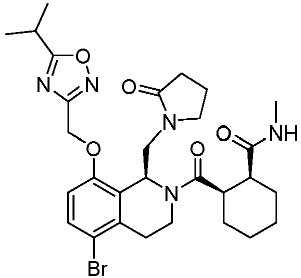 | (1*S*,2*R*)-2-((*S*)-5-bromo-8-((5-isopropyl-1,2,4-oxadiazol-3-yl)methoxy)-1-((2-oxopyrrolidin-1-yl)methyl)-1,2,3,4-tetrahydroisoquinoline-2-carbonyl)-*N-*methylcyclohexane-1-carboxamide | 98 | ¹H NMR (400 MHz, DMSO-d₆) δ 7.55 (d, 1H), 7.26 (m, 1H), 7.06 (d, 1H), 5.86 (dd, 1H), 5.36 (d, 1H), 5.29 (d, 1H), 4.00-3.83 (m, 2H), 3.65-3.53 (m, 2H), 3.39-3.33 (m, 2H), 3.15 (m, 1H), 3.07 (dd, 1H), 2.83-2.65 (m, 2H), 2.42 (d, 3H), 2.18 (m, 3H), 2.02 (m, 1H), 1.94-1.77 (m, 2H), 1.77-1.65 (m, 3H), 1.52-1.37 (m, 2H), 1.36-1.31 (m, 6H), 1.30-1.12 (m, 2H). | Method 3: 4.27 min, 616.2 [M+H]⁺ |
| **Ref. Ex. 56** | 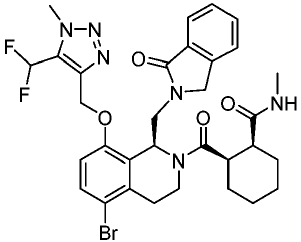 | (1*S*,2*R*)-2-((*S*)-5-bromo-8-((5-(difluoromethyl)-1-methyl-1*H*-1,2,3-triazol-4-yl)methoxy)-1-((1-oxoisoindolin-2-yl)methyl)-1,2,3,4-tetrahydroisoquinoline-2-carbonyl)-*N-*methylcyclohexane-1-carboxamide | 98 | ¹H NMR (400 MHz, DMSO-d₆) δ 7.77-7.49 (m, 5H), 7.43 (m, 1H), 7.21-7.13 (m, 2H), 5.90 (dd, 1H), 5.40-5.30 (m, 2H), 4.50 (d, 1H), 4.22 (s, 3H), 4.14 (dd, 1H), 4.03-3.92 (m, 2H), 3.68 (m, 1H), 3.43 (dd, 1H), 3.27 (m, 1H), 2.86-2.66 (m, 2H), 2.38 (d, 3H), 2.03 (m, 1H), 1.90 (m, 1H), 1.53 (m, 1H), 1.39-1.12 (m, 3H), 0.89 (m, 1H), 0.53 (m, 1H), 0.15 (m, 1H). | Method 3: 4.28 min, 687.2 [M+H]⁺ |
| **Ref. Ex. 57** | 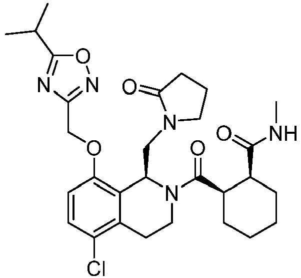 | (1*S*,2*R*)-2-((*S*)-5-chloro-8-((5-isopropyl-1,2,4-oxadiazol-3-yl)methoxy)-1-((2-oxopyrrolidin-1-yl)methyl)-1,2,3,4-tetrahydroisoquinoline-2-carbonyl)-*N-*methylcyclohexane-1-carboxamide | 98 | ¹H NMR (400 MHz, DMSO-d₆) δ 7.39 (d, 1H), 7.26 (m, 1H), 7.11 (d, 1H), 5.86 (dd, 1H), 5.36 (d, 1H), 5.29 (d, 1H), 3.96 (dd, 1H), 3.88 (dd, 1H), 3.65-3.52 (m, 2H), 3.41-3.25 (m, 2H), 3.21-3.11 (m, 1H), 3.08 (dd, 1H), 2.87-2.69 (m, 2H), 2.41 (d, 3H), 2.26-2.10 (m, 3H), 2.02 (m, 1H), 1.94-1.77 (m, 2H), 1.77-1.66 (m, 3H), 1.52-1.38 (m, 2H), 1.37-1.31 (m, 6H), 1.31-1.12 (m, 2H). | Method 3: 4.23 min, 572.4 [M+H]⁺ |
| **Ref. Ex. 58** | 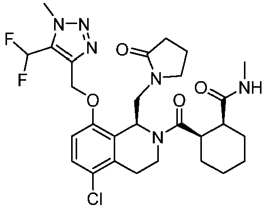 | (1*S*,2*R*)-2-((*S*)-5-chloro-8-((5-(difluoromethyl)-1-methyl-1*H*-1,2,3-triazol-4-yl)methoxy)-1-((2-oxopyrrolidin-1-yl)methyl)-1,2,3,4-tetrahydroisoquinoline-2-carbonyl)-*N-*methylcyclohexane-1-carboxamide | 98 | ¹H NMR (400 MHz, DMSO-d₆) δ 7.58 (t, 1H), 7.39 (d, 1H), 7.26 (m, 1H), 7.16 (d, 1H), 5.75 (dd, 1H), 5.29 (s, 2H), 4.18 (s, 3H), 3.93 (dd, 1H), 3.82 (dd, 1H), 3.56 (m, 1H), 3.43-3.26 (m, 2H), 2.95 (dd, 1H), 2.85 (m, 1H), 2.81-2.68 (m, 2H), 2.41 (d, 3H), 2.23-2.10 (m, 3H), 1.99 (m, 1H), 1.90-1.64 (m, 5H), 1.50-1.36 (m, 2H), 1.29-1.12 (m, 2H). | Method 3: 3.87 min, 615.3 [M+Na]⁺ |
| **Ref. Ex. 59** | 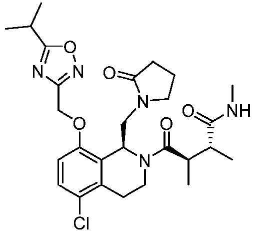 | (2*R*,3*R*)-4-((*S*)-5-chloro-8-((5-isopropyl-1,2,4-oxadiazol-3-yl)methoxy)-1-((2-oxopyrrolidin-1-yl)methyl)-3,4-dihydroisoquinolin-2(1*H*)-yl)-*N*,2,3-trimethyl-4-oxobutanamide | 98 | ¹H NMR (400 MHz, DMSO-d₆) δ 7.55 (m, 1H), 7.39 (d, 1H), 7.10 (d, 1H), 5.82 (dd, 1H), 5.36 (d, 1H), 5.29 (d, 1H), 4.03 (dd, 1H), 3.90 (dd, 1H), 3.66-3.52 (m, 2H), 3.38-3.25 (m, 1H), 3.18-3.06 (m, 2H), 2.97-2.69 (m, 3H), 2.43 (m, 1H), 2.36 (d, 3H), 2.16 (m, 1H), 1.98 (m, 1H), 1.92-1.69 (m, 2H), 1.36-1.30 (m, 6H), 1.00 (d, 3H), 0.93 (d, 3H). | Method 4: 3.13 min, 546.4 [M+H]⁺ |
| **Ref. Ex. 60** | 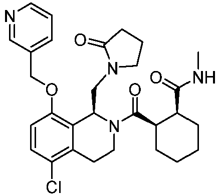 | (1*S*,2*R*)-2-((*S*)-5-chloro-1-((2-oxopyrrolidin-1-yl)methyl)-8-(pyridin-3-ylmethoxy)-1,2,3,4-tetrahydroisoquinoline-2-carbonyl)-*N-*methylcyclohexane-1-carboxamide | 98 | ¹H NMR (400 MHz, DMSO-d₆) δ 8.76 (d, 1H), 8.60 (dd, 1H), 7.97 (m, 1H), 7.49 (m, 1H), 7.40 (d, 1H), 7.27 (m, 1H), 7.09 (d, 1H), 5.84 (dd, 1H), 5.22 (d, 1H), 5.14 (d, 1H), 3.95 (dd, 1H), 3.87 (dd, 1H), 3.59 (m, 1H), 3.42-3.26 (m, 2H), 3.00 (dd, 1H), 2.88-2.69 (m, 3H), 2.41 (d, 3H), 2.26-2.08 (m, 3H), 1.99 (m, 1H), 1.87-1.62 (m, 5H), 1.52-1.32 (m, 2H), 1.29-1.10 (m, 2H). | Method 4: 2.69 min, 539.4 [M+H]⁺ |
| **Ref. Ex. 61** | 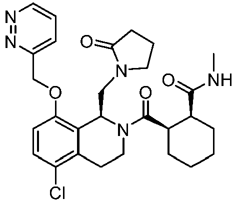 | (1*S*,2*R*)-2-((*S*)-5-chloro-1-((2-oxopyrrolidin-1-yl)methyl)-8-(pyridazin-3-ylmethoxy)-1,2,3,4-tetrahydroisoquinoline-2-carbonyl)-*N-*methylcyclohexane-1-carboxamide | 98 | ¹H NMR (400 MHz, DMSO-d₆) δ 9.27 (m, 1H), 7.94 (m, 1H), 7.84 (dd, 1H), 7.39 (d, 1H), 7.26 (m, 1H), 7.12 (d, 1H), 5.89 (m, 1H), 5.47 (d, 1H), 5.41 (d, 1H), 3.96 (dd, 1H), 3.86 (dd, 1H), 3.61 (m, 1H), 3.43 (m, 1H), 3.39-3.26 (m, 1H), 3.10 (dd, 1H), 2.94 (dd, 1H), 2.87-2.71 (m, 2H), 2.41 (d, 3H), 2.23-2.10 (m, 3H), 2.00 (m, 1H), 1.90-1.65 (m, 5H), 1.52-1.36 (m, 2H), 1.31-1.12 (m, 2H). | Method 3: 3.34 min, 562.2 [M+Na]⁺ |
| **Ref. Ex. 62** | 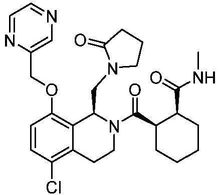 | (1*S*,2*R*)-2-((*S*)-5-chloro-1-((2-oxopyrrolidin-1-yl)methyl)-8-(pyrazin-2-ylmethoxy)-1,2,3,4-tetrahydroisoquinoline-2-carbonyl)-*N-*methylcyclohexane-1-carboxamide | 98 | ¹H NMR (400 MHz, DMSO-d₆) δ 8.92 (d, 1H), 8.72 (dd, 1H), 8.68 (d, 1H), 7.39 (d, 1H), 7.27 (m, 1H), 7.10 (d, 1H), 5.90 (dd, 1H), 5.33 (d, 1H), 5.28 (d, 1H), 3.96 (dd, 1H), 3.88 (dd, 1H), 3.61 (m, 1H), 3.45 (m, 1H), 3.39-3.26 (m, 1H), 3.11 (dd, 1H), 2.96 (m, 1H), 2.88-2.70 (m, 2H), 2.41 (d, 3H), 2.23-2.11 (m, 3H), 2.01 (m, 1H), 1.92-1.66 (m, 5H), 1.53-1.37 (m, 2H), 1.32-1.12 (m, 2H). | Method 3: 3.57 min, 562.2 [M+Na]⁺ |
| **Ref. Ex. 63** | 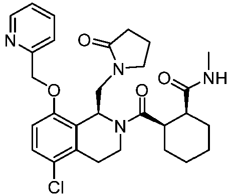 | (1*S*,2*R*)-2-((S)-5-chloro-1-((2-oxopyrrolidin-1-yl)methyl)-8-(pyridin-2-ylmethoxy)-1,2,3,4-tetrahydroisoquinoline-2-carbonyl)-*N-*methylcyclohexane-1-carboxamide | 98 | ¹H NMR (400 MHz, DMSO-d₆) δ 8.62 (m, 1H), 7.91 (m, 1H), 7.63 (m, 1H), 7.42-7.33 (m, 2H), 7.26 (m, 1H), 7.05 (d, 1H), 5.92 (dd, 1H), 5.27-5.16 (m, 2H), 3.96 (dd, 1H), 3.88 (dd, 1H), 3.61 (m, 1H), 3.49 (m, 1H), 3.38-3.29 (m, 1H), 3.13 (dd, 1H), 2.99 (m, 1H), 2.87-2.70 (m, 2H), 2.41 (d, 3H), 2.28-2.10 (m, 3H), 2.01 (m, 1H), 1.91-1.66 (m, 5H), 1.53-1.37 (m, 2H), 1.22 (m, 2H). | Method 3: 3.46 min, 561.3 [M+Na]⁺ |
| **Ref. Ex. 64** | 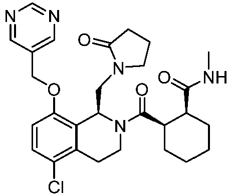 | (1*S*,2*R*)-2-((*S*)-5-chloro-1-((2-oxopyrrolidin-1-yl)methyl)-8-(pyrimidin-5-ylmethoxy)-1,2,3,4-tetrahydroisoquinoline-2-carbonyl)-*N-*methylcyclohexane-1-carboxamide | 98 | ¹H NMR (400 MHz, DMSO-d₆) δ 9.23 (s, 1H), 9.02 (s, 2H), 7.41 (d, 1H), 7.27 (m, 1H), 7.10 (d, 1H), 5.84 (dd, 1H), 5.26 (d, 1H), 5.17 (d, 1H), 3.96 (dd, 1H), 3.86 (dd, 1H), 3.60 (m, 1H), 3.41-3.25 (m, 2H), 3.01 (dd, 1H), 2.89-2.70 (m, 3H), 2.41 (d, 3H), 2.24-2.10 (m, 3H), 1.99 (m, 1H), 1.87-1.65 (m, 5H), 1.51-1.36 (m, 2H), 1.28-1.13 (m, 2H). | Method 3: 3.40 min,562.2 [M+Na]⁺ |
| **Ref. Ex. 65** | 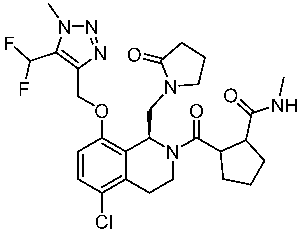 | 2-((*S*)-5-chloro-8-((5-(difluoromethyl)-1-methyl-1*H-*1,2,3-triazol-4-yl)methoxy)-1-((2-oxopyrrolidin-1-yl)methyl)-1,2,3,4-tetrahydroisoquinoline-2-carbonyl)-*N-*methylcyclopentane-1-carboxamide (isomer 1) | 98 | ¹H NMR (400 MHz, DMSO-d₆) δ 7.58 (t, 1H), 7.39 (d, 1H), 7.27 (m, 1H), 7.16 (d, 1H), 5.70 (dd, 1H), 5.29 (s, 2H), 4.17 (s, 3H), 3.96 (dd, 1H), 3.81 (dd, 1H), 3.57 (m, 1H), 3.44-3.24 (m, 2H), 2.92 (dd, 1H), 2.88-2.61 (m, 4H), 2.38 (d, 3H), 2.14 (m, 1H), 2.02-1.49 (m, 9H). | Method 3: 3.67 min, 601.3 [M+Na]⁺ |
| **Ref. Ex. 66** | 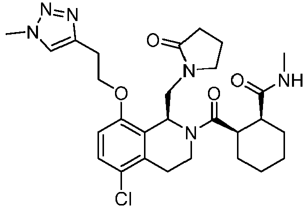 | (1*S*,2*R*)-2-((*S*)-5-chloro-8-(2-(1-methyl-1*H*-1,2,3-triazol-4-yl)ethoxy)-1-((2-oxopyrrolidin-1-yl)methyl)-1,2,3,4-tetrahydroisoquinoline-2-carbonyl)-*N-*methylcyclohexane-1-carboxamide | 98 | ¹H NMR (400 MHz, DMSO-d₆) δ 7.91 (s, 1H), 7.33 (d, 1H), 7.28 (m, 1H), 6.96 (d, 1H), 5.79 (dd, 1H), 4.38 (m, 1H), 4.22 (m, 1H), 4.00 (s, 3H), 3.93 (dd, 1H), 3.77 (dd, 1H), 3.58 (m, 1H), 3.47 (m, 1H), 3.41-3.24 (m, 1H), 3.16 (t, 2H), 3.08 (m, 1H), 2.90 (dd, 1H), 2.84-2.71 (m, 2H), 2.42 (d, 3H), 2.24-2.12 (m, 3H), 2.02 (m, 1H), 1.94-1.64 (m, 5H), 1.52-1.37 (m, 2H), 1.32-1.12 (m, 2H). | Method 3: 3.50 min, 579.3 [M+Na]⁺ |
| **Ref. Ex. 68** | 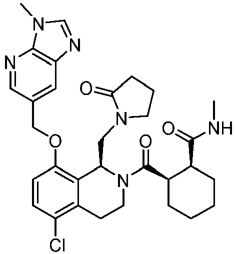 | (1*S*,2*R*)-2-((*S*)-5-chloro-8-((3-methyl-3*H-*imidazo[4,5-*b*]pyridin-6-yl)methoxy)-1-((2-oxopyrrolidin-1-yl)methyl)-1,2,3,4-tetrahydroisoquinoline-2-carbonyl)-*N-*methylcyclohexane-1-carboxamide | 98 | ¹H NMR (400 MHz, DMSO-d₆) δ 8.57 (d, 1H), 8.47 (s, 1H), 8.28 (d, 1H), 7.39 (d, 1H), 7.26 (m, 1H), 7.14 (d, 1H), 5.84 (dd, 1H), 5.34 (d, 1H), 5.26 (d, 1H), 3.95 (dd, 1H), 3.90-3.81 (m, 4H), 3.58 (m, 1H), 3.41-3.24 (m, 2H), 3.02 (dd, 1H), 2.86-2.69 (m, 3H), 2.41 (d, 3H), 2.21-2.07 (m, 3H), 2.01-1.91 (m, 1H), 1.77-1.61 (m, 5H), 1.50-1.35 (m, 2H), 1.27-1.11 (m, 2H). | Method 3: 3.34 min, 593.4 [M+H]⁺ |
| **69** | 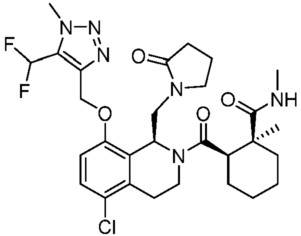 | (1*S*,2*R*)-2-((*S*)-5-chloro-8-((5-(difluoromethyl)-1-methyl-1*H*-1,2,3-triazol-4-yl)methoxy)-1-((2-oxopyrrolidin-1-yl)methyl)-1,2,3,4-tetrahydroisoquinoline-2-carbonyl)-*N*,1-dimethylcyclohexane-1-carboxamide | 98 | ¹H NMR (400 MHz, DMSO-d₆) δ 7.58 (t, 1H), 7.39 (d, 1H), 7.23 (bm, 1H), 7.16 (d, 1H), 5.72 (dd, 1H), 5.29 (s, 2H), 4.17 (s, 3H), 3.93 (m, 1H), 3.82 (dd, 1H), 3.54 (m, 1H), 3.42-3.26 (m, 1H), 3.01 (m, 1H), 2.95 (dd, 1H), 2.89-2.76 (m, 3H), 2.40 (d, 3H), 2.31 (m, 1H), 2.15 (m, 1H), 1.98 (m, 1H), 1.90-1.68 (m, 2H), 1.67-1.27 (m, 6H), 1.17-0.98 (m, 4H). | Method 4: 3.16 min, 607.4 [M+H]⁺ |
| **Ref. Ex. 70** | 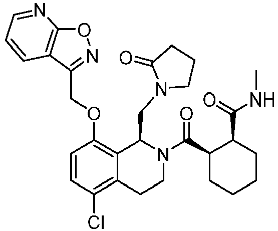 | (1*S*,2*R*)-2-((*S*)-5-chloro-8-(isoxazolo[5,4-b]pyridin-3-ylmethoxy)-1-((2-oxopyrrolidin-1-yl)methyl)-1,2,3,4-tetrahydroisoquinoline-2-carbonyl)-*N-*methylcyclohexane-1-carboxamide | 98 | ¹H NMR (400 MHz, DMSO-d₆) δ 8.74 (dd, 1H), 8.57 (dd, 1H), 7.59 (dd, 1H), 7.43 (d, 1H), 7.27 (m, 1H), 7.18 (d, 1H), 5.87 (dd, 1H), 5.71 (d, 1H), 5.63 (d, 1H), 3.95 (dd, 1H), 3.86 (dd, 1H), 3.60 (m, 1H), 3.42-3.22 (m, 2H), 3.00 (dd, 1H), 2.88-2.63 (m, 3H), 2.42 (d, 3H), 2.27-2.04 (m, 3H), 1.96 (m, 1H), 1.81-1.57 (m, 5H), 1.53-1.32 (m, 2H), 1.31-1.08 (m, 2H). | Method 4: 3.11 min, 580.4 [M+H]⁺ |
| **Ref. Ex. 71** | 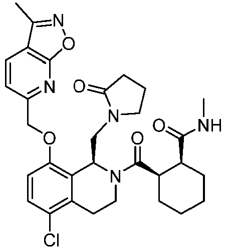 | (1*S*,2*R*)-2-((*S*)-5-chloro-8-((3-methylisoxazolo[5,4-*b*]pyridin-6-yl)methoxy)-1-((2-oxopyrrolidin-1-yl)methyl)-1,2,3,4-tetrahydroisoquinoline-2-carbonyl)-*N-*methylcyclohexane-1-carboxamide | 98 | ¹H NMR (400 MHz, DMSO-d₆) δ 8.53 (d, 1H), 7.71 (d, 1H), 7.37 (d, 1H), 7.27 (m, 1H), 7.05 (d, 1H), 5.98 (dd, 1H), 5.42 (d, 1H), 5.36 (d, 1H), 4.03-3.85 (m, 2H), 3.70-3.51 (m, 2H), 3.40-3.29 (m, 1H), 3.22 (dd, 1H), 3.13 (m, 1H), 2.89-2.72 (m, 2H), 2.59 (s, 3H), 2.41 (d, 3H), 2.25-2.13 (m, 3H), 2.03 (m, 1H), 1.93-1.67 (m, 5H), 1.54-1.38 (m, 2H), 1.33-1.12 (m, 2H). | Method 4: 3.19 min, 594.4 [M+H]⁺ |
| **Ref. Ex. 72** | 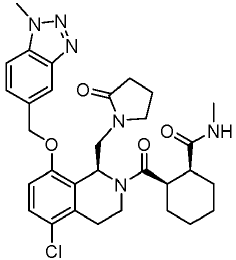 | (1*S*,2*R*)-2-((*S*)-5-chloro-8-((1-methyl-1*H-*benzo[d][1,2,3]triazol-5-yl)methoxy)-1-((2-oxopyrrolidin-1-yl)methyl)-1,2,3,4-tetrahydroisoquinoline-2-carbonyl)-*N-*methylcyclohexane-1-carboxamide | 98 | ¹H NMR (400 MHz, DMSO-d₆) δ 8.20 (m, 1H), 7.93 (m, 1H), 7.73 (m, 1H), 7.39 (d, 1H), 7.27 (m, 1H), 7.11 (d, 1H), 5.90 (dd, 1H), 5.35 (d, 1H), 5.27 (d, 1H), 4.33 (s, 3H), 4.00-3.83 (m, 2H), 3.61 (m, 1H), 3.41 (m, 1H), 3.37-3.26 (m, 1H), 3.07 (dd, 1H), 2.87 (m, 1H), 2.83-2.70 (m, 2H), 2.41 (d, 3H), 2.25-2.08 (m, 3H), 1.98 (m, 1H), 1.82-1.62 (m, 5H), 1.53-1.35 (m, 2H), 1.29-1.11 (m, 2H). | Method 4: 3.08 min, 593.4 [M+H]⁺ |
| **Ref. Ex. 73** | 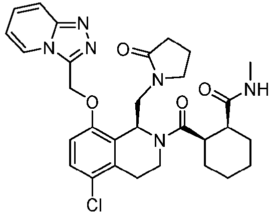 | (1*S*,2*R*)-2-((*S*)-8-([1,2,4]triazolo[4,3-*a*]pyridin-3-ylmethoxy)-5-chloro-1-((2-oxopyrrolidin-1-yl)methyl)-1,2,3,4-tetrahydroisoquinoline-2-carbonyl)-*N-*methylcyclohexane-1-carboxamide | 98 | ¹H NMR (400 MHz, DMSO-d₆) δ 8.71 (m, 1H), 7.87 (m, 1H), 7.48 (m, 1H), 7.44 (d, 1H), 7.30-7.22 (m, 2H), 7.12 (m, 1H), 5.81-5.66 (m, 3H), 3.94 (dd, 1H), 3.80 (dd, 1H), 3.54 (m, 1H), 3.39-3.25 (m, 1H), 3.07 (m, 1H), 2.85-2.68 (m, 3H), 2.41 (d, 3H), 2.34 (m, 1H), 2.21-2.02 (m, 3H), 1.92 (m, 1H), 1.75-1.52 (m, 5H), 1.48-1.32 (m, 2H), 1.26-1.10 (m, 2H). | Method 4: 2.79 min, 579.4 [M+H]⁺ |
| **Ref. Ex. 74** | 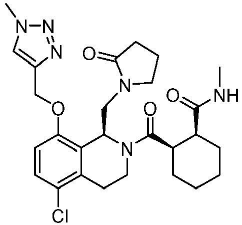 | (1*S*,2*R*)-2-((*S*)-5-chloro-8-((1-methyl-1*H*-1,2,3-triazol-4-yl)methoxy)-1-((2-oxopyrrolidin-1-yl)methyl)-1,2,3,4-tetrahydroisoquinoline-2-carbonyl)-*N-*methylcyclohexane-1-carboxamide | 98 | ¹H NMR (400 MHz, DMSO-d₆) δ 8.24 (s, 1H), 7.36 (d, 1H), 7.25 (m, 1H), 7.13 (d, 1H), 5.78 (dd, 1H), 5.22 (d, 1H), 5.19 (d, 1H), 4.07 (s, 3H), 3.94 (dd, 1H), 3.81 (dd, 1H), 3.56 (m, 1H), 3.47-3.26 (m, 2H), 3.04-2.92 (m, 2H), 2.81-2.69 (m, 2H), 2.41 (d, 3H), 2.23-2.10 (m, 3H), 2.00 (m, 1H), 1.91-1.65 (m, 5H), 1.51-1.36 (m, 2H), 1.30-1.12 (m, 2H). | Method 3: 3.41 min, 565.1 [M+Na]⁺ |
| **Ref. Ex. 75** | 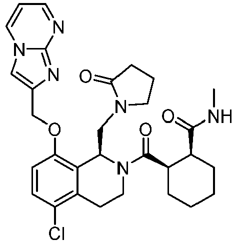 | (1*S*,2*R*)-2-((*S*)-5-chloro-8-(imidazo[1,2-*a*]pyrimidin-2-ylmethoxy)-1-((2-oxopyrrolidin-1-yl)methyl)-1,2,3,4-tetrahydroisoquinoline-2-carbonyl)-*N-*methylcyclohexane-1-carboxamide | 98 | ¹H NMR (400 MHz, DMSO-d₆) δ 9.01 (dd, 1H), 8.55 (dd, 1H), 8.04 (s, 1H), 7.36 (d, 1H), 7.26 (m, 1H), 7.14 (d, 1H), 7.07 (dd, 1H), 5.89 (dd, 1H), 5.31 (s, 2H), 3.95 (dd, 1H), 3.85 (dd, 1H), 3.59 (m, 1H), 3.48 (m, 1H), 3.39-3.25 (m, 1H), 3.19 (dd, 1H), 3.05 (m, 1H), 2.85-2.69 (m, 2H), 2.41 (d, 3H), 2.22-2.10 (m, 3H), 2.00 (m, 1H), 1.85-1.66 (m, 5H), 1.52-1.36 (m, 2H), 1.32-1.12 (m, 2H). | Method 3: 3.07 min, 601.2 [M+Na]⁺ |
| **Ref. Ex. 76** | 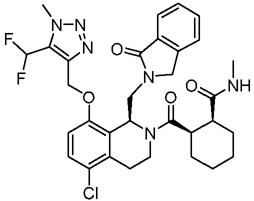 | (1*S*,2*R*)-2-((*S*)-5-chloro-8-((5-(difluoromethyl)-1-methyl-1*H*-1,2,3-triazol-4-yl)methoxy)-1-((1-oxoisoindolin-2-yl)methyl)-1,2,3,4-tetrahydroisoquinoline-2-carbonyl)-*N-*methylcyclohexane-1-carboxamide | 98 | ¹H NMR (400 MHz, DMSO-d₆) δ 7.77-7.48 (m, 4H), 7.48-7.40 (m, 2H), 7.24-7.15 (m, 2H), 5.90 (dd, 1H), 5.40-5.31 (m, 2H), 4.50 (d, 1H), 4.21 (s, 3H), 4.14 (dd, 1H), 4.04-3.93 (m, 2H), 3.68 (m, 1H), 3.44 (dd, 1H), 3.39-3.23 (m, 1H), 2.83 (dd, 1H), 2.75 (m, 1H), 2.38 (d, 3H), 2.03 (m, 1H), 1.91 (m, 1H), 1.53 (m, 1H), 1.38-1.12 (m, 3H), 0.89 (m, 1H), 0.52 (m, 1H), 0.14 (m, 1H). | Method 3: 4.35 min, 663.3 [M+Na]⁺ |
| **Ref. Ex. 77** | 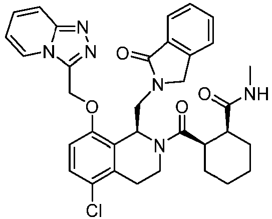 | (1*S*,2*R*)-2-((*S*)-8-([1,2,4]triazolo[4,3-*a*]pyridin-3-ylmethoxy)-5-chloro-1-((1-oxoisoindolin-2-yl)methyl)-1,2,3,4-tetrahydroisoquinoline-2-carbonyl)-*N-*methylcyclohexane-1-carboxamide | 98 | ¹H NMR (400 MHz, DMSO-d₆) δ 8.80 (m, 1H), 7.93 (m, 1H), 7.59-7.48 (m, 3H), 7.46 (d, 1H), 7.41 (t, 1H), 7.37 (d, 1H), 7.32 (d, 1H), 7.21-7.10 (m, 2H), 5.95-5.75 (m, 3H), 4.22 (d, 1H), 4.12 (dd, 1H), 3.98 (dd, 1H), 3.67 (m, 1H), 3.52 (d, 1H), 3.40-3.20 (m, 2H), 2.86 (dd, 1H), 2.75 (m, 1H), 2.38 (d, 3H), 2.03 (m, 1H), 1.89 (m, 1H), 1.52 (m, 1H), 1.36 (m, 1H), 1.30-1.10 (m, 2H), 0.89 (m, 1H), 0.54 (m, 1H), 0.14 (m, 1H). | Method 3: 3.72 min, 649.3 [M+Na]⁺ |
| **Ref. Ex. 78** | 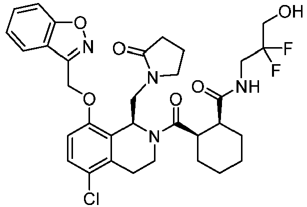 | (1*S*,2*R*)-2-((*S*)-8-(benzo[d]isoxazol-3-ylmethoxy)-5-chloro-1-((2-oxopyrrolidin-1-yl)methyl)-1,2,3,4-tetrahydroisoquinoline-2-carbonyl)-*N*-(2,2-difluoro-3-hydroxypropyl)cyclohexan e-1-carboxamide | 98 | ¹H NMR (400 MHz, DMSO-d₆) δ 8.03 (d, 1H), 7.82 (m, 2H), 7.72 (m, 1H), 7.46 (t, 1H), 7.41 (d, 1H), 7.18 (d, 1H), 5.86 (dd, 1H), 5.71 (d, 1H), 5.61 (d, 1H), 5.12 (t, 1H), 3.95-3.78 (m, 2H), 3.67-3.17 (m, 7H), 2.97 (dd, 1H), 2.88-2.70 (m, 2H), 2.59 (m, 1H), 2.35 (m, 1H), 2.17-2.02 (m, 2H), 1.96 (m, 1H), 1.84 (m, 1H), 1.75-1.58 (m, 4H), 1.55-1.13 (m, 4H). | Method 3: 4.64 min, 681.3 [M+Na]⁺ |
| **Ref. Ex. 79** | 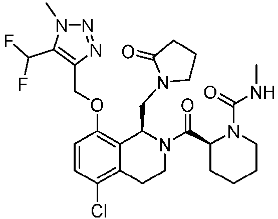 | (*S*)-2-((*S*)-5-chloro-8-((5-(difluoromethyl)-1-methyl-*1H-*1,2,3-triazol-4-yl)methoxy)-1-((2-oxopyrrolidin-1-yl)methyl)-1,2,3,4-tetrahydroisoquinoline-2-carbonyl)-*N-*methylpiperidine-1-carboxamide | 67 | ¹H NMR (400 MHz, DMSO-d₆) δ 7.58 (t, 1H), 7.40 (d, 1H), 7.16 (d, 1H), 6.35 (m, 1H), 5.71 (dd, 1H), 5.35-5.22 (m, 2H), 5.04 (m, 1H), 4.17 (s, 3H), 3.93 (dd, 1H), 3.84 (dd, 1H), 3.58 (m, 1H), 3.48 (m, 1H), 3.42-3.21 (m, 2H), 2.97 (dd, 1H), 2.87 (m, 1H), 2.82-2.65 (m, 2H), 2.47 (d, 3H), 2.18 (m, 1H), 2.00 (m, 1H), 1.92-1.72 (m, 2H), 1.72-1.45 (m, 4H), 1.40-1.13 (m, 2H). | Method 3: 3.71 min, 616.1 [M+Na]⁺ |
| **Ref. Ex. 80** | 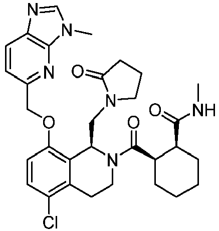 | (1*S*,2*R*)-2-((*S*)-5-chloro-8-((3-methyl-3H-imidazo[4,5-*b*]pyridin-5-yl)methoxy)-1-((2-oxopyrrolidin-1-yl)methyl)-1,2,3,4-tetrahydroisoquinoline-2-carbonyl)-*N-*methylcyclohexane-1-carboxamide | 98 | ¹H NMR (400 MHz, DMSO-d₆) δ 8.45 (s, 1H), 8.17 (d, 1H), 7.54 (d, 1H), 7.36 (d, 1H), 7.26 (m, 1H), 7.10 (d, 1H), 5.93 (dd, 1H), 5.36 (d, 1H), 5.30 (d, 1H), 4.00-3.78 (m, 5H), 3.60 (m, 1H), 3.45 (m, 1H), 3.40-3.20 (m, 1H), 3.18 (dd, 1H), 2.90 (m, 1H), 2.86-2.70 (m, 2H), 2.41 (d, 3H), 2.24-2.08 (m, 3H), 1.99 (m, 1H), 1.86-1.63 (m, 5H), 1.54-1.34 (m, 2H), 1.31-1.11 (m, 2H). | Method 3: 3.38 min, 615.4 [M+Na]⁺ |
| **Ref. Ex. 81** | 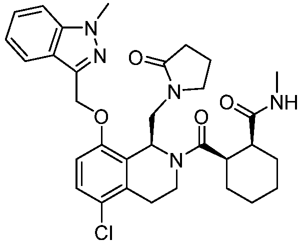 | (1*S*,2*R*)-2-((*S*)-5-chloro-8-((1-methyl-1*H*-indazol-3-yl)methoxy)-1-((2-oxopyrrolidin-1-yl)methyl)-1,2,3,4-tetrahydroisoquinoline-2-carbonyl)-*N-*methylcyclohexane-1-carboxamide | 98 | ¹H NMR (400 MHz, DMSO-d₆) δ 7.91 (d, 1H), 7.67 (d, 1H), 7.44 (m, 1H), 7.39 (d, 1H), 7.29-7.15 (m, 3H), 5.76 (dd, 1H), 5.49 (d, 1H), 5.43 (d, 1H), 4.07 (s, 3H), 3.92 (dd, 1H), 3.80 (dd, 1H), 3.55 (m, 1H), 3.40-3.23 (m, 1H), 3.14 (m, 1H), 2.93 (dd, 1H), 2.86-2.66 (m, 2H), 2.45-2.37 (m, 4H), 2.22-2.02 (m, 3H), 1.92 (m, 1H), 1.78-1.55 (m, 5H), 1.49-1.29 (m, 2H), 1.26-1.08 (m, 2H). | Method 3: 4.40 min, 614.3 [M+Na]⁺ |
| **Ref. Ex. 82** | 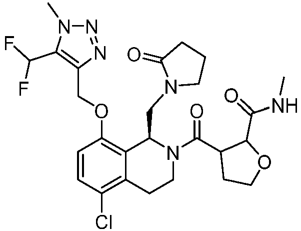 | 3-((*S*)-5-chloro-8-((5-(difluoromethyl)-1-methyl-1*H-*1,2,3-triazol-4-yl)methoxy)-1-((2-oxopyrrolidin-1-yl)methyl)-1,2,3,4-tetrahydroisoquinoline-2-carbonyl)-*N-*methyltetrahydrofuran-2-carboxamide (isomer 1) | 98 | ¹H NMR (400 MHz, DMSO-d₆; rotamers observed, both reported) δ 7.62 (t, 0.3H), 7.56 (t, 0.7H), 7.44 (m, 1H), 7.37 (m, 1H), 7.14 (m, 1H), 5.73 (dd, 0.7H), 5.39-5.29 (m, 0.6H), 5.27 (s, 1.4H), 5.15 (m, 0.3H), 4.44 (m, 0.3H), 4.26 (d, 0.7H), 4.21-4.11 (m, 3.6H), 4.03 (m, 0.7H), 3.92 (m, 0.7H), 3.82-3.58 (m, 3.4H), 3.47-3.17 (m, 1.6H), 3.13-2.65 (m, 4H), 2.61-2.43 (m, 3.1H), 2.26 (d, 0.9H), 2.21-1.66 (m, 5H). | Method 3: 3.57 min, 603.3 [M+Na]⁺ |
| **83** | 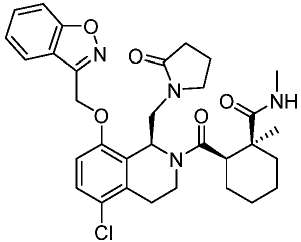 | (1*S*,2*R*)-2-((*S*)-8-(benzo[d]isoxazol-3-ylmethoxy)-5-chloro-1-((2-oxopyrrolidin-1-yl)methyl)-1,2,3,4-tetrahydroisoquinoline-2-carbonyl)-*N*,1-dimethylcyclohexane-1-carboxamide | 98 | ¹H NMR (400 MHz, DMSO-d₆) δ 8.03 (m, 1H), 7.83 (m, 1H), 7.72 (m, 1H), 7.47 (m, 1H), 7.43 (d, 1H), 7.24 (bm, 1H), 7.19 (d, 1H), 5.83 (dd, 1H), 5.71 (d, 1H), 5.62 (d, 1H), 3.94 (m, 1H), 3.85 (dd, 1H), 3.57 (m, 1H), 3.25 (m, 1H), 3.06-2.94 (m, 2H), 2.88-2.77 (m, 2H), 2.60 (m, 1H), 2.40 (d, 3H), 2.34 (m, 1H), 2.10 (m, 1H), 1.94 (m, 1H), 1.76-1.56 (m, 3H), 1.56-1.27 (m, 5H), 1.17-0.98 (m, 4H). | Method 3: 4.64 min, 593.3 [M+H]+ |
| **85** | 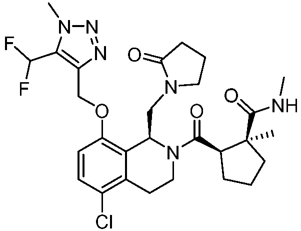 | (1*S*,2*R*)-2-((*S*)-5-chloro-8-((5-(difluoromethyl)-1-methyl-1*H*-1 ,2,3-triazol-4-yl)methoxy)-1-((2-oxopyrrolidin-1-yl)methyl)-1,2,3,4-tetrahydroisoquinoline-2-carbonyl)-*N*,1-dimethylcyclopentane-1-carboxamide | 98 | ¹H NMR (400 MHz, DMSO-d₆) δ 7.57 (t, 1H), 7.39 (d, 1H), 7.16 (d, 1H), 7.03 (m, 1H), 5.66 (dd, 1H), 5.38-5.22 (m, 2H), 4.17 (s, 3H), 3.96 (m, 1H), 3.83 (dd, 1H), 3.51 (m, 1H), 3.44-3.26 (m, 1H), 3.03 (d, 1H), 2.93 (m, 1H), 2.88-2.70 (m, 3H), 2.39 (d, 3H), 2.29 (m, 1H), 2.16 (m, 1H), 2.05-1.89 (m, 2H), 1.88-1.39 (m, 6H), 1.05 (s, 3H). | Method 3: 3.06 min, 593.4 [M+H]⁺ |
| **Ref. Ex. 86** | 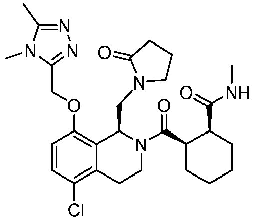 | (1*S*,2*R*)-2-((*S*)-5-chloro-8-((4,5-dimethyl-4H-1,2,4-triazol-3-yl)methoxy)-1-((2-oxopyrrolidin-1-yl)methyl)-1,2,3,4-tetrahydroisoquinoline-2-carbonyl)-*N-*methylcyclohexane-1-carboxamide | 98 | ¹H NMR (400 MHz, DMSO-d₆) δ 7.39 (d, 1H), 7.27 (m, 1H), 7.20 (d, 1H), 5.79 (dd, 1H), 5.40-5.25 (m, 2H), 3.95 (dd, 1H), 3.84 (dd, 1H), 3.70-3.50 (m, 4H), 3.50-3.20 (m, 2H), 2.92 (m, 2H), 2.78 (m, 2H), 2.42 (d, 3H), 2.36 (s, 3H), 2.27-2.10 (m, 3H), 2.01 (m, 1H), 1.94-1.62 (m, 5H), 1.54-1.34 (m, 2H), 1.32-1.09 (m, 2H). | Method 3: 2.94 min, 579.3 [M+Na]⁺ |
| **Ref. Ex. 87** | 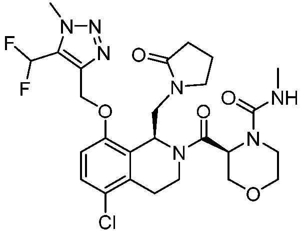 | (*S*)-3-((*S*)-5-chloro-8-((5-(difluoromethyl)-1-methyl-1*H*-1,2,3-triazol-4-yl)methoxy)-1-((2-oxopyrrolidin-1-yl)methyl)-1,2,3,4-tetrahydroisoquinoline-2-carbonyl)-*N-*methylmorpholine-4-carboxamide | 67 | ¹H NMR (400 MHz, DMSO-d₆) δ 7.58 (t, 1H), 7.41 (d, 1H), 7.18 (d, 1H), 6.42 (m, 1H), 5.73 (dd, 1H), 5.30 (s, 2H), 4.82 (d, 1H), 4.17 (s, 3H), 3.91 (dd, 1H), 3.87-3.75 (m, 3H), 3.66-3.49 (m, 3H), 3.40-3.24 (m, 3H), 3.00 (dd, 1H), 2.92-2.77 (m, 2H), 2.70 (m, 1H), 2.52-2.45 (m, 3H), 2.19 (m, 1H), 2.05 (m, 1H), 1.77 (m, 2H). | Method 3: 3.40 min, 618.4 [M+Na]⁺ |
| **Ref. Ex. 89** | 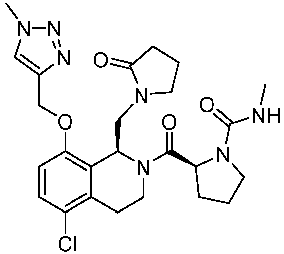 | (*S*)-2-((*S*)-5-chloro-8-((1-methyl-1*H*-1,2,3-triazol-4-yl)methoxy)-1-((2-oxopyrrolidin-1-yl)methyl)-1,2,3,4-tetrahydroisoquinoline-2-carbonyl)-*N-*methylpyrrolidine-1-carboxamide | 88 | ¹H NMR (300 MHz, DMSO-d₆) δ 8.22 (s, 1H), 7.38 (d, 1H), 7.15 (d, 1H), 5.99 (m, 1H), 5.70 (m, 1H), 5.29-5.16 (m, 2H), 4.70 (m, 1H), 4.09-3.96 (m, 4H), 3.86 (dd, 1H), 3.65-3.39 (m, 2H), 3.38-3.14 (m, 2H), 3.06-2.91 (m, 2H), 2.83-2.75 (m, 1H), 2.58-2.42 (m, 4H), 2.18 (m, 1H), 2.06-1.61 (m, 7H). | Method 6: 1.22 min, 530.2 [M+H]⁺ |
| **90** | 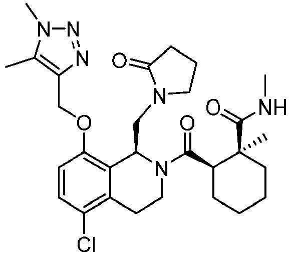 | (1*S*,2*R*)-2-((*S*)-5-chloro-8-((1,5-dimethyl-1*H*-1,2,3-triazol-4-yl)methoxy)-1-((2-oxopyrrolidin-1-yl)methyl)-1,2,3,4-tetrahydroisoquinoline-2-carbonyl)-*N*,1-dimethylcyclohexane-1-carboxamide | 98 | ¹H NMR (300 MHz, CDCl₃) δ 7.54 (bm, 1H), 7.23 (d, 1H), 6.91 (d, 1H), 5.96 (m, 1H), 5.14 (d, 1H), 5.07 (d, 1H), 4.05-3.55 (m, 7H), 3.22-2.72 (m, 5H), 2.69 (d, 3H), 2.49 (m, 1H), 2.35-2.09 (m, 5H), 1.97-1.35 (m, 8H), 1.28-1.08 (m, 4H). | Method 7: 1.95 min, 571.4 [M+H]⁺ |
| **91** | 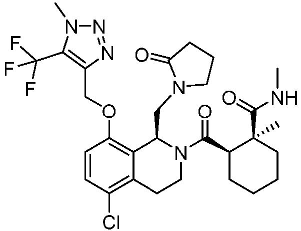 | (1*S*,2*R*)-2-((*S*)-5-chloro-8-((1-methyl-5-(trifluoromethyl)-1*H*-1,2,3-triazol-4-yl)methoxy)-1-((2-oxopyrrolidin-1-yl)methyl)-1,2,3,4-tetrahydroisoquinoline-2-carbonyl)-*N*,1-dimethylcyclohexane-1-carboxamide | 98 | ¹H NMR (300 MHz, CDCl₃) δ 7.31-6.76 (m, 2H), 5.97 (m, 1H), 5.31-4.92 (m, 2H), 4.26-3.39 (m, 7H), 3.21-2.37 (m, 9H), 2.35-2.07 (m, 2H), 1.98-0.52 (m, 12H). | Method 7: 2.33 min, 625.4 [M+H]⁺ |
| **Ref. Ex. 92** | 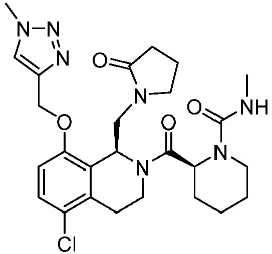 | (*S*)-2-((*S*)-5-chloro-8-((1-methyl-1*H*-1 ,2,3-triazol-4-yl)methoxy)-1-((2-oxopyrrolidin-1-yl)methyl)-1,2,3,4-tetrahydroisoquinoline-2-carbonyl)-*N-*methylpiperidine-1-carboxamide | 88 | ¹H NMR (300 MHz, CDCl₃) δ 7.58 (s, 1H), 7.20 (d, 1H), 6.79 (d, 1H), 5.94 (m, 1H), 5.27 (m, 1H), 5.23 (d, 1H), 5.13 (d, 1H), 4.48 (m, 1H), 4.08 (s, 3H), 4.05-3.88 (m, 2H), 3.87-3.44 (m, 3H), 3.31 (m, 1H), 3.15-3.04 (m, 2H), 2.99-2.87 (m, 2H), 2.73 (d, 3H), 2.39-2.06 (m, 2H), 2.00-1.84 (m, 3H), 1.80-1.26 (m, 5H). | Method 8: 1.61 min, 544.4 [M+H]⁺ |
| **93** | 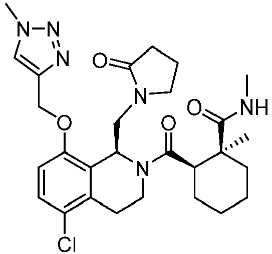 | (1*S*,2*R*)-2-((*S*)-5-chloro-8-((1-methyl-1*H*-1,2,3-triazol-4-yl)methoxy)-1-((2-oxopyrrolidin-1-yl)methyl)-1,2,3,4-tetrahydroisoquinoline-2-carbonyl)-*N*,1-dimethylcyclohexane-1-carboxamide | 98 | ¹H NMR (400 MHz, DMSO-d₆) δ 8.23 (s, 1H), 7.35 (d, 1H), 7.29 (m, 1H), 7.11 (d, 1H), 5.82 (m, 1H), 5.29-5.11 (m, 2H), 4.11-3.98 (m, 4H), 3.81 (dd, 1H), 3.55 (m, 1H), 3.48-3.28 (m, 1H), 3.13 (m, 1H), 3.03-2.86 (m, 2H), 2.80 (m, 2H), 2.61-2.36 (m, 4H), 2.14 (m, 1H), 1.98 (m, 1H), 1.89-1.67 (m, 2H), 1.66-1.36 (m, 6H), 1.35-1.17 (m, 4H). | Method 9 (with CSH column 7 no ammonia): 1.83 min, 557.5 [M+H]⁺ |
| **94** | 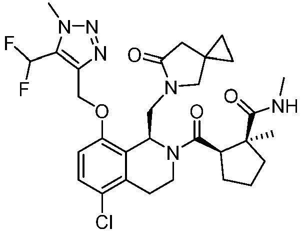 | (1*S*,2*R*)-2-((*S*)-5-chloro-8-((5-(difluoromethyl)-1-methyl-1*H*-1,2,3-triazol-4-yl)methoxy)-1-((6-oxo-5-azaspiro[2.4]heptan-5-yl)methyl)-1,2,3,4-tetrahydroisoquinoline-2-carbonyl)-*N*,1-dimethylcyclopentane-1-carboxamide | 98 | ¹H NMR (400 MHz, DMSO-d₆) δ 7.56 (t, 1H), 7.39 (d, 1H), 7.15 (d, 1H), 7.05 (m, 1H), 5.65 (m, 1H), 5.27 (s, 2H), 4.15 (s, 3H), 4.00 (m, 1H), 3.92 (dd, 1H), 3.52 (m, 1H), 3.39-3.27 (m, 1H), 3.04 (m, 1H), 2.92 (m, 1H), 2.79 (m, 2H), 2.56-2.45 (m, 1H), 2.42-2.24 (m, 4H), 2.17 (d, 1H), 2.08 (d, 1H), 2.01 (m, 1H), 1.79-1.35 (m, 4H), 1.06 (s, 3H), 0.62-0.35 (m, 4H). | Method 11: 1.89 min, 619.3 [M+H]⁺ |
| **95** | 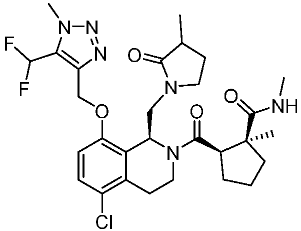 | (1*S*,2*R*)-2-((1*S*)-5-chloro-8-((5-(difluoromethyl)-1-methyl-1*H*-1 ,2,3-triazol-4-yl)methoxy)-1-((3-methyl-2-oxopyrrolidin-1-yl)methyl)-1,2,3,4-tetrahydroisoquinoline-2-carbonyl)-*N*,1-dimethylcyclopentane-1-carboxamide (isomer 1) | 98 | ¹H NMR (300 MHz, CDCl₃; rotamers observed, both reported) δ 7.56 (t, 0.3H), 7.27-7.18 (m, 1H), 7.17-6.76 (m, 1.7H), 5.89 (m, 0.7H), 5.72 (m, 0.7H), 5.59 (m, 0.3H), 5.44-5.15 (m, 2H), 4.64 (m, 0.3H), 4.18 (s, 0.9H), 4.18 (s, 2.1H), 4.03-3.87 (m, 2H), 3.84-3.48 (m, 2H), 3.18-2.84 (m, 5H), 2.79 (d, 0.9H), 2.60 (d, 2.1H), 2.43-2.25 (m, 2H), 2.20-1.34 (m, 7H), 1.27-1.13 (m, 3.9H), 1.10 (d, 2.1H). | Method 13: 2.15 min, 607.3 [M+H]⁺ |
| **96** | 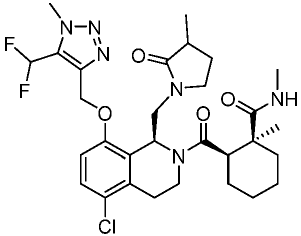 | (1*S*,2*R*)-2-((1*S*)-5-chloro-8-((5-(difluoromethyl)-1-methyl-1*H*-1 ,2,3-triazol-4-yl)methoxy)-1-((3-methyl-2-oxopyrrolidin-1-yl)methyl)-1,2,3,4-tetrahydroisoquinoline-2-carbonyl)-*N*,1-dimethylcyclohexane-1-carboxamide (isomer 1) | 98 | ¹H NMR (400 MHz, CDCl₃) δ 8.31 (bm, 1H), 7.28-7.18 (m, 1H), 6.96 (t, 1H), 6.90 (d, 1H), 6.00 (m, 1H), 5.38-5.20 (m, 2H), 4.19 (s, 3H), 4.04-3.76 (m, 3H), 3.54 (m, 1H), 3.10-2.92 (m, 3H), 2.89-2.76 (m, 2H), 2.70 (d, 3H), 2.50 (m, 1H), 2.35 (m, 1H), 2.14 (m, 1H), 1.78-1.01 (m, 14H). | Method 2: 5.67 min, 621.3 [M+H]⁺ |
| **97** | 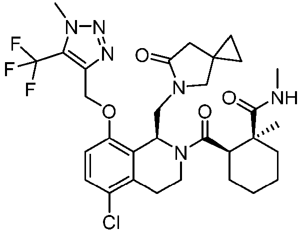 | (1*S*,2*R*)-2-((*S*)-5-chloro-8-((1-methyl-5-(trifluoromethyl)-1*H*-1,2,3-triazol-4-yl)methoxy)-1-((6-oxo-5-azaspiro[2.4]heptan-5-yl)methyl)-1,2,3,4-tetrahydroisoquinoline-2-carbonyl)-*N*,1-dimethylcyclohexane-1-carboxamide | 98 | ¹H NMR (300 MHz, CDCl₃) δ 7.93 (bm, 1H), 7.23 (d, 1H), 6.95 (d, 1H), 6.00 (dd, 1H), 5.38-5.16 (m, 2H), 4.19 (s, 3H), 4.09 (dd, 1H), 4.02-3.74 (m, 2H), 3.59 (d, 1H), 3.23-2.64 (m, 8H), 2.55 (m, 1H), 2.38 (d, 1H), 2.19 (d, 1H), 1.84-1.32 (m, 6H), 1.25-1.03 (m, 4H), 0.75-0.46 (m, 4H). | Method 9: 2.53 min, 651.2 [M+H]+ |
| **99** | 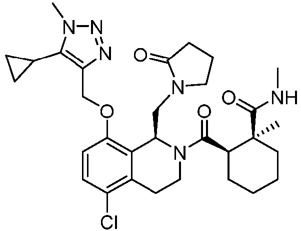 | (1*S*,2*R*)-2-((*S*)-5-chloro-8-((5-cyclopropyl-1-methyl-1*H-*1,2,3-triazol-4-yl)methoxy)-1-((2-oxopyrrolidin-1-yl)methyl)-1,2,3,4-tetrahydroisoquinoline-2-carbonyl)-*N*,1-dimethylcyclohexane-1-carboxamide | 98 | ¹H NMR (300 MHz, CDCl₃) δ 7.73 (bm, 1H), 7.23 (d, 1H), 6.97 (d, 1H), 5.98 (m, 1H), 5.19-5.07 (m, 2H), 4.16-3.74 (m, 6H), 3.64 (m, 1H), 3.16-3.02 (m, 2H), 3.01-2.71 (m, 3H), 2.69 (d, 3H), 2.51 (m, 1H), 2.35-2.08 (m, 2H), 1.96-1.80 (m, 2H), 1.77-1.32 (m, 7H), 1.27-0.99 (m, 6H), 0.84-0.68 (m, 2H). | Method 10: 1.90 min, 597.3 [M+H]⁺ |
| **100** | 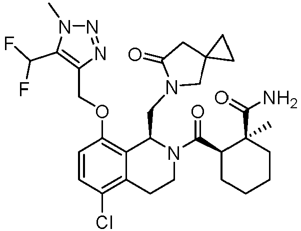 | (1*S*,2*R*)-2-((*S*)-5-chloro-8-((5-(difluoromethyl)-1-methyl-1*H*-1 ,2,3-triazol-4-yl)methoxy)-1-((6-oxo-5-azaspiro[2.4]heptan-5-yl)methyl)-1,2,3,4-tetrahydroisoquinoline-2-carbonyl)-1-methylcyclohexane-1-carboxamide | 98 | ¹H NMR (300 MHz, CDCl₃) δ 7.23 (d, 1H), 6.92 (t, 1H), 6.89 (d, 1H), 5.98 (dd, 1H), 5.30-5.15 (m, 2H), 4.17 (s, 3H), 4.08 (dd, 1H), 4.01-3.75 (m, 2H), 3.52 (d, 1H), 3.08 (dd, 1H), 3.03-2.75 (m, 4H), 2.55 (m, 1H), 2.35 (d, 1H), 2.20 (d, 1H), 1.88-1.33 (m, 6H), 1.32-1.13 (m, 4H), 0.69-0.46 (m, 4H). | Method 9: 2.14 min, 619.2 [M+H]⁺ |
| **101** | 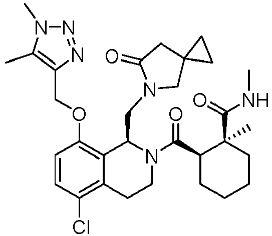 | (1*S*,2*R*)-2-((*S*)-5-chloro-8-((1,5-dimethyl-1*H*-1,2,3-triazol-4-yl)methoxy)-1-((6-oxo-5-azaspiro[2.4]heptan-5-yl)methyl)-1,2,3,4-tetrahydroisoquinoline-2-carbonyl)-*N*,1-dimethylcyclohexane-1-carboxamide | 98 | ¹H NMR (400 MHz, CDCl₃) δ 7.74 (bm, 1H), 7.22 (d, 1H), 6.91 (d, 1H), 5.95 (m, 1H), 5.13 (d, 1H), 5.06 (d, 1H), 4.07 (dd, 1H), 4.00-3.88 (m, 4H), 3.82 (m, 1H), 3.53-3.44 (m, 1H), 3.11 (dd, 1H), 3.01-2.88 (m, 3H), 2.81 (m, 1H), 2.70 (d, 3H), 2.54 (m, 1H), 2.34 (d, 1H), 2.29 (s, 3H), 2.19 (d, 1H), 1.82-1.31 (m, 6H), 1.25-1.01 (m, 4H), 0.68-0.46 (m, 4H). | Method 10: 1.86 min, 597.3 [M+H]⁺ |
| **102** | 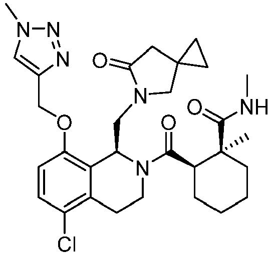 | (1*S*,2*R*)-2-((*S*)-5-chloro-8-((1-methyl-1*H*-1,2,3-triazol-4-yl)methoxy)-1-((6-oxo-5-azaspiro[2.4]heptan-5-yl)methyl)-1,2,3,4-tetrahydroisoquinoline-2-carbonyl)-*N*,1-dimethylcyclohexane-1-carboxamide | 98 | ¹H NMR (300 MHz, CDCl₃) δ 8.00-7.32 (m, 2H), 7.21 (d, 1H), 6.81 (d, 1H), 6.00 (dd, 1H), 5.20 (d, 1H), 5.14 (d, 1H), 4.16-3.90 (m, 5H), 3.82 (m, 1H), 3.54 (d, 1H), 3.13 (dd, 1H), 3.05-2.77 (m, 4H), 2.70 (d, 3H), 2.54 (m, 1H), 2.35 (d, 1H), 2.19 (d, 1H), 1.87-1.32 (m, 6H), 1.28-1.04 (m, 4H), 0.69-0.43 (m, 4H). | Method 11: 1.82 min, 583.3 [M+H]⁺ |
| **103** | 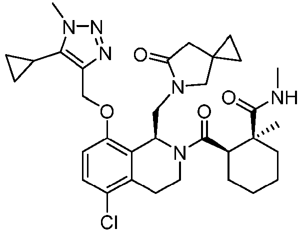 | (1*S*,2*R*)-2-((*S*)-5-chloro-8-((5-cyclopropyl-1-methyl-1*H*-1,2,3-triazol-4-yl)methoxy)-1-((6-oxo-5-azaspiro[2.4]heptan-5-yl)methyl)-1,2,3,4-tetrahydroisoquinoline-2-carbonyl)-*N*,1-dimethylcyclohexane-1-carboxamide | 98 | ¹H NMR (300 MHz, CDCl₃) δ 7.95 (bm, 1H), 7.24 (d, 1H), 6.97 (d, 1H), 5.98 (dd, 1H), 5.22-5.05 (m, 2H), 4.13-4.01 (m, 4H), 3.96 (m, 1H), 3.83 (m, 1H), 3.50 (d, 1H), 3.12 (dd, 1H), 3.02-2.74 (m, 4H), 2.71 (d, 3H), 2.54 (m, 1H), 2.34 (d, 1H), 2.19 (d, 1H), 1.80-0.95 (m, 13H), 0.84-0.67 (m, 2H), 0.66-0.47 (m, 4H). | Method 10: 2.10 min, 623.3 [M+H]⁺ |
| **104** | 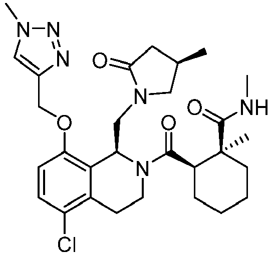 | (1*S*,2*R*)-2-((*S*)-5-chloro-8-((1-methyl-1*H*-1,2,3-triazol-4-yl)methoxy)-1-(((*R*)-4-methyl-2-oxopyrrolidin-1-yl)methyl)-1,2,3,4-tetrahydroisoquinoline-2-carbonyl)-*N*,1-dimethylcyclohexane-1-carboxamide | 98 | ¹H NMR (300 MHz, CDCl₃) δ 7.98 (bm, 1H), 7.62 (s, 1H), 7.22 (d, 1H), 6.83 (d, 1H), 5.98 (m, 1H), 5.22 (d, 1H), 5.16 (d, 1H), 4.10 (s, 3H), 4.06-3.74 (m, 3H), 3.29 (m, 1H), 3.22-3.05 (m, 2H), 3.03-2.77 (m, 3H), 2.69 (dd, 3H), 2.59-2.27 (m, 3H), 1.96-1.28 (m, 7H), 1.25-0.99 (m, 7H). | Method 11: 1.64 min, 571.3 [M+H]⁺ |
| **105** | 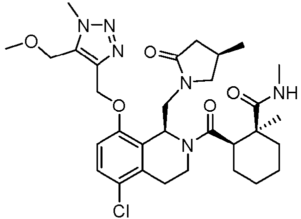 | (1*S*,2*R*)-2-((*S*)-5-chloro-8-((5-(methoxymethyl)-1-methyl-1*H*-1 ,2,3-triazol-4-yl)methoxy)-1-(((*R*)-4-methyl-2-oxopyrrolidin-1-yl)methyl)-1,2,3,4-tetrahydroisoquinoline-2-carbonyl)-*N*,1-dimethylcyclohexane-1-carboxamide | 98 | ¹H NMR (400 MHz, CDCl₃) δ 8.24 (bm, 1H), 7.24 (d, 1H), 6.90 (d, 1H), 5.92 (m, 1H), 5.20 (d, 1H), 5.12 (d, 1H), 4.55 (d, 1H), 4.49 (d, 1H), 4.06 (s, 3H), 4.00 (dd, 1H), 3.93 (m, 1H), 3.83 (m, 1H), 3.33 (s, 3H), 3.22 (dd, 1H), 3.14-3.02 (m, 2H), 2.95 (dd, 1H), 2.87-2.76 (m, 2H), 2.69 (d, 3H), 2.51 (m, 1H), 2.45-2.25 (m, 2H), 1.90-1.20 (m, 7H), 1.17-1.07 (m, 4H), 1.05 (d, 3H). | Method 10: 1.93 min, 615.3 [M+H]⁺ |
| **106** | 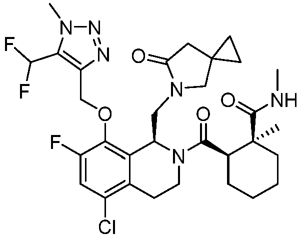 | (1*S*,2*R*)-2-((*S*)-5-chloro-8-((5-(difluoromethyl)-1-methyl-1*H*-1 ,2,3-triazol-4-yl)methoxy)-7-fluoro-1-((6-oxo-5-azaspiro[2.4]heptan-5-yl)methyl)-1,2,3,4-tetrahydroisoquinoline-2-carbonyl)-*N*,1-dimethylcyclohexane-1-carboxamide | 98 | ¹H NMR (400 MHz, DMSO-d₆) δ 7.54 (d, 1H), 7.39-7.05 (m, 2H), 5.45-5.35 (m, 2H), 5.20 (m, 1H), 4.12 (s, 3H), 3.97-3.86 (m, 2H), 3.52 (m, 1H), 3.08-2.98 (m, 2H), 2.85 (dd, 1H), 2.80-2.70 (m, 3H), 2.45 (d, 3H), 2.38 (m, 1H), 2.22 (d, 1H), 2.05 (d, 1H), 1.63 (m, 1H), 1.58-1.29 (m, 5H), 1.18 (m, 1H), 1.07 (s, 3H), 0.66-0.44 (m, 4H). | Method 14: 4.51 min, 651.1 [M+H]⁺ |
| **107** | 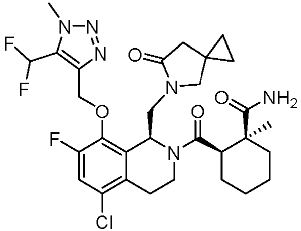 | (1*S*,2*R*)-2-((*S*)-5-chloro-8-((5-(difluoromethyl)-1-methyl-1*H*-1 ,2,3-triazol-4-yl)methoxy)-7-fluoro-1-((6-oxo-5-azaspiro[2.4]heptan-5-yl)methyl)-1,2,3,4-tetrahydroisoquinoline-2-carbonyl)-1-methylcyclohexane-1-carboxamide | 98 | ¹H NMR (400 MHz, DMSO-d₆) δ 7.51 (d, 1H), 7.29 (t, 1H), 6.91 (bm, 1H), 6.55 (bm, 1H), 5.51 (dd, 1H), 5.40 (m, 1H), 5.22 (m, 1H), 4.14 (s, 3H), 3.98-3.86 (m, 2H), 3.52 (m, 1H), 3.10 (d, 1H), 3.04 (m, 1H), 2.91-2.77 (m, 2H), 2.77-2.68 (m, 2H), 2.46-2.34 (m, 1H), 2.21 (d, 1H), 2.07 (d, 1H), 1.63 (m, 1H), 1.57-1.12 (m, 6H), 1.09 (s, 3H), 0.64-0.44 (m, 4H). | Method 14: 4.32 min, 637.1 [M+H]⁺ |
| **108** | 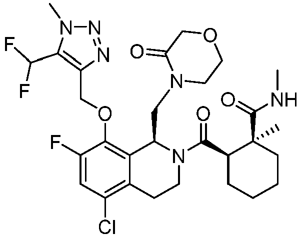 | (1*S*,2*R*)-2-((*S*)-5-chloro-8-((5-(difluoromethyl)-1-methyl-1*H*-1 ,2,3-triazol-4-yl)methoxy)-7-fluoro-1-((3-oxomorpholino)methyl)-1,2,3,4-tetrahydroisoquinoline-2-carbonyl)-*N*,1-dimethylcyclohexane-1-carboxamide | 98 | ¹H NMR (400 MHz, DMSO-d₆) δ 7.55 (d, 1H), 7.29 (bm, 1H), 7.23 (m, 1H), 5.49-5.39 (m, 2H), 5.22 (m, 1H), 4.31 (dd, 1H), 4.13 (s, 3H), 3.96 (d, 1H), 3.88 (m, 1H), 3.78 (d, 1H), 3.72 (m, 1H), 3.64 (m, 1H), 3.53 (m, 1H), 3.19 (m, 1H), 3.01 (m, 1H), 2.84 (m, 1H), 2.80-2.70 (m, 3H), 2.45 (d, 3H), 2.36 (m, 1H), 1.63 (m, 1H), 1.58-1.28 (m, 5H), 1.17 (m, 1H), 1.07 (s, 3H). | Method 14: 4.24 min, 641.1 [M+H]⁺ |
| **109** | 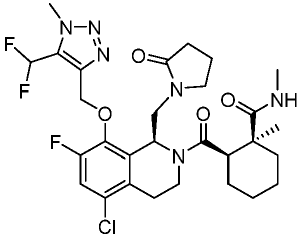 | (1*S*,2*R*)-2-((*S*)-5-chloro-8-((5-(difluoromethyl)-1-methyl-1*H*-1 ,2,3-triazol-4-yl)methoxy)-7-fluoro-1-((2-oxopyrrolidin-1-yl)methyl)-1,2,3,4-tetrahydroisoquinoline-2-carbonyl)-*N*,1-dimethylcyclohexane-1-carboxamide | 98 | ¹H NMR (400 MHz, DMSO-d₆) δ 7.54 (d, 1H), 7.30 (bm, 1H), 7.26 (t, 1H), 5.45-5.36 (m, 2H), 5.22 (m, 1H), 4.13 (s, 3H), 3.93-3.77 (m, 2H), 3.50 (m, 1H), 3.17 (m, 1H), 3.01 (m, 1H), 2.97-2.84 (m, 2H), 2.76 (m, 2H), 2.44 (d, 3H), 2.35 (m, 1H), 2.15 (m, 1H), 2.04-1.79 (m, 2H), 1.78-1.58 (m, 2H), 1.58-1.20 (m, 5H), 1.08 (m, 4H). | Method 14: 4.21 min, 625.1 [M+H]⁺ |
| **110** | 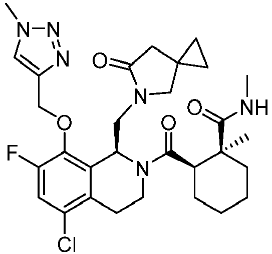 | (1*S*,2*R*)-2-((*S*)-5-chloro-7-fluoro-8-((1-methyl-1*H-*1,2,3-triazol-4-yl)methoxy)-1-((6-oxo-5-azaspiro[2.4]heptan-5-yl)methyl)-1,2,3,4-tetrahydroisoquinoline-2-carbonyl)-*N*,1-dimethylcyclohexane-1-carboxamide | 98 | ¹H NMR (400 MHz, DMSO-d₆) δ 8.17 (s, 1H), 7.52 (d, 1H), 7.30 (bm, 1H), 5.63 (dd, 1H), 5.32-5.20 (m, 2H), 4.04 (s, 3H), 3.98-3.86 (m, 2H), 3.52 (m, 1H), 3.27 (d, 1H), 3.02 (m, 1H), 2.96 (dd, 1H), 2.83-2.68 (m, 3H), 2.43 (d, 3H), 2.38 (m, 1H), 2.22 (d, 1H), 2.09 (d, 1H), 1.63 (m, 1H), 1.57-1.44 (m, 3H), 1.43-1.30 (m, 2H), 1.29-1.14 (m, 1H), 1.05 (s, 3H), 0.65-0.46 (m, 4H). | Method 3: 3.97 min, 601.1 [M+H]⁺ |
| **111** | 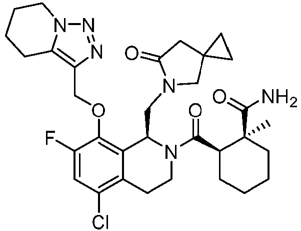 | (1*S*,2*R*)-2-((*S*)-5-chloro-7-fluoro-1-((6-oxo-5-azaspiro[2.4]heptan-5-yl)methyl)-8-((4,5,6,7-tetrahydro-[1,2,3]triazolo[1,5-*a*]pyridin-3-yl)methoxy)-1,2,3,4-tetrahydroisoquinoline-2-carbonyl)-1-methylcyclohexane-1-carboxamide | 98 | ¹H NMR (400 MHz, DMSO-d₆) δ 7.50 (d, 1H), 6.88 (bm, 1H), 6.50 (bm, 1H), 5.62 (dd, 1H), 5.25 (dd, 1H), 5.10 (dd, 1H), 4.28 (m, 2H), 3.99-3.87 (m, 2H), 3.50 (m, 1H), 3.14 (d, 1H), 3.04 (m, 1H), 2.97-2.64 (m, 6H), 2.42 (m, 1H), 2.20 (d, 1H), 2.08 (d, 1H), 1.99 (m, 2H), 1.80 (m, 2H), 1.62 (m, 1H), 1.56-1.42 (m, 3H), 1.42-1.15 (m, 3H), 1.08 (s, 3H), 0.65-0.43 (m, 4H). | Method 14: 4.13 min, 627.1 [M+H]⁺ |
| **112** | 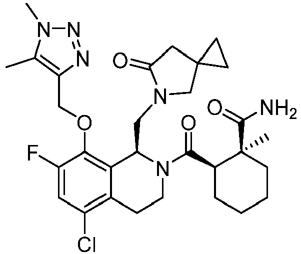 | (1*S*,2*R*)-2-((*S*)-5-chloro-8-((1,5-dimethyl-1*H*-1,2,3-triazol-4-yl)methoxy)-7-fluoro-1-((6-oxo-5-azaspiro[2.4]heptan-5-yl)methyl)-1,2,3,4-tetrahydroisoquinoline-2-carbonyl)-1-methylcyclohexane-1-carboxamide | 98 | ¹H NMR (400 MHz, CDCl₃) δ 7.11 (d, 1H), 5.73 (dd, 1H), 5.24 (d, 1H), 5.14 (dd, 1H), 4.04 (dd, 1H), 3.97-3.84 (m, 4H), 3.76 (m, 1H), 3.28 (d, 1H), 3.10 (dd, 1H), 3.01 (d, 1H), 2.96-2.72 (m, 3H), 2.57 (m, 1H), 2.34 (d, 1H), 2.22-2.12 (m, 4H), 1.82-1.36 (m, 6H), 1.27 (m, 1H), 1.18 (s, 3H), 0.68-0.43 (m, 4H). | Method 3: 3.87 min, 601.0 [M+H]⁺ |
| **113** | 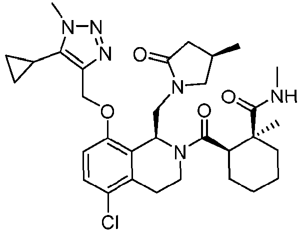 | (1*S*,2*R*)-2-((*S*)-5-chloro-8-((5-cyclopropyl-1-methyl-1*H*-1,2,3-triazol-4-yl)methoxy)-1-(((*R*)-4-methyl-2-oxopyrrolidin-1-yl)methyl)-1,2,3,4-tetrahydroisoquinoline-2-carbonyl)-*N*,1-dimethylcyclohexane-1-carboxamide | 98 | ¹H NMR (300 MHz, CDCl₃) δ 8.29 (bm, 1H), 7.24 (d, 1H), 6.96 (d, 1H), 5.96 (m, 1H), 5.13 (s, 2H), 4.10-3.96 (m, 4H), 3.95-3.75 (m, 2H), 3.24 (m, 1H), 3.18-3.03 (m, 2H), 2.95 (m, 1H), 2.87-2.72 (m, 2H), 2.69 (d, 3H), 2.51 (m, 1H), 2.46-2.24 (m, 2H), 1.93-1.24 (m, 8H), 1.19-0.97 (m, 9H), 0.90-0.68 (m, 2H). | Method 9: 2.27 min, 612.47 [M+H]+ |
| **Ref. Ex. 114** | 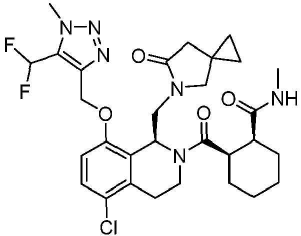 | (1*S*,2*R*)-2-((*S*)-5-chloro-8-((5-(difluoromethyl)-1-methyl-1*H*-1 ,2,3-triazol-4-yl)methoxy)-1-((6-oxo-5-azaspiro[2.4]heptan-5-yl)methyl)-1,2,3,4-tetrahydroisoquinoline-2-carbonyl)-*N-*methylcyclohexane-1-carboxamide | 98 | ¹H NMR (300 MHz, MeOD) δ 7.32 (d, 1H), 7.32 (t, 1H), 7.08 (d, 1H), 5.89 (m, 1H), 5.28 (s, 2H), 4.21 (s, 3H), 4.14-3.98 (m, 2H), 3.74 (m, 1H), 3.50 (m, 1H), 3.44 (d, 1H), 3.16 (dd, 1H), 2.99-2.84 (m, 3H), 2.58 (s, 3H), 2.44-2.17 (m, 4H), 2.07-1.20 (m, 7H), 0.75-0.46 (m, 4H). | Method 9: 2.21 min, 620.6 [M+H]+ |

### Biological Assays

### KEAP1 Kelch fluorescence polarization (FP) assay method

Inhibition of the Kelch domain-NRF2 interaction was determined using a fluorescence polarization-based competition assay in a black 384-well microplate. Compounds were tested at a starting concentration of 10 µM serially diluted 1:3 to generate a 12-point dose response curve on the Biomek FX robot. Each well contained 2 nM FITC-labelled NRF2 peptide (FITC-LDEETGEFL-NH2) and 25nM human KEAP1 (N-term, residues 321-609) enzyme in a final volume of 20 µL of assay buffer (50 mM Tris-HCl pH 8.0, 100 mM NaCl, 5 mM MgCl₂, 0.005% Tween-20, 0.005% BSA, 0.5% DMSO) in the presence of varying concentrations of test compound. Unlabelled peptide (LDEETGEFL-NH2) at 50 µM (negative control) and 0.5% DMSO (positive control) was used to determine the assay window.

After 1 h at room temperature, fluorescence polarization (excitation 470 nm/emission 530 nm) was measured using an Envision plate reader. IC₅₀ values were determined by fitting the data to a four parameter logistic fit using XLfit or XE Runner within ActivityBase. The assay limit is such that compounds below 10 nM cannot be differentiated. IC₅₀ values for the Example compounds are shown in Table 2.

**Table 2**

| **Example No.** | **IC₅₀ (nM)** | **Example No.** | **IC₅₀ (nM)** | **Example No.** | **IC₅₀ (nM)** |
|---|---|---|---|---|---|
| **1** | 901 | **39** | 3009 | **77** | 7.2 |
| **2** | 339 | **40** | 523 | **78** | 110 |
| **3** | 125 | **41** | 788 | **79** | 14 |
| **4** | 161 | **42** | 53 | **80** | 371 |
| **5** | 147 | **43** | 186 | **81** | 723 |
| **6** | 196 | **44** | 1169 | **82** | 4071 |
| **7** | 196 | **45** | 310 | **83** | 12 |
| **8** | 1276 | **46** | 83 | **84** | 161 |
| **9** | 119 | **47** | 5332 | **85** | 7.8 |
| **10** | 38 | **48** | 728 | **86** | 14 |
| **11** | 37 | **49** | 1804 | **87** | 39 |
| **12** | 56 | **50** | 72 | **88** | 67 |
| **13** | 169 | **51** | 1702 | **89** | 19 |
| **14** | 183 | **52** | 38 | **90** | 7.9 |
| **15** | 93 | **53** | 47 | **91** | 9.8 |
| **16** | 366 | **54** | 31 | **92** | 12 |
| **17** | 28 | **55** | 54 | **93** | 851 |
| **18** | 119 | **56** | 13 | **94** | 13 |
| **19** | 196 | **57** | 112 | **95** | 23 |
| **20** | 29 | **58** | 11 | **96** | 37 |
| **21** | 2253 | **59** | 533 | **97** | 8.9 |
| **22** | 32 | **60** | 72 | **98** | 6.0 |
| **23** | 606 | **61** | 34 | **99** | 7.4 |
| **24** | 294 | **62** | 82 | **100** | 6.4 |
| **25** | 292 | **63** | 340 | **101** | 6.1 |
| **26** | 61 | **64** | 41 | **102** | 12 |
| **27** | 565 | **65** | 18 | **103** | 7.1 |
| **28** | 43 | **66** | 151 | **104** | 11 |
| **29** | 166 | **67** | 29 | **105** | 17 |
| **30** | 215 | **68** | 13 | **106** | 6.7 |
| **31** | 539 | **69** | 14 | **107** | 6.2 |
| **32** | 2099 | **70** | 29 | **108** | 7.3 |
| **33** | 1506 | **71** | 17 | **109** | 7.5 |
| **34** | 36 | **72** | 27 | **110** | 7.1 |
| **35** | 185 | **73** | 9.4 | **111** | 7.2 |
| **36** | 126 | **74** | 16 | **112** | 7.0 |
| **37** | 44 | **75** | 73 | **113** | 10.9 |
| **38** | 8204* | **76** | 11 | **114** | 9.1 |

| | | | | | |
|---|---|---|---|---|---|
| * = one one test occasion >10 µM. | | | | | |

### Beas2B NQO1 mRNA cell based assay

The up regulation of the NRF2 mediated gene NAD(P)H:quinone acceptor oxidoreductase 1 (NQO1) was measured using the following assay method: BEAS-2B cells (ATCC CRL-9609) were plated in 96-well clear plates at 20,000 cells/well in 75µL of cell culture media and incubated overnight (37 °C, 5% CO₂). On day 2, 25µL of compound or controls were added to the cells for 24 h. On day 3, the medium was aspirated from the plate and the Cells-to-CT^{™} 1-Step TaqMan^{®} Kit (Ambion A25603) was used to perform expression analysis directly from cultured cells without RNA purification according to the manufacturer's instructions.

Briefly, cells were washed with ice cold PBS and 22.5 µL of room-temperature DNase/Lysis solution was added to the cells and incubated at room temperature for 5 minutes. To stop the reaction, 2.25 µL of stop solution was added to the cell lysate. The samples were diluted 1:5 using nuclease free water and 2.5 µL transferred into the PCR plate. Real-time PCR was performed using the C-1000 Thermal Cycler (Bio-Rad) using human beta actin as the internal control. The cDNA was amplified with a specific primer for NQO1 using the 1-step RT-PCR master mix (Ambion Cells-to-CT^{™} 1-Step TaqMan^{®} Kit A25603). The primers/probes sets that were used for amplification of cDNA were obtained from TaqMan Gene Expression Assays (Applied Biosystems). The comparative CT (ΔΔCT) relative quantification method was used to calculate the relative mRNA level of the target gene NQO1 as described in the Applied Biosystems Chemistry Guide. The data are expressed as an increase in target gene mRNA compared to vehicle (0.1% DMSO) control and the EC₅₀ values were determined by fitting the data to a four parameter logistic fit using XLfit or XE Runner within ActivityBase. EC₅₀ values for the Example compounds are shown in Table 3.

**Table 3**

| **Example No.** | **EC₅₀ (nM)** | **Example No.** | **EC₅₀ (nM)** | **Example No.** | **EC₅₀ (nM)** |
|---|---|---|---|---|---|
| **1** | 2027 | **39** | ND | **77** | 77 |
| **2** | ND | **40** | ND | **78** | >10,000 |
| **3** | ND | **41** | ND | **79** | 336 |
| **4** | ND | **42** | 580 | **80** | 2252 |
| **5** | ND | **43** | ND | **81** | >10,000 |
| **6** | 2358 | **44** | ND | **82** | 1736 |
| **7** | ND | **45** | ND | **83** | 473 |
| **8** | ND | **46** | ND | **84** | 1531 |
| **9** | ND | **47** | ND | **85** | 330 |
| **10** | ND | **48** | ND | **86** | 2417 |
| **11** | ND | **49** | ND | **87** | 4126 |
| **12** | ND | **50** | ND | **88** | 7563** |
| **13** | ND | **51** | ND | **89** | 8717 |
| **14** | ND | **52** | 285* | **90** | 94 |
| **15** | ND | **53** | 189 | **91** | 607 |
| **16** | ND | **54** | 135 | **92** | 565 |
| **17** | 3073 | **55** | 330 | **93** | >10,000 |
| **18** | ND | **56** | 124 | **94** | 148 |
| **19** | ND | **57** | 3996 | **95** | 565 |
| **20** | >500 | **58** | 206 | **96** | 3190 |
| **21** | ND | **59** | ND | **97** | 114 |
| **22** | ND | **60** | 650 | **98** | 41 |
| **23** | ND | **61** | 1083 | **99** | 109 |
| **24** | ND | **62** | 1462 | **100** | 26 |
| **25** | ND | **63** | >10,000 | **101** | 18 |
| **26** | ND | **64** | 5748 | **102** | 59 |
| **27** | ND | **65** | 1865 | **103** | 57 |
| **28** | ND | **66** | 5658 | **104** | 784 |
| **29** | ND | **67** | 1364 | **105** | 790 |
| **30** | ND | **68** | 145 | **106** | 25 |
| **31** | ND | **69** | 239 | **107** | 37 |
| **32** | ND | **70** | 295 | **108** | 654 |
| **33** | ND | **71** | 116 | **109** | 140 |
| **34** | 193 | **72** | 605 | **110** | 66 |
| **35** | ND | **73** | 288 | **111** | 30 |
| **36** | ND | **74** | 334 | **112** | 99 |
| **37** | ND | **75** | 2171 | **113** | 299 |
| **38** | ND | **76** | 166 | **114** | 230 |

| | | | | | |
|---|---|---|---|---|---|
| * = one one or more test occasion >0.5 µM, ** = one one or more test occasion 10 µM. ND = not determined | | | | | |

### PK/PD Method

Male Wistar Han rats (Charles River labs) were administered the test item orally or intravenously at the designated dose concentration. The intravenous dose was administered as a slow bolus *via* the tail vein. The oral formulation was administered by gastric gavage into the stomach. Actual dose times were recorded.

At the designated time points, 2 x 0.25 mL blood samples were collected into K₂EDTA blood tubes via the tail vein. The collected blood samples were then either centrifuged for plasma or decanted into the 1.5 mL PCR RNAlater tubes containing 650 µL of RNAlater.

Immediately following collection, blood samples were placed on wet ice. As soon as practically possible the 0.25 mL blood samples in K₂EDTA were centrifuged (+4°C, 1500 g, 10 min) and the resulting plasma stored in appropriately labelled polypropylene tubes in a freezer set to maintain a temperature of -80°C until determination of plasma pharmacokinetics. The additional 0.25 mL blood samples in the 1.5 mL PCR RNAlater tubes containing 650 µL of RNAlater were stored in a refrigerator at 4°C until determination of blood pharmacodynamics.

Following the last sample collection each animal was sacrificed by anesthetic overdose by IP injection of Pentobarbitone Na as soon as practically possible and death was confirmed by cervical dislocation.

The lungs from each animal were removed and divided into 4 equal pieces immediately after extraction. The first two sections of lung (labelled left and right) were placed in 5 mL RNAlater tissue protect tube containing 5 mL RNAlater stabilisation reagent and stored at 4°C to allow RNA stabilization (PD analysis). The remaining two sections (labelled left and right) were weighed and snap frozen by immersion in liquid nitrogen in polypropylene tubes (PK analysis).

The liver was also collected from each animal. Six representative pieces (of a similar size to the lung pieces) from different areas (no more than 0.5 cm thickness) were collected. Four pieces (no more than 0.5 cm thickness) were placed in two separate (two pieces per tube) 5 mL RNAlater tissue protect tubes containing 5 mL RNAlater stabilisation reagent (tissue sections were completely submerged into the RNA later solution) and stored at 4°C (PD analysis). The remaining two pieces were weighed and snap frozen by immersion in liquid nitrogen in separate polypropylene tubes (a maximum of 0.5 g per tube, PK analysis).

In some studies, the heart, spleen and brain were also collected from each animal. These tissues were sectioned into four equal sized pieces and two pieces placed in a single 5 mL RNA later tissue protect tube containing 5 mL RNAlater stabilisation reagent and stored at 4°C. The remaining two pieces were weighed and placed individually into polypropylene tubes and snap frozen by immersion in liquid nitrogen.

Study sample tubes containing RNAlater RNA Stabilisation Reagent were stored at ca +4 °C to allow RNA stabilisation reagent to perfuse the tissue. Sections snap frozen were stored in a freezer set to maintain a temperature of -80°C.

PK study samples were quantified using a method based on protein precipitation and LC-MS/MS analysis. Prior to analysis defrosted tissue samples were weighed and homogenised following the addition of HPLC grade water using an Omni-Prep Bead Ruptor (Omni Inc., Kennesaw, GA) at 4°C. Plasma and tissue homogenate samples were extracted using protein precipitation with acetonitrile acidified with 0.1% formic acid containing internal standard(s). Samples were mixed and centrifuged at 4000 rpm at 4°C for 30 minutes to remove precipitated proteins, and the supernatant diluted appropriately with HPLC grade water in a 96-well plate. Representative aliquots of plasma and tissue homogenates were assayed for test item by LC-MS/MS using a Waters Xevo TQ-S (Waters, Elstree, UK) against matrix matched calibration curves and quality control standards. The standards were prepared by spiking aliquots of control plasma and tissue homogenate with the test item and extracted as described for the experimental samples.

RNA extraction and real-time PCR analysis for NQO1 gene expression in in-vivo PD studies was performed as detailed below.

Total RNA was isolated using RNeasy plus mini RNA isolation kit (Qiagen) or Mouse RiboPure^{™}-Blood RNA Isolation Kit (ThermoFisher Scientific) according to the manufacturer's instructions and quantified using an Agilent RNA 6000 Nano instrument. Real-time PCR was performed using the C-1000 Thermal Cycler (Bio-Rad) using rat beta actin as the internal control. The cDNA was amplified with specific primer for NQO1/Nqo1 using universal master mix (Applied Biosystems). The primers/probes sets used for amplification of cDNA were obtained from TaqMan Gene Expression Assays (Applied Biosystems). The comparative CT (ΔΔCT) relative quantification method is used to calculate the relative mRNA level of the target gene NQO1 as described in the Applied Biosystems Chemistry Guide. The data is expressed as an increase in target gene mRNA compared to vehicle control treated for each tissue type.

## Claims

1. A pharmaceutical composition comprising a compound according to Formula IC or Formula IF, or a pharmaceutically acceptable salt thereof, and one or more pharmaceutically acceptable excipients: 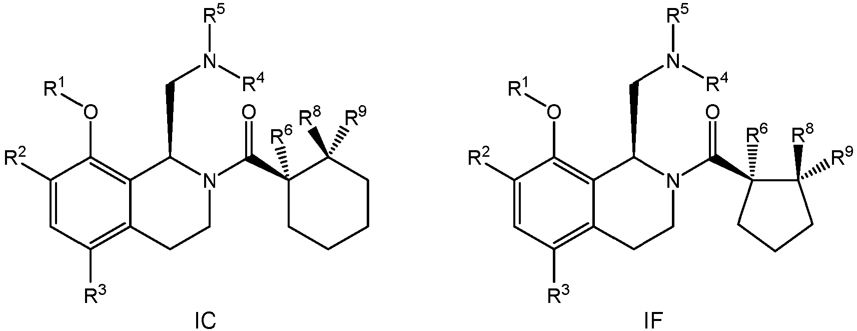 wherein:
R¹ is selected from C₁₋₄alkylene-R¹¹, heterocyclyl and 8-10 membered bicyclic heteroaryl, wherein said heterocyclyl is optionally substituted with one or more substituents independently selected from C₁₋₄alkyl, -C(O)-R¹², SO₂-R¹³, C₁₋₃alkylene-OR¹⁴ and heteroaryl which is optionally substituted with one or more substituents independently selected from C₁₋₄alkyl, C₃₋₇cycloalkyl, halo, OH, C₁₋₃alkoxy and cyano; and wherein said 8-10 membered bicyclic heteroaryl is optionally substituted with one or more substituents independently selected from C₁₋₄alkyl, C₃₋₇cycloalkyl, halo, OH and C₁₋₃alkoxy;
R² is selected from hydrogen, fluoro, chloro and C₁₋₃alkyl;
R³ is selected from hydrogen, fluoro, chloro, bromo, C₁₋₃alkoxy, C₁₋₃alkyl, C₁₋₃haloalkyl and cyano;
R⁴ is hydrogen or C₁₋₄alkyl;
R⁵ is -C(O)-C₁₋₄alkyl, -C(O)-C₃₋₇cycloalkyl, -C(O)-heteroaryl or -C(O)-aryl, wherein said heteroaryl and aryl are optionally substituted with one or more substituents selected from C₁₋₄alkyl, halo, hydroxy, C₁₋₃alkoxy, CO₂R¹⁵ and cyano; or
R⁴ and R⁵, taken together with the nitrogen atom to which they are attached, form a 4-, 5-, or 6-membered heterocyclyl ring, wherein said heterocyclyl ring:
comprises one or more -C(O)- moieties attached to the nitrogen atom;
optionally contains one or more additional heteroatoms selected from oxygen, nitrogen and sulfur;
optionally is fused to an aryl or heteroaryl ring;
optionally is spiro-attached to a C₃₋₇cycloalkyl group or a 3- to 6-membered heterocyclyl ring; and
optionally is substituted with one or more substituents independently selected from C₁₋₄alkyl, halo, OH, C₁₋₃alkoxy, C₁₋₃haloalkyl, cyano, NR¹⁶R¹⁷, C(O)R¹⁸, S(O)R¹⁹ and SO₂R²⁰;
R⁶ is selected from hydrogen, C₁₋₄alkyl, and C₃₋₇cycloalkyl;
R⁸ is C(=O)NR^{8a}R^{8b} or -C(=O)R^{8c};
R^{8a} is hydrogen or C₁₋₆alkyl;
R^{8b} is hydrogen, C₁₋₄alkyl or C₃₋₅cycloalkyl, wherein the C₁₋₄alkyl group is optionally substituted with one or more substituents independently selected from halo, OH, C₁₋₃alkoxy, C₃₋₇cycloalkyl and cyano; or
R^{8a} and R^{8b}, taken together with the nitrogen atom to which they are attached, form a 3-, 4-, 5-, 6-, or 7-membered heterocyclyl ring, wherein the heterocyclyl ring:
optionally contains one or more additional heteroatoms selected from oxygen, nitrogen and sulfur; and
is optionally substituted with one or more substituents independently selected from C₁₋₄alkyl, C₃₋₇cycloalkyl, halo, OH, C₁₋₃alkoxy, C₁₋₃haloalkyl, cyano, NR¹⁶R¹⁷, C(O)R¹⁸, S(O)R¹⁹ and SO₂R²⁰;
R^{8c} is C₁₋₃alkyl or C₁₋₃haloalkyl;
R⁹ is methyl;
R¹¹ is selected from -C(O)-R²⁴, -SO₂-R²⁶, -NR²⁶C(O)-R²⁷, -NR²⁸SO₂-R²⁹, heterocyclyl, aryl and heteroaryl, wherein said aryl and heteroaryl groups are optionally substituted with one or more substituents independently selected from C₁₋₄alkyl, C₁₋₃haloalkyl, C₃₋₇cycloalkyl, C₁₋₄alkylene-R³⁰, halo, OH, C₁₋₃alkoxy, heterocyclyl and cyano; and said heterocyclyl group is optionally substituted with one or more substituents independently selected from C₁₋₄alkyl, C₁₋₃haloalkyl, C₃₋₇cycloalkyl, C₁₋₄alkylene-R³⁰, halo, OH, C₁₋₃alkoxy, oxo and cyano;
R¹² is selected from C₁₋₄alkyl, C₃₋₇cycloalkyl, OR³¹, NR³²R³³, aryl and heteroaryl, wherein said aryl and heteroaryl are optionally substituted with one or more substituents independently selected from C₁₋₄alkyl, halo, OH, C₁₋₃alkoxy and cyano;
R¹³ is selected from C₁₋₄alkyl, C₃₋₇cycloalkyl, heteroaryl, heterocyclyl and NR³⁴R³⁵, wherein said heteroaryl and heterocyclyl are optionally substituted with one or more substituents independently selected from C₁₋₄alkyl, halo, OH, C₁₋₃alkoxy and cyano;
R¹⁷ is selected from hydrogen, C₁₋₄alkyl, C(O)C₁₋₃alkyl and C(O)NR³⁶R³⁷;
R¹⁸, R¹⁹ and R²⁰ are independently selected from C₁₋₄alkyl, OH, C₁₋₃alkoxy and NR³⁸R³⁹;
R²⁴ is selected from C₁₋₄alkyl, NR⁴⁰R⁴¹ and OR⁴²;
R²⁵ is selected from C₁₋₄alkyl and NR⁴³R⁴⁴;
R²⁷ is selected from C₁₋₄alkyl, C₃₋₇cycloalkyl, C₁₋₃haloalkyl, heterocyclyl, aryl and heteroaryl, wherein said aryl and heteroaryl are optionally substituted with one or more substituents independently selected from C₁₋₄alkyl, C₁₋₃haloalkyl, C₃₋₇cycloalkyl, C₁₋₄alkylene-R⁴⁵, halo, OH, C₁₋₃alkoxy and cyano;
R²⁹ is selected from C₁₋₄alkyl, C₃₋₇cycloalkyl, C₁₋₃haloalkyl, aryl and heteroaryl, wherein said aryl and heteroaryl are optionally substituted with one or more substituents independently selected from C₁₋₄alkyl, C₁₋₃haloalkyl, C₃₋₇cycloalkyl, C₁₋₄alkylene-R⁴⁶ halo, OH, C₁₋₃alkoxy and cyano;
R³⁰ is selected from hydroxy, C₁₋₃alkoxy, C₃₋₇cycloalkyl, cyano and NR⁴⁷R⁴⁸;
R⁴⁰ is selected from hydrogen and C₁₋₄alkyl;
R⁴¹ is selected from hydrogen, C₁₋₄alkyl, C₃₋₇cycloalkyl, C₁₋₃alkoxy, aryl and heteroaryl; or
R⁴⁰ and R⁴¹, taken together with the nitrogen atom to which they are attached, form a 4-, 5-, or 6-membered heteroaryl or heterocyclyl ring, wherein said heteroaryl and heterocyclyl rings are optionally substituted with one or more substituents independently selected from C₁₋₄alkyl, halo, OH, C₁₋₃alkoxy, C₃₋₇cycloalkyl and cyano;
R⁴⁵ and R⁴⁶ are independently selected from hydroxy, C₁₋₃alkoxy and C₃₋₇cycloalkyl; and
R¹⁴, R¹⁵, R¹⁶, R²⁶, R²⁸, R³¹, R³², R³³, R³⁴, R³⁵, R³⁶, R³⁷, R³⁸, R³⁹, R⁴², R⁴³, R⁴⁴, R⁴⁷ and R⁴⁸ are independently selected from hydrogen, C₁₋₄alkyl and C₃₋₇cycloalkyl.

2. A pharmaceutical composition according to claim 1, wherein said compound has the structural formulae ID or IG shown below: 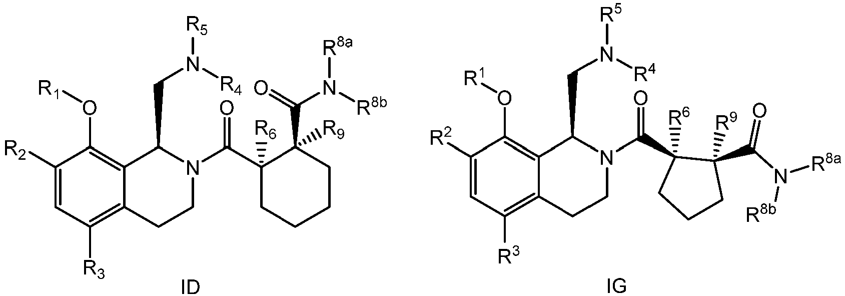 wherein R¹ to R⁶, R^{8a}, R^{8b} and R⁹ are as defined in claim 1.

3. A pharmaceutical composition according to claim 1 or 2, wherein R¹ is C₁₋₄alkylene-R¹¹, such as CH₂-R¹¹.

4. A pharmaceutical composition according to any one of claims 1 to 3, wherein R¹¹ is:
a. selected from -C(O)-R²⁴, -SO₂-R²⁶, -NR²⁶C(O)-R²⁷, -NR²⁸SO₂-R²⁰ and heteroaryl, wherein said heteroaryl is optionally substituted with one or more substituents independently selected from C₁₋₄alkyl, C₁₋₃haloalkyl, C₃₋₇cycloalkyl, C₁₋₄alkylene-R³⁰, halo, OH, C₁₋₃alkoxy, heterocyclyl and cyano;
b. heteroaryl optionally substituted with one or more substituents independently selected from C₁₋₄alkyl, C₁₋₃haloalkyl, C₃₋₇cycloalkyl, C₁₋₄alkylene-R³⁰, halo, OH, C₁₋₃alkoxy, heterocyclyl and cyano;
c. heteroaryl optionally substituted with one or more substituents independently selected from methyl, ethyl, isopropyl, difluoromethyl, trifluoromethyl, chloro, fluoro, cyclopropyl, methoxy, CH₂-R³⁰ and CH₂CH₂-R³⁰;
d. pyridyl, pyrimidinyl, pyridazinyl, pyrazinyl, oxazolyl, isoxazolyl, oxadiazolyl, 1,2,3-triazolyl, 1,2,4-triazolyl, 1,2,4-oxadiazolyl, indazolyl, benzotriazolyl, benzisoxazolyl, isoxazolopyridinyl, imidazopyridinyl or triazolopyridinyl, each optionally substituted with one or more substituents independently selected from C₁₋₄alkyl, C₁₋₃haloalkyl, C₃₋₇cycloalkyl, C₁₋₄alkylene-R³⁰, halo, OH, C₁₋₃alkoxy, heterocyclyl and cyano; or
e. selected from: 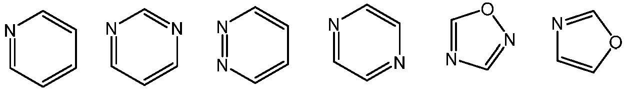 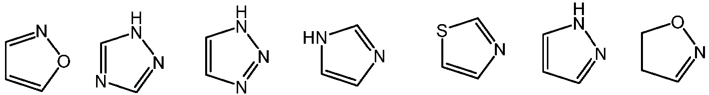 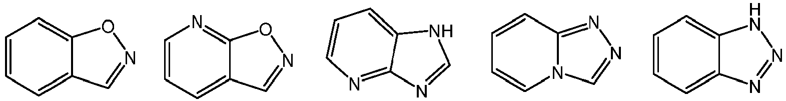 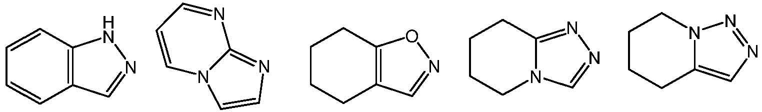 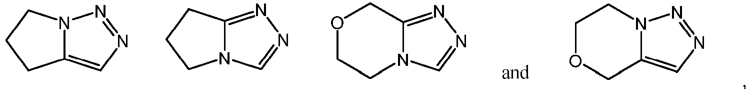 optionally substituted with one or more substituents independently selected from C₁₋₄alkyl, C₁₋₃haloalkyl, C₃₋₇cycloalkyl, C₁₋₄alkylene-R³⁰, halo, OH, C₁₋₃alkoxy, heterocyclyl and cyano.

5. A pharmaceutical composition according to claim 1 or 2, wherein R¹ is:
a. selected from one of the following groups: 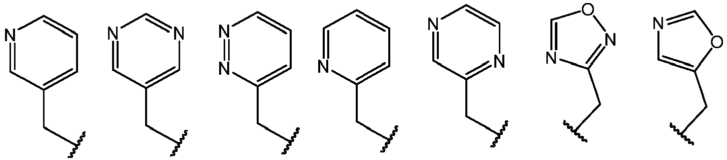 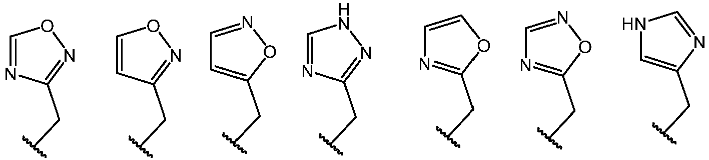 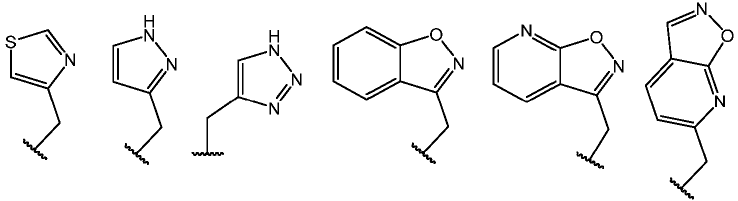 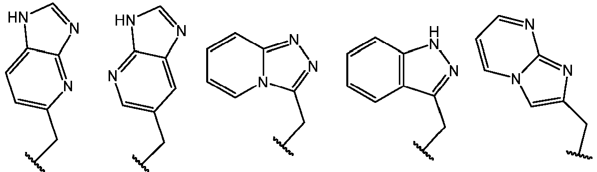 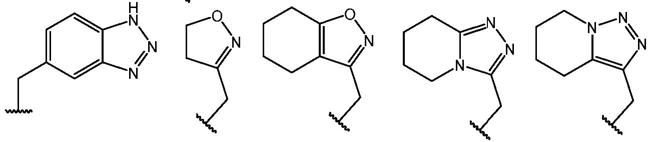 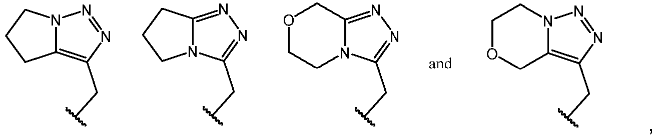 wherein 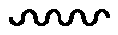 represents the point of attachment of the group to the oxygen atom of the rest of the compound and wherein each group is optionally substituted with one or more substituents independently selected from C₁₋₄alkyl, C₁₋₃haloalkyl, C₃₋₇cycloalkyl, C₁₋₄alkylene-R³⁰, halo, OH, C₁₋₃alkoxy, heterocyclyl and cyano;
b. heterocyclyl optionally substituted with one or more substituents independently selected from C₁₋₄alkyl, -C(O)-R¹², SO₂-R¹³, heteroaryl and C₁₋₃alkylene-OR¹⁴, wherein said heteroaryl is optionally substituted with one or more substituents independently selected from C₁₋₄alkyl, C₃₋₇cycloalkyl, halo, OH, C₁₋₃alkoxy and cyano;
c. piperidinyl or pyrrolidinyl, each optionally substituted with one or more substituents independently selected from -C(O)-R¹², SO₂-R¹³, heteroaryl and C₁₋₃alkylene-OR¹⁴, wherein said heteroaryl is optionally substituted with one or more substituents independently selected from C₁₋₄alkyl, C₃₋₇cycloalkyl, halo, OH, C₁₋₃alkoxy and cyano;
d. pyrrolidinyl optionally substituted with one or more substituents independently selected from -C(O)-R¹², SO₂-R¹³, heteroaryl and C₁₋₃alkylene-OR¹⁴, wherein said heteroaryl is optionally substituted with C₁₋₄alkyl or C₃₋₇cycloalkyl; or
e. selected from one of the following groups: 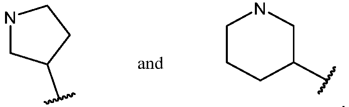 wherein 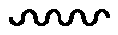 represents the point of attachment of the group to the oxygen atom of the rest of the compound and wherein each group is optionally substituted with one or more substituents independently selected from -C(O)-R¹², SO₂-R¹³, heteroaryl and C₁₋₃alkylene-OR¹⁴, wherein said heteroaryl is optionally substituted with C₁₋₄alkyl or C₃₋₇cycloalkyl.

6. A pharmaceutical composition according to any one of claims 1 to 5, wherein R² is hydrogen or fluoro.

7. A pharmaceutical composition according to any one of claims 1 to 6, wherein R³ is hydrogen or chloro, such as chloro.

8. A pharmaceutical composition according to any one of claims 1 to 7, wherein R⁴ is hydrogen and R⁵ is -C(O)-C₁₋₄alkyl, -C(O)-C₃₋₇cycloalkyl, or -C(O)-aryl, wherein said aryl is optionally substituted with one or more substituents selected from C₁₋₄alkyl, halo, hydroxy, C₁₋₃alkoxy, CO₂R¹⁵ and cyano.

9. A pharmaceutical composition according to any one of claims 1 to 7, wherein R⁴ and R⁵, taken together with the nitrogen atom to which they are attached:
a. form a 5-, or 6-membered heterocyclyl ring, wherein said heterocyclyl ring:
comprises one or more -C(O)- moieties attached to the nitrogen atom; optionally contains one or more additional heteroatoms selected from oxygen, nitrogen and sulfur;
optionally is fused to an aryl ring;
optionally is spiro-attached to a C₃₋₇cycloalkyl group or a 3- to 5-membered heterocyclyl ring; and
optionally is substituted with one or more substituents independently selected from C₁₋₄alkyl, halo, OH, C₁₋₃alkoxy, C₁₋₃haloalkyl, cyano, NR¹⁶R¹⁷, C(O)R¹⁸, S(O)R¹⁹ and SO₂R²⁰;
b. form a heterocyclic moiety selected from one of the following: 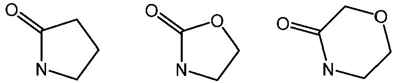 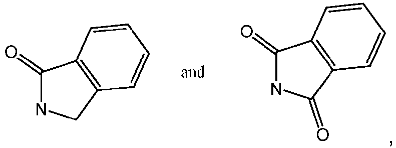 wherein the saturated ring of the heterocyclic moiety is optionally spiro-attached to a C₃₋₇cycloalkyl group, and wherein said heterocyclic moiety is optionally substituted with one or more substituents independently selected from C₁₋₄alkyl, halo and OH; or
c. form the following heterocyclic moiety: 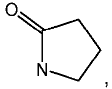 wherein the heterocyclic moiety is optionally spiro-attached to a cyclopropyl group and is optionally substituted with one or more substituents independently selected from methyl and fluoro.

10. A pharmaceutical composition according to any one of claims 1 to 9, wherein R⁶ is hydrogen.

11. A pharmaceutical composition according to any one of claims 1 to 10, wherein R⁸ is C(=O)NR^{8a}R^{8b}.

12. A pharmaceutical composition according to claim 11, wherein R^{8a} is hydrogen.

13. A pharmaceutical composition according to claim 11 or 12, wherein R^{8b} is:
a. C₁₋₄alkyl or C₃₋₅cycloalkyl, wherein the C₁₋₄alkyl group is optionally substituted with one or more substituents independently selected from fluoro, OH, C₁₋₃alkoxy, C₃₋₇cycloalkyl and cyano; or
b. methyl.

14. A pharmaceutical composition according to claim 11, wherein R^{8a} and R^{8b}, taken together with the nitrogen atom to which they are attached, form a 4-, or 5-membered heterocyclyl ring, wherein the heterocyclyl ring is optionally substituted with one or more substituents independently selected from C₁₋₄alkyl, halo, OH, C₁₋₃alkoxy, C₁₋₃haloalkyl, C₃₋₇cycloalkyl, cyano, NR¹⁶R¹⁷, C(O)R¹⁸, S(O)R¹⁹ and SO₂R²⁰.

15. A pharmaceutical composition according to claim 1 or claims 3 to 10, wherein R^{8c} is C₁₋₃fluoroalkyl.

16. A pharmaceutical composition according to claim 1, wherein the compound is selected from any one of the following compounds:
(1S,2R)-2-((S)-5-chloro-8-((5-(difluoromethyl)-1-methyl-1H-1,2,3-triazol-4-yl)methoxy)-1-((2-oxopyrrolidin-1-yl)methyl)-1,2,3,4-tetrahydroisoquinoline-2-carbonyl)-N,1-dimethylcyclohexane-1-carboxamide;
(1S,2R)-2-((S)-8-(benzo[d]isoxazol-3-ylmethoxy)-5-chloro-1-((2-oxopyrrolidin-1-yl)methyl)-1,2,3,4-tetrahydroisoquinoline-2-carbonyl)-N,1-dimethylcyclohexane-1-carboxamide;
(1S,2R)-2-((S)-5-chloro-8-((5-(difluoromethyl)-1-methyl-1H-1,2,3-triazol-4-yl)methoxy)-1-((2-oxopyrrolidin-1-yl)methyl)-1,2,3,4-tetrahydroisoquinoline-2-carbonyl)-N,1-dimethylcyclopentane-1-carboxamide;
(1S,2R)-2-((S)-5-chloro-8-((1,5-dimethyl-1H-1,2,3-triazol-4-yl)methoxy)-1-((2-oxopyrrolidin-1-yl)methyl)-1,2,3,4-tetrahydroisoquinoline-2-carbonyl)-N,1-dimethylcyclohexane-1-carboxamide;
(1S,2R)-2-((S)-5-chloro-8-((1-methyl-5-(trifluoromethyl)-1H-1,2,3-triazol-4-yl)methoxy)-1-((2-oxopyrrolidin-1-yl)methyl)-1,2,3,4-tetrahydroisoquinoline-2-carbonyl)-N,1-dimethylcyclohexane-1-carboxamide;
(1S,2R)-2-((S)-5-chloro-8-((1-methyl-1H-1 ,2,3-triazol-4-yl)methoxy)-1-((2-oxopyrrolidin-1-yl)methyl)-1,2,3,4-tetrahydroisoquinoline-2-carbonyl)-N,1-dimethylcyclohexane-1-carboxamide;
(1S,2R)-2-((S)-5-chloro-8-((5-(difluoromethyl)-1-methyl-1H-1,2,3-triazol-4-yl)methoxy)-1-((6-oxo-5-azaspiro[2.4]heptan-5-yl)methyl)-1,2,3,4-tetrahydroisoquinoline-2-carbonyl)-N,1-dimethylcyclopentane-1-carboxamide;
(1S,2R)-2-((1S)-5-chloro-8-((5-(difluoromethyl)-1-methyl-1H-1,2,3-triazol-4-yl)methoxy)-1-((3-methyl-2-oxopyrrolidin-1-yl)methyl)-1,2,3,4-tetrahydroisoquinoline-2-carbonyl)-N,1-dimethylcyclopentane-1-carboxamide;
(1S,2R)-2-((1S)-5-chloro-8-((5-(difluoromethyl)-1-methyl-1H-1,2,3-triazol-4-yl)methoxy)-1-((3-methyl-2-oxopyrrolidin-1-yl)methyl)-1,2,3,4-tetrahydroisoquinoline-2-carbonyl)-N,1-dimethylcyclohexane-1-carboxamide;
(1S,2R)-2-((S)-5-chloro-8-((1-methyl-5-(trifluoromethyl)-1H-1,2,3-triazol-4-yl)methoxy)-1-((6-oxo-5-azaspiro[2.4]heptan-5-yl)methyl)-1,2,3,4-tetrahydroisoquinoline-2-carbonyl)-N,1-dimethylcyclohexane-1-carboxamide;
(1S,2R)-2-((S)-5-chloro-8-((5-cyclopropyl-1-methyl-1H-1,2,3-triazol-4-yl)methoxy)-1-((2-oxopyrrolidin-1-yl)methyl)-1,2,3,4-tetrahydroisoquinoline-2-carbonyl)-N,1-dimethylcyclohexane-1-carboxamide;
(1S,2R)-2-((S)-5-chloro-8-((5-(difluoromethyl)-1-methyl-1H-1,2,3-triazol-4-yl)methoxy)-1-((6-oxo-5-azaspiro[2.4]heptan-5-yl)methyl)-1,2,3,4-tetrahydroisoquinoline-2-carbonyl)-1-methylcyclohexane-1-carboxamide;
(1S,2R)-2-((S)-5-chloro-8-((1,5-dimethyl-1H-1,2,3-triazol-4-yl)methoxy)-1-((6-oxo-5-azaspiro[2.4]heptan-5-yl)methyl)-1,2,3,4-tetrahydroisoquinoline-2-carbonyl)-N,1-dimethylcyclohexane-1-carboxamide;
(1S,2R)-2-((S)-5-chloro-8-((1-methyl-1H-1,2,3-triazol-4-yl)methoxy)-1-((6-oxo-5-azaspiro[2.4]heptan-5-yl)methyl)-1,2,3,4-tetrahydroisoquinoline-2-carbonyl)-N,1-dimethylcyclohexane-1-carboxamide;
(1S,2R)-2-((S)-5-chloro-8-((5-cyclopropyl-1-methyl-1H-1,2,3-triazol-4-yl)methoxy)-1-((6-oxo-5-azaspiro[2.4]heptan-5-yl)methyl)-1,2,3,4-tetrahydroisoquinoline-2-carbonyl)-N,1-dimethylcyclohexane-1-carboxamide;
(1S,2R)-2-((S)-5-chloro-8-((1-methyl-1H-1,2,3-triazol-4-yl)methoxy)-1-(((R)-4-methyl-2-oxopyrrolidin-1-yl)methyl)-1,2,3,4-tetrahydroisoquinoline-2-carbonyl)-N,1-dimethylcyclohexane-1-carboxamide;
(1S,2R)-2-((S)-5-chloro-8-((5-(methoxymethyl)-1-methyl-1H-1,2,3-triazol-4-yl)methoxy)-1-(((R)-4-methyl-2-oxopyrrolidin-1-yl)methyl)-1,2,3,4-tetrahydroisoquinoline-2-carbonyl)-N,1-dimethylcyclohexane-1-carboxamide;
(1S,2R)-2-((S)-5-chloro-8-((5-(difluoromethyl)-1-methyl-1H-1,2,3-triazol-4-yl)methoxy)-7-fluoro-1-((6-oxo-5-azaspiro[2.4]heptan-5-yl)methyl)-1,2,3,4-tetrahydroisoquinoline-2-carbonyl)-N,1-dimethylcyclohexane-1-carboxamide;
(1S,2R)-2-((S)-5-chloro-8-((5-(difluoromethyl)-1-methyl-1H-1,2,3-triazol-4-yl)methoxy)-7-fluoro-1-((6-oxo-5-azaspiro[2.4]heptan-5-yl)methyl)-1,2,3,4-tetrahydroisoquinoline-2-carbonyl)-1-methylcyclohexane-1-carboxamide;
(1S,2R)-2-((S)-5-chloro-8-((5-(difluoromethyl)-1-methyl-1H-1,2,3-triazol-4-yl)methoxy)-7-fluoro-1-((3-oxomorpholino)methyl)-1,2,3,4-tetrahydroisoquinoline-2-carbonyl)-N,1-dimethylcyclohexane-1-carboxamide;
(1S,2R)-2-((S)-5-chloro-8-((5-(difluoromethyl)-1-methyl-1H-1,2,3-triazol-4-yl)methoxy)-7-fluoro-1-((2-oxopyrrolidin-1-yl)methyl)-1,2,3,4-tetrahydroisoquinoline-2-carbonyl)-N,1-dimethylcyclohexane-1-carboxamide;
(1S,2R)-2-((S)-5-chloro-7-fluoro-8-((1-methyl-1H-1,2,3-triazol-4-yl)methoxy)-1-((6-oxo-5-azaspiro[2.4]heptan-5-yl)methyl)-1,2,3,4-tetrahydroisoquinoline-2-carbonyl)-N,1-dimethylcyclohexane-1-carboxamide;
(1S,2R)-2-((S)-5-chloro-7-fluoro-1-((6-oxo-5-azaspiro[2.4]heptan-5-yl)methyl)-8-((4,5,6,7-tetrahydro-[1,2,3]triazolo[1,5-a]pyridin-3-yl)methoxy)-1,2,3,4-tetrahydroisoquinoline-2-carbonyl)-1-methylcyclohexane-1-carboxamide;
(1S,2R)-2-((S)-5-chloro-8-((1,5-dimethyl-1H-1,2,3-triazol-4-yl)methoxy)-7-fluoro-1-((6-oxo-5-azaspiro[2.4]heptan-5-yl)methyl)-1,2,3,4-tetrahydroisoquinoline-2-carbonyl)-1-methylcyclohexane-1-carboxamide; or
(1S,2R)-2-((S)-5-chloro-8-((5-cyclopropyl-1-methyl-1H-1,2,3-triazol-4-yl)methoxy)-1-(((R)-4-methyl-2-oxopyrrolidin-1-yl)methyl)-1,2,3,4-tetrahydroisoquinoline-2-carbonyl)-N,1-dimethylcyclohexane-1-carboxamide;
or a pharmaceutically acceptable salt thereof.

17. A pharmaceutical composition according to any one of claims 1 to 16, for use in:
a. therapy;
b. the treatment of diseases or disorders mediated by Nrf2 activation; or
c. the treatment of chronic obstructive pulmonary disease, acute, chronic and severe asthma, acute lung injury/acute respiratory distress syndrome with or without accompanying multi organ dysfunction syndrome, pulmonary fibrosis including idiopathic pulmonary fibrosis, cystic fibrosis, COVID-19, diabetes, atherosclerosis, hypertension, heart failure, myocardial infarction and repair, cardiac remodelling, cardiac arrhythmias, cardiac hypertrophy, heart failure with preserved ejection fraction, diabetic cardiomyopathy, sarcopenia, obesity, metabolic syndrome, diabetes mellitus, insulin resistance, pulmonary arterial hypertension, subarachnoid haemorrhage, intracerebral haemorrhage, ischemic stroke, beta-thalassemia, sickle cell disease, rheumatoid arthritis, irritable bowel disorder, ulcerative colitis, Crohn's disease, psoriasis, radiation-induced dermatitis, atopic dermatitis, non-alcoholic fatty liver disease, non-alcoholic steatohepatitis, toxin-induced liver disease, viral hepatitis and cirrhosis, chronic kidney disease, diabetic nephropathy, autosomal dominant polycystic kidney disease, CKD associated with type 1 diabetes (T1D), IgA nephropathy (IgAN), Alport Syndrome, focal segmental glomerulosclerosis, Huntington's disease, Parkinson's disease, Alzheimer's disease, amyotrophic lateral sclerosis, frontotemporal dementia, multiple sclerosis, Friedreich's ataxia, chronic pain, schizophrenia, lung cancer, breast cancer, colon cancer, age related macular degeneration (AMD), Fuchs Endothelial Corneal Dystrophy, or uveitis .

## Patentansprüche

1. Pharmazeutische Zusammensetzung, umfassend eine Verbindung nach Formel IC oder Formel IF oder ein pharmazeutisch unbedenkliches Salz davon und einen oder mehrere pharmazeutisch unbedenkliche Hilfsstoffe; 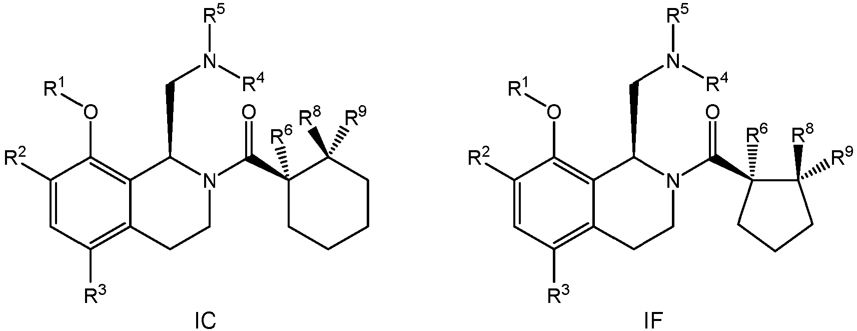 wobei:
R¹ ausgewählt ist aus C₁₋₄-Alkylen-R¹¹, Heterocyclyl und 8-10-gliedrigem bicyclischen Heteroaryl, wobei das Heterocyclyl optional substituiert ist mit einem oder mehreren Substituenten unabhängig ausgewählt aus C₁₋₄-Alkyl, -C(O)-R¹², SO₂-R¹³, C₁₋₃-Alkylen-OR¹⁴ und Heteroaryl, das optional substituiert ist mit einem oder mehreren Substituenten unabhängig ausgewählt aus C₁₋₄-Alkyl, C₃₋₇-Cycloalkyl, Halogen, OH, C₁₋₃-Alkoxy und Cyano; und wobei das 8-10-gliedrige bicyclische Heteroaryl optional substituiert ist mit einem oder mehreren Substituenten unabhängig ausgewählt aus C₁₋₄-Alkyl, C₃₋₇-Cycloalkyl, Halogen, OH und C₁₋₃-Alkoxy;
R² ausgewählt ist aus Wasserstoff, Fluor, Chlor und C₁₋₃-Alkyl;
R³ ausgewählt ist aus Wasserstoff, Fluor, Chlor, Brom, C₁₋₃-Alkoxy, C₁₋₃-Alkyl, C₁₋₃-Halogenalkyl und Cyano;
R⁴ Wasserstoff oder C₁₋₄-Alkyl ist;
R⁵ -C(O)-C₁₋₄-Alkyl, -C(O)-C₃₋₇-Cycloalkyl, -C(O)-Heteroaryl oder-C(O)-Aryl ist, wobei das Heteroaryl und Aryl optional substituiert sind mit einem oder mehreren Substituenten ausgewählt aus C₁₋₄-Alkyl, Halogen, Hydroxy, C₁₋₃-Alkoxy, CO₂R¹⁵ und Cyano; oder
R⁴ und R⁵ zusammengenommen mit dem Stickstoffatom, an das sie gebunden sind, einen 4-, 5- oder 6-gliedrigen Heterocyclylring bilden, wobei der Heterocyclylring:
eine oder mehrere -C(O)--Einheiten umfasst, die an das Stickstoffatom gebunden sind;
optional ein oder mehrere zusätzliche Heteroatome ausgewählt aus Sauerstoff, Stickstoff und Schwefel enthält;
optional an einen Aryl- oder Heteroarylring kondensiert ist;
optional an eine C₃₋₇-Cycloalkylgruppe oder einen 3- bis 6-gliedrigen Heterocyclylring spirogebunden ist; und
optional substituiert ist mit einem oder mehreren Substituenten unabhängig ausgewählt aus C₁₋₄-Alkyl, Halogen, OH, C₁₋₃-Alkoxy, C₁₋₃-Halogenalkyl, Cyano, NR¹⁶R¹⁷, C(O)R¹⁸, S(O)R¹⁹ und SO₂R²⁰;
R⁶ ausgewählt ist aus Wasserstoff, C₁₋₄-Alkyl und C₃₋₇-Cycloalkyl;
R⁸ C(=O)NR^{8a}R^{8b} oder -C(=O)R^{8c} ist;
R^{8a} Wasserstoff oder C₁₋₆-Alkyl ist;
R^{8b} Wasserstoff, C₁₋₄-Alkyl oder C₃₋₅-Cycloalkyl ist, wobei die C₁₋₄-Alkylgruppe optional substituiert ist mit einem oder mehreren Substituenten unabhängig ausgewählt aus Halogen, OH, C₁₋₃-Alkoxy, C₃₋₇-Cycloalkyl und Cyano; oder
R^{8a} und R^{8b} zusammengenommen mit dem Stickstoffatom, an das sie gebunden sind, einen 3-, 4-, 5-, 6- oder 7-gliedrigen Heterocyclylring bilden, wobei der Heterocyclylring:
optional ein oder mehrere zusätzliche Heteroatome ausgewählt aus Sauerstoff, Stickstoff und Schwefel enthält; und
optional substituiert ist mit einem oder mehreren Substituenten unabhängig ausgewählt aus C₁₋₄-Alkyl, C₃₋₇-Cycloalkyl, Halogen, OH, C₁₋₃-Alkoxy, C₁₋₃-Halogenalkyl, Cyano, NR¹⁶R¹⁷, C(O)R¹⁸, S(O)R¹⁹ und SO₂R²⁰;
R^{8c} C₁₋₃-Alkyl oder C₁₋₃-Halogenalkyl ist;
R⁹ Methyl ist;
R¹¹ ausgewählt ist aus -C(O)-R²⁴, -SO₂-R²⁵, -NR²⁶C(O)-R²⁷, -NR²⁸SO₂-R²⁹, Heterocyclyl, Aryl und Heteroaryl, wobei die Aryl- und Heteroarylgruppen optional substituiert sind mit einem oder mehreren Substituenten unabhängig ausgewählt aus C₁₋₄-Alkyl, C₁₋₃-Halogenalkyl, C₃₋₇-Cycloalkyl, C₁₋₄-Alkylen-R³⁰, Halogen, OH, C₁₋₃-Alkoxy, Heterocyclyl und Cyano; und die Heterocyclylgruppe optional substituiert ist mit einem oder mehreren Substituenten unabhängig ausgewählt aus C₁₋₄-Alkyl, C₁₋₃-Halogenalkyl, C₃₋₇-Cycloalkyl, C₁₋₄-Alkylen-R³⁰, Halogen, OH, C₁₋₃-Alkoxy, Oxo und Cyano;
R¹² ausgewählt ist aus C₁₋₄-Alkyl, C₃₋₇-Cycloalkyl, OR³¹, NR³²R³³, Aryl und Heteroaryl, wobei das Aryl und Heteroaryl optional substituiert sind mit einem oder mehreren Substituenten unabhängig ausgewählt aus C₁₋₄-Alkyl, Halogen, OH, C₁₋₃-Alkoxy und Cyano;
R¹³ ausgewählt ist aus C₁₋₄-Alkyl, C₃₋₇-Cycloalkyl, Heteroaryl, Heterocyclyl und NR³⁴R³⁵, wobei das Heteroaryl und Heterocyclyl optional substituiert sind mit einem oder mehreren Substituenten unabhängig ausgewählt aus C₁₋₄-Alkyl, Halogen, OH, C₁₋₃-Alkoxy und Cyano;
R¹⁷ ausgewählt ist aus Wasserstoff, C₁₋₄-Alkyl, C(O)C₁₋₃-Alkyl und C(O)NR³⁶R³⁷;
R¹⁸, R¹⁹ und R²⁰ unabhängig ausgewählt sind aus C₁₋₄-Alkyl, OH, C₁₋₃-Alkoxy und NR³⁸R³⁹;
R²⁴ ausgewählt ist aus C₁₋₄-Alkyl, NR⁴⁰R⁴¹ und OR⁴²;
R²⁵ ausgewählt ist aus C₁₋₄-Alkyl und NR⁴³R⁴⁴;
R²⁷ ausgewählt ist aus C₁₋₄-Alkyl, C₃₋₇-Cycloalkyl, C₁₋₃-Halogenalkyl, Heterocyclyl, Aryl und Heteroaryl, wobei das Aryl und Heteroaryl optional substituiert sind mit einem oder mehreren Substituenten unabhängig ausgewählt aus C₁₋₄-Alkyl, C₁₋₃-Halogenalkyl, C_{3- 7}-Cycloalkyl, C₁₋₄-Alkylen-R⁴⁵, Halogen, OH, C₁₋₃-Alkoxy und Cyano;
R²⁹ ausgewählt ist aus C₁₋₄-Alkyl, C₃₋₇-Cycloalkyl, C₁₋₃-Halogenalkyl, Aryl und Heteroaryl, wobei das Aryl und Heteroaryl optional substituiert sind mit einem oder mehreren Substituenten unabhängig ausgewählt aus C₁₋₄-Alkyl, C₁₋₃-Halogenalkyl, C₃₋₇-Cycloalkyl, C₁₋₄-Alkylen-R⁴⁶-halogen, OH, C₁₋₃-Alkoxy und Cyano;
R³⁰ ausgewählt ist aus Hydroxy, C₁₋₃-Alkoxy, C₃₋₇-Cycloalkyl, Cyano und NR⁴⁷R⁴⁸;
R⁴⁰ ausgewählt ist aus Wasserstoff und C₁₋₄-Alkyl;
R⁴¹ ausgewählt ist aus Wasserstoff, C₁₋₄-Alkyl, C₃₋₇-Cycloalkyl, C₁₋₃-Alkoxy, Aryl und Heteroaryl, oder
R⁴⁰ und R⁴¹ zusammengenommen mit dem Stickstoffatom, an das sie gebunden sind, einen 4-, 5- oder 6-gliedrigen Heteroaryl- oder Heterocyclylring bilden, wobei die Heteroaryl- und Heterocyclylringe optional substituiert sind mit einem oder mehreren Substituenten unabhängig ausgewählt aus C₁₋₄-Alkyl, Halogen, OH, C₁₋₃-Alkoxy, C₃₋₇-Cycloalkyl und Cyano;
R⁴⁵ und R⁴⁶ unabhängig ausgewählt sind aus Hydroxy, C₁₋₃-Alkoxy und C₃₋₇-Cycloalkyl; und
R¹⁴, R¹⁵, R¹⁶, R²⁶, R²⁸, R³¹, R³², R³³, R³⁴, R³⁵, R³⁶, R³⁷, R³⁸, R³⁹, R⁴², R⁴³, R⁴⁴, R⁴⁷ und R⁴⁸ unabhängig ausgewählt sind aus Wasserstoff, C₁₋₄-Alkyl und C₃₋₇-Cycloalkyl.

2. Pharmazeutische Zusammensetzung nach Anspruch 1, wobei die Verbindung die unten gezeigten Strukturformeln ID oder IG aufweist: 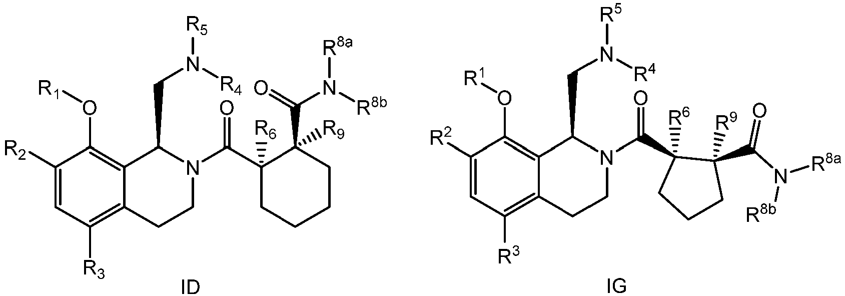 wobei R¹ bis R⁶, R^{8a}, R^{8b} und R⁹ wie in Anspruch 1 definiert sind.

3. Pharmazeutische Zusammensetzung nach Anspruch 1 oder 2, wobei R¹ C₁₋₄-Alkylen-R¹¹ ist, wie CH₂-R¹¹.

4. Pharmazeutische Zusammensetzung nach einem der Ansprüche 1 bis 3, wobei R¹¹ wie folgt ist:
a. ausgewählt aus -C(O)-R²⁴, -SO₂-R²⁵, -NR²⁶C(O)-R²⁷, -NR²⁸SO₂-R²⁹ und Heteroaryl, wobei das Heteroaryl optional substituiert ist mit einem oder mehreren Substituenten unabhängig ausgewählt aus C₁₋₄-Alkyl, C₁₋₃-Halogenalkyl, C₃₋₇-Cycloalkyl, C₁₋₄-Alkylen-R³⁰, Halogen, OH, C₁₋₃-Alkoxy, Heterocyclyl und Cyano;
b. Heteroaryl optional substituiert mit einem oder mehreren Substituenten unabhängig ausgewählt aus C₁₋₄-Alkyl, C₁₋₃-Halogenalkyl, C₃₋₇-Cycloalkyl, C₁₋₄-Alkylen-R³⁰, Halogen, OH, C₁₋₃-Alkoxy, Heterocyclyl und Cyano;
c. Heteroaryl optional substituiert mit einem oder mehreren Substituenten unabhängig ausgewählt aus Methyl, Ethyl, Isopropyl, Difluormethyl, Trifluormethyl, Chlor, Fluor, Cyclopropyl, Methoxy, CH₂-R³⁰ und CH₂CH₂-R³⁰;
d. Pyridyl, Pyrimidinyl, Pyridazinyl, Pyrazinyl, Oxazolyl, Isoxazolyl, Oxadiazolyl, 1,2,3-Triazolyl, 1,2,4-Triazolyl, 1,2,4-Oxadiazolyl, Indazolyl, Benzotriazolyl, Benzisoxazolyl, Isoxazolopyridinyl, Imidazopyridinyl oder Triazolopyridinyl, jeweils optional substituiert mit einem oder mehreren Substituenten unabhängig ausgewählt aus C₁₋₄-Alkyl, C₁₋₃-Halogenalkyl, C₃₋₇-Cycloalkyl, C₁₋₄-Alkylen-R³⁰, Halogen, OH, C₁₋₃-Alkoxy, Heterocyclyl und Cyano; oder
e. ausgewählt aus: 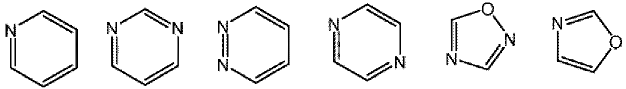 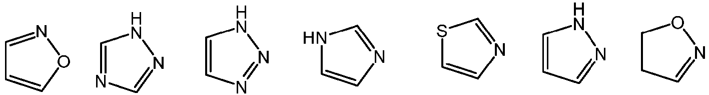 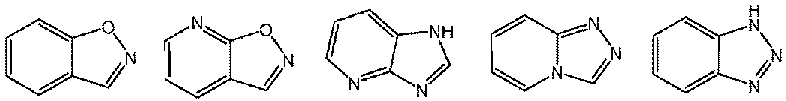 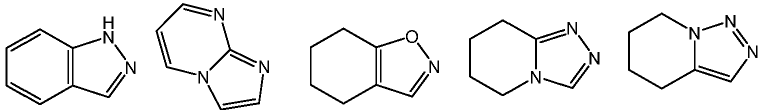 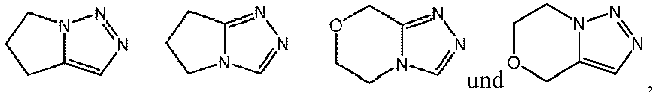 optional substituiert mit einem oder mehreren Substituenten unabhängig ausgewählt aus C₁₋₄-Alkyl, C₁₋₃-Halogenalkyl, C₃₋₇-Cycloalkyl, C₁₋₄-Alkylen-R³⁰, Halogen, OH, C₁₋₃-Alkoxy, Heterocyclyl und Cyano.

5. Pharmazeutische Zusammensetzung nach Anspruch 1 oder 2, wobei R¹ wie folgt ist:
a. ausgewählt aus einer der folgenden Gruppen: 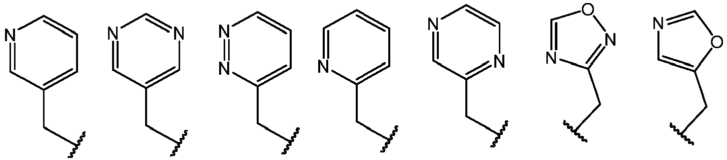 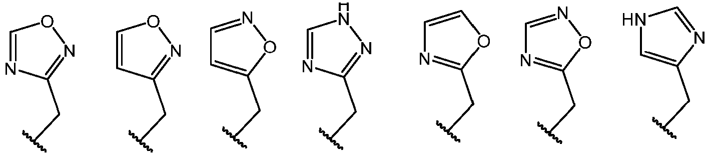 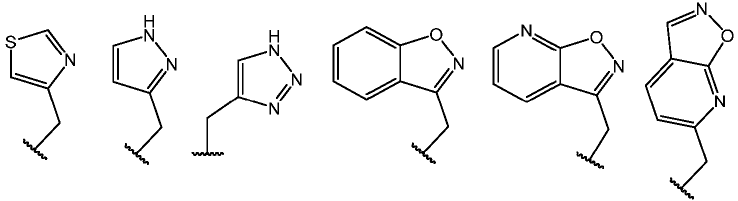 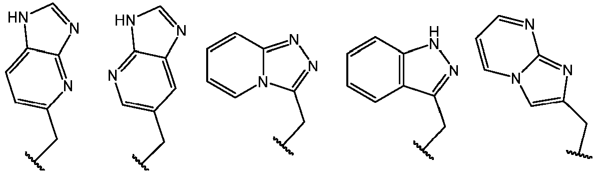 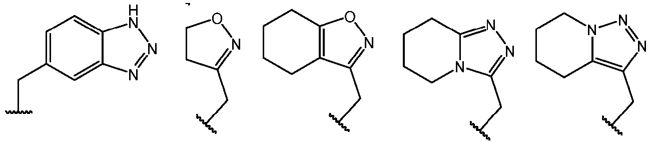 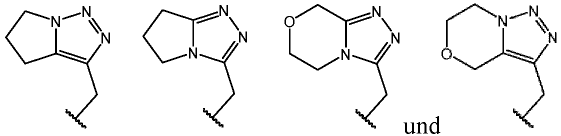 wobei 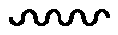 den Bindungspunkt der Gruppe an das Sauerstoffatom des Rests der Verbindung darstellt und wobei jede Gruppe optional substituiert ist mit einem oder mehreren Substituenten unabhängig ausgewählt aus C₁₋₄-Alkyl, C₁₋₃-Halogenalkyl, C₃₋₇-Cycloalkyl, C₁₋₄-Alkylen-R³⁰, Halogen, OH, C₁₋₃-Alkoxy, Heterocyclyl und Cyano;
b. Heterocyclyl optional substituiert mit einem oder mehreren Substituenten unabhängig ausgewählt aus C₁₋₄-Alkyl, -C(O)-R¹², SO₂-R¹³, Heteroaryl und C₁₋₃-Alkylen-OR¹⁴, wobei das Heteroaryl optional substituiert ist mit einem oder mehreren Substituenten unabhängig ausgewählt aus C₁₋₄-Alkyl, C₃₋₇-Cycloalkyl, Halogen, OH, C₁₋₃-Alkoxy und Cyano;
c. Piperidinyl oder Pyrrolidinyl, jeweils optional substituiert mit einem oder mehreren Substituenten unabhängig ausgewählt aus -C(O)-R¹², SO₂-R¹³, Heteroaryl und C₁₋₃-Alkylen-OR¹⁴, wobei das Heteroaryl optional substituiert ist mit einem oder mehreren Substituenten unabhängig ausgewählt aus C₁₋₄-Alkyl, C₃₋₇-Cycloalkyl, Halogen, OH, C₁₋₃-Alkoxy und Cyano;
d. Pyrrolidinyl optional substituiert mit einem oder mehreren Substituenten unabhängig ausgewählt aus -C(O)-R¹², SO₂-R¹³, Heteroaryl und C₁₋₃-Alkylen-OR¹⁴, wobei das Heteroaryl optional substituiert ist mit C₁₋₄-Alkyl oder C₃₋₇-Cycloalkyl; oder
e. ausgewählt aus einer der folgenden Gruppen: 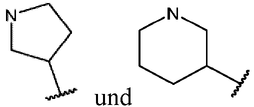 wobei 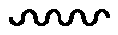 den Bindungspunkt der Gruppe an das Sauerstoffatom des Rests der Verbindung darstellt und wobei jede Gruppe optional substituiert ist mit einem oder mehreren Substituenten unabhängig ausgewählt aus -C(O)-R¹², SO₂-R¹³, Heteroaryl und C₁₋₃-Alkylen-OR¹⁴, wobei das Heteroaryl optional substituiert ist mit C₁₋₄-Alkyl oder C₃₋₇-Cycloalkyl.

6. Pharmazeutische Zusammensetzung nach einem der Ansprüche 1 bis 5, wobei R² Wasserstoff oder Fluor ist.

7. Pharmazeutische Zusammensetzung nach einem der Ansprüche 1 bis 6, wobei R³ Wasserstoff oder Chlor ist, wie Chlor.

8. Pharmazeutische Zusammensetzung nach einem der Ansprüche 1 bis 7, wobei R⁴ Wasserstoff ist und R⁵ -C(O)-C₁₋₄-Alkyl, -C(O)-C₃₋₇-Cycloalkyl oder -C(O)-Aryl ist, wobei das Aryl optional substituiert ist mit einem oder mehreren Substituenten ausgewählt aus C₁₋₄-Alkyl, Halogen, Hydroxy, C₁₋₃-Alkoxy, CO₂R¹⁵ und Cyano.

9. Pharmazeutische Zusammensetzung nach einem der Ansprüche 1 bis 7, wobei R⁴ und R⁵ zusammengenommen mit dem Stickstoffatom, an das sie gebunden sind:
a. einen 5- oder 6-gliedrigen Heterocyclylring bilden, wobei der Heterocyclylring: eine oder mehrere -C(O)--Einheiten umfasst, die an das Stickstoffatom gebunden sind; optional ein oder mehrere zusätzliche Heteroatome ausgewählt aus Sauerstoff, Stickstoff und Schwefel enthält;
optional an einen Arylring kondensiert ist;
optional an eine C₃₋₇-Cycloalkylgruppe oder einen 3- bis 5-gliedrigen Heterocyclylring spirogebunden ist; und
optional substituiert ist mit einem oder mehreren Substituenten unabhängig ausgewählt aus C₁₋₄-Alkyl, Halogen, OH, C₁₋₃-Alkoxy, C₁₋₃-Halogenalkyl, Cyano, NR¹⁶R¹⁷, C(O)R¹⁸, S(O)R¹⁹ und SO₂R²⁰;
b. eine heterocyclische Einheit bilden, die aus einem des Folgenden ausgewählt ist: 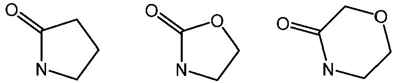 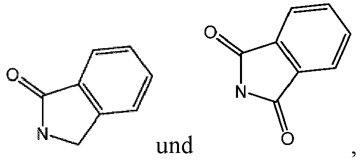 wobei der gesättigte Ring der heterocyclischen Einheit optional an eine C₃₋₇-Cycloalkylgruppe spirogebunden ist und wobei die heterocyclische Einheit optional substituiert ist mit einem oder mehreren Substituenten unabhängig ausgewählt aus C₁₋₄-Alkyl, Halogen und OH; oder
c. die folgende heterocyclische Einheit bilden: 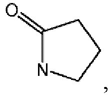 wobei die heterocyclische Einheit optional an eine Cyclopropylgruppe spirogebunden ist und optional substituiert ist mit einem oder mehreren Substituenten unabhängig ausgewählt aus Methyl und Fluor.

10. Pharmazeutische Zusammensetzung nach einem der Ansprüche 1 bis 9, wobei R⁶ Wasserstoff ist.

11. Pharmazeutische Zusammensetzung nach einem der Ansprüche 1 bis 10, wobei R⁸ C(=O)NR^{8a}R^{8b} ist.

12. Pharmazeutische Zusammensetzung nach Anspruch 11, wobei R^{8a} Wasserstoff ist.

13. Pharmazeutische Zusammensetzung nach Anspruch 11 oder 12, wobei R^{8b} wie folgt ist:
a. C₁₋₄-Alkyl oder C₃₋₅-Cycloalkyl, wobei die C₁₋₄-Alkylgruppe optional substituiert ist mit einem oder mehreren Substituenten unabhängig ausgewählt aus Fluor, OH, C₁₋₃-Alkoxy, C₃₋₇-Cycloalkyl und Cyano; oder
b. Methyl.

14. Pharmazeutische Zusammensetzung nach Anspruch 11, wobei R^{8a} und R^{8b} zusammengenommen mit dem Stickstoffatom, an das sie gebunden sind, einen 4- oder 5-gliedrigen Heterocyclylring bilden, wobei der Heterocyclylring optional substituiert ist mit einem oder mehreren Substituenten unabhängig ausgewählt aus C₁₋₄-Alkyl, Halogen, OH, C₁₋₃-Alkoxy, C₁₋₃-Halogenalkyl, C₃₋₇-Cycloalkyl, Cyano, NR¹⁶R¹⁷, C(O)R¹⁸, S(O)R¹⁹ und SO₂R²⁰.

15. Pharmazeutische Zusammensetzung nach Anspruch 1 oder Anspruch 3 bis 10, wobei R^{8c} C₁₋₃-Fluoralkyl ist.

16. Pharmazeutische Zusammensetzung nach Anspruch 1, wobei die Verbindung ausgewählt ist aus einer beliebigen der folgenden Verbindungen:
(1S,2R)-2-((S)-5-Chlor-8-((5-(difluormethyl)-1-methyl-1H-1,2,3-triazol-4-yl)methoxy)-1-((2-oxopyrrolidin-1-yl)methyl)-1,2,3,4-tetrahydroisochinolin-2-carbonyl)-N,1-dimethylcyclohexan-1-carboxamid;
(1S,2R)-2-((S)-8-(Benzo[d]isoxazol-3-ylmethoxy)-5-chlor-1-((2-oxopyrrolidin-1-yl)methyl)-1,2,3,4-tetrahydroisochinolin-2-carbonyl)-N,1-dimethylcyclohexan-1-carboxamid;
(1S,2R)-2-((S)-5-Chlor-8-((5-(difluormethyl)-1-methyl-1H-1,2,3-triazol-4-yl)methoxy)-1-((2-oxopyrrolidin-1-yl)methyl)-1,2,3,4-tetrahydroisochinolin-2-carbonyl)-N,1-dimethylcyclopentan-1-carboxamid;
(1S,2R)-2-((S)-5-Chlor-8-((1,5-dimethyl-1H-1,2,3-triazol-4-yl)methoxy)-1-((2- oxopyrrolidin-1-yl)methyl)-1,2,3,4-tetrahydroisochinolin-2-carbonyl)-N,1-dimethylcyclohexan-1-carboxamid;
(1S,2R)-2-((S)-5-Chlor-8-((1-methyl-5-(trifluormethyl)-1H-1,2,3-triazol-4-yl)methoxy)-1-((2-oxopyrrolidin-1-yl)methyl)-1,2,3,4-tetrahydroisochinolin-2-carbonyl)-N,1-dimethylcyclohexan-1-carboxamid;
(1S,2R)-2-((S)-5-Chlor-8-((1-methyl-1H-1,2,3-triazol-4-yl)methoxy)-1-((2-oxopyrrolidin-1-yl)methyl)-1,2,3,4-tetrahydroisochinolin-2-carbonyl)-N,1-dimethylcyclohexan-1-carboxamid;
(1S,2R)-2-((S)-5-Chlor-8-((5-(difluormethyl)-1-methyl-1H-1,2,3-triazol-4-yl)methoxy)-1-((6-oxo-5-azaspiro[2.4]heptan-5-yl)methyl)-1,2,3,4-tetrahydroisochinolin-2-carbonyl)-N,1-dimethylcyclopentan-1-carboxamid;
(1S,2R)-2-((1S)-5-Chlor-8-((5-(difluormethyl)-1-methyl-1H-1,2,3-triazol-4-yl)methoxy)-1-((3-methyl-2-oxopyrrolidin-1-yl)methyl)-1,2,3,4-tetrahydroisochinolin-2-carbonyl)-N,1-dimethylcyclopentan-1-carboxamid;
(1S,2R)-2-((1S)-5-Chlor-8-((5-(difluormethyl)-1-methyl-1H-1,2,3-triazol-4-yl)methoxy)-1-((3-methyl-2-oxopyrrolidin-1-yl)methyl)-1,2,3,4-tetrahydroisochinolin-2-carbonyl)-N,1-dimethylcyclohexan-1-carboxamid;
(1S,2R)-2-((S)-5-Chlor-8-((1-methyl-5-(trifluormethyl)-1H-1,2,3-triazol-4-yl)methoxy)-1-((6-oxo-5-azaspiro[2.4]heptan-5-yl)methyl)-1,2,3,4-tetrahydroisochinolin-2-carbonyl)-N,1-dimethylcyclohexan-1-carboxamid;
(1S,2R)-2-((S)-5-Chlor-8-((5-cyclopropyl-1-methyl-1H-1,2,3-triazol-4-yl)methoxy)-1-((2-oxopyrrolidin-1-yl)methyl)-1,2,3,4-tetrahydroisochinolin-2-carbonyl)-N,1-dimethylcyclohexan-1-carboxamid;
(1S,2R)-2-((S)-5-Chlor-8-((5-(difluormethyl)-1-methyl-1H-1,2,3-triazol-4-yl)methoxy)-1-((6-oxo-5-azaspiro[2.4]heptan-5-yl)methyl)-1,2,3,4-tetrahydroisochinolin-2-carbonyl)-1-methylcyclohexan-1-carboxamid;
(1S,2R)-2-((S)-5-Chlor-8-((1,5-dimethyl-1H-1,2,3-triazol-4-yl)methoxy)-1-((6-oxo-5-azaspiro[2.4]heptan-5-yl)methyl)-1,2,3,4-tetrahydroisochinolin-2-carbonyl)-N,1-dimethylcyclohexan-1-carboxamid;
(1S,2R)-2-((S)-5-Chlor-8-((1-methyl-1H-1,2,3-triazol-4-yl)methoxy)-1-((6-oxo-5-azaspiro[2.4]heptan-5-yl)methyl)-1,2,3,4-tetrahydroisochinolin-2-carbonyl)-N,1-dimethylcyclohexan-1-carboxamid;
(1S,2R)-2-((S)-5-Chlor-8-((5-cyclopropyl-1-methyl-1H-1,2,3-triazol-4-yl)methoxy)-1-((6-oxo-5-azaspiro[2.4]heptan-5-yl)methyl)-1,2,3,4-tetrahydroisochinolin-2-carbonyl)-N,1-dimethylcyclohexan-1-carboxamid;
(1S,2R)-2-((S)-5-Chlor-8-((1-methyl-1H-1,2,3-triazol-4-yl)methoxy)-1-(((R)-4-methyl-2-oxopyrrolidin-1-yl)methyl)-1,2,3,4-tetrahydroisochinolin-2-carbonyl)-N,1-dimethylcyclohexan-1-carboxamid;
(1S,2R)-2-((S)-5-Chlor-8-((5-(methoxymethyl)-1-methyl-1H-1,2,3-triazol-4-yl)methoxy)-1-(((R)-4-methyl-2-oxopyrrolidin-1-yl)methyl)-1,2,3,4-tetrahydroisochinolin-2-carbonyl)-N,1-dimethylcyclohexan-1-carboxamid;
(1S,2R)-2-((S)-5-Chlor-8-((5-(difluormethyl)-1-methyl-1H-1,2,3-triazol-4-yl)methoxy)-7-fluor-1-((6-oxo-5-azaspiro[2.4]heptan-5-yl)methyl)-1,2,3,4-tetrahydroisochinolin-2-carbonyl)-N,1-dimethylcyclohexan-1-carboxamid;
(1S,2R)-2-((S)-5-Chlor-8-((5-(difluormethyl)-1-methyl-1H-1,2,3-triazol-4-yl)methoxy)-7-fluor-1-((6-oxo-5-azaspiro[2.4]heptan-5-yl)methyl)-1,2,3,4-tetrahydroisochinolin-2-carbonyl)-1-methylcyclohexan-1-carboxamid;
(1S,2R)-2-((S)-5-Chlor-8-((5-(difluormethyl)-1-methyl-1H-1,2,3-triazol-4-yl)methoxy)-7-fluor-1-((3-oxomorpholino)methyl)-1,2,3,4-tetrahydroisochinolin-2-carbonyl)-N,1-dimethylcyclohexan-1-carboxamid;
(1S,2R)-2-((S)-5-Chlor-8-((5-(difluormethyl)-1-methyl-1H-1,2,3-triazol-4-yl)methoxy)-7-fluor-1-((2-oxopyrrolidin-1-yl)methyl)-1,2,3,4-tetrahydroisochinolin-2-carbonyl)-N,1-dimethylcyclohexan-1-carboxamid;
(1S,2R)-2-((S)-5-Chlor-7-fluor-8-((1-methyl-1H-1,2,3-triazol-4-yl)methoxy)-1-((6-oxo-5-azaspiro[2.4]heptan-5-yl)methyl)-1,2,3,4-tetrahydroisochinolin-2-carbonyl)-N,1-dimethylcyclohexan-1-carboxamid;
(1S,2R)-2-((S)-5-Chlor-7-fluor-1-((6-oxo-5-azaspiro[2.4]heptan-5-yl)methyl)-8-((4,5,6,7-tetrahydro-[1,2,3]triazolo[1, 5-a]pyridin-3-yl)methoxy)-1,2,3,4-tetrahydroisochinolin-2-carbonyl)-1-methylcyclohexan-1-carboxamid;
(1S,2R)-2-((S)-5-Chlor-8-((1,5-dimethyl-1H-1,2,3-triazol-4-yl)methoxy)-7-fluor-1-((6-oxo-5-azaspiro[2.4]heptan-5-yl)methyl)-1,2,3,4-tetrahydroisochinolin-2-carbonyl)-1-methylcyclohexan-1-carboxamid; oder
(1S,2R)-2-((S)-5-Chlor-8-((5-cyclopropyl-1-methyl-1H-1,2,3-triazol-4-yl)methoxy)-1-(((R)-4-methyl-2-oxopyrrolidin-1-yl)methyl)-1,2,3,4-tetrahydroisochinolin-2-carbonyl)-N,1-dimethylcyclohexan-1-carboxamid;
oder einem pharmazeutisch unbedenklichen Salz davon.

17. Pharmazeutische Zusammensetzung nach einem der Ansprüche 1 bis 16 zur Verwendung in:
a. Therapie;
b. der Behandlung von Erkrankungen oder Störungen vermittelt durch Nrf2-Aktivierung; oder
c. der Behandlung von chronisch obstruktiver pulmonaler Erkrankung, akutem, chronischem und schwerem Asthma, akuter Lungenverletzung/akutem Atemnotsyndrom mit oder ohne begleitendem Multiorganfehlfunktionssyndrom, pulmonaler Fibrose beinhaltend idiopathische pulmonale Fibrose, zystische Fibrose, COVID-19, Diabetes, Atherosklerose, Bluthochdruck, Herzinsuffizienz, Myokardinfarkt und -reparatur, kardialem Remodelling, Herzrhythmusstörungen, Herzhypertrophie, Herzinsuffizienz mit konservierter Ejektionsfraktion, diabetischer Kardiomyopathie, Sarkopenie, Adipositas, metabolischem Syndrom, Diabetes mellitus, Insulinresistenz, pulmonaler arterieller Hypertonie, Subarachnoidalblutung, intrazerebraler Blutung, ischämischem Schlaganfall, Betathalassämie, Sichelzellerkrankung, rheumatoider Arthritis, Reizdarmstörung, Colitis ulcerosa, Crohn-Erkrankung, Psoriasis, strahleninduzierter Dermatitis, atopischer Dermatitis, nichtalkoholischer Fettlebererkrankung, nichtalkoholischer Steatohepatitis, toxininduzierter Lebererkrankung, Virushepatitis und - zirrhose, chronischer Nierenerkrankung, diabetischer Nephropathie, autosomal-dominanter polyzystischer Nierenerkrankung, CKD assoziiert mit Typ-1-Diabetes (T1D), IgA-Nephropathie (IgAN), Alport-Syndrom, fokaler segmentaler Glomerulosklerose, Huntington-Erkrankung, Parkinson-Erkrankung, Alzheimer-Erkrankung, amyotropher Lateralsklerose, frontotemporaler Demenz, multipler Sklerose, Friedreich-Ataxie, chronischen Schmerzen, Schizophrenie, Lungenkrebs, Brustkrebs, Darmkrebs, altersbedingter Makuladegeneration (AMD), Fuchs-Endothelhornhautdystrophie oder Uveitis.

## Revendications

1. Composition pharmaceutique comprenant un composé selon la formule IC ou la formule IF, ou un sel pharmaceutiquement acceptable de celui-ci, et un ou plusieurs excipients pharmaceutiquement acceptables : 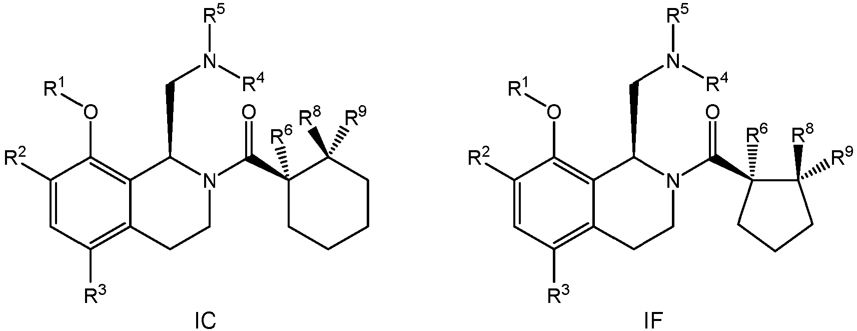 dans laquelle :
R¹ est choisi parmi C₁₋₄alkylène-R¹¹, hétérocyclyle et hétéroaryle bicyclique à 8-10 chaînons, ledit hétérocyclyle étant éventuellement substitué par un ou plusieurs substituants choisis indépendamment parmi C₁₋₄alkyle, -C(O)-R¹², SO₂-R¹³, C₁₋₃alkylène-OR¹⁴ et hétéroaryle qui est éventuellement substitué par un ou plusieurs substituants indépendamment choisis parmi C₁₋₄alkyle, C₃₋₇cycloalkyle, halo, OH, C₁₋₃alkoxy et cyano ; et ledit hétéroaryle bicyclique à 8-10 chaînons étant éventuellement substitué par un ou plusieurs substituants indépendamment choisis parmi C₁₋₄alkyle, C₃₋₇cycloalkyle, halo, OH et C₁₋₃alkoxy ;
R² est choisi parmi hydrogène, fluoro, chloro et C₁₋₃alkyle ;
R³ est choisi parmi hydrogène, fluoro, chloro, bromo, C₁₋₃alkoxy, C₁₋₃alkyle, C₁₋₃haloalkyle et cyano ;
R⁴ est hydrogène ou C₁₋₄alkyle ;
R⁵ est -C(O)-C₁₋₄alkyle, -C(O)-C₃₋₇cycloalkyle, -C(O)-hétéroaryle ou -C(O)-aryle, lesdits hétéroaryle et aryle étant éventuellement substitués par un ou plusieurs substituants choisis parmi C₁₋₄alkyle, halo, hydroxy, C₁₋₃alkoxy, CO₂R¹⁵ et cyano ; ou
R⁴ et R⁵, pris conjointement avec l'atome d'azote auquel ils sont liés, forment un cycle hétérocyclyle à 4, 5 ou 6 chaînons, ledit cycle hétérocyclyle :
comprenant une ou plusieurs fractions -C(O)- liées à l'atome d'azote ;
contenant éventuellement un ou plusieurs hétéroatomes supplémentaires choisis parmi oxygène, azote et soufre ;
étant éventuellement fusionné à un cycle aryle ou hétéroaryle ;
étant éventuellement spiro-lié à un groupe C₃₋₇cycloalkye ou à un cycle hétérocyclyle à 3 à 6 chaînons ; et
étant éventuellement substitué par un ou plusieurs substituants indépendamment choisis parmi C₁₋₄alkyle, halo, OH, C₁₋₃alkoxy, C₁₋₃haloalkyle, cyano, NR¹⁶R¹⁷, C(O)R¹⁸, S(O)R¹⁹ et SO₂R²⁰ ;
R⁶ est choisi parmi hydrogène, C₁₋₄alkyle et C₃₋₇cycloalkyle ;
R⁸ est C(=O)NR^{8a}R^{8b} ou -C(=O)R^{8c} ;
R^{8a} est hydrogène ou C₁₋₆alkyle ;
R^{8b} est hydrogène, C₁₋₄alkyle ou C₃₋₅cycloalkyle, le groupe C₁₋₄alkyle étant éventuellement substitué par un ou plusieurs substituants indépendamment choisis parmi halo, OH, C₁₋₃alkoxy, C₃₋₇cycloalkyle et cyano ; ou
R^{8a} et R^{8b}, pris conjointement avec l'atome d'azote auquel ils sont liés, forment un cycle hétérocyclyle à 3, 4, 5, 6 ou 7 chaînons, ledit cycle hétérocyclyle :
contenant éventuellement un ou plusieurs hétéroatomes supplémentaires choisis parmi oxygène, azote et soufre ; et
étant éventuellement substitué par un ou plusieurs substituants indépendamment choisis parmi C₁₋₄alkyle, C₃₋₇cycloalkyle, halo, OH, C₁₋₃alkoxy, C₁₋₃haloalkyle, cyano, NR¹⁶R¹⁷, C(O)R¹⁸, S(O)R¹⁹ et SO₂R²⁰ ;
R^{8c} est C₁₋₃alkyle ou C₁₋₃haloalkyle ;
R⁹ est méthyle ;
R¹¹ est choisi parmi -C(O)-R²⁴, -SO₂-R²⁵, -NR²⁶C(O)-R²⁷, -NR²⁸SO₂-R²⁹, hétérocyclyle, aryle et hétéroaryle, lesdits groupes aryle et hétéroaryle étant éventuellement substitués par un ou plusieurs substituants indépendamment choisis parmi C₁₋₄alkyle, C₁₋₃haloalkyle, C₃₋₇cycloalkyle, C₁₋₄alkylène-R³⁰, halo, OH, C₁₋₃alkoxy, hétérocyclyle et cyano ; et ledit groupe hétérocyclyle étant éventuellement substitué par un ou plusieurs substituants indépendamment choisis parmi C₁₋₄alkyle, C₁₋₃haloalkyle, C₃₋₇cycloalkyle, C₁₋₄ alkylène-R³⁰, halo, OH, C₁₋₃alkoxy, oxo et cyano ;
R¹² est choisi parmi C₁₋₄alkyle, C₃₋₇cycloalkyle, OR³¹, NR³²R³³, aryle et hétéroaryle, lesdits aryle et hétéroaryle étant éventuellement substitués par un ou plusieurs substituants indépendamment choisis parmi C₁₋₄alkyle, halo, OH, C₁₋₃alkoxy et cyano ;
R¹³ est choisi parmi C₁₋₄alkyle, C₃₋₇cycloalkyle, hétéroaryle, hétérocyclyle et NR³⁴R³⁵, lesdits hétéroaryle et hétérocyclyle étant éventuellement substitués par un ou plusieurs substituants indépendamment choisis parmi C₁₋₄alkyle, halo, OH, C₁₋₃alkoxy et cyano ;
R¹⁷ est choisi parmi hydrogène, C₁₋₄alkyle, C(O)C₁₋₃alkyle et C(O)NR³⁶R³⁷ ;
R¹⁸, R¹⁹ et R²⁰ sont indépendamment choisis parmi C₁₋₄alkyle, OH, C₁₋₃alkoxy et NR³⁸R³⁹ ;
R²⁴ est choisi parmi C_{1 4}alkyle, NR⁴⁰R⁴¹ et OR⁴² ;
R²⁵ est choisi parmi C₁₋₄alkyle et NR⁴³R⁴⁴ ;
R²⁷ est choisi parmi C₁₋₄alkyle, C₃₋₇cycloalkyle, C₁₋₃haloalkyl, hétérocyclyle, aryle et hétéroaryle, lesdits aryle et hétéroaryle étant éventuellement substitués par un ou plusieurs substituants indépendamment choisis parmi C₁₋₄alkyle, C₁₋₃haloalkyle, C₃₋₇cycloalkyle, C₁₋₄alkylène-R⁴⁵, halo, OH, C₁₋₃alkoxy et cyano ;
R²⁹ est choisi parmi C₁₋₄alkyle, C₃₋₇cycloalkyle, C₁₋₃haloalkyle, aryle et hétéroaryle, lesdits aryle et hétéroaryle étant éventuellement substitués par un ou plusieurs substituants indépendamment choisis parmi C₁₋₄alkyle, C₁₋₃haloalkyle, C₃₋₇cycloalkyle, C₁₋₄alkylène-R⁴⁶ halo, OH, C₁₋₃alkoxy et cyano ;
R³⁰ est choisi parmi hydroxy, C₁₋₃alkoxy, C₃₋₇cycloalkyle, cyano et NR⁴⁷R⁴⁸ ;
R⁴⁰ est choisi parmi hydrogène et C₁₋₄alkyle ;
R⁴¹ est choisi parmi hydrogène, C₁₋₄alkyle, C₃₋₇cycloalkyle, C₁₋₃alkoxy, aryle et hétéroaryle ; ou
R⁴⁰ et R⁴¹, pris conjointement avec l'atome d'azote auquel ils sont liés, forment un cycle hétéroaryle ou hétérocyclyle à 4, 5 ou 6 chaînons, lesdits cycles hétéroaryle et hétérocyclyle étant éventuellement substitués par un ou plusieurs substituants indépendamment choisis parmi C₁₋₄alkyle, halo, OH, C₁₋₃alkoxy, C₃₋₇cycloalkyle et cyano ;
R⁴⁵ et R⁴⁶ sont indépendamment choisis parmi hydroxy, C₁₋₃alkoxy et C₃₋₇cycloalkyle ; et
R¹⁴, R¹⁵, R¹⁶, R²⁶, R²⁸, R³¹, R³², R³³, R³⁴, R³⁵, R³⁶, R³⁷, R³⁸, R³⁹, R⁴², R⁴³, R⁴⁴, R⁴⁷ et R⁴⁸ sont indépendamment choisis parmi hydrogène, C₁₋₄alkyle et C₃₋₇cycloalkyle.

2. Composition pharmaceutique selon la revendication 1, dans laquelle le composé comporte les formules structurelles ID ou IG indiquées ci-dessous : 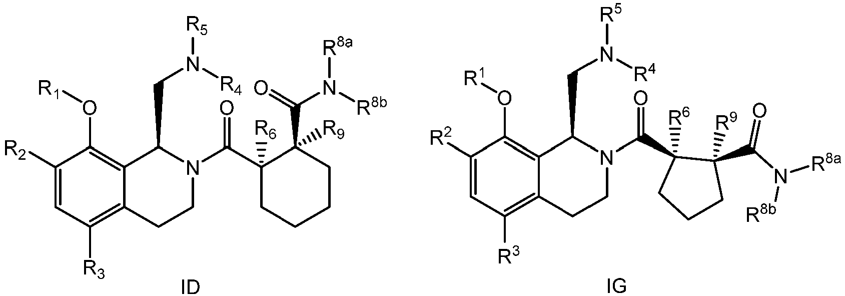 R¹ à R⁶, R^{8a}, R^{8b} et R⁹ étant tels que définis dans la revendication 1.

3. Composition pharmaceutique selon la revendication 1 ou 2, dans laquelle R¹ est C₁₋₄alkylène-R¹¹, tel que CH₂-R¹¹.

4. Composition pharmaceutique selon l'une quelconque des revendications 1 à 3, dans laquelle R¹¹ est :
a. choisi parmi -C(O)-R²⁴, -SO₂-R²⁵, -NR²⁶C(O)-R²⁷, -NR²⁸SO₂-R²⁹ et hétéroaryle, ledit hétéroaryle étant éventuellement substitué par un ou plusieurs substituants indépendamment choisis parmi C₁₋₄alkyle, C₁₋₃haloalkyle, C₃₋₇cycloalkyle, C₁₋₄alkylène- R³⁰, halo, OH, C₁₋₃alkoxy, hétérocyclyle et cyano ;
b. hétéroaryle éventuellement substitué par un ou plusieurs substituants indépendamment choisis parmi C₁₋₄alkyle, C₁₋₃haloalkyle, C₃₋₇cycloalkyle, C₁₋₄ alkylène-R³⁰, halo, OH, C₁₋₃alkoxy, hétérocyclyle et cyano ;
c. hétéroaryle éventuellement substitué par un ou plusieurs substituants indépendamment choisis parmi méthyle, éthyle, isopropyle, difluorométhyle, trifluorométhyle, chloro, fluoro, cyclopropyle, méthoxy, CH₂-R³⁰ et CH₂CH₂-R³⁰ ;
d. pyridyle, pyrimidinyle, pyridazinyle, pyrazinyle, oxazolyle, isoxazolyle, oxadiazolyle, 1,2,3-triazolyle, 1,2,4-triazolyle, 1,2,4-oxadiazolyle, indazolyle, benzotriazolyle, benzisoxazolyle, isoxazolopyridinyle, imidazopyridinyle ou triazolopyridinyle, chacun éventuellement substitué par un ou plusieurs substituants indépendamment choisis parmi C₁₋₄alkyle, C₁₋₃haloalkyle, C₃₋₇cycloalkyle, C₁₋₄ alkylène-R³⁰, halo, OH, C₁₋₃alkoxy, hétérocyclyle et cyano ; ou
e. choisi parmi : 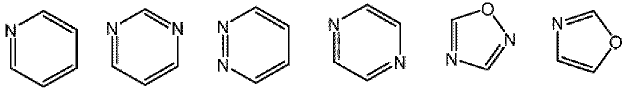 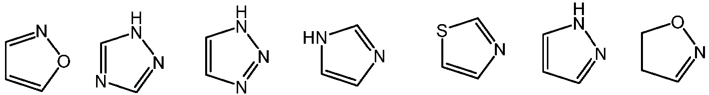 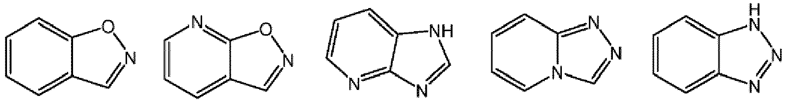 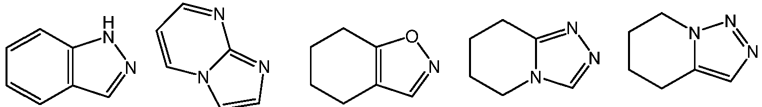 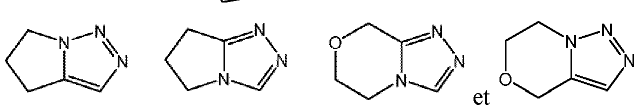 éventuellement substitué par un ou plusieurs substituants indépendamment choisis parmi C₁₋₄alkyle, C₁₋₃haloalkyle, C₃₋₇cycloalkyle, C₁₋₄ alkylène-R³⁰, halo, OH, C₁₋₃alkoxy, hétérocyclyle et cyano.

5. Composition pharmaceutique selon la revendication 1 ou 2, dans laquelle R¹ est :
a. choisi parmi l'un des groupes suivants : 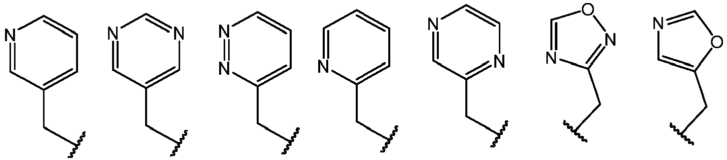 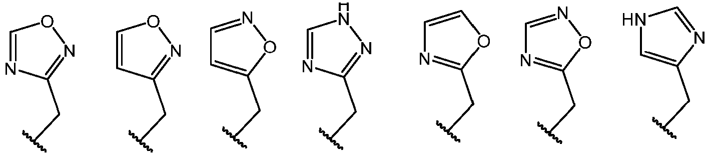 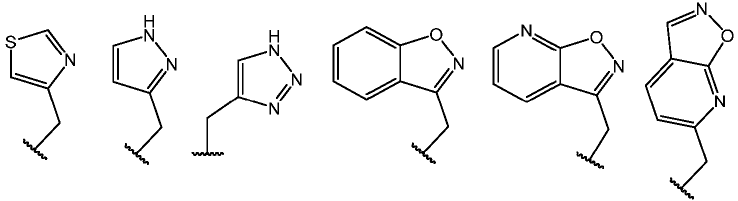 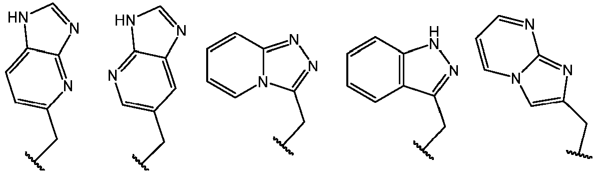 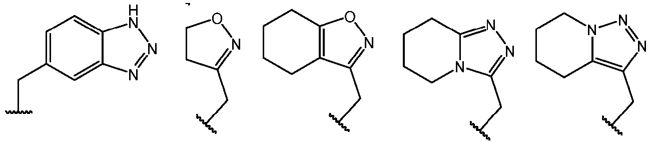 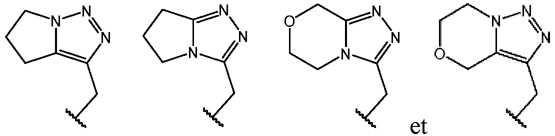 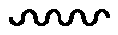 représentant le point de fixation du groupe à l'atome d'oxygène du reste du composé et chaque groupe étant éventuellement substitué par un ou plusieurs substituants indépendamment choisis parmi C₁₋₄alkyle, C₁₋₃haloalkyle, C₃₋₇cycloalkyle, C₁₋₄ alkylène-R³⁰, halo, OH, C₁₋₃alkoxy, hétérocyclyle et cyano ;
b. hétérocyclyle éventuellement substitué par un ou plusieurs substituants indépendamment choisis parmi C₁₋₄alkyle, -C(O)-R¹², SO₂-R¹³, hétéroaryle et C₁₋₃alkylène-OR¹⁴, ledit hétéroaryle étant éventuellement substitué par un ou plusieurs substituants indépendamment choisis parmi C₁₋₄alkyle, C₃₋₇cycloalkyle, halo, OH, C₁₋₃alkoxy et cyano ;
c. pipéridinyle ou pyrrolidinyle, chacun étant éventuellement substitué par un ou plusieurs substituants indépendamment choisis parmi -C(O)-R¹², SO₂-R¹³, hétéroaryle et C₁₋₃alkylène-OR¹⁴, ledit hétéroaryle étant éventuellement substitué par un ou plusieurs substituants indépendamment choisis parmi C₁₋₄alkyle, C₃₋₇cycloalkyle, halo, OH, C₁₋₃alkoxy et cyano ;
d. pyrrolidinyle éventuellement substitué par un ou plusieurs substituants indépendamment choisis parmi -C(O)-R¹², SO₂-R¹³, hétéroaryle et C₁₋₃alkylène-OR¹⁴, ledit hétéroaryle étant éventuellement substitué par C₁₋₄alkyle ou C₃₋₇cycloalkyle ; ou
e. choisi parmi l'un des groupes suivants : 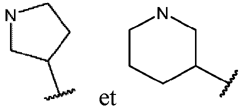 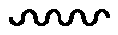 représentant le point de fixation du groupe à l'atome d'oxygène du reste du composé et chaque groupe étant éventuellement substitué par un ou plusieurs substituants indépendamment choisis parmi -C(O)-R¹², SO₂-R¹³, hétéroaryle et C₁₋₃alkylène-OR¹⁴, ledit hétéroaryle étant éventuellement substitué par C₁₋₄alkyle ou C₃₋₇cycloalkyle.

6. Composition pharmaceutique selon l'une quelconque des revendications 1 à 5, dans laquelle R² est hydrogène ou fluoro.

7. Composition pharmaceutique selon l'une quelconque des revendications 1 à 6, dans laquelle R³ est hydrogène ou chloro, tel que chloro.

8. Composition pharmaceutique selon l'une quelconque des revendications 1 à 7, dans laquelle R⁴ est hydrogène et R⁵ est -C(O)-C₁₋₄alkyle, -C(O)-C₃₋₇cycloalkyle ou -C(O)-aryle, ledit aryle étant éventuellement substitué par un ou plusieurs substituants choisis parmi C₁₋₄alkyle, halo, hydroxy, C₁₋₃alkoxy, CO₂R¹⁵ et cyano.

9. Composition pharmaceutique selon l'une quelconque des revendications 1 à 7, dans laquelle R⁴ et R⁵, pris conjointement avec l'atome d'azote auquel ils sont liés :
a. forment un cycle hétérocyclyle à 5 ou 6 chaînons, ledit cycle hétérocyclyle : comprenant une ou plusieurs fractions -C(O)- liées à l'atome d'azote ;
contenant éventuellement un ou plusieurs hétéroatomes supplémentaires choisis parmi oxygène, azote et soufre ;
étant éventuellement fusionné à un cycle aryle ;
étant éventuellement spiro-lié à un groupe C₃₋₇cycloalkye ou à un cycle hétérocyclyle à 3 à 5 chaînons ; et
étant éventuellement substitué par un ou plusieurs substituants indépendamment choisis parmi C₁₋₄alkyle, halo, OH, C₁₋₃alkoxy, C₁₋₃haloalkyle, cyano, NR¹⁶R¹⁷, C(O)R¹⁸, S(O)R¹⁹ et SO₂R²⁰ ;
b. forment une fraction hétérocyclique choisie parmi l'un des suivants : 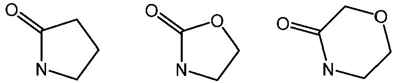 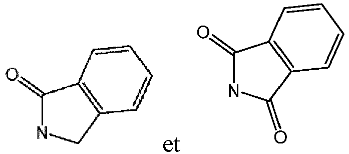 ledit cycle saturé de la fraction hétérocyclique étant éventuellement spiro-lié à un groupe C₃₋₇cycloalkyle, et ladite fraction hétérocyclique étant éventuellement substituée par un ou plusieurs substituants indépendamment choisis parmi C₁₋₄alkyle, halo et OH ; ou
c. forment la fraction hétérocyclique suivante : 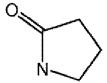 ladite fraction hétérocyclique étant éventuellement spiro-liée à un groupe cyclopropyle et étant éventuellement substituée par un ou plusieurs substituants indépendamment choisis parmi méthyle et fluoro.

10. Composition pharmaceutique selon l'une quelconque des revendications 1 à 9, dans laquelle R⁶ est hydrogène.

11. Composition pharmaceutique selon l'une quelconque des revendications 1 à 10, dans laquelle R⁸ est C(=O)NR^{8a}R^{8b}.

12. Composition pharmaceutique selon la revendication 11, dans laquelle R^{8a} est hydrogène.

13. Composition pharmaceutique selon la revendication 11 ou 12, dans laquelle R^{8b} est :
a. C₁₋₄alkyle ou C₃₋₅cycloalkyle, le groupe C₁₋₄alkyle étant éventuellement substitué par un ou plusieurs substituants indépendamment choisis parmi fluoro, OH, C₁₋₃alkoxy, C₃₋₇cycloalkyle et cyano ; ou
b. méthyle.

14. Composition pharmaceutique selon la revendication 11, dans laquelle R^{8a} et R^{8b}, pris conjointement avec l'atome d'azote auquel ils sont liés, forment un cycle hétérocyclyle à 4 ou 5 chaînons, ledit cycle hétérocyclyle étant éventuellement substitué par un ou plusieurs substituants indépendamment choisis parmi C₁₋₄alkyle, halo, OH, C₁₋₃alkoxy, C₁₋₃haloalkyle, C₃₋₇cycloalkyle, cyano, NR¹⁶R¹⁷, C(O)R¹⁸, S(O)R¹⁹ et SO₂R²⁰.

15. Composition pharmaceutique selon la revendication 1 ou les revendications 3 à 10, dans laquelle R^{8c} est C₁₋₃fluoroalkyle.

16. Composition pharmaceutique selon la revendication 1, dans laquelle le composé est choisi parmi l'un quelconque des composés suivants :
(1S,2R)-2-((S)-5-chloro-8-((5-(difluorométhyl)-1-méthyl-1H-1,2,3-triazol-4-yl)méthoxy)-1-((2-oxopyrrolidin-1-yl)méthyl)-1,2,3,4-tétrahydroisoquinoline-2-carbonyl)-N,1-diméthylcyclohexane-1-carboxamide ;
(1S,2R)-2-((S)-8-(benzo[d]isoxazol-3-ylméthoxy)-5-chloro-1-((2-oxopyrrolidin-1-yl)méthyl)-1,2,3,4-tétrahydroisoquinoline-2-carbonyl)-N,1-diméthylcyclohexane-1-carboxamide ;
(1S,2R)-2-((S)-S-chloro-8-((S-(difluorométhyl)-1-méthyl-1H-1,2,3-triazol-4-yl)méthoxy)-1-((2-oxopyrrolidin-1-yl)méthyl)-1,2,3,4-tétrahydroisoquinoline-2-carbonyl)-N,1-diméthylcyclopentane-1-carboxamide ;
(1S,2R)-2-((S)-5-chloro-8-((1,5-diméthyl-1H-1,2,3-triazol-4-yl)méthoxy)-1-((2-oxopyrrolidin-1-yl)méthyl)-1,2,3,4-tétrahydroisoquinoline-2-carbonyl)-N,1-diméthylcyclohexane-1-carboxamide ;
(1S,2R)-2-((S)-5-chloro-8-((1-méthyl-5-(trifluorométhyl)-1H-1,2,3-triazol-4-yl)méthoxy)-1-((2-oxopyrrolidin-1-yl)méthyl)-1,2,3,4-tétrahydroisoquinoline-2-carbonyl)-N,1-diméthylcyclohexane-1-carboxamide ;
(1S,2R)-2-((S)-5-chloro-8-((1-méthyl-1H-1,2,3-triazol-4-yl)méthoxy)-1-((2-oxopyrrolidin-1-yl)méthyl)-1,2,3,4-tétrahydroisoquinoline-2-carbonyl)-N,1-diméthylcyclohexane-1-carboxamide ;
(1S,2R)-2-((S)-5-chloro-8-((5-(difluorométhyl)-1-méthyl-1H-1,2,3-triazol-4-yl)méthoxy)-1-((6-oxo-5-azaspiro[2.4]heptan-5-yl)méthyl)-1,2,3,4-tétrahydroisoquinoline-2-carbonyl)-N,1-diméthylcyclopentane-1-carboxamide ;
(1S,2R)-2-((1S)-5-chloro-8-((5-(difluorométhyl)-1-méthyl-1H-1,2,3-triazol-4-yl)méthoxy)-1-((3-méthyl-2-oxopyrrolidin-1-yl)méthyl)-1,2,3,4-tetrahydroisoquinoline-2-carbonyl)-N,1-diméthylcyclopentane-1-carboxamide ;
(1S,2R)-2-((1S)-5-chloro-8-((5-(difluorométhyl)-1-méthyl-1H-1,2,3-triazol-4-yl)méthoxy)-1-((3-méthyl-2-oxopyrrolidin-1-yl)méthyl)-1,2,3,4-tetrahydroisoquinoline-2-carbonyl)-N,1-diméthylcyclohexane-1-carboxamide ;
(1S,2R)-2-((S)-5-chloro-8-((1-méthyl-5-(trifluorométhyl)-1H-1,2,3-triazol-4-yl)méthoxy)-1-((6-oxo-5-azaspiro[2.4]heptan-5-yl)méthyl)-1,2,3,4-tetrahydroisoquinoline-2-carbonyl)-N,1-diméthylcyclohexane-1-carboxamide ;
(1S,2R)-2-((S)-5-chloro-8-((5-cyclopropyl-1-méthyl-1H-1,2,3-triazol-4-yl)méthoxy)-1-((2-oxopyrrolidin-1-yl)méthyl)-1,2,3,4-tétrahydroisoquinoline-2-carbonyl)-N,1-diméthylcyclohexane-1-carboxamide ;
(1S,2R)-2-((S)-5-chloro-8-((5-(difluorométhyl)-1-méthyl-1H-1,2,3-triazol-4-yl)méthoxy)-1-((6-oxo-5-azaspiro[2.4]heptan-5-yl)méthyl)-1,2,3,4-tétrahydroisoquinoline-2-carbonyl)-1-méthylcyclohexane-1-carboxamide ;
(1S,2R)-2-((S)-5-chloro-8-((1,5-diméthyl-1H-1,2,3-triazol-4-yl)méthoxy)-1-((6-oxo-5-azaspiro[2.4]heptan-5-yl)méthyl)-1,2,3,4-tétrahydroisoquinoline-2-carbonyl)-N,1-diméthylcyclohexane-1-carboxamide ;
(1S,2R)-2-((S)-5-chloro-8-((1-méthyl-1H-1,2,3-triazol-4-yl)méthoxy)-1-((6-oxo-5-azaspiro[2.4]heptan-5-yl)méthyl)-1,2,3,4-tétrahydroisoquinoline-2-carbonyl)-N,1-diméthylcyclohexane-1-carboxamide ;
(1S,2R)-2-((S)-5-chloro-8-((5-cyclopropyl-1-méthyl-1H-1,2,3-triazol-4-yl)méthoxy)-1-((6-oxo-5-azaspiro[2.4]heptan-5-yl)méthyl)-1,2,3,4-tétrahydroisoquinoline-2-carbonyl)-N,1-diméthylcyclohexane-1-carboxamide ;
(1S,2R)-2-((S)-5-chloro-8-((1-méthyl-1H-1,2,3-triazol-4-yl)méthoxy)-1-(((R)-4-méthyl-2-oxopyrrolidin-1-yl)méthyl)-1,2,3,4-tétrahydroisoquinoline-2-carbonyl)-N,1-diméthylcyclohexane-1-carboxamide ;
(1S,2R)-2-((S)-5-chloro-8-((5-(méthoxyméthyl)-1-méthyl-1H-1,2,3-triazol-4-yl)méthoxy)-1-(((R)-4-méthyl-2-oxopyrrolidin-1-yl)méthyl)-1,2,3,4-tétrahydroisoquinoline-2-carbonyl)-N,1-diméthylcyclohexane-1-carboxamide ;
(1S,2R)-2-((S)-5-chloro-8-((5-(difluorométhyl)-1-méthyl-1H-1,2,3-triazol-4-yl)méthoxy)-7-fluoro-1-((6-oxo-5-azaspiro[2.4]heptan-5-yl)méthyl)-1,2,3,4-tétrahydroisoquinoline-2-carbonyl)-N,1-diméthylcyclohexane-1-carboxamide ;
(1S,2R)-2-((S)-5-chloro-8-((5-(difluorométhyl)-1-méthyl-1H-1,2,3-triazol-4-yl)méthoxy)-7-fluoro-1-((6-oxo-5-azaspiro[2.4]heptan-5-yl)méthyl)-1,2,3,4-tétrahydroisoquinoline-2-carbonyl)-1-méthylcyclohexane-1-carboxamide ;
(1S,2R)-2-((S)-5-chloro-8-((5-(difluorométhyl)-1-méthyl-1H-1,2,3-triazol-4-yl)méthoxy)-7-fluoro-1-((3-oxomorpholino)méthyl)-1,2,3,4-tétrahydroisoquinoline-2-carbonyl)-N,1-diméthylcyclohexane-1-carboxamide ;
(1S,2R)-2-((S)-5-chloro-8-((5-(difluorométhyl)-1-méthyl-1H-1,2,3-triazol-4-yl)méthoxy)-7-fluoro-1-((2-oxopyrrolidin-1-yl)méthyl)-1,2,3,4-tétrahydroisoquinoline-2-carbonyl)-N,1-diméthylcyclohexane-1-carboxamide ;
(1S,2R)-2-((S)-5-chloro-7-fluoro-8-((1-méthyl-1H-1,2,3-triazol-4-yl)méthoxy)-1-((6-oxo-5-azaspiro[2.4]heptan-5-yl)méthyl)-1,2,3,4-tétrahydroisoquinoline-2-carbonyl)-N,1-diméthylcyclohexane-1-carboxamide ;
(1S,2R)-2-((S)-5-chloro-7-fluoro-1-((6-oxo-5-azaspiro[2.4]heptan-5-yl)méthyl)-8-((4,5,6,7-tetrahydro-[1,2,3]triazolo[1,5-a]pyridin-3-yl)méthoxy)-1,2,3,4-tétrahydroisoquinoline-2-carbonyl)-1-méthylcyclohexane-1-carboxamide ;
(1S,2R)-2-((S)-5-chloro-8-((1,5-diméthyl-1H-1,2,3-triazol-4-yl)méthoxy)-7-fluoro-1-((6-oxo-5-azaspiro[2.4]heptan-5-yl)méthyl)-1,2,3,4-tétrahydroisoquinoline-2-carbonyl)-1-méthylcyclohexane-1-carboxamide ; ou
(1S,2R)-2-((S)-5-chloro-8-((5-cyclopropyl-1-méthyl-1H-1,2,3-triazol-4-yl)méthoxy)-1-(((R)-4-méthyl-2-oxopyrrolidin-1-yl)méthyl)-1,2,3,4-tétrahydroisoquinoline-2-carbonyl)-N,1-diméthylcyclohexane-1-carboxamide ;
ou un sel pharmaceutiquement acceptable de ceux-ci.

17. Composition pharmaceutique selon l'une quelconque des revendications 1 à 16, destinée à être utilisée dans :
a. une thérapie ;
b. le traitement de maladies ou de troubles médiés par l'activation de Nrf2 ; ou
c. le traitement de la bronchopneumopathie chronique obstructive, de l'asthme aigu, chronique et sévère, de la lésion pulmonaire aiguë/du syndrome de détresse respiratoire aiguë avec ou sans syndrome de dysfonctionnement multiviscéral, de la fibrose pulmonaire, y compris la fibrose pulmonaire idiopathique, de la mucoviscidose, de la COVID-19, du diabète, de l'athérosclérose, de l'hypertension, de l'insuffisance cardiaque, de l'infarctus et de la réparation du myocarde, du remodelage cardiaque, des arythmies cardiaques, de l'hypertrophie cardiaque, de l'insuffisance cardiaque avec fraction d'éjection préservée, de la cardiomyopathie diabétique, de la sarcopénie, de l'obésité, du syndrome métabolique, du diabète sucré, de la résistance à l'insuline, de l'hypertension artérielle pulmonaire, de l'hémorragie sous-arachnoïdienne, de l'hémorragie intracérébrale, d'un AVC ischémique, de la bêta-thalassémie, de la drépanocytose, de la polyarthrite rhumatoïde, du trouble du côlon irritable, de la colite ulcéreuse, de la maladie de Crohn, du psoriasis, de la dermatite induite par les radiations, de la dermatite atopique, de la stéatose hépatique non alcoolique, de la stéatohépatite non alcoolique, d'une maladie hépatique induite par la toxine, de l'hépatite virale et de la cirrhose, d'une maladie rénale chronique, de la néphropathie diabétique, de la polykystose rénale autosomique dominante, de la maladie rénale chronique associée au diabète de type 1 (DT1), de la néphropathie à IgA (IgAN), du syndrome d'Alport, de la glomérulosclérose segmentaire focale, de la maladie de Huntington, de la maladie de Parkinson, de la maladie d'Alzheimer, de la sclérose latérale amyotrophique, de la démence frontotemporale, de la sclérose en plaques, de l'ataxie de Friedreich, de la douleur chronique, de la schizophrénie, du cancer du poumon, du cancer du sein, du cancer du côlon, de la dégénérescence maculaire liée à l'âge (DMLA), de la dystrophie cornéenne endothéliale de Fuchs ou d'une uvéite.
